(19) Europäisches Patentamt
European Patent Office
Office européen des brevets

(11) **EP 2 857 419 B1**

(12) **EUROPEAN PATENT SPECIFICATION**

(45) Date of publication and mention
of the grant of the patent:
**13.01.2021 Bulletin 2021/02**

(51) Int Cl.:
***C07K 16/28*** *(2006.01)*

(21) Application number: **13796975.4**

(22) Date of filing: **30.05.2013**

(86) International application number:
**PCT/JP2013/064979**

(87) International publication number:
**WO 2013/180201 (05.12.2013 Gazette 2013/49)**

(54) **ANTIGEN-BINDING MOLECULE FOR ELIMINATING AGGREGATED ANTIGENS**

ANTIGENBINDENDE MOLEKÜLE ZUR BESEITIGUNG VON AGGREGIERTEN ANTIGENEN

MOLÉCULE DE LIAISON D'ANTIGÈNE SUPPRIMANT UN ANTIGÈNE ASSOCIÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.05.2012 JP 2012123782**

(43) Date of publication of application:
**08.04.2015 Bulletin 2015/15**

(60) Divisional application:
**19177897.6**

(73) Proprietor: **Chugai Seiyaku Kabushiki Kaisha
Kita-ku
Tokyo 115-8543 (JP)**

(72) Inventors:
• **IGAWA, Tomoyuki**
**Gotemba-shi**
**Shizuoka 412-8513 (JP)**
• **HIRONIWA, Naoka**
**Gotemba-shi**
**Shizuoka 412-8513 (JP)**
• **ITO, Eriko**
**Gotemba-shi**
**Shizuoka 412-8513 (JP)**

(74) Representative: **Vossius & Partner
Patentanwälte Rechtsanwälte mbB
Siebertstrasse 3
81675 München (DE)**

(56) References cited:
**EP-A1- 1 787 998      EP-A1- 2 189 526
EP-A1- 2 275 443      WO-A1-2006/016644**

**WO-A1-2009/008529      WO-A1-2009/125825
WO-A1-2010/058860      WO-A1-2012/073992
WO-A2-2006/053301      WO-A2-2011/122011
JP-A- H02 163 085      US-A1- 2007 009 523**

• **R REVERBERI ET AL: "Factors affecting the antigen-antibody reaction", BLOOD TRANSFUS, vol. 5, 1 January 2007 (2007-01-01), pages 227-240, XP055069254, DOI: 10.2450/2007.0047-07**
• **MARTIN L YARMUSH ET AL: "Immunoadsorption: strategies for antigen elution and production of reusable adsorbents", BIOTECHNOLOGY PROGRESS, AMERICAN INSTITUTE OF CHEMICAL ENGINEERS, US, vol. 8, no. 3, 1 January 1992 (1992-01-01), pages 168-178, XP008143270, ISSN: 8756-7938, DOI: 10.1021/BP00015A001 [retrieved on 2008-09-05]**
• **DALL'ACQUA W F ET AL: "Properties of human IgG1s engineered for enhanced binding to the neonatal Fc receptor (FcRn)", JOURNAL OF BIOLOGICAL CHEMISTRY, AMERICAN SOCIETY FOR BIOCHEMISTRY AND MOLECULAR BIOLOGY, vol. 281, no. 33, 21 June 2006 (2006-06-21), pages 23514-23524, XP002404904, ISSN: 0021-9258, DOI: 10.1074/JBC.M604292200**
• **SCHULKE, NORBERT ET AL.: 'The homodimer of prostate-specific membrane antigen is a functional target for cancer therapy.' PROCEEDINGS OF THE NATIONAL ACADEMY OF SCIENCES OF THE UNITED STATES OF AMERICA vol. 100, no. 22, 2003, pages 12590 - 12595, XP002323660**

EP 2 857 419 B1

**(Cont. next page)**

- HAMILTON, ROBERT G.: 'Molecular engineering: applications to the clinical laboratory.' CLINICAL CHEMISTRY vol. 39, no. 9, 1993, pages 1988 - 1997, XP055132119
- YEUNG, YIK ANDY ET AL.: 'Engineering human IgG1 affinity to human neonatal Fc receptor: impact of affinity improvement on pharmacokinetics in primates' JOURNAL OF IMMUNOLOGY vol. 182, no. 12, 2009, pages 7663 - 7671, XP002566420
- SHIELDS, ROBERT L. ET AL.: 'High resolution mapping of the binding site on human IgG1 for Fc gamma RI, Fc gamma RII, Fc gamma RIII, and FcRn and design of IgG1 variants with improved binding to the Fc gamma R.' THE JOURNAL OF BIOLOGICAL CHEMISTRY vol. 276, no. 9, 2001, pages 6591 - 6604, XP002495886
- PRICKETT, KATHRYN S. ET AL.: 'A calcium-dependent antibody for identification and purification of recombinant proteins.' BIOTECHNIQUES vol. 7, no. 6, 1989, pages 580 - 589, XP008174362
- YARMUSH, MARTIN L. ET AL.: 'Immunoadsorption: strategies for antigen elution and production of reusable adsorbents.' BIOTECHNOLOGY PROGRESS vol. 8, no. 3, 1992, pages 168 - 178, XP008143270
- SHIELDS R L ET AL: "High resolution mapping of the binding site on human IgG1 for FcgammaRI, FcgammaRII, FcgammaRIII, and FcRn and design of IgG1 variants with improved binding to the FcgammaR", JOURNAL OF BIOLOGICAL CHEMISTRY, AMERICAN SOCIETY FOR BIOCHEMISTRY AND MOLECULAR BIOLOGY, US, vol. 276, no. 9, 2 March 2001 (2001-03-02), pages 6591-6604, XP002271092, ISSN: 0021-9258, DOI: 10.1074/JBC.M009483200

SEQUENCE LISTING

<110> CHUGAI SEIYAKU KABUSHIKI KAISHA

<120> Antigen Binding Moleculs That Promote Clearance of Aggregated Antigens

<130> C1-A1205P

<150> JP 2012-123782
<151> 2012-05-30

<160> 39

<170> PatentIn version 3.5

<210> 1
<211> 19

<212> PRT
<213> Artificial Sequence

<220>
<223> an artificially synthesized sequence

<400> 1

```
Met Gly Trp Ser Cys Ile Ile Leu Phe Leu Val Ala Thr Ala Thr Gly
1               5                   10                  15

Val His Ser
```

<210> 2
<211> 107
<212> PRT
<213> Artificial Sequence

<220>
<223> an artificially synthesized sequence

<400> 2

```
Glu Thr Thr Leu Thr Gln Ser Pro Ala Phe Met Ser Ala Thr Pro Gly
1               5                   10                  15

Asp Lys Val Asn Ile Ser Cys Lys Ala Ser Gln Asp Ile Asp Asp Asp
            20                  25                  30

Met Asn Trp Tyr Gln Gln Lys Pro Gly Glu Ala Ala Ile Phe Ile Ile
            35                  40                  45

Gln Glu Ala Thr Thr Leu Val Pro Gly Ile Ser Pro Arg Phe Ser Gly
        50                  55                  60

Ser Gly Tyr Gly Thr Asp Phe Thr Leu Thr Ile Asn Asn Ile Glu Ser
65                  70                  75                  80

Glu Asp Ala Ala Tyr Tyr Phe Cys Leu Gln His Asp Asn Phe Pro Tyr
                85                  90                  95

Thr Phe Gly Gln Gly Thr Lys Leu Glu Ile Lys
            100                 105
```

<210> 3
<211> 107
<212> PRT
<213> Artificial Sequence

<220>
<223> an artificially synthesized sequence

<400> 3

```
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Ser Ser Tyr
            20                  25                  30

Leu Asn Trp Tyr Gln Gln Lys Pro Gly Lys Ala Pro Lys Leu Leu Ile
            35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Tyr Ser Thr Pro Phe
                85                  90                  95

Thr Phe Gly Pro Gly Thr Lys Val Asp Ile Lys
            100                 105
```

<210> 4
<211> 112
<212> PRT
<213> Artificial Sequence

<220>
<223> an artificially synthesized sequence

<400> 4

```
Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Leu His Ser
            20                  25                  30
```

```
        Asn Gly Asp Asn Tyr Leu Asp Trp Tyr Leu Gln Lys Pro Gly Gln Ser
                35                  40                  45


        Pro Gln Leu Leu Ile Tyr Leu Gly Ser Asn Arg Ala Ser Gly Val Pro
                50                  55                  60


        Asp Arg Phe Ser Gly Ser Gly Ser Gly Thr Asp Phe Thr Leu Lys Ile
        65                  70                  75                  80


        Ser Arg Val Glu Ala Glu Asp Val Gly Val Tyr Tyr Cys Met Gln Val
                        85                  90                  95


        Leu Arg Asn Pro Leu Thr Phe Gly Gly Gly Thr Lys Val Glu Ile Gln
                        100                 105                 110
```

<210> 5
<211> 107
<212> PRT
<213> Artificial Sequence

<220>
<223> an artificially synthesized sequence

<400> 5

```
        Glu Ile Val Met Thr Gln Ser Pro Ala Thr Leu Ser Leu Ser Pro Gly
        1                   5                   10                  15


        Glu Arg Ala Thr Leu Ser Cys Arg Ala Ser Gln Ser Val Ser Ser Tyr
                        20                  25                  30


        Leu Ala Trp Tyr Gln Gln Lys Pro Gly Gln Ala Pro Arg Leu Leu Ile
                35                  40                  45


        Tyr Asp Ala Ser Asn Arg Ala Thr Gly Ile Pro Ala Arg Phe Ser Gly
                50                  55                  60


        Ser Gly Ser Gly Thr Asp Phe Thr Leu Thr Ile Ser Ser Leu Glu Pro
        65                  70                  75                  80


        Glu Asp Phe Ala Val Tyr Tyr Cys Gln Gln Arg Ser Asn Trp Pro Pro
                        85                  90                  95


        Thr Phe Gly Gln Gly Thr Lys Val Glu Ile Lys
                        100                 105
```

<210> 6
<211> 112
<212> PRT
<213> Artificial Sequence

<220>
<223> an artificially synthesized sequence

<400> 6

```
        Asp Ile Val Met Thr Gln Ser Pro Glu Ser Leu Val Leu Ser Leu Gly
        1               5                  10                 15

        Gly Thr Ala Thr Ile Asn Cys Arg Ser Ser Gln Ser Val Leu Tyr Ser
                    20                  25                 30

        Ser Asn Asn Lys Asn Tyr Leu Thr Trp Tyr Gln Gln Lys Pro Gly Gln
                    35                  40                 45

        Pro Pro Thr Leu Leu Phe Ser Trp Ala Ser Ile Arg Asp Ser Gly Val
                50                  55                 60

        Pro Asp Arg Phe Ser Ala Ser Gly Ser Gly Thr Glu Phe Thr Leu Thr
        65                  70                 75                 80

        Ile Ser Asp Leu Gln Ala Glu Asp Ala Ala Val Tyr Tyr Cys Gln Gln
                        85                  90                 95

        Tyr Tyr Arg Ala Pro Ser Phe Gly Gln Gly Thr Lys Leu Gln Ile Lys
                    100                 105                110
```

<210> 7
<211> 121
<212> PRT
<213> Artificial Sequence

<220>
<223> an artificially synthesized sequence

<400> 7

```
        Gln Val Gln Leu Val Gln Ser Gly Ala Glu Val Lys Lys Pro Gly Ala
        1               5                  10                 15

        Ser Val Lys Val Ser Cys Lys Ala Ser Gly Tyr Thr Phe Thr Ser Tyr
                    20                  25                 30

        Tyr Met His Trp Val Arg Gln Ala Pro Gly Gln Gly Leu Glu Trp Met
                    35                  40                 45

        Gly Ile Ile Asn Pro Ser Gly Gly Ser Thr Ser Tyr Ala Gln Lys Phe
                50                  55                 60

        Gln Gly Arg Val Thr Met Thr Arg Asp Thr Ser Thr Ser Thr Val Tyr
        65                  70                 75                 80
```

```
Met Glu Leu Ser Ser Leu Arg Ser Glu Asp Thr Ala Val Tyr Tyr Cys
                85                  90                  95

Ala Arg Asp Asp Pro Gly Gly Gly Glu Tyr Tyr Phe Asp Tyr Trp Gly
            100                 105                 110

Gln Gly Thr Leu Val Thr Val Ser Ser
            115                 120
```

<210> 8
<211> 126
<212> PRT
<213> Artificial Sequence

<220>
<223> an artificially synthesized sequence

<400> 8

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Glu Met Asn Trp Val Arg Gln Ala Pro Gly Lys Gly Leu Glu Trp Val
            35                  40                  45

Ser Tyr Ile Ser Ser Ser Gly Ser Thr Ile Tyr Tyr Ala Asp Ser Val
        50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Asp Ala Pro Tyr Tyr Tyr Asp Ser Ser Gly Tyr Thr Asp Ala
            100                 105                 110

Phe Asp Ile Trp Gly Gln Gly Thr Met Val Thr Val Ser Ser
            115                 120                 125
```

<210> 9
<211> 330
<212> PRT
<213> Artificial Sequence

<220>
<223> an artificially synthesized sequence

<400> 9

```
Ala Ser Thr Lys Gly Pro Ser Val Phe Pro Leu Ala Pro Ser Ser Lys
1               5               10              15

Ser Thr Ser Gly Gly Thr Ala Ala Leu Gly Cys Leu Val Lys Asp Tyr
            20              25              30

Phe Pro Glu Pro Val Thr Val Ser Trp Asn Ser Gly Ala Leu Thr Ser
        35              40              45

Gly Val His Thr Phe Pro Ala Val Leu Gln Ser Ser Gly Leu Tyr Ser
        50              55              60

Leu Ser Ser Val Val Thr Val Pro Ser Ser Ser Leu Gly Thr Gln Thr
65              70              75              80

Tyr Ile Cys Asn Val Asn His Lys Pro Ser Asn Thr Lys Val Asp Lys
                85              90              95

Lys Val Glu Pro Lys Ser Cys Asp Lys Thr His Thr Cys Pro Pro Cys
            100             105             110

Pro Ala Pro Glu Leu Leu Gly Gly Pro Ser Val Phe Leu Phe Pro Pro
        115             120             125

Lys Pro Lys Asp Thr Leu Met Ile Ser Arg Thr Pro Glu Val Thr Cys
    130             135             140

Val Val Val Asp Val Ser His Glu Asp Pro Glu Val Lys Phe Asn Trp
145             150             155             160

Tyr Val Asp Gly Val Glu Val His Asn Ala Lys Thr Lys Pro Arg Glu
            165             170             175

Glu Gln Tyr Asn Ser Thr Tyr Arg Val Val Ser Val Leu Thr Val Leu
        180             185             190

His Gln Asp Trp Leu Asn Gly Lys Glu Tyr Lys Cys Lys Val Ser Asn
        195             200             205

Lys Ala Leu Pro Ala Pro Ile Glu Lys Thr Ile Ser Lys Ala Lys Gly
    210             215             220

Gln Pro Arg Glu Pro Gln Val Tyr Thr Leu Pro Pro Ser Arg Asp Glu
225             230             235             240

Leu Thr Lys Asn Gln Val Ser Leu Thr Cys Leu Val Lys Gly Phe Tyr
```

                         245                      250                      255

        Pro Ser Asp Ile Ala Val Glu Trp Glu Ser Asn Gly Gln Pro Glu Asn
                    260                  265                  270


        Asn Tyr Lys Thr Thr Pro Pro Val Leu Asp Ser Asp Gly Ser Phe Phe
                    275                  280                  285


        Leu Tyr Ser Lys Leu Thr Val Asp Lys Ser Arg Trp Gln Gln Gly Asn
                    290                  295                  300


        Val Phe Ser Cys Ser Val Met His Glu Ala Leu His Asn His Tyr Thr
        305                  310                  315                  320


        Gln Lys Ser Leu Ser Leu Ser Pro Gly Lys
                    325                  330

<210> 10
<211> 326
<212> PRT
<213> Artificial Sequence

<220>
<223> an artificially synthesized sequence

<400> 10

Ala Ser Thr Lys Gly Pro Ser Val Phe Pro Leu Ala Pro Cys Ser Arg
1               5               10              15

Ser Thr Ser Glu Ser Thr Ala Ala Leu Gly Cys Leu Val Lys Asp Tyr
        20              25              30

Phe Pro Glu Pro Val Thr Val Ser Trp Asn Ser Gly Ala Leu Thr Ser
        35              40              45

Gly Val His Thr Phe Pro Ala Val Leu Gln Ser Ser Gly Leu Tyr Ser
    50              55              60

Leu Ser Ser Val Val Thr Val Pro Ser Ser Asn Phe Gly Thr Gln Thr
65              70              75              80

Tyr Thr Cys Asn Val Asp His Lys Pro Ser Asn Thr Lys Val Asp Lys
            85              90              95

Thr Val Glu Arg Lys Cys Cys Val Glu Cys Pro Pro Cys Pro Ala Pro
            100             105             110

Pro Val Ala Gly Pro Ser Val Phe Leu Phe Pro Pro Lys Pro Lys Asp
        115             120             125

```
Thr Leu Met Ile Ser Arg Thr Pro Glu Val Thr Cys Val Val Val Asp
    130             135             140

Val Ser His Glu Asp Pro Glu Val Gln Phe Asn Trp Tyr Val Asp Gly
    145             150             155             160

Val Glu Val His Asn Ala Lys Thr Lys Pro Arg Glu Glu Gln Phe Asn
                165             170             175

Ser Thr Phe Arg Val Val Ser Val Leu Thr Val Val His Gln Asp Trp
            180             185             190

Leu Asn Gly Lys Glu Tyr Lys Cys Lys Val Ser Asn Lys Gly Leu Pro
            195             200             205

Ala Pro Ile Glu Lys Thr Ile Ser Lys Thr Lys Gly Gln Pro Arg Glu
    210             215             220

Pro Gln Val Tyr Thr Leu Pro Pro Ser Arg Glu Glu Met Thr Lys Asn
225             230             235             240

Gln Val Ser Leu Thr Cys Leu Val Lys Gly Phe Tyr Pro Ser Asp Ile
            245             250             255

Ala Val Glu Trp Glu Ser Asn Gly Gln Pro Glu Asn Asn Tyr Lys Thr
            260             265             270

Thr Pro Pro Met Leu Asp Ser Asp Gly Ser Phe Phe Leu Tyr Ser Lys
            275             280             285

Leu Thr Val Asp Lys Ser Arg Trp Gln Gln Gly Asn Val Phe Ser Cys
    290             295             300

Ser Val Met His Glu Ala Leu His Asn His Tyr Thr Gln Lys Ser Leu
305             310             315             320

Ser Leu Ser Pro Gly Lys
                325
```

<210> 11
<211> 377
<212> PRT
<213> Homo sapiens

<400> 11

```
Ala Ser Thr Lys Gly Pro Ser Val Phe Pro Leu Ala Pro Cys Ser Arg
1               5               10              15
```

```
Ser Thr Ser Gly Gly Thr Ala Ala Leu Gly Cys Leu Val Lys Asp Tyr
            20              25              30

Phe Pro Glu Pro Val Thr Val Ser Trp Asn Ser Gly Ala Leu Thr Ser
            35              40              45

Gly Val His Thr Phe Pro Ala Val Leu Gln Ser Ser Gly Leu Tyr Ser
            50              55              60

Leu Ser Ser Val Val Thr Val Pro Ser Ser Ser Leu Gly Thr Gln Thr
65              70              75              80

Tyr Thr Cys Asn Val Asn His Lys Pro Ser Asn Thr Lys Val Asp Lys
                85              90              95

Arg Val Glu Leu Lys Thr Pro Leu Gly Asp Thr Thr His Thr Cys Pro
            100             105             110

Arg Cys Pro Glu Pro Lys Ser Cys Asp Thr Pro Pro Cys Pro Arg
            115             120             125

Cys Pro Glu Pro Lys Ser Cys Asp Thr Pro Pro Cys Pro Arg Cys
    130             135             140

Pro Glu Pro Lys Ser Cys Asp Thr Pro Pro Cys Pro Arg Cys Pro
145             150             155             160

Ala Pro Glu Leu Leu Gly Gly Pro Ser Val Phe Leu Phe Pro Pro Lys
            165             170             175

Pro Lys Asp Thr Leu Met Ile Ser Arg Thr Pro Glu Val Thr Cys Val
            180             185             190

Val Val Asp Val Ser His Glu Asp Pro Glu Val Gln Phe Lys Trp Tyr
            195             200             205

Val Asp Gly Val Glu Val His Asn Ala Lys Thr Lys Pro Arg Glu Glu
            210             215             220

Gln Tyr Asn Ser Thr Phe Arg Val Val Ser Val Leu Thr Val Leu His
225             230             235             240

Gln Asp Trp Leu Asn Gly Lys Glu Tyr Lys Cys Lys Val Ser Asn Lys
            245             250             255

Ala Leu Pro Ala Pro Ile Glu Lys Thr Ile Ser Lys Thr Lys Gly Gln
```

                    260                    265                    270

        Pro Arg Glu Pro Gln Val Tyr Thr Leu Pro Pro Ser Arg Glu Glu Met
                275                 280                 285

        Thr Lys Asn Gln Val Ser Leu Thr Cys Leu Val Lys Gly Phe Tyr Pro
                290                 295                 300

        Ser Asp Ile Ala Val Glu Trp Glu Ser Ser Gly Gln Pro Glu Asn Asn
        305                 310                 315                 320

        Tyr Asn Thr Thr Pro Pro Met Leu Asp Ser Asp Gly Ser Phe Phe Leu
                        325                 330                 335

        Tyr Ser Lys Leu Thr Val Asp Lys Ser Arg Trp Gln Gln Gly Asn Ile
                340                 345                 350

        Phe Ser Cys Ser Val Met His Glu Ala Leu His Asn Arg Phe Thr Gln
                355                 360                 365

        Lys Ser Leu Ser Leu Ser Pro Gly Lys
                370                 375

<210> 12
<211> 327
<212> PRT
<213> Artificial Sequence

<220>
<223> an artificially synthesized sequence

<400> 12

Ala Ser Thr Lys Gly Pro Ser Val Phe Pro Leu Ala Pro Cys Ser Arg
1               5                   10              15

Ser Thr Ser Glu Ser Thr Ala Ala Leu Gly Cys Leu Val Lys Asp Tyr
            20                  25              30

Phe Pro Glu Pro Val Thr Val Ser Trp Asn Ser Gly Ala Leu Thr Ser
            35                  40              45

Gly Val His Thr Phe Pro Ala Val Leu Gln Ser Ser Gly Leu Tyr Ser
        50                  55              60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys
                85                  90                  95

```
Arg Val Glu Ser Lys Tyr Gly Pro Pro Cys Pro Ser Cys Pro Ala Pro
            100             105             110

Glu Phe Leu Gly Gly Pro Ser Val Phe Leu Phe Pro Pro Lys Pro Lys
            115             120             125

Asp Thr Leu Met Ile Ser Arg Thr Pro Glu Val Thr Cys Val Val Val
    130             135             140

Asp Val Ser Gln Glu Asp Pro Glu Val Gln Phe Asn Trp Tyr Val Asp
145             150             155             160

Gly Val Glu Val His Asn Ala Lys Thr Lys Pro Arg Glu Glu Gln Phe
            165             170             175

Asn Ser Thr Tyr Arg Val Val Ser Val Leu Thr Val Leu His Gln Asp
            180             185             190

Trp Leu Asn Gly Lys Glu Tyr Lys Cys Lys Val Ser Asn Lys Gly Leu
            195             200             205

Pro Ser Ser Ile Glu Lys Thr Ile Ser Lys Ala Lys Gly Gln Pro Arg
    210             215             220

Glu Pro Gln Val Tyr Thr Leu Pro Pro Ser Gln Glu Glu Met Thr Lys
225             230             235             240

Asn Gln Val Ser Leu Thr Cys Leu Val Lys Gly Phe Tyr Pro Ser Asp
            245             250             255

Ile Ala Val Glu Trp Glu Ser Asn Gly Gln Pro Glu Asn Asn Tyr Lys
            260             265             270

Thr Thr Pro Pro Val Leu Asp Ser Asp Gly Ser Phe Phe Leu Tyr Ser
    275             280             285

Arg Leu Thr Val Asp Lys Ser Arg Trp Gln Glu Gly Asn Val Phe Ser
    290             295             300

Cys Ser Val Met His Glu Ala Leu His Asn His Tyr Thr Gln Lys Ser
305             310             315             320

Leu Ser Leu Ser Leu Gly Lys
            325
```

<210> 13
<211> 365

<212> PRT
<213> Homo sapiens

<400> 13

```
Met Gly Val Pro Arg Pro Gln Pro Trp Ala Leu Gly Leu Leu Leu Phe
1               5               10              15

Leu Leu Pro Gly Ser Leu Gly Ala Glu Ser His Leu Ser Leu Leu Tyr
            20              25              30

His Leu Thr Ala Val Ser Ser Pro Ala Pro Gly Thr Pro Ala Phe Trp
        35              40              45

Val Ser Gly Trp Leu Gly Pro Gln Gln Tyr Leu Ser Tyr Asn Ser Leu
    50              55              60

Arg Gly Glu Ala Glu Pro Cys Gly Ala Trp Val Trp Glu Asn Gln Val
65              70              75              80

Ser Trp Tyr Trp Glu Lys Glu Thr Thr Asp Leu Arg Ile Lys Glu Lys
            85              90              95

Leu Phe Leu Glu Ala Phe Lys Ala Leu Gly Gly Lys Gly Pro Tyr Thr
        100             105             110

Leu Gln Gly Leu Leu Gly Cys Glu Leu Gly Pro Asp Asn Thr Ser Val
        115             120             125

Pro Thr Ala Lys Phe Ala Leu Asn Gly Glu Glu Phe Met Asn Phe Asp
    130             135             140

Leu Lys Gln Gly Thr Trp Gly Gly Asp Trp Pro Glu Ala Leu Ala Ile
145             150             155             160

Ser Gln Arg Trp Gln Gln Gln Asp Lys Ala Ala Asn Lys Glu Leu Thr
            165             170             175

Phe Leu Leu Phe Ser Cys Pro His Arg Leu Arg Glu His Leu Glu Arg
        180             185             190

Gly Arg Gly Asn Leu Glu Trp Lys Glu Pro Pro Ser Met Arg Leu Lys
        195             200             205

Ala Arg Pro Ser Ser Pro Gly Phe Ser Val Leu Thr Cys Ser Ala Phe
    210             215             220

Ser Phe Tyr Pro Pro Glu Leu Gln Leu Arg Phe Leu Arg Asn Gly Leu
```

225                    230                      235                      240


Ala Ala Gly Thr Gly Gln Gly Asp Phe Gly Pro Asn Ser Asp Gly Ser
            245                250                255


Phe His Ala Ser Ser Ser Leu Thr Val Lys Ser Gly Asp Glu His His
        260                265                270


Tyr Cys Cys Ile Val Gln His Ala Gly Leu Ala Gln Pro Leu Arg Val
        275                280                285


Glu Leu Glu Ser Pro Ala Lys Ser Ser Val Leu Val Val Gly Ile Val
    290                295                300


Ile Gly Val Leu Leu Leu Thr Ala Ala Ala Val Gly Gly Ala Leu Leu
305                310                315                320


Trp Arg Arg Met Arg Ser Gly Leu Pro Ala Pro Trp Ile Ser Leu Arg
            325                330                335


Gly Asp Asp Thr Gly Val Leu Leu Pro Thr Pro Gly Glu Ala Gln Asp
        340                345                350


Ala Asp Leu Lys Asp Val Asn Val Ile Pro Ala Thr Ala
        355                360                365

<210> 14
<211> 119
<212> PRT
<213> Homo sapiens

<400> 14

```
Met Ser Arg Ser Val Ala Leu Ala Val Leu Ala Leu Leu Ser Leu Ser
1               5               10                  15

Gly Leu Glu Ala Ile Gln Arg Thr Pro Lys Ile Gln Val Tyr Ser Arg
            20              25                  30

His Pro Ala Glu Asn Gly Lys Ser Asn Phe Leu Asn Cys Tyr Val Ser
            35              40                  45

Gly Phe His Pro Ser Asp Ile Glu Val Asp Leu Leu Lys Asn Gly Glu
        50              55                  60

Arg Ile Glu Lys Val Glu His Ser Asp Leu Ser Phe Ser Lys Asp Trp
65                  70                  75                  80

Ser Phe Tyr Leu Leu Tyr Tyr Thr Glu Phe Thr Pro Thr Glu Lys Asp
                85                  90                  95

Glu Tyr Ala Cys Arg Val Asn His Val Thr Leu Ser Gln Pro Lys Ile
            100             105                 110

Val Lys Trp Asp Arg Asp Met
            115
```

<210> 15
<211> 1125
<212> DNA
<213> Homo sapiens

<220>
<221> CDS
<222> (1)..(1125)

<400> 15

```
atg tgg ttc ttg aca act ctg ctc ctt tgg gtt cca gtt gat ggg caa        48
Met Trp Phe Leu Thr Thr Leu Leu Leu Trp Val Pro Val Asp Gly Gln
1               5                   10                  15

gtg gac acc aca aag gca gtg atc act ttg cag cct cca tgg gtc agc        96
Val Asp Thr Thr Lys Ala Val Ile Thr Leu Gln Pro Pro Trp Val Ser
                20                  25                  30

gtg ttc caa gag gaa acc gta acc ttg cac tgt gag gtg ctc cat ctg       144
Val Phe Gln Glu Glu Thr Val Thr Leu His Cys Glu Val Leu His Leu
            35                  40                  45

cct ggg agc agc tct aca cag tgg ttt ctc aat ggc aca gcc act cag       192
Pro Gly Ser Ser Ser Thr Gln Trp Phe Leu Asn Gly Thr Ala Thr Gln
        50                  55                  60

acc tcg acc ccc agc tac aga atc acc tct gcc agt gtc aat gac agt       240
Thr Ser Thr Pro Ser Tyr Arg Ile Thr Ser Ala Ser Val Asn Asp Ser
65                  70                  75                  80

ggt gaa tac agg tgc cag aga ggt ctc tca ggg cga agt gac ccc ata       288
Gly Glu Tyr Arg Cys Gln Arg Gly Leu Ser Gly Arg Ser Asp Pro Ile
                85                  90                  95

cag ctg gaa atc cac aga ggc tgg cta cta ctg cag gtc tcc agc aga       336
Gln Leu Glu Ile His Arg Gly Trp Leu Leu Leu Gln Val Ser Ser Arg
            100                 105                 110

gtc ttc acg gaa gga gaa cct ctg gcc ttg agg tgt cat gcg tgg aag       384
Val Phe Thr Glu Gly Glu Pro Leu Ala Leu Arg Cys His Ala Trp Lys
            115                 120                 125

gat aag ctg gtg tac aat gtg ctt tac tat cga aat ggc aaa gcc ttt       432
Asp Lys Leu Val Tyr Asn Val Leu Tyr Tyr Arg Asn Gly Lys Ala Phe
        130                 135                 140

aag ttt ttc cac tgg aat tct aac ctc acc att ctg aaa acc aac ata       480
Lys Phe Phe His Trp Asn Ser Asn Leu Thr Ile Leu Lys Thr Asn Ile
145                 150                 155                 160

agt cac aat ggc acc tac cat tgc tca ggc atg gga aag cat cgc tac       528
```

```
Ser His Asn Gly Thr Tyr His Cys Ser Gly Met Gly Lys His Arg Tyr
            165             170             175

aca tca gca gga ata tct gtc act gtg aaa gag cta ttt cca gct cca    576
Thr Ser Ala Gly Ile Ser Val Thr Val Lys Glu Leu Phe Pro Ala Pro
            180             185             190

gtg ctg aat gca tct gtg aca tcc cca ctc ctg gag ggg aat ctg gtc    624
Val Leu Asn Ala Ser Val Thr Ser Pro Leu Leu Glu Gly Asn Leu Val
            195             200             205

acc ctg agc tgt gaa aca aag ttg ctc ttg cag agg cct ggt ttg cag    672
Thr Leu Ser Cys Glu Thr Lys Leu Leu Leu Gln Arg Pro Gly Leu Gln
            210             215             220

ctt tac ttc tcc ttc tac atg ggc agc aag acc ctg cga ggc agg aac    720
Leu Tyr Phe Ser Phe Tyr Met Gly Ser Lys Thr Leu Arg Gly Arg Asn
225             230             235             240

aca tcc tct gaa tac caa ata cta act gct aga aga gaa gac tct ggg    768
Thr Ser Ser Glu Tyr Gln Ile Leu Thr Ala Arg Arg Glu Asp Ser Gly
            245             250             255

tta tac tgg tgc gag gct gcc aca gag gat gga aat gtc ctt aag cgc    816
Leu Tyr Trp Cys Glu Ala Ala Thr Glu Asp Gly Asn Val Leu Lys Arg
            260             265             270

agc cct gag ttg gag ctt caa gtg ctt ggc ctc cag tta cca act cct    864
Ser Pro Glu Leu Glu Leu Gln Val Leu Gly Leu Gln Leu Pro Thr Pro
            275             280             285

gtc tgg ttt cat gtc ctt ttc tat ctg gca gtg gga ata atg ttt tta    912
Val Trp Phe His Val Leu Phe Tyr Leu Ala Val Gly Ile Met Phe Leu
            290             295             300

gtg aac act gtt ctc tgg gtg aca ata cgt aaa gaa ctg aaa aga aag    960
Val Asn Thr Val Leu Trp Val Thr Ile Arg Lys Glu Leu Lys Arg Lys
305             310             315             320

aaa aag tgg gat tta gaa atc tct ttg gat tct ggt cat gag aag aag   1008
Lys Lys Trp Asp Leu Glu Ile Ser Leu Asp Ser Gly His Glu Lys Lys
            325             330             335

gta att tcc agc ctt caa gaa gac aga cat tta gaa gaa gag ctg aaa   1056
Val Ile Ser Ser Leu Gln Glu Asp Arg His Leu Glu Glu Glu Leu Lys
            340             345             350

tgt cag gaa caa aaa gaa gaa cag ctg cag gaa ggg gtg cac cgg aag   1104
Cys Gln Glu Gln Lys Glu Glu Gln Leu Gln Glu Gly Val His Arg Lys
            355             360             365

gag ccc cag ggg gcc acg tag                                        1125
Glu Pro Gln Gly Ala Thr
            370
```

<210> 16
<211> 374
<212> PRT
<213> Homo sapiens

<400> 16

```
Met Trp Phe Leu Thr Thr Leu Leu Leu Trp Val Pro Val Asp Gly Gln
1               5                   10                  15

Val Asp Thr Thr Lys Ala Val Ile Thr Leu Gln Pro Pro Trp Val Ser
            20                  25                  30

Val Phe Gln Glu Glu Thr Val Thr Leu His Cys Glu Val Leu His Leu
        35                  40                  45

Pro Gly Ser Ser Ser Thr Gln Trp Phe Leu Asn Gly Thr Ala Thr Gln
        50                  55                  60

Thr Ser Thr Pro Ser Tyr Arg Ile Thr Ser Ala Ser Val Asn Asp Ser
65                  70                  75                  80

Gly Glu Tyr Arg Cys Gln Arg Gly Leu Ser Gly Arg Ser Asp Pro Ile
                85                  90                  95

Gln Leu Glu Ile His Arg Gly Trp Leu Leu Leu Gln Val Ser Ser Arg
            100                 105                 110

Val Phe Thr Glu Gly Glu Pro Leu Ala Leu Arg Cys His Ala Trp Lys
        115                 120                 125

Asp Lys Leu Val Tyr Asn Val Leu Tyr Tyr Arg Asn Gly Lys Ala Phe
    130                 135                 140

Lys Phe Phe His Trp Asn Ser Asn Leu Thr Ile Leu Lys Thr Asn Ile
145                 150                 155                 160

Ser His Asn Gly Thr Tyr His Cys Ser Gly Met Gly Lys His Arg Tyr
                165                 170                 175

Thr Ser Ala Gly Ile Ser Val Thr Val Lys Glu Leu Phe Pro Ala Pro
            180                 185                 190

Val Leu Asn Ala Ser Val Thr Ser Pro Leu Leu Glu Gly Asn Leu Val
        195                 200                 205

Thr Leu Ser Cys Glu Thr Lys Leu Leu Leu Gln Arg Pro Gly Leu Gln
    210                 215                 220

Leu Tyr Phe Ser Phe Tyr Met Gly Ser Lys Thr Leu Arg Gly Arg Asn
225                 230                 235                 240

Thr Ser Ser Glu Tyr Gln Ile Leu Thr Ala Arg Arg Glu Asp Ser Gly
                245                 250                 255
```

```
Leu Tyr Trp Cys Glu Ala Ala Thr Glu Asp Gly Asn Val Leu Lys Arg
        260             265             270

Ser Pro Glu Leu Glu Leu Gln Val Leu Gly Leu Gln Leu Pro Thr Pro
        275             280             285

Val Trp Phe His Val Leu Phe Tyr Leu Ala Val Gly Ile Met Phe Leu
    290             295             300

Val Asn Thr Val Leu Trp Val Thr Ile Arg Lys Glu Leu Lys Arg Lys
305             310             315             320

Lys Lys Trp Asp Leu Glu Ile Ser Leu Asp Ser Gly His Glu Lys Lys
            325             330             335

Val Ile Ser Ser Leu Gln Glu Asp Arg His Leu Glu Glu Glu Leu Lys
        340             345             350

Cys Gln Glu Gln Lys Glu Glu Gln Leu Gln Glu Gly Val His Arg Lys
        355             360             365

Glu Pro Gln Gly Ala Thr
        370
```

<210> 17
<211> 951
<212> DNA
<213> Homo sapiens

<220>
<221> CDS
<222> (1)..(951)

<400> 17

```
atg act atg gag acc caa atg tct cag aat gta tgt ccc aga aac ctg      48
Met Thr Met Glu Thr Gln Met Ser Gln Asn Val Cys Pro Arg Asn Leu
1               5               10                  15

tgg ctg ctt caa cca ttg aca gtt ttg ctg ctg ctg gct tct gca gac      96
Trp Leu Leu Gln Pro Leu Thr Val Leu Leu Leu Leu Ala Ser Ala Asp
            20              25                  30

agt caa gct gct ccc cca aag gct gtg ctg aaa ctt gag ccc ccg tgg     144
Ser Gln Ala Ala Pro Pro Lys Ala Val Leu Lys Leu Glu Pro Pro Trp
        35              40                  45

atc aac gtg ctc cag gag gac tct gtg act ctg aca tgc cag ggg gct     192
Ile Asn Val Leu Gln Glu Asp Ser Val Thr Leu Thr Cys Gln Gly Ala
        50              55                  60

cgc agc cct gag agc gac tcc att cag tgg ttc cac aat ggg aat ctc     240
Arg Ser Pro Glu Ser Asp Ser Ile Gln Trp Phe His Asn Gly Asn Leu
65              70                  75                  80
```

```
att ccc acc cac acg cag ccc agc tac agg ttc aag gcc aac aac aat          288
Ile Pro Thr His Thr Gln Pro Ser Tyr Arg Phe Lys Ala Asn Asn Asn
            85              90                  95

gac agc ggg gag tac acg tgc cag act ggc cag acc agc ctc agc gac          336
Asp Ser Gly Glu Tyr Thr Cys Gln Thr Gly Gln Thr Ser Leu Ser Asp
            100             105                 110

cct gtg cat ctg act gtg ctt tcc gaa tgg ctg gtg ctc cag acc cct          384
Pro Val His Leu Thr Val Leu Ser Glu Trp Leu Val Leu Gln Thr Pro
            115             120                 125

cac ctg gag ttc cag gag gga gaa acc atc atg ctg agg tgc cac agc          432
His Leu Glu Phe Gln Glu Gly Glu Thr Ile Met Leu Arg Cys His Ser
            130             135                 140

tgg aag gac aag cct ctg gtc aag gtc aca ttc ttc cag aat gga aaa          480
Trp Lys Asp Lys Pro Leu Val Lys Val Thr Phe Phe Gln Asn Gly Lys
145             150                 155                 160

tcc cag aaa ttc tcc cat ttg gat ccc acc ttc tcc atc cca caa gca          528
Ser Gln Lys Phe Ser His Leu Asp Pro Thr Phe Ser Ile Pro Gln Ala
                165             170                 175

aac cac agt cac agt ggt gat tac cac tgc aca gga aac ata ggc tac          576
Asn His Ser His Ser Gly Asp Tyr His Cys Thr Gly Asn Ile Gly Tyr
                180             185                 190

acg ctg ttc tca tcc aag cct gtg acc atc act gtc caa gtg ccc agc          624
Thr Leu Phe Ser Ser Lys Pro Val Thr Ile Thr Val Gln Val Pro Ser
            195             200                 205

atg ggc agc tct tca cca atg ggg gtc att gtg gct gtg gtc att gcg          672
Met Gly Ser Ser Ser Pro Met Gly Val Ile Val Ala Val Val Ile Ala
            210             215                 220

act gct gta gca gcc att gtt gct gct gta gtg gcc ttg atc tac tgc          720
Thr Ala Val Ala Ala Ile Val Ala Ala Val Val Ala Leu Ile Tyr Cys
225             230                 235                 240

agg aaa aag cgg att tca gcc aat tcc act gat cct gtg aag gct gcc          768
Arg Lys Lys Arg Ile Ser Ala Asn Ser Thr Asp Pro Val Lys Ala Ala
                245             250                 255

caa ttt gag cca cct gga cgt caa atg att gcc atc aga aag aga caa          816
Gln Phe Glu Pro Pro Gly Arg Gln Met Ile Ala Ile Arg Lys Arg Gln
            260             265                 270

ctt gaa gaa acc aac aat gac tat gaa aca gct gac ggc ggc tac atg          864
Leu Glu Glu Thr Asn Asn Asp Tyr Glu Thr Ala Asp Gly Gly Tyr Met
            275             280                 285

act ctg aac ccc agg gca cct act gac gat gat aaa aac atc tac ctg          912
Thr Leu Asn Pro Arg Ala Pro Thr Asp Asp Asp Lys Asn Ile Tyr Leu
            290             295                 300

act ctt cct ccc aac gac cat gtc aac agt aat aac taa                      951
Thr Leu Pro Pro Asn Asp His Val Asn Ser Asn Asn
305             310                 315
```

<210> 18

<211> 316
<212> PRT
<213> Homo sapiens

<400> 18

Met Thr Met Glu Thr Gln Met Ser Gln Asn Val Cys Pro Arg Asn Leu
1               5               10          15

Trp Leu Leu Gln Pro Leu Thr Val Leu Leu Leu Ala Ser Ala Asp
          20          25              30

Ser Gln Ala Ala Pro Pro Lys Ala Val Leu Lys Leu Glu Pro Pro Trp
        35          40              45

Ile Asn Val Leu Gln Glu Asp Ser Val Thr Leu Thr Cys Gln Gly Ala
    50              55              60

Arg Ser Pro Glu Ser Asp Ser Ile Gln Trp Phe His Asn Gly Asn Leu
65              70              75              80

Ile Pro Thr His Thr Gln Pro Ser Tyr Arg Phe Lys Ala Asn Asn Asn
            85              90              95

Asp Ser Gly Glu Tyr Thr Cys Gln Thr Gly Gln Thr Ser Leu Ser Asp
        100             105             110

Pro Val His Leu Thr Val Leu Ser Glu Trp Leu Val Leu Gln Thr Pro
        115             120             125

His Leu Glu Phe Gln Glu Gly Glu Thr Ile Met Leu Arg Cys His Ser
    130             135             140

Trp Lys Asp Lys Pro Leu Val Lys Val Thr Phe Phe Gln Asn Gly Lys
145             150             155             160

Ser Gln Lys Phe Ser His Leu Asp Pro Thr Phe Ser Ile Pro Gln Ala
            165             170             175

Asn His Ser His Ser Gly Asp Tyr His Cys Thr Gly Asn Ile Gly Tyr
        180             185             190

Thr Leu Phe Ser Ser Lys Pro Val Thr Ile Thr Val Gln Val Pro Ser
        195             200             205

Met Gly Ser Ser Ser Pro Met Gly Val Ile Val Ala Val Val Ile Ala
    210             215             220

Thr Ala Val Ala Ala Ile Val Ala Ala Val Val Ala Leu Ile Tyr Cys

```
            225                230                235                240

            Arg Lys Lys Arg Ile Ser Ala Asn Ser Thr Asp Pro Val Lys Ala Ala
                        245                250                255

            Gln Phe Glu Pro Pro Gly Arg Gln Met Ile Ala Ile Arg Lys Arg Gln
                    260                265                270

            Leu Glu Glu Thr Asn Asn Asp Tyr Glu Thr Ala Asp Gly Gly Tyr Met
                    275                280                285

            Thr Leu Asn Pro Arg Ala Pro Thr Asp Asp Asp Lys Asn Ile Tyr Leu
                290                295                300

            Thr Leu Pro Pro Asn Asp His Val Asn Ser Asn Asn
            305                310                315
```

<210> 19
<211> 876
<212> DNA
<213> Homo sapiens

<220>
<221> CDS
<222> (1)..(876)

<400> 19

```
atg gga atc ctg tca ttc tta cct gtc ctt gcc act gag agt gac tgg        48
Met Gly Ile Leu Ser Phe Leu Pro Val Leu Ala Thr Glu Ser Asp Trp
1               5                   10                  15

gct gac tgc aag tcc ccc cag cct tgg ggt cat atg ctt ctg tgg aca        96
Ala Asp Cys Lys Ser Pro Gln Pro Trp Gly His Met Leu Leu Trp Thr
            20                  25                  30

gct gtg cta ttc ctg gct cct gtt gct ggg aca cct gca gct ccc cca       144
Ala Val Leu Phe Leu Ala Pro Val Ala Gly Thr Pro Ala Ala Pro Pro
        35                  40                  45

aag gct gtg ctg aaa ctc gag ccc cag tgg atc aac gtg ctc cag gag       192
Lys Ala Val Leu Lys Leu Glu Pro Gln Trp Ile Asn Val Leu Gln Glu
    50                  55                  60

gac tct gtg act ctg aca tgc cgg ggg act cac agc cct gag agc gac       240
Asp Ser Val Thr Leu Thr Cys Arg Gly Thr His Ser Pro Glu Ser Asp
65                  70                  75                  80

tcc att cag tgg ttc cac aat ggg aat ctc att ccc acc cac acg cag       288
Ser Ile Gln Trp Phe His Asn Gly Asn Leu Ile Pro Thr His Thr Gln
                85                  90                  95

ccc agc tac agg ttc aag gcc aac aac aat gac agc ggg gag tac acg       336
Pro Ser Tyr Arg Phe Lys Ala Asn Asn Asn Asp Ser Gly Glu Tyr Thr
            100                 105                 110

tgc cag act ggc cag acc agc ctc agc gac cct gtg cat ctg act gtg       384
```

```
    Cys Gln Thr Gly Gln Thr Ser Leu Ser Asp Pro Val His Leu Thr Val
            115                 120                 125

    ctt tct gag tgg ctg gtg ctc cag acc cct cac ctg gag ttc cag gag    432
    Leu Ser Glu Trp Leu Val Leu Gln Thr Pro His Leu Glu Phe Gln Glu
        130                 135                 140

    gga gaa acc atc gtg ctg agg tgc cac agc tgg aag gac aag cct ctg    480
    Gly Glu Thr Ile Val Leu Arg Cys His Ser Trp Lys Asp Lys Pro Leu
    145                 150                 155                 160

    gtc aag gtc aca ttc ttc cag aat gga aaa tcc aag aaa ttt tcc cgt    528
    Val Lys Val Thr Phe Phe Gln Asn Gly Lys Ser Lys Lys Phe Ser Arg
                        165                 170                 175

    tcg gat ccc aac ttc tcc atc cca caa gca aac cac agt cac agt ggt    576
    Ser Asp Pro Asn Phe Ser Ile Pro Gln Ala Asn His Ser His Ser Gly
                    180                 185                 190

    gat tac cac tgc aca gga aac ata ggc tac acg ctg tac tca tcc aag    624
    Asp Tyr His Cys Thr Gly Asn Ile Gly Tyr Thr Leu Tyr Ser Ser Lys
            195                 200                 205

    cct gtg acc atc act gtc caa gct ccc agc tct tca ccg atg ggg atc    672
    Pro Val Thr Ile Thr Val Gln Ala Pro Ser Ser Ser Pro Met Gly Ile
        210                 215                 220

    att gtg gct gtg gtc act ggg att gct gta gcg gcc att gtt gct gct    720
    Ile Val Ala Val Val Thr Gly Ile Ala Val Ala Ala Ile Val Ala Ala
    225                 230                 235                 240

    gta gtg gcc ttg atc tac tgc agg aaa aag cgg att tca gcc aat ccc    768
    Val Val Ala Leu Ile Tyr Cys Arg Lys Lys Arg Ile Ser Ala Asn Pro
                        245                 250                 255

    act aat cct gat gag gct gac aaa gtt ggg gct gag aac aca atc acc    816
    Thr Asn Pro Asp Glu Ala Asp Lys Val Gly Ala Glu Asn Thr Ile Thr
                    260                 265                 270

    tat tca ctt ctc atg cac ccg gat gct ctg gaa gag cct gat gac cag    864
    Tyr Ser Leu Leu Met His Pro Asp Ala Leu Glu Glu Pro Asp Asp Gln
            275                 280                 285

    aac cgt att tag                                                    876
    Asn Arg Ile
            290
```

<210> 20
<211> 291
<212> PRT
<213> Homo sapiens

<400> 20

```
    Met Gly Ile Leu Ser Phe Leu Pro Val Leu Ala Thr Glu Ser Asp Trp
    1               5                   10                  15


    Ala Asp Cys Lys Ser Pro Gln Pro Trp Gly His Met Leu Leu Trp Thr
                20                  25                  30
```

```
            Ala Val Leu Phe Leu Ala Pro Val Ala Gly Thr Pro Ala Ala Pro Pro
                    35                  40                  45

            Lys Ala Val Leu Lys Leu Glu Pro Gln Trp Ile Asn Val Leu Gln Glu
                    50                  55                  60

            Asp Ser Val Thr Leu Thr Cys Arg Gly Thr His Ser Pro Glu Ser Asp
            65                  70                  75                  80

            Ser Ile Gln Trp Phe His Asn Gly Asn Leu Ile Pro Thr His Thr Gln
                        85                  90                  95

            Pro Ser Tyr Arg Phe Lys Ala Asn Asn Asn Asp Ser Gly Glu Tyr Thr
                        100                 105                 110

            Cys Gln Thr Gly Gln Thr Ser Leu Ser Asp Pro Val His Leu Thr Val
                        115                 120                 125

            Leu Ser Glu Trp Leu Val Leu Gln Thr Pro His Leu Glu Phe Gln Glu
                    130                 135                 140

            Gly Glu Thr Ile Val Leu Arg Cys His Ser Trp Lys Asp Lys Pro Leu
            145                 150                 155                 160

            Val Lys Val Thr Phe Phe Gln Asn Gly Lys Ser Lys Lys Phe Ser Arg
                            165                 170                 175

            Ser Asp Pro Asn Phe Ser Ile Pro Gln Ala Asn His Ser His Ser Gly
                        180                 185                 190

            Asp Tyr His Cys Thr Gly Asn Ile Gly Tyr Thr Leu Tyr Ser Ser Lys
                    195                 200                 205

            Pro Val Thr Ile Thr Val Gln Ala Pro Ser Ser Ser Pro Met Gly Ile
                    210                 215                 220

            Ile Val Ala Val Val Thr Gly Ile Ala Val Ala Ala Ile Val Ala Ala
            225                 230                 235                 240

            Val Val Ala Leu Ile Tyr Cys Arg Lys Lys Arg Ile Ser Ala Asn Pro
                            245                 250                 255

            Thr Asn Pro Asp Glu Ala Asp Lys Val Gly Ala Glu Asn Thr Ile Thr
                        260                 265                 270

            Tyr Ser Leu Leu Met His Pro Asp Ala Leu Glu Glu Pro Asp Asp Gln
                    275                 280                 285
```

137

```
                                    Asn Arg Ile
                                        290
```

<210> 21
<211> 765
<212> DNA
<213> Homo sapiens

<220>
<221> CDS
<222> (1)..(765)

<400> 21

```
atg tgg cag ctg ctc ctc cca act gct ctg cta ctt cta gtt tca gct        48
Met Trp Gln Leu Leu Leu Pro Thr Ala Leu Leu Leu Leu Val Ser Ala
1               5                   10                  15

ggc atg cgg act gaa gat ctc cca aag gct gtg gtg ttc ctg gag cct        96
Gly Met Arg Thr Glu Asp Leu Pro Lys Ala Val Val Phe Leu Glu Pro
                20                  25                  30

caa tgg tac agg gtg ctc gag aag gac agt gtg act ctg aag tgc cag       144
Gln Trp Tyr Arg Val Leu Glu Lys Asp Ser Val Thr Leu Lys Cys Gln
            35                  40                  45

gga gcc tac tcc cct gag gac aat tcc aca cag tgg ttt cac aat gag       192
Gly Ala Tyr Ser Pro Glu Asp Asn Ser Thr Gln Trp Phe His Asn Glu
        50                  55                  60

agc ctc atc tca agc cag gcc tcg agc tac ttc att gac gct gcc aca       240
Ser Leu Ile Ser Ser Gln Ala Ser Ser Tyr Phe Ile Asp Ala Ala Thr
65                  70                  75                  80

gtt gac gac agt gga gag tac agg tgc cag aca aac ctc tcc acc ctc       288
Val Asp Asp Ser Gly Glu Tyr Arg Cys Gln Thr Asn Leu Ser Thr Leu
                85                  90                  95

agt gac ccg gtg cag cta gaa gtc cat atc ggc tgg ctg ttg ctc cag       336
Ser Asp Pro Val Gln Leu Glu Val His Ile Gly Trp Leu Leu Leu Gln
                100                 105                 110

gcc cct cgg tgg gtg ttc aag gag gaa gac cct att cac ctg agg tgt       384
Ala Pro Arg Trp Val Phe Lys Glu Glu Asp Pro Ile His Leu Arg Cys
            115                 120                 125

cac agc tgg aag aac act gct ctg cat aag gtc aca tat tta cag aat       432
His Ser Trp Lys Asn Thr Ala Leu His Lys Val Thr Tyr Leu Gln Asn
        130                 135                 140

ggc aaa ggc agg aag tat ttt cat cat aat tct gac ttc tac att cca       480
Gly Lys Gly Arg Lys Tyr Phe His His Asn Ser Asp Phe Tyr Ile Pro
145                 150                 155                 160

aaa gcc aca ctc aaa gac agc ggc tcc tac ttc tgc agg ggg ctt gtt       528
Lys Ala Thr Leu Lys Asp Ser Gly Ser Tyr Phe Cys Arg Gly Leu Val
                165                 170                 175

ggg agt aaa aat gtg tct tca gag act gtg aac atc acc atc act caa       576
Gly Ser Lys Asn Val Ser Ser Glu Thr Val Asn Ile Thr Ile Thr Gln
                180                 185                 190
```

```
ggt ttg tca gtg tca acc atc tca tca ttc ttt cca cct ggg tac caa      624
Gly Leu Ser Val Ser Thr Ile Ser Ser Phe Phe Pro Pro Gly Tyr Gln
        195                 200                 205

gtc tct ttc tgc ttg gtg atg gta ctc ctt ttt gca gtg gac aca gga      672
Val Ser Phe Cys Leu Val Met Val Leu Leu Phe Ala Val Asp Thr Gly
        210                 215                 220

cta tat ttc tct gtg aag aca aac att cga agc tca aca aga gac tgg      720
Leu Tyr Phe Ser Val Lys Thr Asn Ile Arg Ser Ser Thr Arg Asp Trp
225                 230                 235                 240

aag gac cat aaa ttt aaa tgg aga aag gac cct caa gac aaa tga         765
Lys Asp His Lys Phe Lys Trp Arg Lys Asp Pro Gln Asp Lys
                    245                 250
```

<210> 22
<211> 254
<212> PRT
<213> Homo sapiens

<400> 22

Met Trp Gln Leu Leu Leu Pro Thr Ala Leu Leu Leu Val Ser Ala
1                   5                   10                  15

Gly Met Arg Thr Glu Asp Leu Pro Lys Ala Val Val Phe Leu Glu Pro
            20                  25                  30

Gln Trp Tyr Arg Val Leu Glu Lys Asp Ser Val Thr Leu Lys Cys Gln
            35                  40                  45

Gly Ala Tyr Ser Pro Glu Asp Asn Ser Thr Gln Trp Phe His Asn Glu
    50                  55                  60

Ser Leu Ile Ser Ser Gln Ala Ser Ser Tyr Phe Ile Asp Ala Ala Thr
65                  70                  75                  80

Val Asp Asp Ser Gly Glu Tyr Arg Cys Gln Thr Asn Leu Ser Thr Leu
            85                  90                  95

Ser Asp Pro Val Gln Leu Glu Val His Ile Gly Trp Leu Leu Leu Gln
            100                 105                 110

Ala Pro Arg Trp Val Phe Lys Glu Glu Asp Pro Ile His Leu Arg Cys
            115                 120                 125

His Ser Trp Lys Asn Thr Ala Leu His Lys Val Thr Tyr Leu Gln Asn
    130                 135                 140

Gly Lys Gly Arg Lys Tyr Phe His His Asn Ser Asp Phe Tyr Ile Pro
145                 150                 155                 160

Lys Ala Thr Leu Lys Asp Ser Gly Ser Tyr Phe Cys Arg Gly Leu Val
            165                 170                 175

Gly Ser Lys Asn Val Ser Ser Glu Thr Val Asn Ile Thr Ile Thr Gln
            180                 185                 190

Gly Leu Ser Val Ser Thr Ile Ser Ser Phe Phe Pro Pro Gly Tyr Gln
            195                 200                 205

Val Ser Phe Cys Leu Val Met Val Leu Leu Phe Ala Val Asp Thr Gly
    210                 215                 220

Leu Tyr Phe Ser Val Lys Thr Asn Ile Arg Ser Ser Thr Arg Asp Trp
225                 230                 235                 240

Lys Asp His Lys Phe Lys Trp Arg Lys Asp Pro Gln Asp Lys
            245                 250

140

EP 2 857 419 B1

<210> 23
<211> 702
<212> DNA
<213> Homo sapiens

<220>
<221> CDS
<222> (1)..(702)

<400> 23

```
atg tgg cag ctg ctc ctc cca act gct ctg cta ctt cta gtt tca gct      48
Met Trp Gln Leu Leu Leu Pro Thr Ala Leu Leu Leu Leu Val Ser Ala
1               5                   10                  15

ggc atg cgg act gaa gat ctc cca aag gct gtg gtg ttc ctg gag cct      96
Gly Met Arg Thr Glu Asp Leu Pro Lys Ala Val Val Phe Leu Glu Pro
                20                  25                  30

caa tgg tac agc gtg ctt gag aag gac agt gtg act ctg aag tgc cag     144
Gln Trp Tyr Ser Val Leu Glu Lys Asp Ser Val Thr Leu Lys Cys Gln
            35                  40                  45

gga gcc tac tcc cct gag gac aat tcc aca cag tgg ttt cac aat gag     192
Gly Ala Tyr Ser Pro Glu Asp Asn Ser Thr Gln Trp Phe His Asn Glu
        50                  55                  60

agc ctc atc tca agc cag gcc tcg agc tac ttc att gac gct gcc aca     240
Ser Leu Ile Ser Ser Gln Ala Ser Ser Tyr Phe Ile Asp Ala Ala Thr
65                  70                  75                  80

gtc aac gac agt gga gag tac agg tgc cag aca aac ctc tcc acc ctc     288
Val Asn Asp Ser Gly Glu Tyr Arg Cys Gln Thr Asn Leu Ser Thr Leu
                85                  90                  95

agt gac ccg gtg cag cta gaa gtc cat atc ggc tgg ctg ttg ctc cag     336
Ser Asp Pro Val Gln Leu Glu Val His Ile Gly Trp Leu Leu Leu Gln
```

141

```
                    100                   105                   110

        gcc cct cgg tgg gtg ttc aag gag gaa gac cct att cac ctg agg tgt        384
        Ala Pro Arg Trp Val Phe Lys Glu Glu Asp Pro Ile His Leu Arg Cys
            115                   120                   125

        cac agc tgg aag aac act gct ctg cat aag gtc aca tat tta cag aat        432
        His Ser Trp Lys Asn Thr Ala Leu His Lys Val Thr Tyr Leu Gln Asn
            130                   135                   140

        ggc aaa gac agg aag tat ttt cat cat aat tct gac ttc cac att cca        480
        Gly Lys Asp Arg Lys Tyr Phe His His Asn Ser Asp Phe His Ile Pro
        145                   150                   155                   160

        aaa gcc aca ctc aaa gat agc ggc tcc tac ttc tgc agg ggg ctt gtt        528
        Lys Ala Thr Leu Lys Asp Ser Gly Ser Tyr Phe Cys Arg Gly Leu Val
                        165                   170                   175

        ggg agt aaa aat gtg tct tca gag act gtg aac atc acc atc act caa        576
        Gly Ser Lys Asn Val Ser Ser Glu Thr Val Asn Ile Thr Ile Thr Gln
                        180                   185                   190

        ggt ttg gca gtg tca acc atc tca tca ttc tct cca cct ggg tac caa        624
        Gly Leu Ala Val Ser Thr Ile Ser Ser Phe Ser Pro Pro Gly Tyr Gln
                    195                   200                   205

        gtc tct ttc tgc ttg gtg atg gta ctc ctt ttt gca gtg gac aca gga        672
        Val Ser Phe Cys Leu Val Met Val Leu Leu Phe Ala Val Asp Thr Gly
                    210                   215                   220

        cta tat ttc tct gtg aag aca aac att tga                                702
        Leu Tyr Phe Ser Val Lys Thr Asn Ile
        225                   230
```

<210> 24
<211> 233
<212> PRT
<213> Homo sapiens

<400> 24

```
Met Trp Gln Leu Leu Leu Pro Thr Ala Leu Leu Leu Leu Val Ser Ala
1               5               10              15

Gly Met Arg Thr Glu Asp Leu Pro Lys Ala Val Val Phe Leu Glu Pro
        20              25              30

Gln Trp Tyr Ser Val Leu Glu Lys Asp Ser Val Thr Leu Lys Cys Gln
        35              40              45

Gly Ala Tyr Ser Pro Glu Asp Asn Ser Thr Gln Trp Phe His Asn Glu
    50              55              60

Ser Leu Ile Ser Ser Gln Ala Ser Ser Tyr Phe Ile Asp Ala Ala Thr
65              70              75              80

Val Asn Asp Ser Gly Glu Tyr Arg Cys Gln Thr Asn Leu Ser Thr Leu
                85              90              95

Ser Asp Pro Val Gln Leu Glu Val His Ile Gly Trp Leu Leu Leu Gln
        100             105             110

Ala Pro Arg Trp Val Phe Lys Glu Glu Asp Pro Ile His Leu Arg Cys
        115             120             125

His Ser Trp Lys Asn Thr Ala Leu His Lys Val Thr Tyr Leu Gln Asn
    130             135             140

Gly Lys Asp Arg Lys Tyr Phe His His Asn Ser Asp Phe His Ile Pro
145             150             155             160

Lys Ala Thr Leu Lys Asp Ser Gly Ser Tyr Phe Cys Arg Gly Leu Val
        165             170             175

Gly Ser Lys Asn Val Ser Ser Glu Thr Val Asn Ile Thr Ile Thr Gln
        180             185             190

Gly Leu Ala Val Ser Thr Ile Ser Ser Phe Ser Pro Pro Gly Tyr Gln
        195             200             205

Val Ser Phe Cys Leu Val Met Val Leu Leu Phe Ala Val Asp Thr Gly
    210             215             220

Leu Tyr Phe Ser Val Lys Thr Asn Ile
225             230
```

<210> 25

<211> 4
<212> PRT
<213> Artificial Sequence

<220>
<223> an artificially synthesized sequence

<400> 25

```
                                    Gly Gly Gly Ser
                                    1
```

<210> 26
<211> 4
<212> PRT
<213> Artificial Sequence

<220>
<223> an artificially synthesized sequence

<400> 26

```
                                Ser Gly Gly Gly
                                1
```

<210> 27
<211> 5
<212> PRT
<213> Artificial Sequence

<220>
<223> an artificially synthesized sequence

<400> 27

```
                            Gly Gly Gly Gly Ser
                            1               5
```

<210> 28
<211> 5
<212> PRT
<213> Artificial Sequence

<220>
<223> an artificially synthesized sequence

<400> 28

```
                            Ser Gly Gly Gly Gly
                            1               5
```

<210> 29
<211> 6
<212> PRT
<213> Artificial Sequence

<220>
<223> an artificially synthesized sequence

<400> 29

```
                                Gly Gly Gly Gly Gly Ser
                                1                   5
```

<210> 30
<211> 6
<212> PRT
<213> Artificial Sequence

<220>
<223> an artificially synthesized sequence

<400> 30

```
                                Ser Gly Gly Gly Gly Gly
                                1                   5
```

<210> 31
<211> 7
<212> PRT
<213> Artificial Sequence

<220>
<223> an artificially synthesized sequence

<400> 31

```
                                Gly Gly Gly Gly Gly Gly Ser
                                1                   5
```

<210> 32
<211> 7
<212> PRT
<213> Artificial Sequence

<220>
<223> an artificially synthesized sequence

<400> 32

```
                                Ser Gly Gly Gly Gly Gly Gly
                                1                   5
```

<210> 33
<211> 464
<212> PRT
<213> Artificial Sequence

<220>
<223> an artificially synthesized sequence

<400> 33

Gln Val Gln Leu Gln Glu Ser Gly Pro Gly Leu Val Arg Pro Ser Gln
1                   5                   10                  15

Thr Leu Ser Leu Thr Cys Thr Val Ser Gly Tyr Ser Ile Thr Ser Asp
            20                  25                  30

His Ala Trp Ser Trp Val Arg Gln Pro Pro Gly Arg Gly Leu Glu Trp
            35                  40                  45

Ile Gly Tyr Ile Ser Tyr Ser Gly Ile Thr Thr Tyr Asn Pro Ser Leu
        50                  55                  60

Lys Ser Arg Val Thr Met Leu Arg Asp Thr Ser Lys Asn Gln Phe Ser
65                  70                  75                  80

Leu Arg Leu Ser Ser Val Thr Ala Ala Asp Thr Ala Val Tyr Tyr Cys
                85                  90                  95

Ala Arg Ser Leu Ala Arg Thr Thr Ala Met Asp Tyr Trp Gly Gln Gly
            100                 105                 110

```
Ser Leu Val Thr Val Ser Ser Ala Ser Pro Thr Ser Pro Lys Val Phe
        115             120             125

Pro Leu Ser Leu Cys Ser Thr Gln Pro Asp Gly Asn Val Val Ile Ala
        130             135             140

Cys Leu Val Gln Gly Phe Phe Pro Gln Glu Pro Leu Ser Val Thr Trp
145             150             155             160

Ser Glu Ser Gly Gln Gly Val Thr Ala Arg Asn Phe Pro Pro Ser Gln
                165             170             175

Asp Ala Ser Gly Asp Leu Tyr Thr Thr Ser Ser Gln Leu Thr Leu Pro
        180             185             190

Ala Thr Gln Cys Leu Ala Gly Lys Ser Val Thr Cys His Val Lys His
        195             200             205

Tyr Thr Asn Pro Ser Gln Asp Val Thr Val Pro Cys Pro Val Pro Ser
210             215             220

Thr Pro Pro Thr Pro Ser Pro Ser Thr Pro Pro Thr Pro Ser Pro Ser
225             230             235             240

Cys Cys His Pro Arg Leu Ser Leu His Arg Pro Ala Leu Glu Asp Leu
            245             250             255

Leu Leu Gly Ser Glu Ala Asn Leu Thr Cys Thr Leu Thr Gly Leu Arg
        260             265             270

Asp Ala Ser Gly Val Thr Phe Thr Trp Thr Pro Ser Ser Gly Lys Ser
        275             280             285

Ala Val Gln Gly Pro Pro Glu Arg Asp Leu Cys Gly Cys Tyr Ser Val
        290             295             300

Ser Ser Val Leu Pro Gly Cys Ala Glu Pro Trp Asn His Gly Lys Thr
305             310             315             320

Phe Thr Cys Thr Ala Ala Tyr Pro Glu Ser Lys Thr Pro Leu Thr Ala
            325             330             335

Thr Leu Ser Lys Ser Gly Asn Thr Phe Arg Pro Glu Val His Leu Leu
        340             345             350

Pro Pro Pro Ser Glu Glu Leu Ala Leu Asn Glu Leu Val Thr Leu Thr
        355             360             365
```

147

```
Cys Leu Ala Arg Gly Phe Ser Pro Lys Asp Val Leu Val Arg Trp Leu
    370             375             380

Gln Gly Ser Gln Glu Leu Pro Arg Glu Lys Tyr Leu Thr Trp Ala Ser
385             390             395             400

Arg Gln Glu Pro Ser Gln Gly Thr Thr Thr Phe Ala Val Thr Ser Ile
            405             410             415

Leu Arg Val Ala Ala Glu Asp Trp Lys Lys Gly Asp Thr Phe Ser Cys
        420             425             430

Met Val Gly His Glu Ala Leu Pro Leu Ala Phe Thr Gln Lys Thr Ile
        435             440             445

Asp Arg Leu Ala Gly Lys Glu Gln Lys Leu Ile Ser Glu Glu Asp Leu
    450             455             460
```

<210> 34
<211> 458
<212> PRT
<213> Artificial Sequence

<220>
<223> an artificially synthesized sequence

<400> 34

```
Gln Val Gln Leu Val Gln Ser Gly Ala Glu Val Lys Lys Pro Gly Ala
1               5               10              15

Ser Val Lys Val Ser Cys Lys Ala Ser Gly Tyr Thr Phe Thr Asp Tyr
            20              25              30

Glu Met His Trp Ile Arg Gln Pro Pro Gly Gln Gly Leu Glu Trp Ile
        35              40              45

Gly Ala Ile Asp Pro Lys Thr Gly Asp Thr Ala Tyr Ser Gln Lys Phe
    50              55              60

Lys Gly Arg Val Thr Leu Thr Ala Asp Lys Ser Thr Ser Thr Ala Tyr
65              70              75              80

Met Glu Leu Ser Ser Leu Thr Ser Glu Asp Thr Ala Val Tyr Tyr Cys
            85              90              95

Thr Arg Phe Tyr Ser Tyr Thr Tyr Trp Gly Gln Gly Thr Leu Val Thr
        100             105             110
```

```
Val Ser Ser Ala Ser Pro Thr Ser Pro Lys Val Phe Pro Leu Ser Leu
    115                 120                 125

Cys Ser Thr Gln Pro Asp Gly Asn Val Val Ile Ala Cys Leu Val Gln
    130                 135                 140

Gly Phe Phe Pro Gln Glu Pro Leu Ser Val Thr Trp Ser Glu Ser Gly
145                 150                 155                 160

Gln Gly Val Thr Ala Arg Asn Phe Pro Pro Ser Gln Asp Ala Ser Gly
                165                 170                 175

Asp Leu Tyr Thr Thr Ser Ser Gln Leu Thr Leu Pro Ala Thr Gln Cys
            180                 185                 190

Leu Ala Gly Lys Ser Val Thr Cys His Val Lys His Tyr Thr Asn Pro
            195                 200                 205

Ser Gln Asp Val Thr Val Pro Cys Pro Val Pro Ser Thr Pro Pro Thr
    210                 215                 220

Pro Ser Pro Ser Thr Pro Pro Thr Pro Ser Pro Ser Cys Cys His Pro
225                 230                 235                 240

Arg Leu Ser Leu His Arg Pro Ala Leu Glu Asp Leu Leu Leu Gly Ser
                245                 250                 255

Glu Ala Asn Leu Thr Cys Thr Leu Thr Gly Leu Arg Asp Ala Ser Gly
            260                 265                 270

Val Thr Phe Thr Trp Thr Pro Ser Ser Gly Lys Ser Ala Val Gln Gly
            275                 280                 285

Pro Pro Glu Arg Asp Leu Cys Gly Cys Tyr Ser Val Ser Ser Val Leu
    290                 295                 300

Pro Gly Cys Ala Glu Pro Trp Asn His Gly Lys Thr Phe Thr Cys Thr
305                 310                 315                 320

Ala Ala Tyr Pro Glu Ser Lys Thr Pro Leu Thr Ala Thr Leu Ser Lys
            325                 330                 335

Ser Gly Asn Thr Phe Arg Pro Glu Val His Leu Leu Pro Pro Pro Ser
            340                 345                 350

Glu Glu Leu Ala Leu Asn Glu Leu Val Thr Leu Thr Cys Leu Ala Arg
            355                 360                 365
```

```
Gly Phe Ser Pro Lys Asp Val Leu Val Arg Trp Leu Gln Gly Ser Gln
    370             375             380

Glu Leu Pro Arg Glu Lys Tyr Leu Thr Trp Ala Ser Arg Gln Glu Pro
385             390             395                 400

Ser Gln Gly Thr Thr Thr Phe Ala Val Thr Ser Ile Leu Arg Val Ala
                405             410             415

Ala Glu Asp Trp Lys Lys Gly Asp Thr Phe Ser Cys Met Val Gly His
            420             425             430

Glu Ala Leu Pro Leu Ala Phe Thr Gln Lys Thr Ile Asp Arg Leu Ala
        435             440             445

Gly Lys Asp Tyr Lys Asp Asp Asp Lys
    450             455
```

<210> 35
<211> 460
<212> PRT
<213> Artificial Sequence

<220>
<223> an artificially synthesized sequence

<400> 35

```
Gln Val Gln Leu Val Gln Ser Gly Ala Glu Val Lys Lys Pro Gly Ala
1               5               10              15

Ser Val Lys Val Ser Cys Lys Ala Ser Gly Tyr Thr Phe Thr Asp Tyr
            20              25              30

Glu Met His Trp Ile Arg Gln Pro Pro Gly Gln Gly Leu Glu Trp Ile
        35              40              45

Gly Ala Ile Asp Pro Lys Thr Gly Asp Thr Ala Tyr Ser Gln Lys Phe
    50              55              60

Lys Gly Arg Val Thr Leu Thr Ala Asp Lys Ser Thr Ser Thr Ala Tyr
65              70              75              80

Met Glu Leu Ser Ser Leu Thr Ser Glu Asp Thr Ala Val Tyr Tyr Cys
            85              90              95

Thr Arg Phe Tyr Ser Tyr Thr Tyr Trp Gly Gln Gly Thr Leu Val Thr
        100             105             110
```

150

Val Ser Ser Ala Ser Pro Thr Ser Pro Lys Val Phe Pro Leu Ser Leu
        115                 120                 125

Cys Ser Thr Gln Pro Asp Gly Asn Val Val Ile Ala Cys Leu Val Gln
        130                 135                 140

Gly Phe Phe Pro Gln Glu Pro Leu Ser Val Thr Trp Ser Glu Ser Gly
    145                 150                 155                 160

Gln Gly Val Thr Ala Arg Asn Phe Pro Pro Ser Gln Asp Ala Ser Gly
                165                 170                 175

Asp Leu Tyr Thr Thr Ser Ser Gln Leu Thr Leu Pro Ala Thr Gln Cys
            180                 185                 190

Leu Ala Gly Lys Ser Val Thr Cys His Val Lys His Tyr Thr Asn Pro
            195                 200                 205

Ser Gln Asp Val Thr Val Pro Cys Pro Val Pro Ser Thr Pro Pro Thr
    210                 215                 220

Pro Ser Pro Ser Thr Pro Pro Thr Pro Ser Pro Ser Cys Cys His Pro
225                 230                 235                 240

Arg Leu Ser Leu His Arg Pro Ala Leu Glu Asp Leu Leu Leu Gly Ser
                245                 250                 255

Glu Ala Asn Leu Thr Cys Thr Leu Thr Gly Leu Arg Asp Ala Ser Gly
            260                 265                 270

Val Thr Phe Thr Trp Thr Pro Ser Ser Gly Lys Ser Ala Val Gln Gly
    275                 280                 285

Pro Pro Glu Arg Asp Leu Cys Gly Cys Tyr Ser Val Ser Ser Val Leu
    290                 295                 300

Pro Gly Cys Ala Glu Pro Trp Asn His Gly Lys Thr Phe Thr Cys Thr
305                 310                 315                 320

Ala Ala Tyr Pro Glu Ser Lys Thr Pro Leu Thr Ala Thr Leu Ser Lys
                325                 330                 335

Ser Gly Asn Thr Phe Arg Pro Glu Val His Leu Leu Pro Pro Pro Ser
        340                 345                 350

Glu Glu Leu Ala Leu Asn Glu Leu Val Thr Leu Thr Cys Leu Ala Arg
        355                 360                 365

151

```
Gly Phe Ser Pro Lys Asp Val Leu Val Arg Trp Leu Gln Gly Ser Gln
    370             375             380

Glu Leu Pro Arg Glu Lys Tyr Leu Thr Trp Ala Ser Arg Gln Glu Pro
385             390             395             400

Ser Gln Gly Thr Thr Thr Phe Ala Val Thr Ser Ile Leu Arg Val Ala
            405             410             415

Ala Glu Asp Trp Lys Lys Gly Asp Thr Phe Ser Cys Met Val Gly His
            420             425             430

Glu Ala Leu Pro Leu Ala Phe Thr Gln Lys Thr Ile Asp Arg Leu Ala
        435             440             445

Gly Lys Glu Gln Lys Leu Ile Ser Glu Glu Asp Leu
    450             455             460
```

<210> 36
<211> 214
<212> PRT
<213> Artificial Sequence

<220>
<223> an artificially synthesized sequence

<400> 36

```
Asp Ile Gln Met Thr Gln Ser Pro Ser Ser Leu Ser Ala Ser Val Gly
1               5               10              15

Asp Arg Val Thr Ile Thr Cys Arg Ala Ser Gln Asp Ile Ser Ser Tyr
            20              25              30

Leu Asn Trp Tyr Gln Gln Lys Pro Gly Lys Ala Pro Lys Leu Leu Ile
        35              40              45

Tyr Tyr Thr Ser Arg Leu His Ser Gly Val Pro Ser Arg Phe Ser Gly
    50              55              60

Ser Gly Ser Gly Thr Asp Phe Thr Phe Thr Ile Ser Ser Leu Gln Pro
65              70              75              80

Glu Asp Ile Ala Thr Tyr Tyr Cys Gln Gln Gly Asn Thr Leu Pro Tyr
            85              90              95

Thr Phe Gly Gln Gly Thr Lys Val Glu Ile Lys Arg Thr Val Ala Ala
        100             105             110
```

152

```
        Pro Ser Val Phe Ile Phe Pro Pro Ser Asp Glu Gln Leu Lys Ser Gly
            115                 120                 125


        Thr Ala Ser Val Val Cys Leu Leu Asn Asn Phe Tyr Pro Arg Glu Ala
            130                 135                 140


        Lys Val Gln Trp Lys Val Asp Asn Ala Leu Gln Ser Gly Asn Ser Gln
        145                 150                 155                 160


        Glu Ser Val Thr Glu Gln Asp Ser Lys Asp Ser Thr Tyr Ser Leu Ser
                        165                 170                 175


        Ser Thr Leu Thr Leu Ser Lys Ala Asp Tyr Glu Lys His Lys Val Tyr
                180                 185                 190


        Ala Cys Glu Val Thr His Gln Gly Leu Ser Ser Pro Val Thr Lys Ser
            195                 200                 205


        Phe Asn Arg Gly Glu Cys
            210
```

<210> 37
<211> 219
<212> PRT
<213> Artificial Sequence

<220>
<223> an artificially synthesized sequence

<400> 37

```
Asp Ile Val Met Thr Gln Ser Pro Leu Ser Leu Pro Val Thr Pro Gly
1               5               10              15

Glu Pro Ala Ser Ile Ser Cys Arg Ser Ser Gln Ser Leu Val His Ser
            20              25              30

Asn Arg Asn Thr Tyr Leu His Trp Tyr Gln Gln Lys Pro Gly Gln Ala
            35              40              45

Pro Arg Leu Leu Ile Tyr Lys Val Ser Asn Arg Phe Ser Gly Val Pro
    50              55              60

Asp Arg Phe Ser Gly Ser Gly Ser Gly Thr Asp Phe Thr Leu Lys Ile
65              70              75              80

Ser Arg Val Glu Ala Glu Asp Val Gly Val Tyr Tyr Cys Ser Gln Asn
            85              90              95

Thr His Val Pro Pro Thr Phe Gly Gln Gly Thr Lys Leu Glu Ile Lys

            100             105             110

Arg Thr Val Ala Ala Pro Ser Val Phe Ile Phe Pro Pro Ser Asp Glu
        115             120             125

Gln Leu Lys Ser Gly Thr Ala Ser Val Val Cys Leu Leu Asn Asn Phe
    130             135             140

Tyr Pro Arg Glu Ala Lys Val Gln Trp Lys Val Asp Asn Ala Leu Gln
145             150             155             160

Ser Gly Asn Ser Gln Glu Ser Val Thr Glu Gln Asp Ser Lys Asp Ser
            165             170             175

Thr Tyr Ser Leu Ser Ser Thr Leu Thr Leu Ser Lys Ala Asp Tyr Glu
            180             185             190

Lys His Lys Val Tyr Ala Cys Glu Val Thr His Gln Gly Leu Ser Ser
            195             200             205

Pro Val Thr Lys Ser Phe Asn Arg Gly Glu Cys
    210             215
```

<210> 38
<211> 451
<212> PRT
<213> Artificial Sequence

<220>
<223> an artificially synthesized sequence

<400> 38

```
Gln Val Gln Leu Val Gln Ser Gly Ala Glu Val Lys Lys Pro Gly Ser
1               5               10              15

Ser Val Lys Val Ser Cys Lys Ala Ser Gly Gly Thr Phe Ser Ser Tyr
        20              25              30

Ala Ile Ser Trp Val Arg Gln Ala Pro Gly Gln Gly Leu Glu Trp Met
        35              40              45

Gly Gly Ile Ile Pro Ile Phe Gly Thr Ala Asn Tyr Ala Gln Lys Phe
    50              55              60

Gln Gly Arg Val Thr Ile Thr Ala Asp Glu Ser Thr Ser Thr Ala Tyr
65              70              75              80

Met Glu Leu Ser Ser Leu Arg Ser Glu Asp Thr Ala Val Tyr Tyr Cys
            85              90              95
```

```
Ala Arg Pro Arg Trp Glu Thr Ala Ile Ser Ser Asp Ala Phe Asp Ile
        100             105             110

Trp Gly Gln Gly Thr Met Val Thr Val Ser Ser Ala Ser Thr Lys Gly
        115             120             125

Pro Ser Val Phe Pro Leu Ala Pro Ser Ser Lys Ser Thr Ser Gly Gly
        130             135             140

Thr Ala Ala Leu Gly Cys Leu Val Lys Asp Tyr Phe Pro Glu Pro Val
145             150             155             160

Thr Val Ser Trp Asn Ser Gly Ala Leu Thr Ser Gly Val His Thr Phe
                165             170             175

Pro Ala Val Leu Gln Ser Ser Gly Leu Tyr Ser Leu Ser Ser Val Val
                180             185             190

Thr Val Pro Ser Ser Ser Leu Gly Thr Gln Thr Tyr Ile Cys Asn Val
        195             200             205

Asn His Lys Pro Ser Asn Thr Lys Val Asp Lys Lys Val Glu Pro Lys
    210             215             220

Ser Cys Asp Lys Thr His Thr Cys Pro Pro Cys Pro Ala Pro Glu Leu
225             230             235             240

Leu Gly Gly Pro Ser Val Phe Leu Phe Pro Pro Lys Pro Lys Asp Thr
                245             250             255

Leu Met Ile Ser Arg Thr Pro Glu Val Thr Cys Val Val Val Asp Val
                260             265             270

Ser His Glu Asp Pro Glu Val Lys Phe Asn Trp Tyr Val Asp Gly Val
        275             280             285

Glu Val His Asn Ala Lys Thr Lys Pro Arg Glu Glu Gln Tyr Asn Ser
        290             295             300

Thr Tyr Arg Val Val Ser Val Leu Thr Val Leu His Gln Asp Trp Leu
305             310             315             320

Asn Gly Lys Glu Tyr Lys Cys Lys Val Ser Asn Lys Ala Leu Pro Ala
                325             330             335

Pro Ile Glu Lys Thr Ile Ser Lys Ala Lys Gly Gln Pro Arg Glu Pro
```

340                     345                     350

Gln Val Tyr Thr Leu Pro Pro Ser Arg Asp Glu Leu Thr Lys Asn Gln
        355             360                 365

Val Ser Leu Thr Cys Leu Val Lys Gly Phe Tyr Pro Ser Asp Ile Ala
        370             375             380

Val Glu Trp Glu Ser Asn Gly Gln Pro Glu Asn Asn Tyr Lys Thr Thr
385             390             395             400

Pro Pro Val Leu Asp Ser Asp Gly Ser Phe Phe Leu Tyr Ser Lys Leu
            405             410             415

Thr Val Asp Lys Ser Arg Trp Gln Gln Gly Asn Val Phe Ser Cys Ser
        420             425             430

Val Met His Glu Ala Leu His Asn His Tyr Thr Gln Lys Ser Leu Ser
        435             440             445

Leu Ser Pro
        450

<210> 39
<211> 214
<212> PRT
<213> Artificial Sequence

<220>
<223> an artificially synthesized sequence

<400> 39

```
Asp Ile Gln Met Thr Gln Ser Pro Ser Ser Leu Ser Ala Ser Val Gly
1               5               10              15

Asp Arg Val Thr Ile Thr Cys Arg Ala Ser Gln Ser Ile Ser Asp Asp
            20              25              30

Leu Asn Trp Tyr Gln Gln Lys Pro Gly Lys Ala Pro Lys Leu Leu Ile
        35              40              45

Tyr Glu Ala Ser Asn Leu Gln Ser Gly Val Pro Ser Arg Phe Ser Gly
    50              55              60

Ser Gly Ser Gly Thr Asp Phe Thr Leu Thr Ile Ser Ser Leu Gln Pro
65              70              75              80

Glu Asp Phe Ala Thr Tyr Tyr Cys Gln Gln His Ser Ser Ser Pro Leu
        85              90              95

Thr Phe Gly Gln Gly Thr Lys Val Glu Ile Lys Arg Thr Val Ala Ala
        100             105             110

Pro Ser Val Phe Ile Phe Pro Pro Ser Asp Glu Gln Leu Lys Ser Gly
        115             120             125

Thr Ala Ser Val Val Cys Leu Leu Asn Asn Phe Tyr Pro Arg Glu Ala
    130             135             140

Lys Val Gln Trp Lys Val Asp Asn Ala Leu Gln Ser Gly Asn Ser Gln
145             150             155             160

Glu Ser Val Thr Glu Gln Asp Ser Lys Asp Ser Thr Tyr Ser Leu Ser
            165             170             175

Ser Thr Leu Thr Leu Ser Lys Ala Asp Tyr Glu Lys His Lys Val Tyr
        180             185             190

Ala Cys Glu Val Thr His Gln Gly Leu Ser Ser Pro Val Thr Lys Ser
        195             200             205

Phe Asn Arg Gly Glu Cys
        210
```

**Description**

Technical Field

Disclosed herein are uses of antigen-binding molecules for eliminating aggregated antigens from plasma; methods for eliminating aggregated antigens from plasma, which comprise administering antigen-binding molecules; pharmaceutical compositions comprising antigen-binding molecules that are capable of eliminating aggregated antigens from plasma; methods of screening for antigen-binding molecules for eliminating aggregated antigens from plasma; and methods for producing antigen-binding molecules for eliminating aggregated antigens from plasma.

Background Art

When proteins form aggregates due to various factors such as gene mutations and environmental changes, they are known to become causes of various diseases by reducing physiological functions of proteins or by posing toxic effects on cells. For example, when amyloid-β aggregates and accumulates in the brain, nerve cells degenerate and Alzheimer's disease develops. Furthermore, when immunoglobulin L chain aggregates and deposits in each organ to cause organ failure, AL amyloidosis develops. Similar to these diseases, a group of diseases characterized by extracellular accumulation of various protein aggregates is called amyloidosis, and the classification by The Research Committees on intractable Diseases Specified by the Japanese Ministry of Health and Welfare (now Japanese Ministry of Health, Labour and Welfare) reports that there are ten disease types of systemic amyloidosis and ten disease types of localized amyloidosis. Besides amyloidosis, α-synuclein disease is known as a disease where aggregated α-synuclein is deposited in nerve cells. Some inherited Parkinson's diseases are caused by deposition of α-synuclein in cerebral neurons, and they are considered to be a type of α-synuclein disease. As described above, many diseases caused by protein aggregates are known in the world, but the mechanism of protein aggregation is still unclear; and for many of these diseases, an ultimate therapeutic agent does not exist.
Recently, antibodies are drawing attention as pharmaceuticals as they have a high stability in plasma and have few side effects. At present, a number of IgG-type antibody pharmaceuticals are available on the market and many antibody pharmaceuticals are currently under development (Non-patent Documents 1 and 2).
Meanwhile, the antigen-neutralizing capacity of a single antibody molecule depends on its affinity. By increasing the affinity, an antigen can be neutralized by a smaller amount of an antibody. Various methods can be used to enhance antibody affinity (Non-patent Document 6). Furthermore, if the affinity could be made infinite by covalently binding the antibody to the antigen, a single antibody molecule could neutralize one antigen molecule (a divalent antibody can neutralize two antigen molecules). However, the stoichiometric neutralization of one antibody against one antigen (one divalent antibody against two antigens) is the limit of preexisting methods, and thus it was impossible to completely neutralize antigen with an amount of antibody smaller than the amount of antigen. In other words, the affinity-enhancing effect has a limit (Non-patent Document 9). To prolong the neutralization effect of a neutralizing antibody for a certain period, the antibody must be administered at a dose higher than the amount of antigen produced in the body during the same period. Therefore, with just the above-described improvement of antibody pharmacokinetics or affinity maturation technology, there were limitations when it comes to reduction of the required antibody dose. Accordingly, in order to sustain antibody's antigen-neutralizing effect for a target period with an amount of the antibody smaller than the amount of antigen, a single antibody must neutralize multiple antigens.
As a novel method for achieving this objective, use of an antibody that binds to an antigen in a pH-dependent manner has been reported recently to enable a single antibody molecule to bind to multiple antigen molecules (Patent Document 1 and Non-patent Document 5). Antibodies with pH-dependent antigen binding, which bind strongly to an antigen under the neutral condition in plasma, and dissociate from the antigen under the acidic condition in the endosome, can dissociate from the antigen in the endosome. When an antibody with pH-dependent antigen binding that has dissociated from the antigen is recycled into plasma by FcRn, the antibody can again bind to an antigen; therefore, a single pH-dependent antigen-binding antibody molecule can repeatedly bind to multiple antigens. Such a recycling antibody will be very useful as a pharmaceutical since a single antibody molecule can repeatedly bind to multiple antigens.
In addition, the plasma retention of an antigen is very short when compared to that of antibodies that are recycled by binding to FcRn. When a typical antibody with long plasma retention binds to such an antigen, the plasma retention of the antigen-antibody complex is prolonged to the same as that of the antibody. Thus, when a typical antibody is administered, the antibody binds to an antigen, the plasma retention of the antigen is prolonged (becomes difficult to be eliminated from plasma) by binding to the antibody, and thus the plasma antigen concentration is increased. On the other hand, antibodies with pH-dependent antigen binding can suppress increase of antigen concentration in plasma by dissociating from the antigen in the endosome. However, even with such a pH-dependent antigen-binding antibody, the plasma antigen concentration may be increased by antibody administration compared to before antibody administration.

Recently, antibodies were produced by enhancing the FcRn-binding property of antibodies with pH-dependent antigen binding under a neutral condition, and it was found that administration of such antibodies can decrease the antigen concentration in plasma compared to before antibody administration (Patent Document 2). While antibody administration of recycled antibodies such as antibodies with pH-dependent antigen binding and typical antibodies result in an increase of antigen concentration in plasma, pH-dependent antigen-binding antibodies with enhanced FcRn-binding under a neutral condition can decrease antigen concentration in plasma by antibody administration. Such antibodies are very useful as pharmaceuticals since they can actively eliminate antigens from plasma.

However, for diseases where protein aggregates is a cause of disease, monomers that have normal function co-exist in plasma with the disease-causing aggregates, and antibodies having a property of selectively eliminating aggregates from plasma are desired.

Prior art documents are shown below.

Prior Art Documents

[Patent Documents]

[Patent Document 1] WO 2009/125825, ANTIGEN-BINDING MOLECULE CAPABLE OF BINDING TO TWO OR MORE ANTIGEN MOLECULES REPEATEDLY
[Patent Document 2] WO 2011/122011, ANTIBODIES WITH MODIFIED AFFINITY TO FCRN THAT PROMOTE ANTIGEN CLEARANCE

[Non-patent Documents]

[Non-patent Document 1] Monoclonal antibody successes in the clinic, Janice M Reichert, Clark J Rosensweig, Laura B Faden & Matthew C Dewitz, Nature Biotechnology 23, 1073 - 1078 (2005)
[Non-patent Document 2] Pavlou AK, Belsey MJ., The therapeutic antibodies market to 2008., Eur J Pharm Biopharm. 2005 Apr; 59(3): 389-96.
[Non-patent Document 3] Rajpal A, Beyaz N, Haber L, Cappuccilli G, Yee H, Bhatt RR, Takeuchi T, Lerner RA, Crea R., A general method for greatly improving the affinity of antibodies by using combinatorial libraries., Proc. Natl. Acad. Sci. U. S. A. (2005) 102(24), 8466-8471
[Non-patent Document 4] Rathanaswami P, Roalstad S, Roskos L, Su QJ, Lackie S, Babcook J., Demonstration of an in vivo generated sub-picomolar affinity fully human monoclonal antibody to interleukin-8., Biochem. Biophys. Res. Commun. (2005) 334(4), 1004-1013
[Non-patent Document 5] Igawa T, et al., Antibody recycling by engineered pH-dependent antigen binding improves the duration of antigen neutralization. Nat Biotechnol. 2010, 28, 1203-7.

[Summary]

[Problems to be Solved]

For treatment of diseases where protein aggregates is a cause of the disease, antibodies having a property of selectively eliminating aggregates from plasma are desired. However, typical antibodies of which FcRn-binding is not enhanced under neutral conditions may possibly increase antigen concentration in plasma as described in the Background Art. In such cases, elimination of proteins causing the disease is delayed, and such proteins will tend to accumulate, causing negative effects such as enhanced cytotoxicity.

On the other hand, while pH-dependent antigen-binding antibodies with enhanced FcRn binding can eliminate antigens from plasma, in diseases where aggregates is the cause, monomers having normal functions coexist in plasma with aggregates that cause the disease, and therefore even if a pH-dependent antigen-binding antibody with enhanced FcRn binding is used, not only the aggregates but also normal monomers may be eliminated as well. Furthermore, when the proportion of the monomers present in plasma is overwhelmingly large relative to the aggregates, there is also a possibility that elimination of the monomers might be carried out preferentially and that elimination of the aggregates may become difficult.

The present invention was made in view of such circumstances. An objective of the present invention is to provide methods of producing antigen-binding molecules that can eliminate disease-causing protein aggregates in preference to protein monomers from plasma. Also disclosed herein are pharmaceutical compositions comprising the antigen-binding molecules.

[Means for Solving the Problems]

The present inventors conducted dedicated studies to solve the above-mentioned objectives. As a result, the present inventors successfully produced antigen-binding molecules that can eliminate protein aggregates in preference to monomers from plasma, by introducing an Fc region and an antigen-binding domain whose antigen-binding activity varies depending on ion concentration into an antigen-binding molecule that binds to an aggregate-forming antigen.

More specifically, the present invention relates to the following:

[1] a method for producing an antibody which is capable of selectively eliminating disease-causing protein aggregates in preference to protein monomers from plasma, said method comprising the following steps:

(a) selecting an antibody:

(A) wherein the ratio of the KD (dissociation constant) of the antibody to said protein aggregates under an intracellular pH 5.8 and a calcium ion concentration of 3 μM, and an extracellular pH 7.4 and a calcium ion concentration of 1.2 mM is 2 or more;

(B) wherein the binding activity to the FcRn receptor of a complex formed between said protein aggregates and the antibody is higher than the binding activity to the FcRn receptor of a complex formed between the unaggregated protein and the antibody; and

(C) wherein the ratio of plasma clearance of said aggregated protein in the absence of said antibody to plasma clearance of said aggregated protein in the presence of said antibody is 1.5 times or more than the same plasma clearance ratio for said unaggregated protein;

(b) culturing a host cell comprising a vector that carries a gene encoding the antibody selected in step (a); and
(c) isolating the antibody from the culture obtained in step (b);

[2] the method of [1], wherein the protein is huntingtin, ataxin-1, ataxin-2, Ca channel α1A, ataxin-7, TATA binding protein, MDJ, DRPLA, androgen receptor, α1-antitrypsin, α1-antichymotrypsin, neuroserpin, C1 inhibitor, antithrombin III, Aβ, L-ch, transthyretin, SAA, β2M, H-ch, cystatin C, α synuclein, amylin, hemoglobin, crystalline, IgA, Tau protein, TAR DNA-binding protein 43 kDa (TDP-43), Superoxide dismutase (SOD1), FUS (Fused in Sarcoma gene), Prion, PHOX2B, ARX, poly-adenylate binding protein nuclear 1 (PABPN1), dysferlin, desmin, GFAP, or keratin 5/14;

[3] the method of [1] or [2], wherein the antibody comprises an Fc region which is an Fc region represented by any one of SEQ ID NO: 9, 10, 11, or 12;

[4] the method of [1] or [2], wherein under an acidic pH condition, FcRn-binding activity of the Fc region comprised in the antibody is enhanced compared to that of the Fc region represented by any one of SEQ ID NO: 9, 10, 11, or 12;

[5] the method of [4], wherein the Fc region is an Fc region in which at least one or more amino acids selected from the group consisting of amino acids at positions 238, 244, 245, 249, 250, 251, 252, 253, 254, 255, 256, 257, 258, 260, 262, 265, 270, 272, 279, 283, 285, 286, 288, 293, 303, 305, 307, 308, 309, 311, 312, 314, 316, 317, 318, 332, 339, 340, 341, 343, 356, 360, 362, 375, 376, 377, 378, 380, 382, 385, 386, 387, 388, 389, 400, 413, 415, 423, 424, 427, 428, 430, 431, 433, 434, 435, 436, 438, 439, 440, 442, and 447, according to EU numbering, are substituted in the amino acid sequence of the Fc region represented by any one of SEQ ID NO: 9, 10, 11, or 12;

[6] the method of [5], wherein the Fc region comprises at least one or more amino acids selected from the group consisting of:

Leu for the amino acid at position 238;
Leu for the amino acid at position 244;
Arg for the amino acid at position 245;
Pro for the amino acid at position 249;
Gln or Glu for the amino acid at position 250, or
Arg, Asp, Glu, or Leu for the amino acid at position 251;

Phe, Ser, Thr, or Tyr for the amino acid at position 252;
Ser or Thr for the amino acid at position 254;
Arg, Gly, Ile, or Leu for the amino acid at position 255;
Ala, Arg, Asn, Asp, Gln, Glu, Pro, or Thr for the amino acid at position 256;
Ala, Ile, Met, Asn, Ser, or Val for the amino acid at position 257;
Asp for the amino acid at position 258;
Ser for the amino acid at position 260;
Leu for the amino acid at position 262;
Lys for the amino acid at position 270;
Leu or Arg for the amino acid at position 272;
Ala, Asp, Gly, His, Met, Asn, Gln, Arg, Ser, Thr, Trp, or Tyr for the amino acid at position 279;
Ala, Asp, Phe, Gly, His, Ile, Lys, Leu, Asn, Pro, Gln, Arg, Ser, Thr, Trp, or Tyr for the amino acid at position 283;
Asn for the amino acid at position 285;
Phe for the amino acid at position 286;
Asn or Pro for the amino acid at position 288;
Val for the amino acid at position 293;
Ala, Glu, Gln, or Met for the amino acid at position 307;
Ala, Glu, Ile, Lys, Leu, Met, Ser, Val, or Trp for the amino acid at position 311;
Pro for the amino acid at position 309;
Ala, Asp, or Pro for the amino acid at position 312;
Ala or Leu for the amino acid at position 314;
Lys for the amino acid at position 316;
Pro for the amino acid at position 317;
Asn or Thr for the amino acid at position 318;
Phe, His, Lys, Leu, Met, Arg, Ser, or Trp for the amino acid at position 332;
Asn, Thr, or Trp for the amino acid at position 339;
Pro for the amino acid at position 341;
Glu, His, Lys, Gln, Arg, Thr, or Tyr for the amino acid at position 343;
Arg for the amino acid at position 375;
Gly, Ile, Met, Pro, Thr, or Val for the amino acid at position 376;
Lys for the amino acid at position 377;
Asp, Asn, or Val for the amino acid at position 378;
Ala, Asn, Ser, or Thr for the amino acid at position 380;
Phe, His, Ile, Lys, Leu, Met, Asn, Gln, Arg, Ser, Thr, Val, Trp, or Tyr for the amino acid at position 382;
Ala, Arg, Asp, Gly, His, Lys, Ser, or Thr for the amino acid at position 385;
Arg, Asp, Ile, Lys, Met, Pro, Ser, or Thr for the amino acid at position 386;
Ala, Arg, His, Pro, Ser, or Thr for the amino acid at position 387;
Asn, Pro, or Ser for the amino acid at position 389;
Asn for the amino acid at position 423;
Asn for the amino acid at position 427;
Leu, Met, Phe, Ser, or Thr for the amino acid at position 428;
Ala, Phe, Gly, His, Ile, Lys, Leu, Met, Asn, Gln, Arg, Ser, Thr, Val, or Tyr for the amino acid at position 430;
His or Asn for the amino acid at position 431;
Arg, Gln, His, Ile, Lys, Pro, or Ser for the amino acid at position 433;
Ala, Gly, His, Phe, Ser, Trp, or Tyr for the amino acid at position 434;
Arg, Asn, His, Ile, Leu, Lys, Met, or Thr for the amino acid at position 436;
Lys, Leu, Thr, or Trp for the amino acid at position 438;
Lys for the amino acid at position 440, or
Lys for the amino acid at position 442; and
Ile, Pro, or Thr for the amino acid at position 308;
as indicated by EU numbering, in the amino acid sequence of the Fc region represented by any one of SEQ ID NO: 9, 10, 11, or 12;

[7] the method of [1] or [2], wherein under a neutral pH range condition, an FcRn-binding activity of the Fc region comprised in the antibody is enhanced compared to that of the Fc region represented by any one of SEQ ID NO: 9, 10, 11, or 12;

[8] the method of [7], wherein the Fc region is an Fc region in which at least one or more amino acids selected from the group consisting of amino acids at positions 237, 248, 250, 252, 254, 255, 256, 257, 258, 265, 286, 289, 297, 298, 303, 305, 307, 308, 309, 311, 312, 314, 315, 317, 332, 334, 360, 376, 380, 382, 384, 385, 386, 387, 389, 424, 428, 433, 434, and 436, according to EU numbering, are substituted in the amino acid sequence of the Fc region represented by any one of SEQ ID NO: 9, 10, 11, or 12;

[9] the method of [8], wherein the Fc region comprises at least one or more amino acids selected from the group of:

Met for the amino acid at position 237;
Ile for the amino acid at position 248;
Ala, Phe, Ile, Met, Gln, Ser, Val, Trp, or Tyr for the amino acid at position 250;
Phe, Trp, or Tyr for the amino acid at position 252;
Thr for the amino acid at position 254;
Glu for the amino acid at position 255;
Asp, Asn, Glu, or Gln for the amino acid at position 256;
Ala, Gly, Ile, Leu, Met, Asn, Ser, Thr, or Val for the amino acid at position 257;
His for the amino acid at position 258;
Ala for the amino acid at position 265;
Ala or Glu for the amino acid at position 286;
His for the amino acid at position 289;
Ala for the amino acid at position 297;
Ala for the amino acid at position 303;
Ala for the amino acid at position 305;
Ala, Asp, Phe, Gly, His, Ile, Lys, Leu, Met, Asn, Pro, Gln, Arg, Ser, Val, Trp, or Tyr for the amino acid at position 307;
Ala, Phe, Ile, Leu, Met, Pro, Gln, or Thr for the amino acid at position 308;
Ala, Asp, Glu, Pro, or Arg for the amino acid at position 309;
Ala, His, or Ile for the amino acid at position 311;
Ala or His for the amino acid at position 312;
Lys or Arg for the amino acid at position 314;
Ala, Asp, or His for the amino acid at position 315;
Ala for the amino acid at position 317;
Val for the amino acid at position 332;
Leu for the amino acid at position 334;
His for the amino acid at position 360;
Ala for the amino acid at position 376;
Ala for the amino acid at position 380;
Ala for the amino acid at position 382;
Ala for the amino acid at position 384;
Asp or His for the amino acid at position 385;
Pro for the amino acid at position 386;
Glu for the amino acid at position 387;
Ala or Ser for the amino acid at position 389;
Ala for the amino acid at position 424;
Ala, Asp, Phe, Gly, His, Ile, Lys, Leu, Asn, Pro, Gln, Ser, Thr, Val, Trp, or Tyr for the amino acid at position 428;
Lys for the amino acid at position 433;
Ala, Phe, His, Ser, Trp, or Tyr for the amino acid at position 434; and
His, Ile, Leu, Phe, Thr, or Val for the amino acid at position 436;
as indicated by EU numbering in the amino acid sequence of the Fc region represented by any one of SEQ ID NOs: 9, 10, 11, and 12;

[10] the method of any one of [1] to [6] wherein the antibody comprises an Fc region which includes an Fc region that has a higher Fcγ receptor-binding activity than that of the Fc region of a native human IgG;

[11] the method of [10], wherein the Fc region comprises in its amino acid sequence at least one or more amino acids that are different from amino acids of the native human IgG Fc region selected from the group of positions 221, 222, 223, 224, 225, 227, 228, 230, 231, 232, 233, 234, 235, 236, 237, 238, 239, 240, 241, 243, 244, 245, 246, 247, 249, 250, 251, 254, 255, 256, 258, 260, 262, 263, 264, 265, 266, 267, 268, 269, 270, 271, 272, 273, 274, 275, 276, 278, 279, 280, 281, 282, 283, 284, 285, 286, 288, 290, 291, 292, 293, 294, 295, 296, 297, 298, 299, 300, 301, 302, 303, 304, 305, 311, 313, 315, 317, 318, 320, 322, 323, 324, 325, 326, 327, 328, 329, 330, 331, 332, 333, 334, 335, 336, 337, 339, 376, 377, 378, 379, 380, 382, 385, 392, 396, 421, 427, 428, 429, 434, 436, and 440 (EU numbering);

[12] the method of [11], wherein the Fc region comprises in its amino acid sequence at least one or more amino acids selected from the group of:

Lys or Tyr for the amino acid at position 221;
Phe, Trp, Glu, or Tyr for the amino acid at position 222;
Phe, Trp, Glu, or Lys for the amino acid at position 223;
Phe, Trp, Glu, or Tyr for the amino acid at position 224;
Glu, Lys, or Trp for the amino acid at position 225;
Glu, Gly, Lys, or Tyr for the amino acid at position 227;
Glu, Gly, Lys, or Tyr for the amino acid at position 228;
Ala, Glu, Gly, or Tyr for the amino acid at position 230;
Glu, Gly, Lys, Pro, or Tyr for the amino acid at position 231;
Glu, Gly, Lys, or Tyr for the amino acid at position 232;
Ala, Asp, Phe, Gly, His, Ile, Lys, Leu, Met, Asn, Gln, Arg, Ser, Thr, Val, Trp, or Tyr for the amino acid at position 233;
Ala, Asp, Glu, Phe, Gly, His, Ile, Lys, Met, Asn, Pro, Gln, Arg, Ser, Thr, Val, Trp, or Tyr for the amino acid at position 234;
Ala, Asp, Glu, Phe, Gly, His, Ile, Lys, Met, Asn, Pro, Gln, Arg, Ser, Thr, Val, Trp, or Tyr for the amino acid at position 235;
Ala, Asp, Glu, Phe, His, Ile, Lys, Leu, Met, Asn, Pro, Gln, Arg, Ser, Thr, Val, Trp, or Tyr for the amino acid at position 236;
Asp, Glu, Phe, His, Ile, Lys, Leu, Met, Asn, Pro, Gln, Arg, Ser, Thr, Val, Trp, or Tyr for the amino acid at position 237;
Asp, Glu, Phe, Gly, His, Ile, Lys, Leu, Met, Asn, Gln, Arg, Ser, Thr, Val, Trp, or Tyr for the amino acid at position 238;
Asp, Glu, Phe, Gly, His, Ile, Lys, Leu, Met, Asn, Pro, Gln, Arg, Thr, Val, Trp, or Tyr for the amino acid at position 239;
Ala, Ile, Met, or Thr for the amino acid at position 240;
Asp, Glu, Leu, Arg, Trp, or Tyr for the amino acid at position 241;
Leu, Glu, Leu, Gln, Arg, Trp, or Tyr for the amino acid at position 243;
His for the amino acid at position 244;
Ala for the amino acid at position 245;
Asp, Glu, His, or Tyr for the amino acid at position 246;
Ala, Phe, Gly, His, Ile, Leu, Met, Thr, Val, or Tyr for the amino acid at position 247;
Glu, His, Gln, or Tyr for the amino acid at position 249;
Glu or Gln for the amino acid at position 250;
Phe for the amino acid at position 251;
Phe, Met, or Tyr for the amino acid at position 254;
Glu, Leu, or Tyr for the amino acid at position 255;
Ala, Met, or Pro for the amino acid at position 256;
Asp, Glu, His, Ser, or Tyr for the amino acid at position 258;
Asp, Glu, His, or Tyr for the amino acid at position 260;
Ala, Glu, Phe, Ile, or Thr for the amino acid at position 262;
Ala, Ile, Met, or Thr for the amino acid at position 263;
Asp, Glu, Phe, Gly, His, Ile, Lys, Leu, Met, Asn, Pro, Gln, Arg, Ser, Thr, Trp, or Tyr for the amino acid at position 264;
Ala, Leu, Phe, Gly, His, Ile, Lys, Leu, Met, Asn, Pro, Gln, Arg, Ser, Thr, Val, Trp, or Tyr for the amino acid at position 265;
Ala, Ile, Met, or Thr for the amino acid at position 266;
Asp, Glu, Phe, His, Ile, Lys, Leu, Met, Asn, Pro, Gln, Arg, Thr, Val, Trp, or Tyr for the amino acid at position 267;
Asp, Glu, Phe, Gly, Ile, Lys, Leu, Met, Pro, Gln, Arg, Thr, Val, or Trp for the amino acid at position 268;
Phe, Gly, His, Ile, Lys, Leu, Met, Asn, Pro, Arg, Ser, Thr, Val, Trp, or Tyr for the amino acid at position 269;
Glu, Phe, Gly, His, Ile, Leu, Met, Pro, Gln, Arg, Ser, Thr, Trp, or Tyr for the amino acid at position 270;
Ala, Asp, Glu, Phe, Gly, His, Ile, Lys, Leu, Met, Asn, Gln, Arg, Ser, Thr, Val, Trp, or Tyr for the amino acid at position 271;
Asp, Phe, Gly, His, Ile, Lys, Leu, Met, Pro, Arg, Ser, Thr, Val, Trp, or Tyr for the amino acid at position 272;
Phe or Ile for the amino acid at position 273;
Asp, Glu, Phe, Gly, His, Ile, Leu, Met, Asn, Pro, Arg, Ser, Thr, Val, Trp, or Tyr for the amino acid at position 274;

Leu or Trp for the amino acid at position 275;

Asp, Glu, Phe, Gly, His, Ile, Leu, Met, Pro, Arg, Ser, Thr, Val, Trp, or Tyr for the amino acid at position 276;

Asp, Glu, Gly, His, Ile, Lys, Leu, Met, Asn, Pro, Gln, Arg, Ser, Thr, Val, or Trp for the amino acid at position 278;

Ala for the amino acid at position 279;

Ala, Gly, His, Lys, Leu, Pro, Gln, Trp, or Tyr for the amino acid at position 280;

Asp, Lys, Pro, or Tyr for the amino acid at position 281;

Glu, Gly, Lys, Pro, or Tyr for the amino acid at position 282;

Ala, Gly, His, Ile, Lys, Leu, Met, Pro, Arg, or Tyr for the amino acid at position 283;

Asp, Glu, Leu, Asn, Thr, or Tyr for the amino acid at position 284;

Asp, Glu, Lys, Gln, Trp, or Tyr for the amino acid at position 285;

Glu, Gly, Pro, or Tyr for the amino acid at position 286;

Asn, Asp, Glu, or Tyr for the amino acid at position 288;

Asp, Gly, His, Leu, Asn, Ser, Thr, Trp, or Tyr for the amino acid at position 290;

Asp, Glu, Gly, His, Ile, Gln, or Thr for the amino acid at position 291;

Ala, Asp, Glu, Pro, Thr, or Tyr for the amino acid at position 292;

Phe, Gly, His, Ile, Leu, Met, Asn, Pro, Arg, Ser, Thr, Val, Trp, or Tyr for the amino acid at position 293;

Phe, Gly, His, Ile, Lys, Leu, Met, Asn, Pro, Arg, Ser, Thr, Val, Trp, or Tyr for the amino acid at position 294;

Asp, Glu, Phe, Gly, His, Ile, Lys, Met, Asn, Pro, Arg, Ser, Thr, Val, Trp, or Tyr for the amino acid at position 295;

Ala, Asp, Glu, Gly, His, Ile, Lys, Leu, Met, Asn, Gln, Arg, Ser, Thr, or Val for the amino acid at position 296;

Asp, Glu, Phe, Gly, His, Ile, Lys, Leu, Met, Pro, Gln, Arg, Ser, Thr, Val, Trp, or Tyr for the amino acid at position 297;

Ala, Asp, Glu, Phe, His, Ile, Lys, Met, Asn, Gln, Arg, Thr, Val, Trp, or Tyr for the amino acid at position 298;

Ala, Asp, Glu, Phe, Gly, His, Ile, Lys, Leu, Met, Asn, Pro, Gln, Arg, Ser, Val, Trp, or Tyr for the amino acid at position 299;

Ala, Asp, Glu, Gly, His, Ile, Lys, Leu, Met, Asn, Pro, Gln, Arg, Ser, Thr, Val, or Trp for the amino acid at position 300;

Asp, Glu, His, or Tyr for the amino acid at position 301;

Ile for the amino acid at position 302;

Asp, Gly, or Tyr for the amino acid at position 303;

Asp, His, Leu, Asn, or Thr for the amino acid at position 304;

Glu, Ile, Thr, or Tyr for the amino acid at position 305;

Ala, Asp, Asn, Thr, Val, or Tyr for the amino acid at position 311;

Phe for the amino acid at position 313;

Leu for the amino acid at position 315;

Glu or Gln for the amino acid at position 317;

His, Leu, Asn, Pro, Gln, Arg, Thr, Val, or Tyr for the amino acid at position 318;

Asp, Phe, Gly, His, Ile, Leu, Asn, Pro, Ser, Thr, Val, Trp, or Tyr for the amino acid at position 320;

Ala, Asp, Phe, Gly, His, Ile, Pro, Ser, Thr, Val, Trp, or Tyr for the amino acid at position 322;

Ile for the amino acid at position 323;

Asp, Phe, Gly, His, Ile, Leu, Met, Pro, Arg, Thr, Val, Trp, or Tyr for the amino acid at position 324;

Ala, Asp, Glu, Phe, Gly, His, Ile, Lys, Leu, Met, Pro, Gln, Arg, Ser, Thr, Val, Trp, or Tyr for the amino acid at position 325;

Ala, Asp, Glu, Gly, Ile, Leu, Met, Asn, Pro, Gln, Ser, Thr, Val, Trp, or Tyr for the amino acid at position 326;

Ala, Asp, Glu, Phe, Gly, His, Ile, Lys, Leu, Met, Asn, Pro, Arg, Thr, Val, Trp, or Tyr for the amino acid at position 327;

Ala, Asp, Glu, Phe, Gly, His, Ile, Lys, Met, Asn, Pro, Gln, Arg, Ser, Thr, Val, Trp, or Tyr for the amino acid at position 328;

Asp, Glu, Phe, Gly, His, Ile, Lys, Leu, Met, Asn, Gln, Arg, Ser, Thr, Val, Trp, or Tyr for the amino acid at position 329;

Cys, Glu, Phe, Gly, His, Ile, Lys, Leu, Met, Asn, Pro, Arg, Ser, Thr, Val, Trp, or Tyr for the amino acid at position 330;

Asp, Phe, His, Ile, Leu, Met, Gln, Arg, Thr, Val, Trp, or Tyr for the amino acid at position 331; Ala, Asp, Glu, Phe, Gly, His, Lys, Leu, Met, Asn, Pro, Gln, Arg, Ser, Thr, Val, Trp, or Tyr for the amino acid at position 332;

Ala, Asp, Glu, Phe, Gly, His, Ile, Leu, Met, Pro, Ser, Thr, Val, or Tyr for the amino acid at position 333;

Ala, Glu, Phe, Ile, Leu, Pro, or Thr for the amino acid at position 334;

Asp, Phe, Gly, His, Ile, Leu, Met, Asn, Pro, Arg, Ser, Val, Trp, or Tyr for the amino acid at position 335;

Glu, Lys, or Tyr for the amino acid at position 336;

Glu, His, or Asn for the amino acid at position 337;

Asp, Phe, Gly, Ile, Lys, Met, Asn, Gln, Arg, Ser, or Thr for the amino acid at position 339;

Ala or Val for the amino acid at position 376;

Gly or Lys for the amino acid at position 377;

Asp for the amino acid at position 378;

Asn for the amino acid at position 379;

Ala, Asn, or Ser for the amino acid at position 380;
Ala or Ile for the amino acid at position 382;
Glu for the amino acid at position 385;
Thr for the amino acid at position 392;
Leu for the amino acid at position 396;
Lys for the amino acid at position 421;
Asn for the amino acid at position 427;
Phe or Leu for the amino acid at position 428;
Met for the amino acid at position 429;
Trp for the amino acid at position 434;
Ile for the amino acid at position 436; and
Gly, His, Ile, Leu, or Tyr for the amino acid at position 440;
as indicated by EU numbering;

[13] the method of any one of [1] to [9], wherein the antibody comprises an Fc region which has a higher binding activity toward an inhibitory Fcγ receptor than toward an activating Fcγ receptor;

[14] the method of [13], wherein the inhibitory Fcγ receptor is human FcγRIIb;

[15] the method of [13] or [14], wherein the activating Fcγ receptor is human FcγRIa, human FcγRIIa (R), human FcγRIIa (H), human FcγRIIIa (V), or human FcyRIIIa (F);

[16] the method of any one of [13] to [15], wherein the amino acid at position 238 or 328 (EU numbering) in the Fc region is different from the amino acid in the native human IgG Fc region;

[17] the method of [16], wherein the amino acid at position 238 of the Fc region is Asp or the amino acid at position 328 of the Fc region is Glu as indicated by EU numbering; and

[18] the method of [16] or [17], wherein the amino acid sequence of the Fc region comprises at least one or more amino acids selected from the group consisting of:

Asp for the amino acid at position 233;
Trp or Tyr for the amino acid at position 234;
Ala, Asp, Glu, Leu, Met, Phe, Trp, or Tyr for the amino acid at position 237;
Asp for the amino acid at position 239;
Ala, Gln, or Val for the amino acid at position 267;
Asn, Asp, or Glu for the amino acid at position 268;
Gly for the amino acid at position 271;
Ala, Asn, Asp, Gln, Glu, Leu, Met, Ser, or Thr for the amino acid at position 326;
Arg, Lys, or Met for the amino acid at position 330;
Ile, Leu, or Met for the amino acid at position 323; and
Asp for the amino acid at position 296;
as indicated by EU numbering.

[Brief Description of the Drawings]

Fig. 1 is a diagram showing that an antibody with pH-dependent binding repeatedly binds to soluble antigens. (i) An antibody binds to soluble antigens; (ii) the antibody is non-specifically internalized into a cell by pinocytosis; (iii) the antibody binds to FcRn within the endosome, and the soluble antigens dissociate from the antibody; (iv) the soluble antigens are transferred to the lysosome and degraded; (v) after dissociation from the soluble antigens, the antibody is recycled to the plasma *via* FcRn; (vi) the recycled antibody can bind to soluble antigens again.

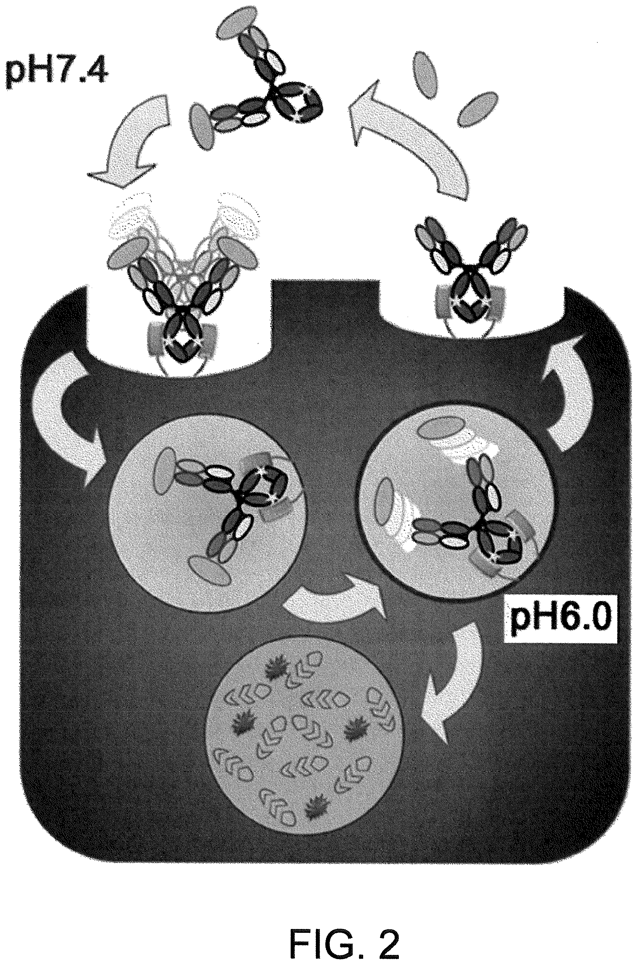

Fig. 2 is a diagram showing that enhancing FcRn binding under neutral conditions results in improving the effect of an antibody with pH-dependent binding to repeatedly bind to antigens: (i) an antibody binds to soluble antigens; (ii) the antibody is internalized into a cell by pinocytosis *via* FcRn; (iii) the soluble antigens dissociate from the antibody in the endosome; (iv) the soluble antigens are transferred into the lysosome and degraded; (v) after dissociation of the soluble antigens, the antibody is recycled to the plasma *via* FcRn; and (vi) the recycled antibody can bind to soluble antigens again.

Fig. 3 shows a result of SEC analysis performed on aggregated human IgA.

Fig. 4 presents Biacore sensorgrams showing the interaction of anti-human IgA antibodies with monomeric human IgA and with aggregated human IgA.

Fig. 5 shows a time course of the plasma antibody concentration in normal mice of the GA2-IgG1 antibody-administered group.

Fig. 6 shows a time course of the plasma monomeric human IgA concentration in normal mice of the group administered only with monomeric human IgA, and in normal mice of the monomeric human IgA+ GA2-IgG1 antibody-administered group.

Fig. 7 shows a time course of the aggregated human IgA concentration in plasma of normal mice in the group administered only with aggregated human IgA, and in the aggregated human IgA+ GA2-IgG1 antibody-administered group.

Fig. 8 shows a time course of "human IgA concentration when human IgA+ GA2-IgG1 is administration / human IgA concentration when only human IgA is administered"

Fig. 9 is a diagram showing an example that an aggregated antigen is efficiently incorporated into the cell by binding to receptors multivalently and strongly through formation of a large immune complex by polyvalent binding of multiple antibodies to the aggregated antigen.

Fig. 10 is a diagram showing an example that incorporation of unaggregated antigens into the cell is not efficient because the unaggregated antigens do not form a large immune complex and their binding to the receptor is weak.

[Detailed Description]

The definitions and detailed description below are provided to help the understanding of the present disclosure.

Aggregated antigen

As used herein, aggregated antigen refers to a molecule in a state at which two or more of a molecule (monomer) present in a normal biological fluid have become aggregated or multimerized. An aggregated antigen may be a molecule in which monomers having the same three-dimensional structure (protein secondary structure or tertiary structure) are aggregated or multimerized as compared to antigens normally present in in biological fluid, or it may be a molecule in which partially or totally degenerated molecules as compared to the monomer are aggregated or multimerized. Furthermore, an aggregated antigen may be a molecule in which a mixture of two is aggregated or multimerized. In the aggregated antigens, another type of antigen that does not bind to the antigen-binding molecule may also be present. A target antigen of an antigen-binding molecule of the present disclosure is not particularly limited as long as the antigen aggregates, but an antigen that aggregates in a pathological condition is preferred, and an antigen whose aggregated form is a disease-causing substance is more preferred. Examples of such antigens include the antigens shown later in Table 7. Herein, the antigen that has aggregated may be referred to as an aggregated antigen or a multimeric antigen.

Biological fluid as used herein refers to all fluids that fill the space between the vasculature or tissues/cells in an organism where an aggregated antigen is present in its pathological condition. Specific examples include plasma, interstitial fluid, cerebrospinal fluid, spinal fluid, puncture liquid, synovial fluid, alveolar fluid (bronchoalveolar lavage fluid), lymph, ascites, pleural fluid, pericardial fluid, cyst fluid, aqueous humor (hydatoid), and such.

Methods of preparing such aggregated antigens include the methods below: (1) purifying an aggregated antigen by chromatography or such from plasma containing the aggregated antigen; (2) chemically crosslinking a monomeric antigen by chemical crosslinking agents such as SPDP (*N*-Succinimidyl 3-(2-pyridyldithio)propionate), and purifying the aggregated antigen by chromatography or such; and (3) purifying by chromatography or such an aggregated antigen formed through overall or partial chemical degeneration of a monomeric antigen by thermal treatment or acid treatment.

Amino acids

Herein, amino acids are described in one- or three-letter codes or both, for example, Ala/A, Leu/L, Arg/R, Lys/K, Asn/N, Met/M, Asp/D, Phe/F, Cys/C, Pro/P, Gln/Q, Ser/S, Glu/E, Thr/T, Gly/G, Trp/W, His/H, Tyr/Y, Ile/I, or Val/V.

Alteration of amino acids

For amino acid alteration in the amino acid sequence of an antigen-binding molecule, known methods such as site-directed mutagenesis methods (Kunkel et al. (Proc. Natl. Acad. Sci. USA (1985) 82: 488-492)) and overlap extension PCR may be appropriately adopted. Furthermore, several known methods may also be adopted as amino acid alteration methods for substitution to non-natural amino acids (Annu. Rev. Biophys. Biomol. Struct. (2006) 35: 225-249; and Proc.

Natl. Acad. Sci. U.S.A. (2003) 100(11): 6353-6357). For example, it is suitable to use a cell-free translation system (Clover Direct (Protein Express)) containing a tRNA that has a non-natural amino acid bound to a complementary amber suppressor tRNA of the UAG codon (amber codon) which is one of the stop codons.

Herein, the meaning of the term "and/or" when describing the site of amino acid alteration includes every combination where "and" and "or" are suitably combined. Specifically, for example, "the amino acids at positions 33, 55, and/or 96 are substituted" includes the following variation of amino acid alterations:

amino acid(s) at (a) position 33; (b) position 55; (c) position 96; (d) positions 33 and 55; (e) positions 33 and 96; (f) positions 55 and 96; and (g) positions 33, 55, and 96.

Epitopes

"Epitope" means an antigenic determinant in an antigen, and refers to an antigen site to which the antigen-binding domain of an antigen-binding molecule disclosed herein binds. Thus, for example, the epitope can be defined according to its structure. Alternatively, the epitope may be defined according to the antigen-binding activity of an antigen-binding molecule that recognizes the epitope. When the antigen is a peptide or polypeptide, the epitope can be specified by the amino acid residues forming the epitope. Alternatively, when the epitope is a sugar chain, the epitope can be specified by its specific sugar chain structure.

A linear epitope is an epitope that contains an epitope whose primary amino acid sequence has been recognized. Such a linear epitope typically contains at least three and most commonly at least five, for example, about 8 to about 10 or 6 to 20 amino acids in a specific sequence.

In contrast to the linear epitope, a "conformational epitope" is an epitope in which the primary amino acid sequence containing the epitope is not the only determinant of the recognized epitope (for example, the primary amino acid sequence of a conformational epitope is not necessarily recognized by an epitope-defining antibody). Conformational epitopes may contain a greater number of amino acids compared to linear epitopes. A conformational epitope-recognizing antibody recognizes the three-dimensional structure of a peptide or protein. For example, when a protein molecule folds and forms a three-dimensional structure, amino acids and/or polypeptide main chains that form a conformational epitope become aligned, and the epitope is made recognizable by the antibody. Methods for determining epitope conformations include, for example, X ray crystallography, two-dimensional nuclear magnetic resonance, site-specific spin labeling, and electron paramagnetic resonance, but are not limited thereto. See, for example, Epitope Mapping Protocols in Methods in Molecular Biology (1996), Vol. 66, Morris (ed.).

The structure of the antigen-binding domain which binds to an epitope is called a paratope. An epitope and a paratope bind with stability through the action of hydrogen bonds, electrostatic force, van der Waals force, hydrophobic bonds, and such between the epitope and the paratope. This strength of binding between the epitope and paratope is called affinity. The total sum of binding strength when a plurality of epitopes and a plurality of paratopes bind is referred to as avidity. When an antibody comprising a plurality of paratopes (*i.e.,* multivalent antibody) or such binds to a plurality of epitopes, the affinity acts additively or synergistically, and therefore avidity becomes higher than affinity.

Binding activity

Examples of a method for assessing epitope binding by a test antigen-binding molecule containing an antigen-binding domain directed to an antigen are described below; but a method is not limited thereto.

For example, whether a test antigen-binding molecule containing an antigen-binding domain against an antigen recognizes a linear epitope in the antigen molecule can be confirmed for example as mentioned below. For example, a linear peptide comprising an amino acid sequence forming the antigen is synthesized for the above purpose. The peptide can be synthesized chemically, or obtained by genetic engineering techniques using a cDNA encoding the antigen. Then, a test antigen-binding molecule containing an antigen-binding domain toward the antigen is assessed for its binding activity towards the linear peptide. For example, an ELISA using an immobilized linear peptide as an antigen can be performed to evaluate the binding activity of the antigen-binding molecule towards the peptide. Alternatively, the binding activity towards a linear peptide can be assessed based on the level of inhibition by the linear peptide of the binding of the antigen-binding molecule toward cells expressing on its surface the antigen. These tests can demonstrate the binding activity of the antigen-binding molecule towards the linear peptide.

Recognition of a conformational epitope by a test antigen-binding molecule comprising an antigen-binding domain targeting an antigen may be confirmed as stated below. For the above-mentioned objective, as described herein, a general genetic recombination technique is used to transfer an antigen-encoding recombinant gene into host cells (for example, animal cells, insect cells, or yeast cells) that enable formation of the native conformational epitope in the antigen. Antigen containing the conformational epitope is prepared from the culture of recombinant cells produced in this manner. Recognition of a conformational epitope by a test antigen-binding molecule comprising an antigen-binding domain targeting antigen is, for example, when the test antigen-binding molecule binds strongly to the antigen when it is contacted with immobilized antigen containing the conformational epitope, while the antigen-binding molecule does not substantively bind to a linear peptide comprising an amino acid sequence constituting the amino acid sequence of the immobilized antigen. Alternatively, it is also possible to use, instead of the above-mentioned linear peptide, the test IgA-targeting antigen-binding molecule that has been denatured by a reducing agent that cleaves disulfide bonds, such as dithiothreitol, dithioerythritol, $\beta$-mercaptoethanol, phosphines, and sodium borohydride, and/or chaotropic agents such as surfactants including guanidine hydrochloride, urea, and sodium lauryl sulfate. Here, the phrase "does not substantively bind" refers to a binding activity not greater than 80%, normally not greater than 50%, preferably not greater than 30%, or particularly preferably not greater than 15% of the human-IgA-binding activity.

Methods for assaying the binding activity toward an antigen of a test antigen-binding molecule containing an antigen-binding domain against the antigen include, for example, the methods described in Antibodies: A Laboratory Manual (Ed Harlow, David Lane, Cold Spring Harbor Laboratory (1988) 359-420). Specifically, the assessment can be performed based on the principle of ELISA or EIA using IgA as antigen.

In the ELISA format, the binding activity of a test antigen-binding molecule containing an antigen-binding domain towards the antigen can be assessed quantitatively by comparing the levels of signal generated by enzymatic reaction. Specifically, a test antigen-binding molecule is added to an ELISA plate onto which an antigen has been immobilized. Then, the test antigen-binding molecule that bound to an antigen immobilized on the plate is detected using an enzyme-labeled antibody that recognizes the test antigen-binding molecule. In the ELISA, a serial dilution of the test antigen-binding molecule can be prepared and the antibody binding titer toward an antigen is determined to compare the binding activity of the test antigen-binding molecule towards the antigen.

The binding of a test antigen-binding molecule towards an antigen expressed on the surface of cells suspended in buffer or the like can be detected using a flow cytometer. Known flow cytometers include, for example, the following devices:

FACSCanto™ II
FACSAria™
FACSArray™
FACSVantage™ SE
FACSCalibur™ (all are trade names of BD Biosciences)
EPICS ALTRA HyPerSort
Cytomics FC 500
EPICS XL-MCL ADC EPICS XL ADC
Cell Lab Quanta/Cell Lab Quanta SC (all are trade names of Beckman Coulter).

Alternatively, preferable methods for assaying the binding activity towards an antigen of a test antigen-binding molecule containing an antigen-binding domain against an antigen include, for example, the following method. First, antigen-expressing cells are reacted with a test antigen-binding molecule, and then this is stained with an FITC-labeled secondary antibody that recognizes the antigen-binding molecule. The test antigen-binding molecule is appropriately diluted with a suitable buffer to prepare the molecule at a desired concentration. For example, the molecule can be used at a concentration within the range of 10 $\mu$g/ml to 10 ng/ml. Then, the fluorescence intensity and cell count are determined using FACSCalibur (BD). The fluorescence intensity obtained by analysis using the CELL QUEST Software (BD), *i.e.,* the Geometric Mean value, reflects the quantity of antibody bound to cells. That is, the binding activity of a test antigen-binding molecule, which is represented by the quantity of the test antigen-binding molecule bound, can be determined by measuring the Geometric Mean value.

Whether a test antigen-binding molecule containing a binding domain towards an antigen shares a common epitope with another antigen-binding molecule can be assessed based on the competition between the two molecules for the same epitope. The competition between antigen-binding molecules can be detected by cross-blocking assay or the like. For example, the competitive ELISA assay is a preferred cross-blocking assay.

Specifically, in cross-blocking assay, the antigen immobilized to the wells of a microtiter plate is pre-incubated in the presence or absence of a candidate competitor antigen-binding molecule, and then a test antigen-binding molecule is added thereto. The quantity of test antigen-binding molecule bound to the antigen in the wells is indirectly correlated with the binding ability of a candidate competitor antigen-binding molecule that competes for the binding to the same epitope. That is, the greater the affinity of the competitor antigen-binding molecule for the same epitope, the lower the binding activity of the test antigen-binding molecule towards the antigen-coated wells.

The quantity of the test antigen-binding molecule bound to the wells *via* the antigen can be readily determined by labeling the antigen-binding molecule in advance. For example, a biotin-labeled antigen-binding molecule is measured using an avidin/peroxidase conjugate and appropriate substrate. In particular, cross-blocking assay that uses enzyme labels such as peroxidase is called "competitive ELISA assay". The antigen-binding molecule can also be labeled with other labeling substances that enable detection or measurement. Specifically, radiolabels, fluorescent labels, and such are known.

When the candidate competitor antigen-binding molecule can block the antigen binding by a test antigen-binding molecule by at least 20%, preferably at least 20 to 50%, and more preferably at least 50% compared to the binding activity in a control experiment conducted in the absence of the candidate competitor antigen-binding molecule, the test antigen-binding molecule is determined to substantially bind to the same epitope bound by the competitor antigen-binding molecule, or compete for the binding to the same epitope.

When the structure of an epitope bound by a test antigen-binding molecule containing a binding domain towards an antigen has already been identified, whether the test and control antigen-binding molecules share a common epitope can be assessed by comparing the binding activities of the two antigen-binding molecules towards a peptide prepared by introducing amino acid mutations into the peptide forming the epitope.

To measure the above binding activities, for example, the binding activities of test and control antigen-binding molecules towards a linear peptide into which a mutation is introduced are compared in the above ELISA format. Besides the ELISA methods, the binding activity towards the mutant peptide bound to a column can be determined by flowing test and control antigen-binding molecules in the column, and then quantifying the antigen-binding molecule eluted in the elution solution. Methods for adsorbing a mutant peptide to a column, for example, in the form of a GST fusion peptide, are known.

Alternatively, when the identified epitope in an antigen-binding molecule expressed on a cell is a conformational epitope, whether test and control antigen-binding molecules share a common epitope can be assessed, for example, by the following method. First, cells expressing an antigen of interest and cells expressing an antigen with a mutation introduced into the epitope are prepared. The test and control antigen-binding molecules are added to a cell suspension prepared by suspending these cells in an appropriate buffer such as PBS. Then, the cell suspensions are appropriately washed with a buffer, and an FITC-labeled antibody that recognizes the test and control antigen-binding molecules is added thereto. The fluorescence intensity and number of cells stained with the labeled antibody are determined using FACSCalibur (BD). The test and control antigen-binding molecules are appropriately diluted using a suitable buffer, and used at desired concentrations. For example, they may be used at a concentration within the range of 10 $\mu$g/ml to 10 ng/ml. The fluorescence intensity determined by analysis using the CELL QUEST Software (BD), *i.e.*, the Geometric Mean value, reflects the quantity of labeled antibody bound to cells. That is, the binding activities of the test and control antigen-binding molecules, which are represented by the quantity of labeled antibody bound, can be determined by measuring the Geometric Mean value.

Whether the binding activity to an aggregated antigen is higher than the binding activity to an unaggregated antigen can be confirmed by comparing the binding activity to the aggregated antigen and the binding activity to the unaggregated antigen according to the above-described method.

Antigen-binding domain

Herein, an "antigen-binding domain" may be of any structure as long as it binds to an antigen of interest. Such domains preferably include, for example:

antibody heavy-chain and light-chain variable regions;
a module of about 35 amino acids called A domain which is contained in the *in vivo* cell membrane protein Avimer (International Publication No. WO 2004/044011, International Publication No. WO 2005/040229);
Adnectin containing the 10Fn3 domain which binds to the protein moiety of fibronectin, a glycoprotein expressed on cell membrane (International Publication No. WO 2002/032925);
Affibody which is composed of a 58-amino acid three-helix bundle based on the scaffold of the IgG-binding domain of Protein A (International Publication No. WO 1995/001937);
Designed Ankyrin Repeat proteins (DARPins) which are a region exposed on the molecular surface of ankyrin repeats (AR) having a structure in which a subunit consisting of a turn comprising 33 amino acid residues, two antiparallel helices, and a loop is repeatedly stacked (International Publication No. WO 2002/020565);
Anticalins and such, which are domains consisting of four loops that support one side of a barrel structure composed of eight circularly arranged antiparallel strands that are highly conserved among lipocalin molecules such as neutrophil gelatinase-associated lipocalin (NGAL) (International Publication No. WO 2003/029462); and
the concave region formed by the parallel-sheet structure inside the horseshoe-shaped structure constituted by stacked repeats of the leucine-rich-repeat (LRR) module of the variable lymphocyte receptor (VLR) which does not have the immunoglobulin structure and is used in the system of acquired immunity in jawless vertebrate such as lampery and hagfish (International Publication No. WO 2008/016854). Preferred antigen-binding domains include, for example, those having antibody heavy-chain and light-chain variable regions. Preferred examples of antigen-binding domains include "single chain Fv (scFv)", "single chain antibody", "Fv", "single chain Fv 2 (scFv2)", "Fab", and "F(ab')2".

Immune complex

Immune complex refers to a structure produced when at least one unaggregated antigen or aggregated antigen and at least one antigen-binding molecule bind with each other to form a larger molecular-weight complex consisting of antigen(s) and antigen-binding molecule(s).

Antibody

Herein, "antibody" refers to a natural immunoglobulin or an immunoglobulin produced by partial or complete synthesis. Antibodies can be isolated from natural sources such as naturally-occurring plasma and serum, or culture supernatants of antibody-producing hybridomas. Alternatively, antibodies can be partially or completely synthesized using techniques such as genetic recombination. Preferred antibodies include, for example, antibodies of an immunoglobulin isotype or subclass belonging thereto. Known human immunoglobulins include antibodies of the following nine classes (isotypes): IgG1, IgG2, IgG3, IgG4, IgA1, IgA2, IgD, IgE, and IgM. Of these isotypes, antibodies of the present disclosure include IgG1, IgG2, IgG3, and IgG4. A number of allotype sequences of human IgG1, human IgG2, human IgG3, and human IgG4 constant regions due to gene polymorphisms are described in "Sequences of proteins of immunological interest", NIH Publication No. 91-3242. Any of such sequences may be used as disclosed herein. In particular, for the human IgG1 sequence, the amino acid sequence at positions 356 to 358 as indicated by EU numbering may be DEL or EEM. Several allotype sequences due to genetic polymorphisms have been described in "Sequences of proteins of immunological interest", NIH Publication No. 91-3242 for the human Igκ (Kappa) constant region and human Igλ (Lambda) constant region, and any of the sequences may be used as disclosed herein.

Methods for producing an antibody with desired binding activity are known to those skilled in the art.

Antibodies can be obtained as polyclonal or monoclonal antibodies using known methods. Mammal-derived monoclonal antibodies include antibodies produced by hybridomas or host cells transformed with an expression vector carrying an antibody gene by genetic engineering techniques. Meanwhile, "humanized antibodies" or "chimeric antibodies" are included in the monoclonal antibodies disclosed herein.

Monoclonal antibody-producing hybridomas can be produced using known techniques, for example, as described below. Specifically, mammals are immunized by conventional immunization methods using a sensitizing antigen. Resulting immune cells are fused with known parental cells by conventional cell fusion methods. Then, hybridomas producing an antibody of interest can be selected by screening for monoclonal antibody-producing cells using conventional screening methods. There is no particular limitation on the mammals to be immunized with the sensitizing antigen. However, it is preferable to select the mammals by considering their compatibility with the parent cells to be used for cell fusion. In general, rodents such as mice, rats, and hamsters, rabbits, and monkeys are preferably used.

The above animals are immunized with a sensitizing antigen by known methods. Generally performed immunization methods include, for example, intraperitoneal or subcutaneous injection of a sensitizing antigen into mammals. Specifically, a sensitizing antigen is appropriately diluted with PBS (Phosphate-Buffered Saline), physiological saline, or the like. If desired, a conventional adjuvant such as Freund's complete adjuvant is mixed with the antigen, and the mixture is emulsified. Then, the sensitizing antigen is administered to a mammal several times at 4- to 21-day intervals. Appropriate carriers may be used in immunization with the sensitizing antigen. In particular, when a low-molecular-weight partial peptide is used as the sensitizing antigen, it is sometimes desirable to couple the sensitizing antigen peptide to a carrier protein such as albumin or keyhole limpet hemocyanin for immunization.

Alternatively, hybridomas producing a desired antibody can be prepared using DNA immunization as mentioned below. DNA immunization is an immunization method that confers immunostimulation by expressing a sensitizing antigen in an animal immunized as a result of administering a vector DNA constructed to allow expression of an antigen protein-encoding gene in the animal. As compared to conventional immunization methods in which a protein antigen is administered to animals to be immunized, DNA immunization is expected to be superior in that:

- in the case the antigen is a membrane protein, immunostimulation can be provided while retaining the structure of the membrane protein; and
- there is no need to purify the antigen for immunization.

In order to prepare a monoclonal antibody using DNA immunization, first, a DNA expressing an antigen protein is administered to an animal to be immunized. The antigen protein-encoding DNA can be synthesized by known methods such as PCR. The obtained DNA is inserted into an appropriate expression vector, and then this is administered to an animal to be immunized. Preferably used expression vectors include, for example, commercially-available expression vectors such as pcDNA3.1. Vectors can be administered to an organism using conventional methods. For example, DNA immunization is performed by using a gene gun to introduce expression vector-coated gold particles into cells in the body of an animal to be immunized.

After immunizing a mammal as described above, an increase in the titer of an antibody against a desired antigen is confirmed in the serum. Then, immune cells are collected from the mammal, and then subjected to cell fusion. In particular, splenocytes are preferably used as immune cells.

A mammalian myeloma cell is used as a cell to be fused with the above-mentioned immune cells. The myeloma cells preferably comprise a suitable selection marker for screening. A selection marker confers characteristics to cells for their survival (or death) under a specific culture condition. Hypoxanthine-guanine-phosphoribosyltransferase deficiency (hereinafter abbreviated as HGPRT deficiency) and thymidine kinase deficiency (hereinafter abbreviated as TK deficiency) are known as selection markers. Cells with HGPRT or TK deficiency have hypoxanthine-aminopterin-thymidine sensitivity (hereinafter abbreviated as HAT sensitivity). HAT-sensitive cells cannot synthesize DNA in a HAT selection medium, and are thus killed. However, when the cells are fused with normal cells, they can continue DNA synthesis using the salvage pathway of the normal cells, and therefore they can grow even in the HAT selection medium.

HGPRT-deficient and TK-deficient cells can be selected in a medium containing 6-thioguanine, 8-azaguanine (hereinafter abbreviated as 8AG), or 5'-bromodeoxyuridine, respectively. Normal cells are killed because they incorporate these pyrimidine analogs into their DNA. Meanwhile, cells that are deficient in these enzymes can survive in the selection medium, since they cannot incorporate these pyrimidine analogs. In addition, a selection marker referred to as G418 resistance provided by the neomycin-resistant gene confers resistance to 2-deoxystreptamine antibiotics (gentamycin analogs). Various types of myeloma cells that are suitable for cell fusion are known.

For example, myeloma cells including the following cells can be preferably used:

P3(P3x63Ag8.653) (J. Immunol. (1979) 123 (4), 1548-1550);
P3x63Ag8U.1 (Current Topics in Microbiology and Immunology (1978)81, 1-7);
NS-1 (C. Eur. J. Immunol. (1976)6 (7), 511-519);
MPC-11 (Cell (1976) 8 (3), 405-415);
SP2/0 (Nature (1978) 276 (5685), 269-270);
FO (J. Immunol. Methods (1980) 35 (1-2), 1-21);
S194/5.XX0.BU.1 (J. Exp. Med. (1978) 148 (1), 313-323);
R210 (Nature (1979) 277 (5692), 131-133), etc.

Cell fusions between the immunocytes and myeloma cells are essentially carried out using known methods, for example, a method by Kohler and Milstein et al. (Methods Enzymol. (1981) 73: 3-46).

More specifically, cell fusion can be carried out, for example, in a conventional culture medium in the presence of a cell fusion-promoting agent. The fusion-promoting agents include, for example, polyethylene glycol (PEG) and Sendai virus (HVJ). If required, an auxiliary substance such as dimethyl sulfoxide is also added to improve fusion efficiency.

The ratio of immune cells to myeloma cells may be determined at one's own discretion, preferably, for example, one myeloma cell for every one to ten immunocytes. Culture media to be used for cell fusions include, for example, media that are suitable for the growth of myeloma cell lines, such as RPMI1640 medium and MEM medium, and other conventional culture medium used for this type of cell culture. In addition, serum supplements such as fetal calf serum (FCS) may be preferably added to the culture medium.

For cell fusion, predetermined amounts of the above immune cells and myeloma cells are mixed well in the above culture medium. Then, a PEG solution (for example, the average molecular weight is about 1000 to 6000) prewarmed to about 37°C is added thereto at a concentration of generally 30% to 60% (w/v). This is gently mixed to produce desired fusion cells (hybridomas). Then, an appropriate culture medium mentioned above is gradually added to the cells, and this is repeatedly centrifuged to remove the supernatant. Thus, cell fusion agents and such which are unfavorable to hybridoma growth can be removed.

The hybridomas thus obtained can be selected by culture using a conventional selective medium, for example, HAT medium (a culture medium containing hypoxanthine, aminopterin, and thymidine). Cells other than the desired hybridomas (non-fused cells) can be killed by continuing culture in the above HAT medium for a sufficient period of time (typically, the period is several days to several weeks). Then, hybridomas producing the desired antibody are screened and singly cloned by conventional limiting dilution methods.

The hybridomas thus obtained can be selected using a selection medium based on the selection marker possessed by the myeloma used for cell fusion. For example, HGPRT- or TK-deficient cells can be selected by culture using the HAT medium (a culture medium containing hypoxanthine, aminopterin, and thymidine). Specifically, when HAT-sensitive myeloma cells are used for cell fusion, cells successfully fused with normal cells can selectively proliferate in the HAT medium. Cells other than the desired hybridomas (non-fused cells) can be killed by continuing culture in the above HAT medium for a sufficient period of time. Specifically, desired hybridomas can be selected by culture for generally several days to several weeks. Then, hybridomas producing the desired antibody are screened and singly cloned by conventional limiting dilution methods.

Desired antibodies can be preferably selected and singly cloned by screening methods based on known antigen/antibody reaction. For example, the activity of an antibody to bind to immobilized antigen can be assessed based on the principle of ELISA. For example, antigen is immobilized to the wells of an ELISA plate. Culture supernatants of hybridomas are contacted with the IgA in the wells, and antibodies that bind to the IgA are detected. When the monoclonal antibodies are derived from mouse, antibodies bound to the cells can be detected using an antimouse immunoglobulin antibody. Hybridomas producing a desired antibody having the antigen-binding ability are selected by the above screening, and they can be cloned by a limiting dilution method or the like.

Monoclonal antibody-producing hybridomas thus prepared can be passaged in a conventional culture medium, and stored in liquid nitrogen for a long period.

The above hybridomas are cultured by a conventional method, and desired monoclonal antibodies can be prepared from the culture supernatants. Alternatively, the hybridomas are administered to and grown in compatible mammals, and monoclonal antibodies are prepared from the ascites. The former method is suitable for preparing antibodies with high purity.

Antibodies encoded by antibody genes that are cloned from antibody-producing cells such as the above hybridomas can also be preferably used. A cloned antibody gene is inserted into an appropriate vector, and this is introduced into a host to express the antibody encoded by the gene. Methods for isolating antibody genes, inserting the genes into vectors, and transforming host cells have already been established, for example, by Vandamme et al. (Eur. J. Biochem. (1990) 192(3), 767-775). Methods for producing recombinant antibodies are also known as described below.

For example, a cDNA encoding the variable region (V region) of an antibody of interest is prepared from hybridoma cells producing the antibody. For this purpose, total RNA is first extracted from hybridomas. Methods used for extracting mRNAs from cells include, for example:

- the guanidine ultracentrifugation method (Biochemistry (1979) 18(24), 5294-5299), and
- the AGPC method (Anal. Biochem. (1987) 162(1), 156-159)

Extracted mRNAs can be purified using the mRNA Purification Kit (GE Healthcare Bioscience) or such. Alternatively, kits for extracting total mRNA directly from cells, such as the QuickPrep mRNA Purification Kit (GE Healthcare Bioscience), are also commercially available. mRNAs can be prepared from hybridomas using such kits. cDNAs encoding the antibody V region can be synthesized from the prepared mRNAs using a reverse transcriptase. cDNAs can be synthesized using the AMV Reverse Transcriptase First-strand cDNA Synthesis Kit (Seikagaku Co.) or such. Furthermore, the SMART RACE cDNA amplification kit (Clontech) and the PCR-based 5'-RACE method (Proc. Natl. Acad. Sci. USA (1988) 85(23), 8998-9002; Nucleic Acids Res. (1989) 17(8), 2919-2932) can be appropriately used to synthesize and amplify cDNAs. In such a cDNA synthesis process, appropriate restriction enzyme sites described below may be introduced into both ends of a cDNA.

The cDNA fragment of interest is purified from the resulting PCR product, and then this is ligated to a vector DNA. A recombinant vector is thus constructed, and introduced into *E. coli* or such. After colony selection, the desired recombinant vector can be prepared from the colony-forming *E. coli.* Then, whether the recombinant vector has the cDNA nucleotide sequence of interest is tested by a known method such as the dideoxy nucleotide chain termination method.

The 5'-RACE method which uses primers to amplify the variable region gene is conveniently used for isolating the gene encoding the variable region. First, a 5'-RACE cDNA library is constructed by cDNA synthesis using RNAs extracted from hybridoma cells as a template. A commercially available kit such as the SMART RACE cDNA amplification kit is appropriately used to synthesize the 5'-RACE cDNA library.

The antibody gene is amplified by PCR using the prepared 5'-RACE cDNA library as a template. Primers for amplifying the mouse antibody gene can be designed based on known antibody gene sequences. The nucleotide sequences of the primers vary depending on the immunoglobulin subclass. Therefore, it is preferable that the subclass is determined in advance using a commercially available kit such as the Iso Strip mouse monoclonal antibody isotyping kit (Roche Diagnostics).

Specifically, for example, primers that allow amplification of genes encoding γ1, γ2a, γ2b, and γ3 heavy chains and κ and λ light chains are used to isolate mouse IgG-encoding genes. In general, a primer that anneals to a constant region site close to the variable region is used as a 3'-side primer to amplify an IgG variable region gene. Meanwhile, a primer attached to a 5' RACE cDNA library construction kit is used as a 5'-side primer.

PCR products thus amplified are used to reshape immunoglobulins composed of a combination of heavy and light chains. A desired antibody can be selected using the antigen-binding activity of a reshaped immunoglobulin as an indicator. For example, when the objective is to isolate an antibody against a desired antigen, it is more preferred that the binding of the antibody to antigen is specific. An antibody that binds to the antigen can be screened, for example, by the following steps:

(1) contacting a desired antigen with an antibody comprising the V region encoded by a cDNA isolated from a hybridoma;
(2) detecting the binding of the antibody to the antigen; and
(3) selecting an antibody that binds to the antigen-expressing cell.

Methods for detecting the binding of an antibody to antigen are known. Specifically, the binding of an antibody to antigen-expressing cells can be detected by the above-described techniques such as ELISA.

Preferred antibody screening methods that use the binding activity as an indicator also include panning methods using phage vectors. Screening methods using phage vectors are advantageous when the antibody genes are isolated from heavy-chain and light-chain subclass libraries from a polyclonal antibody-expressing cell population. Genes encoding the heavy-chain and light-chain variable regions can be linked by an appropriate linker sequence to form a single-chain Fv (scFv). Phages presenting scFv on their surface can be produced by inserting a gene encoding scFv into a phage vector. The phages are contacted with an antigen of interest. Then, a DNA encoding scFv having the binding activity of interest can be isolated by collecting phages bound to the antigen. This process can be repeated as necessary to enrich scFv having the binding activity of interest.

After isolation of the cDNA encoding the V region of the antibody of interest, the cDNA is digested with restriction enzymes that recognize the restriction sites introduced into both ends of the cDNA. Preferred restriction enzymes recognize and cleave a nucleotide sequence that occurs in the nucleotide sequence of the antibody gene at a low frequency. Furthermore, a restriction site for an enzyme that produces a sticky end is preferably introduced into a vector to insert a single-copy digested fragment in the correct orientation. The cDNA encoding the V region of the antibody is digested as described above, and this is inserted into an appropriate expression vector to construct an antibody expression vector. In this case, if a gene encoding the antibody constant region (C region) and a gene encoding the above V region are fused in-frame, a chimeric antibody is obtained. Herein, "chimeric antibody" means that the origin of the constant region is different from that of the variable region. Thus, in addition to mouse-human heterochimeric antibodies, human-human allochimeric antibodies are included in the chimeric antibodies disclosed herein. A chimeric antibody expression vector can be constructed by inserting the above V region gene into an expression vector that already has the constant region. Specifically, for example, a recognition sequence for a restriction enzyme that excises the above V region gene can be appropriately placed on the 5' side of an expression vector carrying a DNA encoding a desired antibody constant region (C region). A chimeric antibody expression vector is constructed by fusing in frame the two genes digested with the same combination of restriction enzymes.

To produce a monoclonal antibody, antibody genes are inserted into an expression vector so that the genes are expressed under the control of an expression regulatory region. The expression regulatory region for antibody expression includes, for example, enhancers and promoters. Furthermore, an appropriate signal sequence may be attached to the amino terminus so that the expressed antibody is secreted to the outside of cells. The expressed polypeptide is cleaved at the carboxyl terminus of the above sequence, and the resulting polypeptide is secreted to the outside of cells as a mature polypeptide. Then, appropriate host cells are transformed with the expression vector, and recombinant cells expressing the DNA encoding a desired antibody are obtained.

DNAs encoding the antibody heavy chain (H chain) and light chain (L chain) are separately inserted into different expression vectors to express the antibody gene. An antibody molecule having the H and L chains can be expressed by co-transfecting the same host cell with vectors into which the H-chain and L-chain genes are respectively inserted. Alternatively, host cells can be transformed with a single expression vector into which DNAs encoding the H and L chains are inserted (see International Publication No. WO 1994/011523).

There are various known host cell/expression vector combinations for antibody preparation by introducing isolated antibody genes into appropriate hosts. All of these expression systems are applicable to isolation of the antigen-binding molecules disclosed herein. Appropriate eukaryotic cells used as host cells include animal cells, plant cells, and fungal cells. Specifically, the animal cells include, for example, the following cells.

(1) Mammalian cells: CHO (Chinese hamster ovary cell line), COS (Monkey kidney cell line), myeloma (Sp2/O, NS0, etc.), BHK (baby hamster kidney cell line), HEK293 (human embryonic kidney cell line with sheared adenovirus (Ad)5 DNA), PER.C6 cell (human embryonic retinal cell line transformed with the Adenovirus Type 5 (Ad5) E1A and E1B genes), Hela, Vero, or such (Current Protocols in Protein Science (May, 2001, Unit 5.9, Table 5.9.1)).
(2) Amphibian cells: Xenopus oocytes, or such.
(3) Insect cells: sf9, sf21, Tn5, or such.

In addition, as a plant cell, an antibody gene expression system using cells derived from the *Nicotiana* genus such as *Nicotiana tabacum* is known. Callus cultured cells can be appropriately used to transform plant cells.

Furthermore, the following cells can be used as fungal cells:

yeasts: the *Saccharomyces* genus such as *Saccharomyces serevisiae,* and the *Pichia* genus such as *Pichia pastoris;* and

filamentous fungi: the *Aspergillus* genus such as *Aspergillus niger.*

Furthermore, antibody gene expression systems that utilize prokaryotic cells are also known. For example, when using bacterial cells, *E. coli* cells, *Bacillus subtilis* cells, and such can suitably be utilized as disclsoed herein. Expression vectors carrying the antibody genes of interest are introduced into these cells by transfection. The transfected cells are cultured *in vitro,* and the desired antibody can be prepared from the culture of transformed cells.

In addition to the above-described host cells, transgenic animals can also be used to produce a recombinant antibody. That is, the antibody can be obtained from an animal into which the gene encoding the antibody of interest is introduced. For example, the antibody gene can be constructed as a fusion gene by inserting in frame into a gene that encodes a protein produced specifically in milk. Goat β-casein or such can be used, for example, as the protein secreted in milk. DNA fragments containing the fused gene inserted with the antibody gene is injected into a goat embryo, and then this embryo is introduced into a female goat. Desired antibodies can be obtained as a protein fused with the milk protein from milk produced by the transgenic goat born from the embryo-recipient goat (or progeny thereof). In addition, to increase the volume of milk containing the desired antibody produced by the transgenic goat, hormones can be administered to the transgenic goat as necessary (Ebert, K. M. et al., Bio/Technology (1994) 12(7): 699-702).

When an antigen-binding molecule described herein is administered to human, an antigen-binding domain derived from a genetically recombinant antibody that has been artificially modified to reduce the heterologous antigenicity against human and such, can be appropriately used as the antigen-binding domain of the antigen-binding molecule. Such genetically recombinant antibodies include, for example, humanized antibodies. These modified antibodies are appropriately produced by known methods.

An antibody variable region used to produce the antigen-binding domain of an antigen-binding molecule described herein is generally formed by three complementarity-determining regions (CDRs) that are separated by four framework regions (FRs). CDR is a region that substantially determines the binding specificity of an antibody. The amino acid sequences of CDRs are highly diverse. On the other hand, the FR-forming amino acid sequences often have high identity even among antibodies with different binding specificities. Therefore, generally, the binding specificity of a certain antibody can be introduced to another antibody by CDR grafting.

A humanized antibody is also called a reshaped human antibody. Specifically, humanized antibodies prepared by applying the CDR grafting technology, which grafts the CDRs of a non-human animal antibody such as a mouse antibody to a human antibody, and such are known. Common genetic engineering techniques for obtaining humanized antibodies are also known. Specifically, for example, overlap extension PCR is known as a method for grafting a mouse antibody CDR to a human FR. In overlap extension PCR, a nucleotide sequence encoding a mouse antibody CDR to be grafted is added to primers for synthesizing a human antibody FR. Primers are prepared for each of the four FRs. It is generally considered that when grafting a mouse CDR to a human FR, selecting a human FR that has high identity to a mouse FR is advantageous for maintaining the CDR function. That is, it is generally preferable to use a human FR comprising an amino acid sequence which has high identity to the amino acid sequence of the FR adjacent to the mouse CDR to be grafted.

Nucleotide sequences to be ligated are designed so that they will be connected to each other in frame. Human FRs are individually synthesized using the respective primers. As a result, products in which the mouse CDR-encoding DNA is attached to the individual FR-encoding DNAs are obtained. Nucleotide sequences encoding the mouse CDR of each product are designed so that they overlap with each other. Then, complementary strand synthesis reaction is conducted to anneal the overlapping CDR regions of the products synthesized using a human antibody gene as template. Human FRs are ligated *via* the mouse CDR sequences by this reaction.

The full length V region gene, in which three CDRs and four FRs are ultimately ligated, is amplified using primers that anneal to its 5'- or 3'-end, which are added with suitable restriction enzyme recognition sequences. An expression vector for humanized antibody can be produced by inserting the DNA obtained as described above and a DNA that encodes a human antibody C region into an expression vector so that they will ligate in frame. After the recombinant vector is transfected into a host to establish recombinant cells, the recombinant cells are cultured, and the DNA encoding the humanized antibody is expressed to produce the humanized antibody in the cell culture (see, European Patent Publication No. EP 239400 and International Patent Publication No. WO 1996/002576).

By qualitatively or quantitatively measuring and evaluating the antigen-binding activity of the humanized antibody produced as described above, one can suitably select human antibody FRs that allow CDRs to form a favorable antigen-binding site when ligated through the CDRs. Amino acid residues in FRs may be substituted as necessary, so that the CDRs of a reshaped human antibody form an appropriate antigen-binding site. For example, amino acid sequence mutations can be introduced into FRs by applying the PCR method used for grafting a mouse CDR into a human FR. More specifically, partial nucleotide sequence mutations can be introduced into primers that anneal to the FR. Nucleotide sequence mutations are introduced into the FRs synthesized by using such primers. Mutant FR sequences having the desired characteristics can be selected by measuring and evaluating the activity of the amino acid-substituted mutant antibody to bind to the antigen by the above-mentioned method (Cancer Res. (1993) 53: 851-856).

Alternatively, desired human antibodies can be obtained by immunizing transgenic animals having the entire repertoire of human antibody genes (see International Publication Nos. WO 1993/012227; WO 1992/003918; WO 1994/002602; WO 1994/025585; WO 1996/034096; WO 1996/033735) by DNA immunization.

Furthermore, techniques for preparing human antibodies by panning using human antibody libraries are also known. For example, the V region of a human antibody is expressed as a single-chain antibody (scFv) on phage surface by the phage display method. Phages expressing an scFv that binds to the antigen can be selected. The DNA sequence encoding the human antibody V region that binds to the antigen can be determined by analyzing the genes of selected phages. The DNA sequence of the scFv that binds to the antigen is determined. An expression vector is prepared by fusing the V region sequence in frame with the C region sequence of a desired human antibody, and inserting this into an appropriate expression vector. The expression vector is introduced into cells appropriate for expression such as those described above. The human antibody can be produced by expressing the human antibody-encoding gene in the cells. These methods are already known (see International Publication Nos. WO 1992/001047; WO 1992/020791; WO 1993/006213; WO 1993/011236; WO 1993/019172; WO 1995/001438; WO 1995/015388).

In addition to the techniques described above, techniques of B cell cloning (identification of each antibody-encoding sequence, cloning and its isolation; use in constructing expression vector in order to prepare each antibody (IgG1, IgG2, IgG3, or IgG4 in particular); and such) such as described in Bernasconi et al. (Science (2002) 298: 2199-2202) or in International Publication No. WO 2008/081008 can be appropriately used to isolate antibody genes.

EU numbering and Kabat numbering

According to the methods used in the present disclosure, amino acid positions assigned to antibody CDR and FR are specified according to Kabat's numbering (Sequences of Proteins of Immunological Interest (National Institute of Health, Bethesda, Md., 1987 and 1991)). Herein, when an antigen-binding molecule is an antibody or antigen-binding fragment, variable region amino acids are indicated according to Kabat's numbering system (Kabat numbering), while constant region amino acids are indicated according to EU numbering system based on Kabat's amino acid positions.

Conditions of ion concentration

Conditions of metal ion concentration

In one embodiment, the ion concentration refers to a metal ion concentration. "Metal ions" refer to ions of group I elements except hydrogen such as alkaline metals and copper group elements, group II elements such as alkaline earth metals and zinc group elements, group III elements except boron, group IV elements except carbon and silicon, group VIII elements such as iron group and platinum group elements, elements belonging to subgroup A of groups V, VI, and VII, and metal elements such as antimony, bismuth, and polonium. Metal atoms have the property of releasing valence electrons to become cations. This is referred to as ionization tendency. Metals with strong ionization tendency are deemed to be chemically active.

In the present invention, a preferred metal ion is calcium ion. Calcium ion is involved in modulation of many biological phenomena, including contraction of muscles such as skeletal, smooth, and cardiac muscles; activation of movement, phagocytosis, and the like of leukocytes; activation of shape change, secretion, and the like of platelets; activation of lymphocytes; activation of mast cells including secretion of histamine; cell responses mediated by catecholamine $\alpha$ receptor or acetylcholine receptor; exocytosis; release of transmitter substances from neuron terminals; and axoplasmic flow in neurons. Known intracellular calcium ion receptors include troponin C, calmodulin, parvalbumin, and myosin light chain, which have several calcium ion-binding sites and are believed to be derived from a common origin in terms of molecular evolution. There are also many known calcium-binding motifs. Such well-known motifs include, for example, cadherin domains, EF-hand of calmodulin, C2 domain of Protein kinase C, Gla domain of blood coagulation protein Factor IX, C-type lectins of asialoglycoprotein receptor and mannose-binding receptor, A domains of LDL receptors, annexin, thrombospondin type 3 domain, and EGF-like domains.

In the present invention, when the metal ion is calcium ion, the conditions of calcium ion concentration include low calcium ion concentration conditions and high calcium ion concentration conditions. "The binding activity varies depending on calcium ion concentration conditions" means that the antigen-binding activity of an antigen-binding molecule varies due to the difference in the conditions between low and high calcium ion concentration conditions. For example, the antigen-binding activity of an antigen-binding molecule may be higher under a high calcium ion concentration condition than under a low calcium ion concentration condition. Alternatively, the antigen-binding activity of an antigen-binding molecule may be higher under a low calcium ion concentration condition than under a high calcium ion concentration condition.

Herein, the high calcium ion concentration is not particularly limited to a specific value; however, the concentration may preferably be selected between 100 μM and 10 mM. In another embodiment, the concentration may be selected between 200 μM and 5 mM. In an alternative embodiment, the concentration may be selected between 400 μM and 3 mM. In still another embodiment, the concentration may be selected between 200 μM and 2 mM. Furthermore, the concentration may be selected between 400 μM and 1 mM. In particular, a concentration selected between 500 μM and 2.5 mM, which is close to the plasma (blood) concentration of calcium ion *in vivo,* is preferred.

Herein, the low calcium ion concentration is not particularly limited to a specific value; however, the concentration may preferably be selected between 0.1 μM and 30 μM. In another embodiment, the concentration may be selected between 0.2 μM and 20 μM. In still another embodiment, the concentration may be selected between 0.5 μM and 10 μM. In an alternative embodiment, the concentration may be selected between 1 μM and 5 μM. Furthermore, the concentration may be selected between 2 μM and 4 μM. In particular, a concentration selected between 1 μM and 5 μM, which is close to the concentration of ionized calcium in early endosomes *in vivo,* is preferred.

As used herein, "the antigen-binding activity is lower at a low calcium ion concentration condition than at a high calcium ion concentration condition" means that the antigen-binding activity of an antigen-binding molecule is weaker at a calcium ion concentration selected between 0.1 μM and 30 μM than at a calcium ion concentration selected between 100 μM and 10 mM. Preferably, it means that the antigen-binding activity of an antigen-binding molecule is weaker at a calcium ion concentration selected between 0.5 μM and 10 μM than at a calcium ion concentration selected between 200 μM and 5 mM. It particularly preferably means that the antigen-binding activity at the calcium ion concentration in the early endosome *in vivo* is weaker than that at the *in vivo* plasma calcium ion concentration; and specifically, it means that the antigen-binding activity of an antigen-binding molecule is weaker at a calcium ion concentration selected between 1 μM and 5 μM than at a calcium ion concentration selected between 500 μM and 2.5 mM.

Whether the antigen-binding activity of an antigen-binding molecule is changed depending on metal ion concentration conditions can be determined, for example, by the use of known measurement methods such as those described in the section "Binding Activity" above. For example, in order to confirm that the antigen-binding activity of an antigen-binding molecule becomes higher under a high calcium ion concentration condition than under a low calcium ion concentration condition, the antigen-binding activity of the antigen-binding molecule under low and high calcium ion concentration conditions is compared.

As used herein, the expression "the antigen-binding activity is lower at a low calcium ion concentration condition than at a high calcium ion concentration condition" can also be expressed as "the antigen-binding activity of an antigen-binding molecule is higher under a high calcium ion concentration condition than under a low calcium ion concentration condition". As used herein, "the antigen-binding activity is lower at a low calcium ion concentration condition than at a high calcium ion concentration condition" is sometimes written as "the antigen-binding ability is weaker under a low calcium ion concentration condition than under a high calcium ion concentration condition". Also, "the antigen-binding activity at a low calcium ion concentration condition is reduced to be lower than that at a high calcium ion concentration condition" may be written as "the antigen-binding ability under a low calcium ion concentration condition is made weaker than that under a high calcium ion concentration condition".

When determining the antigen-binding activity, the conditions other than calcium ion concentration can be appropriately selected by those skilled in the art, and are not particularly limited. For example, the activity can be determined at 37°C in HEPES buffer. For example, Biacore (GE Healthcare) or such can be used for the determination. When the antigen is a soluble antigen, the antigen-binding activity of an antigen-binding molecule can be assessed by flowing the antigen as an analyte over a chip onto which the antigen-binding molecule is immobilized. When the antigen is a membrane antigen, the binding activity of an antigen-binding molecule to the membrane antigen can be assessed by flowing the antigen-binding molecule as an analyte over a chip onto which the antigen is immobilized.

As long as the antigen-binding activity of an antigen-binding molecule is weaker at a low calcium ion concentration condition than at a high calcium ion concentration condition, the ratio of the antigen-binding activity between under low and high calcium ion concentration conditions is not particularly limited. However, the ratio of the KD (dissociation constant) of the antigen-binding molecule for an antigen at a low calcium ion concentration condition with respect to the KD at a high calcium ion concentration condition, *i.e.,* the value of KD (3 μM Ca)/KD (2 mM Ca), is preferably 2 or more, more preferably 10 or more, and still more preferably 40 or more. The upper limit of the KD (3 μM Ca)/KD (2 mM Ca) value is not particularly limited, and may be any value such as 400, 1000, or 10000 as long as the molecule can be produced by techniques known to those skilled in the art.

When the antigen is a soluble antigen, KD (dissociation constant) can be used to represent the antigen-binding activity. Meanwhile, when the antigen is a membrane antigen, apparent KD (apparent dissociation constant) can be used to represent the activity. KD (dissociation constant) and apparent KD (apparent dissociation constant) can be determined by methods known to those skilled in the art, for example, using Biacore (GE healthcare), Scatchard plot, or flow cytometer.

Alternatively, for example, the dissociation rate constant (kd) can also be preferably used as an index to represent the ratio of the antigen-binding activity of an antigen-binding molecule disclosed herein between low and high calcium concentrations. When the dissociation rate constant (kd) is used instead of the dissociation constant (KD) as an index to represent the binding activity ratio, the ratio of the dissociation rate constant (kd) between low and high calcium concentration conditions, *i.e.,* the value of kd (low calcium concentration condition)/kd (high calcium concentration condition), is preferably 2 or more, more preferably 5 or more, still more preferably 10 or more, and yet more preferably 30 or more. The upper limit of the Kd (low calcium concentration condition)/kd (high calcium concentration condition) value is not particularly limited, and can be any value such as 50, 100, or 200 as long as the molecule can be produced by techniques known to those skilled in the art.

When the antigen is a soluble antigen, kd (dissociation rate constant) can be used to represent the antigen-binding activity. Meanwhile, when the antigen is a membrane antigen, apparent kd (apparent dissociation rate constant) can be used to represent the antigen-binding activity. The kd (dissociation rate constant) and apparent kd (apparent dissociation rate constant) can be determined by methods known to those skilled in the art, for example, using Biacore (GE healthcare) or flow cytometer. When the antigen-binding activity of an antigen-binding molecule is determined at different calcium ion concentrations, it is preferable to use the same conditions except for the calcium concentrations.

For example, an antigen-binding domain (or antigen-binding molecule) whose antigen-binding activity is lower at a low calcium ion concentration condition than at a high calcium ion concentration condition, can be obtained *via* screening of antigen-binding domains (or antigen-binding molecules) including the steps of (a) to (c) below:

(a) determining the antigen-binding activity of an antigen-binding domain (or antigen-binding molecule) at a low calcium concentration condition;
(b) determining the antigen-binding activity of an antigen-binding domain (or antigen-binding molecule) at a high calcium concentration condition; and
(c) selecting an antigen-binding domain (or antigen-binding molecule) whose antigen-binding activity is lower at a low calcium concentration condition than at a high calcium concentration condition.

Moreover, an antigen-binding domain (or antigen-binding molecule) whose antigen-binding activity is lower at a low calcium ion concentration condition than at a high calcium ion concentration condition, can be obtained *via* screening of antigen-binding domains (or antigen-binding molecules) or a library thereof, including the steps of (a) to (c) below:

(a) contacting an antigen with an antigen-binding domain (or antigen-binding molecule), or a library thereof at a high calcium concentration condition;
(b) incubating under a low calcium concentration condition an antigen-binding domain (or antigen-binding molecule) that has bound to the antigen in step (a); and
(c) isolating an antigen-binding domain (or antigen-binding molecule) dissociated in step (b).

Furthermore, an antigen-binding domain (or antigen-binding molecule) whose antigen-binding activity is lower at a low calcium ion concentration condition than at a high calcium ion concentration condition, can be obtained *via* screening of antigen-binding domains (or antigen-binding molecules) or a library thereof, including the steps of (a) to (d) below:

(a) contacting an antigen with a library of antigen-binding domains (or antigen-binding molecules) under a low calcium concentration condition;
(b) selecting an antigen-binding domain (or antigen-binding molecule) which does not bind to the antigen in step (a);
(c) allowing the antigen-binding domain (or antigen-binding molecule) selected in step (b) to bind to the antigen under a high calcium concentration condition; and
(d) isolating an antigen-binding domain (or antigen-binding molecule) that has bound to the antigen in step (c).

In addition, an antigen-binding domain (or antigen-binding molecule) whose antigen-binding activity is lower at a low calcium ion concentration condition than at a high calcium ion concentration condition, can be obtained by a screening method comprising the steps of (a) to (c) below:

(a) contacting under a high calcium concentration condition a library of antigen-binding domains (or antigen-binding molecules) with a column onto which an antigen is immobilized;
(b) eluting an antigen-binding domain (or antigen-binding molecule) that has bound to the column in step (a) from the column under a low calcium concentration condition; and
(c) isolating the antigen-binding domain (or antigen-binding molecule) eluted in step (b).

Furthermore, an antigen-binding domain (or antigen-binding molecule) whose antigen-binding activity is lower at a low calcium ion concentration condition than at a high calcium ion concentration condition, can be obtained by a screening method comprising the steps of (a) to (d) below:

> (a) allowing under a low calcium concentration condition a library of antigen-binding domains (or antigen-binding molecules) to pass through a column onto which an antigen is immobilized;
> (b) collecting an antigen-binding domain (or antigen-binding molecule) that has been eluted without binding to the column in step (a);
> (c) allowing the antigen-binding domain (or antigen-binding molecule) collected in step (b) to bind to the antigen under a high calcium concentration condition; and
> (d) isolating an antigen-binding domain (or antigen-binding molecule) that has bound to the antigen in step (c).

Moreover, an antigen-binding domain (or antigen-binding molecule) whose antigen-binding activity is lower at a low calcium ion concentration condition than at a high calcium ion concentration condition, can be obtained by a screening method comprising the steps of (a) to (d) below:

> (a) contacting an antigen with a library of antigen-binding domains (or antigen-binding molecules) under a high calcium concentration condition;
> (b) obtaining an antigen-binding domain (or antigen-binding molecule) that has bound to the antigen in step (a);
> (c) incubating under a low calcium concentration condition the antigen-binding domain (or antigen-binding molecule) obtained in step (b); and
> (d) isolating an antigen-binding domain (or antigen-binding molecule) whose antigen-binding activity in step (c) is weaker than the criterion for the selection of step (b).

The above-described steps may be repeated twice or more times. Thus, the present disclosure provides antigen-binding domains (or antigen-binding molecules) whose antigen-binding activity is lower at a low calcium ion concentration condition than at a high calcium ion concentration condition, which are obtained by screening methods that further comprises the step of repeating twice or more times steps (a) to (c) or (a) to (d) in the above-described screening methods. The number of cycles of steps (a) to (c) or (a) to (d) is not particularly limited, but generally is 10 or less.

In the screening methods disclosed herein, the antigen-binding activity of an antigen-binding domain (or antigen-binding molecule) under a low calcium concentration condition is not particularly limited as long as it is antigen-binding activity at an ionized calcium concentration of between 0.1 $\mu$M and 30 $\mu$M, but preferably is antigen-binding activity at an ionized calcium concentration of between 0.5 $\mu$M and 10 $\mu$M. More preferably, it is antigen-binding activity at the ionized calcium concentration in the cell *in vivo,* in particular at the ionized calcium concentration in the early endosome, specifically, between 1 $\mu$M and 5 $\mu$M. Meanwhile, the antigen-binding activity of an antigen-binding domain (or antigen-binding molecule) under a high calcium concentration condition is not particularly limited, as long as it is antigen-binding activity at an ionized calcium concentration of between 100 $\mu$M and 10 mM, but preferably is antigen-binding activity at an ionized calcium concentration of between 200 $\mu$M and 5 mM. More preferably, it is antigen-binding activity at the ionized calcium concentration in the cell *in vivo,* in particular at the ionized calcium concentration in plasma, specifically, between 0.5 mM and 2.5 mM.

The antigen-binding activity of an antigen-binding domain (or antigen-binding molecule) can be measured by methods known to those skilled in the art. Conditions other than the ionized calcium concentration can be determined by those skilled in the art. The antigen-binding activity of an antigen-binding domain (or antigen-binding molecule) can be evaluated as a dissociation constant (KD), apparent dissociation constant (apparent KD), dissociation rate constant (kd), apparent dissociation constant (apparent kd), and such. These can be determined by methods known to those skilled in the art, for example, using Biacore (GE healthcare), Scatchard plot, or FACS.

As disclosed herein, the step of selecting an antigen-binding domain (or antigen-binding molecule) whose antigen-binding activity is higher under a high calcium concentration condition than under a low calcium concentration condition is synonymous with the step of selecting an antigen-binding domain (or antigen-binding molecule) whose antigen-binding activity is lower under a low calcium concentration condition than under a high calcium concentration condition.

As long as the antigen-binding activity is higher under a high calcium concentration condition than under a low calcium concentration condition, the difference in the antigen-binding activity between high and low calcium concentration conditions is not particularly limited; however, the antigen-binding activity under a high calcium concentration condition is preferably twice or more, more preferably 10 times or more, and still more preferably 40 times or more than that under a low calcium concentration condition.

Antigen-binding domains (or antigen-binding molecules) disclosed herein to be screened by the screening methods described above may be any antigen-binding domains (or antigen-binding molecules). For example, it is possible to screen the above-described antigen-binding domains (or antigen-binding molecules). For example, antigen-binding domains (or antigen-binding molecules) having natural sequences or substituted amino acid sequences may be screened.

<u>Libraries (Library)</u>

In an embodiment, an antigen-binding domain (or antigen-binding molecule) as disclosed herei can be obtained from a library that is mainly composed of a plurality of antigen-binding molecules whose sequences are different from one another and whose antigen-binding domains have at least one amino acid residue that alters the antigen-binding activity of the antigen-binding molecules depending on ion concentration conditions. The ion concentrations preferably include, for example, metal ion concentration and proton concentration.

Herein, a "library" refers to a plurality of antigen-binding molecules or a plurality of fusion polypeptides containing antigen-binding molecules, or nucleic acids or polynucleotides encoding their sequences. The sequences of a plurality of antigen-binding molecules or a plurality of fusion polypeptides containing antigen-binding molecules in a library are not identical, but are different from one another.

Herein, the phrase "sequences are different from one another" in the expression "a plurality of antigen-binding molecules whose sequences are different from one another" means that the sequences of antigen-binding molecules in a library are different from one another. Specifically, in a library, the number of sequences different from one another reflects the number of independent clones with different sequences, and may also be referred to as "library size". The library size of a conventional phage display library ranges from $10^6$ to $10^{12}$. The library size can be increased up to $10^{14}$ by the use of known techniques such as ribosome display. However, the actual number of phage particles used in panning selection of a phage library is in general 10-10000 times greater than the library size. This excess multiplicity is also referred to as "the number of library equivalents", and means that there are 10 to 10000 individual clones that have the same amino acid sequence. Thus, the phrase "sequences are different from one another" means that the sequences of independent antigen-binding molecules in a library, excluding library equivalents, are different from one another. More specifically, the above means that there are $10^6$ to $10^{14}$ antigen-binding molecules whose sequences are different from one another, preferably $10^7$ to $10^{12}$ molecules, more preferably $10^8$ to $10^{11}$ molecules, and particularly preferably $10^8$ to $10^{10}$ molecules whose sequences are different from one another.

As used herein, the phrase "a plurality of' in the expression "a library mainly composed of a plurality of antigen-binding molecules" generally refers to, in the case of, for example, antigen-binding molecules, fusion polypeptides, polynucleotide molecules, vectors, or viruses as disclosed herein, a group of two or more types of the substance. For example, when two or more substances are different from one another in a particular characteristic, this means that there are two or more types of the substance. Such examples may include, for example, mutant amino acids observed at specific amino acid positions in an amino acid sequence. For example, when there are two or more antigen-binding molecules whose sequences are substantially the same or preferably the same except for flexible residues or except for particular mutant amino acids at hypervariable positions exposed on the surface, there are a plurality of antigen-binding molecules of the present disclosure. In another Example, when there are two or more polynucleotide molecules whose sequences are substantially the same or preferably the same except for nucleotides encoding flexible residues or nucleotides encoding mutant amino acids of hypervariable positions exposed on the surface, there are a plurality of polynucleotide molecules of the present disclosure.

In addition, the phrase "mainly composed of' in the expression "a library mainly composed of a plurality of antigen-binding molecules" reflects the number of antigen-binding molecules whose antigen-binding activity varies depending on ion concentration conditions, among independent clones with different sequences in a library. Specifically, it is preferable that there are at least $10^4$ antigen-binding molecules having such binding activity in a library. More preferably, antigen-binding domains as disclosed herein can be obtained from a library containing at least $10^5$ antigen-binding molecules having such binding activity. Still more preferably, antigen-binding domains as disclosed herein can be obtained from a library containing at least $10^6$ antigen-binding molecules having such binding activity. Particularly preferably, antigen-binding domains as disclosed herein can be obtained from a library containing at least $10^7$ antigen-binding molecules having such binding activity. Yet more preferably, antigen-binding domains as dsiclsoed herein can be obtained from a library containing at least $10^8$ antigen-binding molecules having such binding activity. Alternatively, this may also be preferably expressed as the ratio of the number of antigen-binding molecules whose antigen-binding activity varies depending on ion concentration conditions with respect to the number of independent clones having different sequences in a library. Specifically, antigen-binding domains as disclosed herein can be obtained from a library in which antigen-binding molecules having such binding activity account for 0.1% to 80%, preferably 0.5% to 60%, more preferably 1% to 40%, still more preferably 2% to 20%, and particularly preferably 4% to 10% of independent clones with different sequences in the library. In the case of fusion polypeptides, polynucleotide molecules, or vectors, similar expressions may be possible using the number of molecules or the ratio to the total number of molecules. In the case of viruses, similar expressions may also be possible using the number of virions or the ratio to total number of virions.

Amino acids that alter the antigen-binding activity of antigen-binding domains depending on calcium ion concentration conditions

Antigen-binding domains (or antigen-binding molecules) to be screened by the above-described screening methods may be prepared in any manner. For example, when the metal ion is calcium ion, it is possible to use preexisting antibodies, preexisting libraries (phage library, etc.), antibodies or libraries prepared from hybridomas obtained by immunizing animals or from B cells of immunized animals, antibodies or libraries obtained by introducing amino acids capable of chelating calcium (for example, aspartic acid and glutamic acid) or unnatural amino acid mutations into the above-described antibodies or libraries (calcium-chelatable amino acids (such as aspartic acid and glutamic acid), libraries with increased content of unnatural amino acids, libraries prepared by introducing calcium-chelatable amino acids (such as aspartic acid and glutamic acid) or unnatural amino acid mutations at particular positions, or the like.

Examples of the amino acids that alter the antigen-binding activity of antigen-binding molecules depending on ion concentration conditions as described above may be any types of amino acids as long as the amino acids form a calcium-binding motif. Calcium-binding motifs are well known to those skilled in the art and have been described in details (for example, Springer et al. (Cell (2000) 102, 275-277); Kawasaki and Kretsinger (Protein Prof. (1995) 2, 305-490); Moncrief et al. (J. Mol. Evol. (1990) 30, 522-562); Chauvaux et al. (Biochem. J. (1990) 265, 261-265); Bairoch and Cox (FEBS Lett. (1990) 269, 454-456); Davis (New Biol. (1990) 2, 410-419); Schaefer et al. (Genomics (1995) 25, 638-643); Economou et al. (EMBO J. (1990) 9, 349-354); Wurzburg et al. (Structure. (2006) 14, 6, 1049-1058)). Specifically, any known calcium-binding motifs, including type C lectins such as ASGPR, CD23, MBR, and DC-SIGN, can be included in antigen-binding molecules of the present disclosure. Preferred examples of such preferred calcium-binding motifs also include, in addition to those described above, for example, the calcium-binding motif in the antigen-binding domain of SEQ ID NO: 2.

Furthermore, as amino acids that alter the antigen-binding activity of antigen-binding molecules depending on calcium ion concentration conditions, for example, amino acids having metal-chelating activity may also be preferably used. Examples of such metal-chelating amino acids include, for example, serine (Ser(S)), threonine (Thr(T)), asparagine (Asn(N)), glutamine (Gln(Q)), aspartic acid (Asp(D)), and glutamic acid (Glu(E)).

Positions in the antigen-binding domains at which the above-described amino acids are contained are not particularly limited to particular positions, and may be any positions within the heavy chain variable region or light chain variable region that forms an antigen-binding domain, as long as they alter the antigen-binding activity of antigen-binding molecules depending on calcium ion concentration conditions. More specifically, antigen-binding domains as disclosed herein can be obtained from a library mainly composed of antigen-binding molecules whose sequences are different from one another and whose heavy chain antigen-binding domains contain amino acids that alter the antigen-binding activity of the antigen-binding molecules depending on calcium ion concentration conditions. In another embodiment, antigen-binding domains as disclosed herein can be obtained from a library mainly composed of antigen-binding molecules whose sequences are different from one another and whose heavy chain CDR3 domains contain the above-mentioned amino acids. In still another embodiment, antigen-binding domains as disclosed herein can be obtained from a library mainly composed of antigen-binding molecules whose sequences are different from one another and whose heavy chain CDR3 domains contain the above-mentioned amino acids at positions 95, 96, 100a, and/or 101 as indicated according to the Kabat numbering system.

Meanwhile, antigen-binding domains as disclosed herein can be obtained from a library mainly composed of antigen-binding molecules whose sequences are different from one another and whose light chain antigen-binding domains contain amino acids that alter the antigen-binding activity of antigen-binding molecules depending on calcium ion concentration conditions. In another embodiment, antigen-binding domains as disclosed herein can be obtained from a library mainly composed of antigen-binding molecules whose sequences are different from one another and whose light chain CDR1 domains contain the above-mentioned amino acids. In still another embodiment, antigen-binding domains as disclosed hereincan be obtained from a library mainly composed of antigen-binding molecules whose sequences are different from one another and whose light chain CDR1 domains contain the above-mentioned amino acids at positions 30, 31, and/or 32 as indicated according to the Kabat numbering system.

In another embodiment, antigen-binding domains as disclosed herein can be obtained from a library mainly composed of antigen-binding molecules whose sequences are different from one another and whose light chain CDR2 domains contain the above-mentioned amino acid residues. In yet another embodiment, disclosed herein are libraries mainly composed of antigen-binding molecules whose sequences are different from one another and whose light chain CDR2 domains contain the above-mentioned amino acid residues at position 50 as indicated according to the Kabat numbering system.

In still another embodiment, antigen-binding domains as disclosed herein can be obtained from a library mainly composed of antigen-binding molecules whose sequences are different from one another and whose light chain CDR3 domains contain the above-mentioned amino acid residues. In an alternative embodiment, antigen-binding domains as disclosed herein can be obtained from a library mainly composed of antigen-binding molecules whose sequences are different from one another and whose light chain CDR3 domains contain the above-mentioned amino acid residues at position 92 as indicated according to the Kabat numbering system.

Furthermore, in a different embodiment, antigen-binding domains as disclosed herein can be obtained from a library mainly composed of antigen-binding molecules whose sequences are different from one another and in which two or three CDRs selected from the above-described light chain CDR1, CDR2, and CDR3 contain the aforementioned amino acid residues. Moreover, antigen-binding domains as disclosed herein can be obtained from a library mainly composed of antigen-binding molecules whose sequences are different from one another and whose light chains contain the aforementioned amino acid residues at any one or more of positions 30, 31, 32, 50, and/or 92 as indicated according to the Kabat numbering system.

In a particularly preferred embodiment, the framework sequences of the light chain and/or heavy chain variable region of an antigen-binding molecule preferably contain human germ line framework sequences. Thus, when the framework sequences are completely human sequences, it is expected that when such an antigen-binding molecule as disclosed herein is administered to humans (for example, to treat diseases), it induces little or no immunogenic response. In the above sense, the phrase "containing a germ line sequence" as used herein means that a part of the framework sequences is identical to a part of any human germ line framework sequences. For example, when the heavy chain FR2 sequence of an antigen-binding molecule as disclosed herein is a combination of heavy chain FR2 sequences of different human germ line framework sequences, such a molecule is also an antigen-binding molecule as disclosed herein "containing a germ line sequence".

Preferred examples of the frameworks include, for example, fully human framework region sequences currently known, which are included in the website of V-Base (http://vbase.mrc-cpe.cam.ac.uk/) or others. Those framework region sequences can be appropriately used as a germ line sequence contained in an antigen-binding molecule as disclosed herein. The germ line sequences may be categorized according to their similarity (Tomlinson et al. (J. Mol. Biol. (1992) 227, 776-798); Williams and Winter (Eur. J. Immunol. (1993) 23, 1456-1461); Cox et al. (Nat. Genetics (1994) 7, 162-168)). Appropriate germ line sequences can be selected from Vκ, which is grouped into seven subgroups; Vλ, which is grouped into ten subgroups; and VH, which is grouped into seven subgroups.

Fully human VH sequences preferably include, but are not limited to, for example, VH sequences of:

   subgroup VH1 (for example, VH1-2, VH1-3, VH1-8, VH1-18, VH1-24, VH1-45, VH1-46, VH1-58, and VH1-69);
   subgroup VH2 (for example, VH2-5, VH2-26, and VH2-70);
   subgroup VH3 (VH3-7, VH3-9, VH3-11, VH3-13, VH3-15, VH3-16, VH3-20, VH3-21, VH3-23, VH3-30, VH3-33, VH3-35, VH3-38, VH3-43, VH3-48, VH3-49, VH3-53, VH3-64, VH3-66, VH3-72, VH3-73, and VH3-74);
   subgroup VH4 (VH4-4, VH4-28, VH4-31, VH4-34, VH4-39, VH4-59, and VH4-61);
   subgroup VH5 (VH5-51);
   subgroup VH6 (VH6-1); and
   subgroup VH7 (VH7-4 and VH7-81).

These are also described in known documents (Matsuda et al. (J. Exp. Med. (1998) 188, 1973-1975)) and such, and thus persons skilled in the art can appropriately design antigen-binding molecules of the present disclosure based on the information of these sequences. It is also preferable to use other fully human frameworks or framework sub-regions.

Fully human Vκ sequences preferably include, but are not limited to, for example:

   A20, A30, L1, L4, L5, L8, L9, L11, L12, L14, L15, L18, L19, L22, L23, L24, O2, O4, O8, O12, 014, and 018 grouped into subgroup Vk1;
   A1, A2, A3, A5, A7, A17, A18, A19, A23, O1, and O11, grouped into subgroup Vk2;
   A11, A27, L2, L6, L10, L16, L20, and L25, grouped into subgroup Vk3;
   B3, grouped into subgroup Vk4;
   B2 (herein also referred to as Vk5-2), grouped into subgroup Vk5; and
   A10, A14, and A26, grouped into subgroup Vk6
   (Kawasaki et al. (Eur. J. Immunol. (2001) 31, 1017-1028); Schable and Zachau (Biol. Chem. Hoppe Seyler (1993) 374, 1001-1022); Brensing-Kuppers et al. (Gene (1997) 191, 173-181)).

Fully human Vλ sequences preferably include, but are not limited to, for example:

   V1-2, V1-3, V1-4, V1-5, V1-7, V1-9, V1-11, V1-13, V1-16, V1-17, V1-18, V1-19, V1-20, and V1-22, grouped into subgroup VL1;
   V2-1, V2-6, V2-7, V2-8, V2-11, V2-13, V2-14, V2-15, V2-17, and V2-19, grouped into subgroup VL1;
   V3-2, V3-3, and V3-4, grouped into subgroup VL3;
   V4-1, V4-2, V4-3, V4-4, and V4-6, grouped into subgroup VL4; and

V5-1, V5-2, V5-4, and V5-6, grouped into subgroup VL5 (Kawasaki et al. (Genome Res. (1997) 7, 250-261)).

Normally, these framework sequences are different from one another at one or more amino acid residues. These framework sequences can be used in combination with "at least one amino acid residue that alters the antigen-binding activity of an antigen-binding molecule depending on ion concentration conditions" as disclosed herein. Other examples of the fully human frameworks used in combination with "at least one amino acid residue that alters the antigen-binding activity of an antigen-binding molecule depending on ion concentration conditions" include, but are not limited to, for example, KOL, NEWM, REI, EU, TUR, TEI, LAY, and POM (for example, Kabat *et al.* (1991) *supra;* Wu et al. (J. Exp. Med. (1970) 132, 211-250)).

Without being bound by a particular theory, one reason for the expectation that the use of germ line sequences precludes adverse immune responses in most individuals is believed to be as follows. As a result of the process of affinity maturation during normal immune responses, somatic mutation occurs frequently in the variable regions of immunoglobulin. Such mutations mostly occur around CDRs whose sequences are hypervariable, but also affect residues of framework regions. Such framework mutations do not exist on the germ line genes, and also they are less likely to be immunogenic in patients. On the other hand, the normal human population is exposed to most of the framework sequences expressed from the germ line genes. As a result of immunotolerance, these germ line frameworks are expected to have low or no immunogenicity in patients. To maximize the possibility of immunotolerance, variable region-encoding genes may be selected from a group of commonly occurring functional germ line genes.

Known methods such as site-directed mutagenesis (Kunkel et al. (Proc. Natl. Acad. Sci. USA (1985) 82, 488-492)) and overlap extension PCR can be appropriately employed to produce the antigen-binding molecules as disclosed herein in which the above-described framework sequences contain amino acids that alter the antigen-binding activity of the antigen-binding molecules depending on calcium ion concentration conditions.

For example, a library which contains a plurality of antigen-binding molecules of the present disclosure whose sequences are different from one another can be constructed by combining heavy chain variable regions prepared as a randomized variable region sequence library with a light chain variable region selected as a framework sequence originally containing at least one amino acid residue that alters the antigen-binding activity of the antigen-binding molecule depending on calcium ion concentration conditions. As a non-limiting example, when the ion concentration is calcium ion concentration, such preferred libraries include, for example, those constructed by combining the light chain variable region sequence of SEQ ID NO: 2 (Vk5-2) and the heavy chain variable region produced as a randomized variable region sequence library.

Alternatively, a light chain variable region sequence selected as a framework region originally containing at least one amino acid residue that alters the antigen-binding activity of an antigen-binding molecule depending on ion concentration conditions as mentioned above can be designed to contain various amino acid residues other than the above amino acid residues. As used herein, such residues are referred to as flexible residues. The number and position of flexible residues are not particularly limited as long as the antigen-binding activity of the antigen-binding molecule varies depending on ion concentration conditions. Specifically, the CDR sequences and/or FR sequences of the heavy chain and/or light chain may contain one or more flexible residues. For example, when the ion concentration is calcium ion concentration, non-limiting examples of flexible residues to be introduced into the light chain variable region sequence of SEQ ID NO: 2 (Vk5-2) include the amino acid residues listed in Table 1 or 2.

[Table 1]

| CDR | Position | 70% of the total | | | | |
|---|---|---|---|---|---|---|
| | 28 | S ; 100% | | | | |
| | 29 | I ; 100% | | | | |
| | 30 | E ; 72% | H; 14% | S ; 14% | | |
| CDR1 | 31 | D; 100% | | | | |
| | 32 | D ; 100% | | | | |
| | 33 | L ; 100% | | | | |
| | 34 | A ; 70% | N ; 30% | | | |

(continued)

| CDR | Position | 70% of the total | | | | |
|---|---|---|---|---|---|---|
| CDR2 | 50 | E ; 100% | | | | |
| | 51 | A ; 100% | | | | |
| | 52 | S ; 100% | | | | |
| | 53 | H ; 5% | N ; 25% | S ; 45% | T ; 25% | |
| | 54 | L ; 100% | | | | |
| | 55 | Q ; 100% | | | | |
| | 56 | S ; 100% | | | | |
| CDR3 | 90 | Q ; 100% | | | | |
| | 91 | H ; 25% | S ; 15% | R; 15% | Y; 45% | |
| | 92 | D ; 80% | N ; 10% | S ; 10% | | |
| | 93 | D ; 5% | G ; 10% | N; 25% | S ; 50% | R; 10% |
| | 94 | S ; 50% | Y; 50% | | | |
| | 95 | P ; 100% | | | | |
| | 96 | L ; 50% | Y ; 50% | | | |

(Position indicates Kabat numbering)
When position 92 as indicated by Kabat numbering is Asn (N), position 94 may be Leu (L) rather than Ser (S).

[Table 2]

| CDR | Kabat numbering | 30% amino acids of the total | | | | |
|---|---|---|---|---|---|---|
| CDR1 | 28 | S : 100% | | | | |
| | 29 | I : 100% | | | | |
| | 30 | E : 83% | S : 17% | | | |
| | 31 | D: 100% | | | | |
| | 32 | D : 100% | | | | |
| | 33 | L : 100% | | | | |
| | 34 | A : 70% | N : 30% | | | |
| CDR2 | 50 | H : 100% | | | | |
| | 51 | A: 100% | | | | |
| | 52 | S : 100% | | | | |
| | 53 | H : 5% | N : 25% | S : 45% | T : 25% | |
| | 54 | L : 100% | | | | |
| | 55 | Q : 100% | | | | |
| | 56 | S : 100% | | | | |

(continued)

| CDR | Kabat numbering | 30% amino acids of the total | | | | |
|---|---|---|---|---|---|---|
| CDR3 | 90 | Q : 100% | | | | |
| | 91 | H : 25% | S : 15% | R : 15% | Y : 45% | |
| | 92 | D : 80% | N : 10% | S : 10% | | |
| | 93 | D : 5% | G : 10% | N : 25% | S : 50% | R : 10% |
| | 94 | S : 50% | Y : 50% | | | |
| | 95 | P : 100% | | | | |
| | 96 | L : 50% | Y : 50% | | | |
| (Position indicates Kabat numbering) When position 92 as indicated by Kabat numbering is Asn (N), position 94 may be Leu (L) rather than Ser (S). | | | | | | |

Herein, flexible residues refer to amino acid residue variations present at hypervariable positions at which several different amino acids are present on the light chain and heavy chain variable regions when the amino acid sequences of known and/or native antibodies or antigen-binding domains are compared. Hypervariable positions are generally located in the CDR regions. In an embodiment, the data provided by Kabat, Sequences of Proteins of Immunological Interest (National Institute of Health Bethesda Md.) (1987 and 1991) is useful to determine hypervariable positions in known and/or native antibodies. Furthermore, databases on the Internet (http://vbase.mrc-cpe.cam.ac.uk/, http://www.bioinf.org.uk/abs/index.html) provide the collected sequences of many human light chains and heavy chains and their locations. The information on the sequences and locations is useful to determine hypervariable positions. According to the present disclosure, when a certain amino acid position has preferably about 2 to about 20 possible amino acid residue variations, preferably about 3 to about 19, preferably about 4 to about 18, preferably 5 to 17, preferably 6 to 16, preferably 7 to 15, preferably 8 to 14, preferably 9 to 13, and preferably 10 to 12 possible amino acid residue variations, the position is hypervariable. In some embodiments, a certain amino acid position may have preferably at least about 2, preferably at least about 4, preferably at least about 6, preferably at least about 8, preferably about 10, and preferably about 12 amino acid residue variations.

Alternatively, a library containing a plurality of antigen-binding molecules as disclosed herein whose sequences are different from one another can be constructed by combining heavy chain variable regions produced as a randomized variable region sequence library with light chain variable regions into which at least one amino acid residue that alters the antigen-binding activity of antigen-binding molecules depending on ion concentration conditions as mentioned above is introduced. When the ion concentration is calcium ion concentration, non-limiting examples of such libraries preferably include, for example, libraries in which heavy chain variable regions produced as a randomized variable region sequence library are combined with light chain variable region sequences in which a particular residue(s) in a germ line sequence such as SEQ ID NO: 3 (Vk1), SEQ ID NO: 4 (Vk2), SEQ ID NO: 5 (Vk3), or SEQ ID NO: 6 (Vk4) has been substituted with at least one amino acid residue that alters the antigen-binding activity of an antigen-binding molecule depending on calcium ion concentration conditions. Non-limiting examples of such amino acid residues include amino acid residues in light chain CDR1. Furthermore, non-limiting examples of such amino acid residues include amino acid residues in light chain CDR2. In addition, non-limiting examples of such amino acid residues also include amino acid residues in light chain CDR3.

Non-limiting examples of such amino acid residues contained in light chain CDR1 include those at positions 30, 31, and/or 32 in the CDR1 of light chain variable region as indicated by EU numbering. Furthermore, non-limiting examples of such amino acid residues contained in light chain CDR2 include an amino acid residue at position 50 in the CDR2 of light chain variable region as indicated by Kabat numbering. Moreover, non-limiting examples of such amino acid residues contained in light chain CDR3 include an amino acid residue at position 92 in the CDR3 of light chain variable region as indicated by Kabat numbering. These amino acid residues can be contained alone or in combination as long as they form a calcium-binding motif and/or as long as the antigen-binding activity of an antigen-binding molecule varies depending on calcium ion concentration conditions. Meanwhile, as troponin C, calmodulin, parvalbumin, and myosin light chain, which have several calcium ion-binding sites and are believed to be derived from a common origin in terms of molecular evolution, are known, the light chain CDR1, CDR2, and/or CDR3 can be designed to have their binding motifs. For example, it is possible to use cadherin domains, EF hand of calmodulin, C2 domain of Protein kinase C, Gla domain of blood coagulation protein FactorIX, C type lectins of asialoglycoprotein receptor and mannose-binding receptor, A domains of LDL receptors, annexin, thrombospondin type 3 domain, and EGF-like domains in an appropriate manner for the above purposes.

When heavy chain variable regions produced as a randomized variable region sequence library and light chain variable regions into which at least one amino acid residue that alters the antigen-binding activity of an antigen-binding molecule depending on ion concentration conditions has been introduced are combined as described above, the sequences of the light chain variable regions can be designed to contain flexible residues in the same manner as described above. The number and position of such flexible residues are not particularly limited to particular embodiments as long as the antigen-binding activity of antigen-binding molecules as disclosed herein varies depending on ion concentration conditions. Specifically, the CDR sequences and/or FR sequences of heavy chain and/or light chain can contain one or more flexible residues. When the ion concentration is calcium ion concentration, non-limiting examples of flexible residues to be introduced into the sequence of light chain variable region include the amino acid residues listed in Tables 1 and 2.

The preferred heavy chain variable regions to be combined include, for example, randomized variable region libraries. Known methods are combined as appropriate to produce a randomized variable region library. In a non-limiting embodiment, an immune library constructed based on antibody genes derived from lymphocytes of animals immunized with a specific antigen, patients with infections, persons with an elevated antibody titer in blood as a result of vaccination, cancer patients, or auto immune disease patients, may be preferably used as a randomized variable region library.

In another non-limiting embodiment, a synthetic library produced by replacing the CDR sequences of V genes in genomic DNA or functional reshaped V genes with a set of synthetic oligonucleotides containing sequences encoding codon sets of an appropriate length can also be preferably used as a randomized variable region library. In this case, since sequence diversity is observed in the heavy chain CDR3 sequence, it is also possible to replace the CDR3 sequence only. A criterion of giving rise to diversity in amino acids in the variable region of an antigen-binding molecule is that diversity is given to amino acid residues at surface-exposed positions in the antigen-binding molecule. The surface-exposed position refers to a position that is considered to be able to be exposed on the surface and/or contacted with an antigen, based on structure, ensemble of structures, and/or modeled structure of an antigen-binding molecule. In general, such positions are CDRs. Preferably, surface-exposed positions are determined using coordinates from a three-dimensional model of an antigen-binding molecule using a computer program such as the InsightII program (Accelrys). Surface-exposed positions can be determined using algorithms known in the art (for example, Lee and Richards (J. Mol. Biol. (1971) 55, 379-400); Connolly (J. Appl. Cryst. (1983) 16, 548-558)). Determination of surface-exposed positions can be performed using software suitable for protein modeling and three-dimensional structural information obtained from an antibody. Software that can be used for these purposes preferably includes SYBYL Biopolymer Module software (Tripos Associates). Generally or preferably, when an algorithm requires a user input size parameter, the "size" of a probe which is used in the calculation is set at about 1.4 Angstrom or smaller in radius. Furthermore, methods for determining surface-exposed regions and areas using software for personal computers are described by Pacios (Comput. Chem. (1994) 18 (4), 377-386; J. Mol. Model. (1995) 1, 46-53).

In another non-limiting embodiment, a naive library, which is constructed from antibody genes derived from lymphocytes of healthy persons and whose repertoire consists of naive sequences, which are antibody sequences with no bias, can also be particularly preferably used as a randomized variable region library (Gejima et al. (Human Antibodies (2002) 11, 121-129); Cardoso et al. (Scand. J. Immunol. (2000) 51, 337-344)). Herein, an amino acid sequence comprising a naive sequence refers to an amino acid sequence obtained from such a naive library.

In one embodiment, an antigen-binding domain as disclosed herein can be obtained from a library containing a plurality of antigen-binding molecules whose sequences are different from one another, prepared by combining light chain variable regions constructed as a randomized variable region sequence library with a heavy chain variable region selected as a framework sequence that originally contains "at least one amino acid residue that alters the antigen-binding activity of an antigen-binding molecule depending on ion concentration conditions". When the ion concentration is calcium ion concentration, non-limiting examples of such libraries preferably include those constructed by combining light chain variable regions constructed as a randomized variable region sequence library with the sequence of heavy chain variable region of SEQ ID NO: 7 (6RL#9-IgG1) or SEQ ID NO: 8 (6KC4-1#85-IgG1). Alternatively, such a library can be constructed by selecting appropriate light chain variable regions from those having germ line sequences, instead of light chain variable regions constructed as a randomized variable region sequence library. Such preferred libraries include, for example, those in which the sequence of heavy chain variable region of SEQ ID NO: 7 (6RL#9-IgG1) or SEQ ID NO: 8 (6KC4-1#85-IgG1) is combined with light chain variable regions having germ line sequences.

Alternatively, the sequence of a heavy chain variable region selected as a framework sequence that originally contains "at least one amino acid residue that alters the antigen-binding activity of an antigen-binding molecule depending on ion concentration conditions" as mentioned above can be designed to contain flexible residues. The number and position of the flexible residues are not particularly limited as long as the antigen-binding activity of an antigen-binding molecule as disclosed herein varies depending on ion concentration conditions. Specifically, the CDR and/or FR sequences of heavy chain and/or light chain can contain one or more flexible residues. When the ion concentration is calcium ion concentration, non-limiting examples of flexible residues to be introduced into the sequence of heavy chain variable region of SEQ ID NO: 7 (6RL#9-IgG1) include all amino acid residues of heavy chain CDR1 and CDR2 and the amino acid residues of the heavy chain CDR3 except those at positions 95, 96, and/or 100a. Alternatively, non-limiting examples of flexible residues to be introduced into the sequence of heavy chain variable region of SEQ ID NO: 8 (6KC4-1#85-IgG1) include all amino acid residues of heavy chain CDR1 and CDR2 and the amino acid residues of the heavy chain CDR3 except those at amino acid positions 95 and/or 101.

Alternatively, a library containing a plurality of antigen-binding molecules whose sequences are different from one another can be constructed by combining light chain variable regions constructed as a randomized variable region sequence library or light chain variable regions having germ line sequences with heavy chain variable regions into which "at least one amino acid residue responsible for the ion concentration condition-dependent change in the antigen-binding activity of an antigen-binding molecule" has been introduced as mentioned above. When the ion concentration is calcium ion concentration, non-limiting examples of such libraries preferably include those in which light chain variable regions constructed as a randomized variable region sequence library or light chain variable regions having germ line sequences are combined with the sequence of a heavy chain variable region in which a particular residue(s) has been substituted with at least one amino acid residue that alters the antigen-binding activity of an antigen-binding molecule depending on calcium ion concentration conditions. Non-limiting examples of such amino acid residues include amino acid residues of the heavy chain CDR1. Further non-limiting examples of such amino acid residues include amino acid residues of the heavy chain CDR2. In addition, non-limiting examples of such amino acid residues also include amino acid residues of the heavy chain CDR3. Non-limiting examples of such amino acid residues of heavy chain CDR3 include the amino acids of positions 95, 96, 100a, and/or 101 in the CDR3 of heavy chain variable region as indicated by the Kabat numbering. Furthermore, these amino acid residues can be contained alone or in combination as long as they form a calcium-binding motif and/or the antigen-binding activity of an antigen-binding molecule varies depending on calcium ion concentration conditions.

When light chain variable regions constructed as a randomized variable region sequence library or light chain variable regions having germ line sequence are combined with a heavy chain variable region into which at least one amino acid residue that alter the antigen-binding activity of an antigen-binding molecule depending on ion concentration conditions as mentioned above has been introduced, the sequence of the heavy chain variable region can also be designed to contain flexible residues in the same manner as described above. The number and position of flexible residues are not particularly limited as long as the antigen-binding activity of an antigen-binding molecule as disclosed herein varies depending on ion concentration conditions. Specifically, the heavy chain CDR and/or FR sequences may contain one or more flexible residues. Furthermore, randomized variable region libraries can be preferably used as amino acid sequences of CDR1, CDR2, and/or CDR3 of the heavy chain variable region other than the amino acid residues that alter the antigen-binding activity of an antigen-binding molecule depending on ion concentration condition. When germ line sequences are used as light chain variable regions, non-limiting examples of such sequences include those of SEQ ID NO: 3 (Vk1), SEQ ID NO: 4 (Vk2), SEQ ID NO: 5 (Vk3), and SEQ ID NO: 6 (Vk4).

Any of the above-described amino acids that alter the antigen-binding activity of an antigen-binding molecule depending on calcium ion concentration conditions can be preferably used, as long as they form a calcium-binding motif. Specifically, such amino acids include electron-donating amino acids. Preferred examples of such electron-donating amino acids include serine, threonine, asparagine, glutamic acid, aspartic acid, and glutamic acid.

Condition of proton concentrations

In an embodiment, the condition of ion concentrations refers to the condition of proton concentrations or pH condition. As used herein, the concentration condition of proton, *i.e.,* the nucleus of hydrogen atom, is treated as synonymous with condition of hydrogen index (pH). When the activity of proton in an aqueous solution is represented as $aH+$, pH is defined as $-\log_{10} aH+$. When the ionic strength of the aqueous solution is low (for example, lower than $10^{-3}$), $aH+$ is nearly equal to the proton strength. For example, the ionic product of water at 25°C and 1 atmosphere is $Kw = aH+ \; aOH = 10^{-14}$, and therefore in pure water, $aH+ = aOH = 10^{-7}$. In this case, pH = 7 is neutral; an aqueous solution whose pH is lower than 7 is acidic or whose pH is greater than 7 is alkaline.

When pH condition is used as the ion concentration condition, pH conditions include condition of high proton concentrations or low pHs, *i.e.,* an acidic pH range condition, and condition of low proton concentrations or high pHs, *i.e.,* a neutral pH range condition. "The binding activity varies depending on pH condition" means that the antigen-binding activity of an antigen-binding molecule varies due to the difference in conditions of a high proton concentration or low pH (an acidic pH range) and a low proton concentration or high pH (a neutral pH range). This includes, for example, the case where the antigen-binding activity of an antigen-binding molecule is higher at a neutral pH range condition than at an acidic pH range condition and the case where the antigen-binding activity of an antigen-binding molecule is higher at an acidic pH range condition than at a neutral pH range condition.

Herein, neutral pH range is not limited to a specific value and is preferably selected from between pH 6.7 and pH 10.0. In another embodiment, the pH can be selected from between pH 6.7 and pH 9.5. In still another embodiment, the pH can be selected from between pH 7.0 and pH 9.0. In yet another embodiment, the pH can be selected from between pH 7.0 and pH 8.0. In particular, the preferred pH includes the extracellular pH *in vivo,* especially pH 7.4, which is close to the pH of plasma (blood).

Herein, an acidic pH range is not limited to a specific value and is preferably selected from between pH 4.0 and pH 6.5. In another embodiment, the pH can be selected from between pH 4.5 and pH 6.5. In still another embodiment, the pH can be selected from between pH 5.0 and pH 6.5. In yet another embodiment, the pH can be selected from between pH 5.5 and pH 6.5. In particular, the preferred pH includes the extracellular pH *in vivo,* especially pH 5.8, which is close to the pH in the early endosome *in vivo.*

As used herein, "the antigen-binding activity of an antigen-binding molecule at a high proton concentration or low pH (an acidic pH range) condition is lower than that at a low proton concentration or high pH (a neutral pH range) condition" means that the antigen-binding activity of an antigen-binding molecule at a pH selected from between pH 4.0 and pH 6.5 is weaker than that at a pH selected from between pH 6.7 and pH 10.0; preferably means that the antigen-binding activity of an antigen-binding molecule at a pH selected from between pH 4.5 and pH 6.5 is weaker than that at a pH selected from between pH 6.7 and pH 9.5; more preferably, means that the antigen-binding activity of an antigen-binding molecule at a pH selected from between pH 5.0 and pH 6.5 is weaker than that at a pH selected from between pH 7.0 and pH 9.0; still more preferably means that the antigen-binding activity of an antigen-binding molecule at a pH selected from between pH 5.5 and pH 6.5 is weaker than that at a pH selected from between pH 7.0 and pH 8.0; particularly preferably means that the antigen-binding activity at the pH in the early endosome *in vivo* is weaker than the antigen-binding activity at the pH of plasma *in vivo;* and specifically means that the antigen-binding activity of an antigen-binding molecule at pH 5.8 is weaker than the antigen-binding activity at pH 7.4.

Whether the antigen-binding activity of an antigen-binding molecule has changed by the pH condition can be determined, for example, by the use of known measurement methods such as those described in the section "Binding Activity" above. Specifically, the binding activity is measured under different pH conditions using the measurement methods described above. For example, the antigen-binding activity of an antigen-binding molecule is compared under the conditions of acidic pH range and neutral pH range to confirm that the antigen-binding activity of the antigen-binding molecule changes to be higher under the condition of neutral pH range than that under the condition of acidic pH range.

Furthermore, the expression "the antigen-binding activity at a condition of high proton concentration or low pH, *i.e.,* in an acidic pH range condition, is lower than that at a condition of low proton concentration or high pH, *i.e.,* in a neutral pH range condition" can also be expressed as "the antigen-binding activity of an antigen-binding molecule at a condition of low proton concentration or high pH, *i.e.,* in a neutral pH range condition, is higher than that at a condition of high proton concentration or low pH, *i.e.,* in an acidic pH range condition". As used herein, "the antigen-binding activity at a condition of high proton concentration or low pH, *i.e.,* in an acidic pH range condition, is lower than that at a condition of low proton concentration or high pH, *i.e.,* in a neutral pH range condition" may be described as "the antigen-binding activity at a condition of high proton concentration or low pH, *i.e.,* in an acidic pH range condition, is weaker than the antigen-binding ability at a condition of low proton concentration or high pH, *i.e.,* in a neutral pH range condition". Alternatively, "the antigen-binding activity at a condition of high proton concentration or low pH, *i.e.,* in an acidic pH range condition, is reduced to be lower than that at a condition of low proton concentration or high pH, *i.e.,* in a neutral pH range condition" may be described as "the antigen-binding activity at a condition of high proton concentration or low pH, *i.e.,* in an acidic pH range condition, is reduced to be weaker than the antigen-binding ability at a condition of low proton concentration or high pH, *i.e.,* in a neutral pH range condition".

The conditions other than proton concentration or pH for measuring the antigen-binding activity may be suitably selected by those skilled in the art and are not particularly limited. Measurements can be carried out, for example, at 37°C using HEPES buffer. Measurements can be carried out, for example, using Biacore (GE Healthcare). When the antigen is a soluble antigen, the antigen-binding activity of an antigen-binding molecule can be determined by assessing the binding activity to the soluble antigen by pouring the antigen as an analyte into a chip immobilized with the antigen-binding molecule. When the antigen is a membrane antigen, the binding activity to the membrane antigen can be assessed by pouring the antigen-binding molecule as an analyte into a chip immobilized with the antigen.

As long as the antigen-binding activity of an antigen-binding molecule as disclosed herein at a condition of high proton concentration or low pH, *i.e.,* in an acidic pH range condition is weaker than that at a condition of low proton concentration or high pH, *i.e.,* in a neutral pH range condition, the ratio of the antigen-binding activity between that under a condition of high proton concentration or low pH, *i.e.,* under an acidic pH range condition, and under a condition of low proton concentration or high pH, *i.e.,* under a neutral pH range condition, is not particularly limited, and the value of KD (pH 5.8) / KD (pH 7.4), which is the ratio of the dissociation constant (KD) for an antigen at a condition of high proton concentration or low pH, *i.e.,* in an acidic pH range condition to the KD at a condition of low proton concentration or high pH, *i.e.,* in a neutral pH range condition, is preferably 2 or more; more preferably the value of KD (pH 5.8) / KD (pH 7.4) is 10 or more; and still more preferably the value of KD (pH 5.8) / KD (pH 7.4) is 40 or more. The upper limit of KD (pH 5.8) / KD (pH 7.4) value is not particularly limited, and may be any value such as 400, 1000, or 10000, as long as the molecule can be produced by the techniques of those skilled in the art.

When the antigen is a soluble antigen, the dissociation constant (KD) can be used as the value for antigen-binding activity. Meanwhile, when the antigen is a membrane antigen, the apparent dissociation constant (KD) can be used. The dissociation constant (KD) and apparent dissociation constant (KD) can be measured by methods known to those skilled in the art, and Biacore (GE healthcare), Scatchard plot, flow cytometer, and such can be used.

Alternatively, for example, the dissociation rate constant (kd) can be suitably used as an index for indicating the ratio of the antigen-binding activity of an antigen-binding molecule as disclosed herein between that at a condition of high proton concentration or low pH, *i.e.,* an acidic pH range and a condition of low proton concentration or high pH, *i.e.,* a neutral pH range. When kd (dissociation rate constant) is used as an index for indicating the binding activity ratio instead of KD (dissociation constant), the value of kd (in an acidic pH range) / kd (in a neutral pH range), which is the ratio of kd (dissociation rate constant) for the antigen at a condition of high proton concentration or low pH, *i.e.,* in an acidic pH range to kd (dissociation rate constant) at a condition of low proton concentration or high pH, *i.e.,* in a neutral pH range, is preferably 2 or more, more preferably 5 or more, still more preferably 10 or more, and yet more preferably 30 or more. The upper limit of kd (in an acidic pH range condition) / kd (in a neutral pH range condition) value is not particularly limited, and may be any value such as 50, 100, or 200, as long as the molecule can be produced by the techniques of those skilled in the art.

When the antigen is a soluble antigen, the dissociation rate constant (kd) can be used as the value for antigen-binding activity and when the antigen is a membrane antigen, the apparent dissociation rate constant (kd) can be used. The dissociation rate constant (kd) and apparent dissociation rate constant (kd) can be determined by methods known to those skilled in the art, and Biacore (GE healthcare), flow cytometer, and such may be used. When the antigen-binding activity of an antigen-binding molecule is measured at different proton concentrations, *i.e.,* pHs, conditions other than the proton concentration, *i.e.,* pH, are preferably the same.

For example, an antigen-binding domain (or antigen-binding molecule) whose antigen-binding activity at a condition of high proton concentration or low pH, *i.e.,* in an acidic pH range condition is lower than that at a condition of low proton concentration or high pH, *i.e.,* in a neutral pH range condition, can be obtained *via* screening of antigen-binding domains (or antigen-binding molecules), comprising the following steps (a) to (c):

(a) obtaining the antigen-binding activity of an antigen-binding domain (or antigen-binding molecule) in an acidic pH range condition;
(b) obtaining the antigen-binding activity of an antigen-binding domain (or antigen-binding molecule) in a neutral pH range condition; and
(c) selecting an antigen-binding domain (or antigen-binding molecule) whose antigen-binding activity in the acidic pH range condition is lower than that in the neutral pH range condition.

Alternatively, an antigen-binding domain (or antigen-binding molecule) whose antigen-binding activity at a condition of high proton concentration or low pH, *i.e.,* in an acidic pH range condition, is lower than that at a condition of low proton concentration or high pH, *i.e.,* in a neutral pH range condition, can be obtained *via* screening of antigen-binding domains (or antigen-binding molecules), or a library thereof, comprising the following steps (a) to (c):

(a) contacting an antigen-binding domain (or antigen-binding molecule), or a library thereof, in a neutral pH range condition with an antigen;
(b) placing in an acidic pH range condition the antigen-binding domain (or antigen-binding molecule) bound to the antigen in step (a); and
(c) isolating the antigen-binding domain (or antigen-binding molecule) dissociated in step (b).

An antigen-binding domain (or antigen-binding molecule) whose antigen-binding activity at a condition of high proton concentration or low pH, *i.e.,* in an acidic pH range condition is lower than that at a condition of low proton concentration or high pH, *i.e.,* in a neutral pH range condition, can be obtained *via* screening of antigen-binding domains (or antigen-binding molecules), or a library thereof, comprising the following steps (a) to (d):

(a) contacting in an acidic pH range an antigen with a library of antigen-binding domains (or antigen-binding molecules);
(b) selecting the antigen-binding domain (or antigen-binding molecule) which does not bind to the antigen in step (a);
(c) allowing the antigen-binding domain (or antigen-binding molecule) selected in step (b) to bind with the antigen in a neutral pH range; and
(d) isolating the antigen-binding domain (or antigen-binding molecule) bound to the antigen in step (c).

An antigen-binding domain (or antigen-binding molecule) whose antigen-binding activity at a condition of high proton concentration or low pH, *i.e.,* in an acidic pH range condition, is lower than that at a condition of low proton concentration or high pH, *i.e.,* in a neutral pH range condition, can be obtained by a screening method comprising the following steps (a) to (c):

(a) contacting in a neutral pH range condition a library of antigen-binding domains (or antigen-binding molecules) with a column immobilized with an antigen;
(b) eluting in an acidic pH range condition from the column the antigen-binding domain (or antigen-binding molecule) bound to the column in step (a); and
(c) isolating the antigen-binding domain (or antigen-binding molecule) eluted in step (b).

An antigen-binding domain (or antigen-binding molecule) whose antigen-binding activity at a condition of high proton concentration or low pH, *i.e.,* in an acidic pH condition, range is lower than that at a condition of low proton concentration or high pH, *i.e.,* in a neutral pH range condition, can be obtained by a screening method comprising the following steps (a) to (d):

(a) allowing, in an acidic pH range condition, a library of antigen-binding domains (or antigen-binding molecules) to pass a column immobilized with an antigen;
(b) collecting the antigen-binding domain (or antigen-binding molecule) eluted without binding to the column in step (a);
(c) allowing the antigen-binding domain (or antigen-binding molecule) collected in step (b) to bind with the antigen in a neutral pH range condition; and
(d) isolating the antigen-binding domain (or antigen-binding molecule) bound to the antigen in step (c).

An antigen-binding domain (or antigen-binding molecule) whose antigen-binding activity at a condition of high proton concentration or low pH, *i.e.,* in an acidic pH range condition, is lower than that at a condition of low proton concentration or high pH, *i.e.,* in a neutral pH range condition, can be obtained by a screening method comprising the following steps (a) to (d):

(a) contacting an antigen with a library of antigen-binding domains (or antigen-binding molecules) in a neutral pH range condition;
(b) obtaining the antigen-binding domain (or antigen-binding molecule) bound to the antigen in step (a);
(c) placing in an acidic pH range condition the antigen-binding domain (or antigen-binding molecule) obtained in step (b); and
(d) isolating the antigen-binding domain (or antigen-binding molecule) whose antigen-binding activity in step (c) is weaker than the standard selected in step (b).

The above-described steps may be repeated twice or more times. Thus, the present disclosure provides antigen-binding domains (or antigen-binding molecules) whose antigen-binding activity in an acidic pH range condition is lower than that in a neutral pH range condition, which are obtained by a screening method that further comprises the steps of repeating twice or more times steps (a) to (c) or (a) to (d) in the above-described screening methods. The number of times that steps (a) to (c) or (a) to (d) is repeated is not particularly limited; however, the number is 10 or less in general.

In the screening methods disclosed herein, the antigen-binding activity of an antigen-binding domain (or antigen-binding molecule) at a condition of high proton concentration or low pH, *i.e.,* in an acidic pH range, is not particularly limited, as long as it is the antigen-binding activity at a pH of between 4.0 and 6.5, and includes the antigen-binding activity at a pH of between 4.5 and 6.6 as the preferred pH. The antigen-binding activity also includes that at a pH of between 5.0 and 6.5, and that at a pH of between 5.5 and 6.5 as another preferred pH. The antigen-binding activity also includes that at the pH in the early endosome *in vivo* as the more preferred pH, and specifically, that at pH 5.8. Meanwhile, the antigen-binding activity of an antigen-binding domain (or antigen-binding molecule) at a condition of low proton concentration or high pH, *i.e.,* in a neutral pH range, is not particularly limited, as long as it is the antigen-binding activity at a pH of between 6.7 and 10, and includes the antigen-binding activity at a pH of between 6.7 and 9.5 as the preferred pH. The antigen-binding activity also includes that at a pH of between 7.0 and 9.5 and that at a pH of between 7.0 and 8.0 as another preferred pH. The antigen-binding activity also includes that at the pH of plasma *in vivo* as the more preferred pH, and specifically, that at pH 7.4.

The antigen-binding activity of an antigen-binding domain (or antigen-binding molecule) can be measured by methods known to those skilled in the art. Those skilled in the art can suitably determine conditions other than ionized calcium concentration. The antigen-binding activity of an antigen-binding domain (or antigen-binding molecule) can be assessed based on the dissociation constant (KD), apparent dissociation constant (KD), dissociation rate constant (kd), apparent dissociation rate constant (kd), and such. These can be determined by methods known to those skilled in the art, for example, using Biacore (GE healthcare), Scatchard plot, or FACS.

The step of selecting an antigen-binding domain (or antigen-binding molecule) whose antigen-binding activity at a condition of low proton concentration or high pH, *i.e.,* in a neutral pH range condition, is higher than that at a condition of high proton concentration or low pH, *i.e.,* in an acidic pH range condition, is synonymous with the step of selecting an antigen-binding domain (or antigen-binding molecule) whose antigen-binding activity at a condition of high proton concentration or low pH, *i.e.,* in an acidic pH range condition, is lower than that at a condition of low proton concentration or high pH, *i.e.,* in a neutral pH range condition.

As long as the antigen-binding activity at a condition of low proton concentration or high pH, *i.e.,* in a neutral pH range condition, is higher than that at a condition of high proton concentration or low pH, *i.e.,* in an acidic pH range condition, the difference between the antigen-binding activity at a condition of low proton concentration or high pH, *i.e.,* a neutral pH range condition, and that at a condition of high proton concentration or low pH, *i.e.,* an acidic pH range condition, is not particularly limited; however, the antigen-binding activity at a condition of low proton concentration or high pH, *i.e.,* in a neutral pH range condition, is preferably twice or more, more preferably 10 times or more, and still more preferably 40 times or more than that at a condition of high proton concentration or low pH, *i.e.,* in an acidic pH range condition.

The antigen binding domain (or antigen-binding molecule) as disclosed herein that is screened by the screening methods described above may be any antigen-binding domain (or antigen-binding molecule), and for example, an above-mentioned antigen-binding domain (or antigen-binding molecule) may be screened. For example, antigen-binding domains (or antigen-binding molecules) having a native sequence may be screened, and antigen-binding domains (or antigen-binding molecules) in which their amino acid sequences have been substituted may also be screened.

The antigen-binding domain (or antigen-binding molecule) as disclosed herein to be screened by the above-described screening methods may be prepared in any manner. For example, preexisting antibodies, preexisting libraries (phage library, etc.), antibodies or libraries prepared from B cells of immunized animals or from hybridomas obtained by immunizing animals, antibodies or libraries (libraries with increased content of amino acids with a side chain pKa of 4.0-8.0 (for example, histidine and glutamic acid) or unnatural amino acids, libraries introduced with amino acids with a side chain pKa of 4.0-8.0 (for example, histidine and glutamic acid) or unnatural amino acid mutations at specific positions, etc.) obtained by introducing amino acids with a side chain pKa of 4.0-8.0 (for example, histidine and glutamic acid) or unnatural amino acid mutations into the above-described antibodies or libraries may be used.

Methods for obtaining an antigen-binding domain (or antigen-binding molecule) whose antigen-binding activity at a condition of low proton concentration or high pH, *i.e.,* in a neutral pH range condition, is higher than that at a condition of high proton concentration or low pH, *i.e.,* in an acidic pH range condition, from an antigen-binding domains (or antigen-binding molecules) prepared from hybridomas obtained by immunizing animals or from B cells of immunized animals preferably include, for example, the antigen-binding molecule or antibody in which at least one of the amino acids of the antigen-binding domain or antibody is substituted with an amino acid with a side chain pKa of 4.0-8.0 (for example, histidine and glutamic acid) or an unnatural amino acid mutation, or the antigen-binding domain or antibody inserted with an amino acid with a side chain pKa of 4.0-8.0 (for example, histidine and glutamic acid) or unnatural amino acid, such as those described in International Publication No. WO 2009/125825.

The sites of introducing mutations of amino acids with a side chain pKa of 4.0-8.0 (for example, histidine and glutamic acid) or unnatural amino acids are not particularly limited, and may be any position as long as the antigen-binding activity in an acidic pH range becomes weaker than that in a neutral pH range (the value of KD (in an acidic pH range) / KD (in a neutral pH range) or kd (in an acidic pH range) / kd (in a neutral pH range) is increased) as compared to before substitution or insertion. For example, when the antigen-binding molecule is an antibody, antibody variable region and CDRs are suitable. Those skilled in the art can appropriately determine the number of amino acids to be substituted with or the number of amino acids with a side chain pKa of 4.0-8.0 (for example, histidine and glutamic acid) or unnatural amino acids to be inserted. It is possible to substitute with a single amino acid having a side chain pKa of 4.0-8.0 (for example, histidine and glutamic acid) or a single unnatural amino acid; it is possible to insert a single amino acid having a side chain pKa of 4.0-8.0 (for example, histidine and glutamic acid) or a single unnatural amino acid; it is possible to substitute with two or more amino acids having a side chain pKa of 4.0-8.0 (for example, histidine and glutamic acid) or two or more unnatural amino acids; and it is possible to insert two or more amino acids having a side chain pKa of 4.0-8.0 (for example, histidine and glutamic acid) or two or more unnatural amino acids. Alternatively, other amino acids can be deleted, added, inserted, and/or substituted concomitantly, aside from the substitution into amino acids having a side chain pKa of 4.0-8.0 (for example, histidine and glutamic acid) or unnatural amino acids, or the insertion of amino acids having a side chain pKa of 4.0-8.0 (for example, histidine and glutamic acid) or unnatural amino acids. Substitution into or insertion of amino acids with a side chain pKa of 4.0-8.0 (for example, histidine and glutamic acid) or unnatural amino acids can performed randomly by methods such as histidine scanning, in which the alanine of alanine scanning known to those skilled in the art is replaced with histidine. Antigen-binding molecules exhibiting a greater value of KD (in an acidic pH range) / KD (in a neutral pH range) or kd (in an acidic pH range) / kd (in a neutral pH range) as compared to before the mutation can be selected from antigen-binding domains or antibodies introduced with random insertions or substitution mutations of amino acids with a side chain pKa of 4.0-8.0 (for example, histidine and glutamic acid) or unnatural amino acids.

Preferred examples of antigen-binding molecules containing the mutation into amino acids with a side chain pKa of 4.0-8.0 (for example, histidine and glutamic acid) or unnatural amino acids as described above and whose antigen-binding activity in an acidic pH range is lower than that in a neutral pH range include, antigen-binding molecules whose antigen-binding activity in the neutral pH range after the mutation into amino acids with a side chain pKa of 4.0-8.0 (for example, histidine and glutamic acid) or unnatural amino acids is comparable to that before the mutation into amino acids with a side chain pKa of 4.0-8.0 (for example, histidine and glutamic acid) or unnatural amino acids. Herein, "an antigen-binding molecule after the mutation with amino acids having a side chain pKa of 4.0-8.0 (for example, histidine and glutamic acid) or unnatural amino acids has an antigen-binding activity comparable to that before the mutation with amino acids having a side chain pKa of 4.0-8.0 (for example, histidine and glutamic acid) or unnatural amino acids" means that, when taking the antigen-binding activity of an antigen-binding molecule before the mutation with amino acids having a side chain pKa of 4.0-8.0 (for example, histidine and glutamic acid) or unnatural amino acids as 100%, the antigen-binding activity of an antigen-binding molecule after the mutation with amino acids having a side chain pKa of 4.0-8.0 (for example, histidine and glutamic acid) or unnatural amino acids is at least 10% or more, preferably 50% or more, more preferably 80% or more, and still more preferably 90% or more. The antigen-binding activity after the mutation of amino acids with a side chain pKa of 4.0-8.0 (for example, histidine and glutamic acid) or unnatural amino acids at pH 7.4 may be higher than that before the mutation of amino acids with a side chain pKa of 4.0-8.0 (for example, histidine and glutamic acid) or unnatural amino acids at pH 7.4. If the antigen-binding activity of an antigen-binding molecule is decreased due to insertion of or substitution into amino acids with a side chain pKa of 4.0-8.0 (for example, histidine and glutamic acid) or unnatural amino acids, the antigen-binding activity can be made to be comparable to that before the insertion of or substitution into amino acids with a side chain pKa of 4.0-8.0 (for example, histidine and glutamic acid) or unnatural amino acids, by introducing a substitution, deletion, addition, and/or insertion of one or more amino acids of the antigen-binding molecule. The present disclosure also includes antigen-binding molecules whose binding activity has been adjusted to be comparable by substitution, deletion, addition, and/or insertion of one or more amino acids after substitution or insertion of amino acids with a side chain pKa of 4.0-8.0 (for example, histidine and glutamic acid) or unnatural amino acids.

Amino acids that alter the antigen-binding activity of antigen-binding domain depending on the proton concentration conditions

Antigen-binding domains (or antigen-binding molecules) as disclosed herein to be screened by the above-described screening methods may be prepared in any manner. For example, when ion concentration condition is proton concentration condition or pH condition, preexisting antibodies, preexisting libraries (phage library, etc.), antibodies or libraries prepared from B cells of immunized animals or from hybridomas obtained by immunizing animals, antibodies or libraries (libraries with increased content of amino acids with a side chain pKa of 4.0-8.0 (for example, histidine and glutamic acid) or unnatural amino acids, libraries introduced with mutations of amino acids with a side chain pKa of 4.0-8.0 (for example, histidine and glutamic acid) or unnatural amino acids at specific positions, etc.) obtained by introducing mutations of amino acids with a side chain pKa of 4.0-8.0 (for example, histidine and glutamic acid) or unnatural amino acids into the above-described antibodies or libraries may be used.

In one embodiment, a library containing multiple antigen-binding molecules as disclosed herein whose sequences are different from one another can also be constructed by combining heavy chain variable regions, produced as a randomized variable region sequence library, with light chain variable regions introduced with "at least one amino acid residue that changes the antigen-binding activity of an antigen-binding molecule depending on the proton concentration condition".

Such amino acid residues include, but are not limited to, for example, amino acid residues contained in the light chain CDR1. The amino acid residues also include, but are not limited to, for example, amino acid residues contained in the light chain CDR2. The amino acid residues also include, but are not limited to, for example, amino acid residues contained in the light chain CDR3.

The above-described amino acid residues contained in the light chain CDR1 include, but are not limited to, for example, amino acid residues of positions 24, 27, 28, 31, 32, and/or 34 according to Kabat numbering in the CDR1 of light chain variable region. Meanwhile, the amino acid residues contained in the light chain CDR2 include, but are not limited to, for example, amino acid residues of positions 50, 51, 52, 53, 54, 55, and/or 56 according to Kabat numbering in the CDR2 of light chain variable region. Furthermore, the amino acid residues in the light chain CDR3 include, but are not limited to, for example, amino acid residues of positions 89, 90, 91, 92, 93, 94, and/or 95A according to Kabat numbering in the CDR3 of light chain variable region. Moreover, the amino acid residues can be contained alone or can be contained in combination of two or more amino acids as long as they allow the change in the antigen-binding activity of an antigen-binding molecule depending on the proton concentration condition.

Even when the heavy chain variable region produced as a randomized variable region sequence library is combined with the above-described light chain variable region introduced with "at least one amino acid residue that changes the antigen-binding activity of an antigen-binding molecule depending on the proton concentration condition", it is possible to design so that the flexible residues are contained in the sequence of the light chain variable region in the same manner as described above. The number and position of the flexible residues are not particularly limited to a specific embodiment, as long as the antigen-binding activity of an antigen-binding molecule as disclosed herein changes depending on the proton concentration condition. Specifically, the CDR and/or FR sequences of heavy chain and/or light chain can contain one or more flexible residues. For example, flexible residues to be introduced into the sequences of the light chain variable regions include, but are not limited to, for example, the amino acid residues listed in Tables 3 and 4. Meanwhile, amino acid sequences of light chain variable regions other than the flexible residues and amino acid residues that change the antigen-binding activity of an antigen-binding molecule depending on the proton concentration condition suitably include, but are not limited to, germ line sequences such as Vk1 (SEQ ID NO: 3), Vk2 (SEQ ID NO: 4), Vk3 (SEQ ID NO: 5), and Vk4 (SEQ ID NO: 6).

[Table 3]

| Position | Amino acid | | | | | | | | |
|---|---|---|---|---|---|---|---|---|---|
| **CDR1** | | | | | | | | | |
| 28 | S:100% | | | | | | | | |
| 29 | 1:100% | | | | | | | | |
| 30 | N:25% | S:25% | R:25% | H:25% | | | | | |
| 31 | S:100% | | | | | | | | |
| 32 | H:100% | | | | | | | | |
| 33 | L:100% | | | | | | | | |
| 34 | A:50% | N:50% | | | | | | | |
| CDR2 | | | | | | | | | |
| 50 | H:100% | | | | or | A:25% | D:25% | G:25% | K:25% |
| 51 | A:100% | | | | | A:100% | | | |
| 52 | S:100% | | | | | S:100% | | | |
| 53 | K:33.3% | N:33.3 % | S:33.3 % | | | H:100% | | | |
| 54 | L:100% | | | | | **L:** 100% | | | |
| 55 | Q:100% | | | | | Q:100% | | | |
| 56 | S:100% | | | | | S:100% | | | |
| CDR3 | | | | | | | | | |
| 90 | Q:100% | | | | or | Q:100% | | | |
| 91 | H:100% | | | | | S:33.3% | R:33.3 % | Y:33.3 % | |
| 92 | G:25% | N:25% | S:25% | Y:25% | | H:100% | | | |
| 93 | H:33.3% | N:33.3 % | S:33.3 % | | | H:33. 3% | N:33.3 % | S:33.3 % | |
| 94 | S:50% | Y:50% | | | | S:50% | Y:50% | | |
| 95 | P:100% | | | | | P:100% | | | |
| 96 | L:50% | Y:50% | | | | L:50% | Y:50% | | |
| (Position indicates Kabat numbering) When position 92 as indicated by Kabat numbering is Asn (N), Ser (S) at position 94 may be excluded. | | | | | | | | | |

[Table 4]

| CDR | Position | Amino acid | | | | |
|---|---|---|---|---|---|---|
| CDR1 | 28 | S:100% | | | | |
| | 29 | I:100% | | | | |
| | 30 | H:30% | N:10% | S:50% | R:10% | |
| | 31 | N:35% | S:65% | | | |
| | 32 | H:40% | N:20% | Y:40% | | |
| | 33 | L:100% | | | | |
| | 34 | A: 70% | N:30% | | | |
| CDR2 | 50 | A:25% | D:15% | G:25% | H: 30% | K:5% |
| | 51 | A: 100% | | | | |
| | 52 | S:100% | | | | |
| | 53 | H:30% | K:10% | N:15% | S:45% | |
| | 54 | L:100% | | | | |
| | 55 | Q:100% | | | | |
| | 56 | S:100% | | | | |
| CDR3 | 90 | Q:100% | | | | |
| | 91 | H:30% | S:15% | R:10% | Y:45% | |
| | 92 | G:20% | H:30% | N:20% | S:15% | Y:15% |
| | 93 | H: 30% | N:25% | S:45% | | |
| | 94 | S:50% | Y:50% | | | |
| | 95 | P:100% | | | | |
| | 96 | L:50% | Y:50% | | | |
| (Position indicates Kabat numbering) When position 92 as indicated by Kabat numbering is Asn (N), Ser (S) at position 94 may be excluded. | | | | | | |

Any amino acid residue may be suitably used as the above-described amino acid residues that change the antigen-binding activity of an antigen-binding molecule depending on the proton concentration condition. Specifically, such amino acid residues include amino acids with a side chain pKa of 4.0-8.0. Such electron-releasing amino acids preferably include, for example, naturally occurring amino acids such as histidine and glutamic acid, as well as unnatural amino acids such as histidine analogs (US2009/0035836), m-NO2-Tyr (pKa 7.45), 3,5-Br2-Tyr (pKa 7.21), and 3,5-I2-Tyr (pKa 7.38) (Bioorg. Med. Chem. (2003) 11 (17), 3761-3768). Particularly preferred amino acid residues include, for example, amino acids with a side chain pKa of 6.0-7.0. Such electron-releasing amino acid residues preferably include, for example, histidine.

Known methods such as site-directed mutagenesis (Kunkel et al. (Proc. Natl. Acad. Sci. USA (1985) 82, 488-492)) and Overlap extension PCR can be appropriately employed to alter the amino acids of antigen-binding domains. Furthermore, various known methods can also be used as an amino acid alteration method for substituting amino acids by those other than natural amino acids (Annu. Rev. Biophys. Biomol. Struct. (2006) 35: 225-249; Proc. Natl. Acad. Sci. U.S.A. (2003) 100(11): 6353-6357). For example, a cell-free translation system (Clover Direct (Protein Express)) containing tRNAs in which amber suppressor tRNA, which is complementary to UAG codon (amber codon) that is a stop codon, is linked with an unnatural amino acid may be suitably used.

The preferred heavy chain variable region that is used in combination includes, for example, randomized variable region libraries. Known methods are appropriately combined as a method for producing a randomized variable region library. In a non-limiting embodiment, an immune library constructed based on antibody genes derived from animals immunized with specific antigens, patients with infection or persons with an elevated antibody titer in blood as a result of vaccination, cancer patients, or lymphocytes of auto immune diseases may be suitably used as a randomized variable region library.

In another non-limiting embodiment, in the same manner as described above, a synthetic library in which the CDR sequences of V genes from genomic DNA or functional reconstructed V genes are replaced with a set of synthetic oligonucleotides containing the sequences encoding codon sets of an appropriate length can also be suitably used as a randomized variable region library. In this case, the CDR3 sequence alone may be replaced because variety in the gene sequence of heavy chain CDR3 is observed. The basis for giving rise to amino acid variations in the variable region of an antigen-binding molecule is to generate variations of amino acid residues of surface-exposed positions of the antigen-binding molecule. The surface-exposed position refers to a position where an amino acid is exposed on the surface and/or contacted with an antigen based on the conformation, structural ensemble, and/or modeled structure of an antigen-binding molecule, and in general, such positions are the CDRs. The surface-exposed positions are preferably determined using the coordinates derived from a three-dimensional model of the antigen-binding molecule using computer programs such as InsightII program (Accelrys). The surface-exposed positions can be determined using algorithms known in the art (for example, Lee and Richards (J. Mol. Biol. (1971) 55, 379-400); Connolly (J. Appl. Cryst. (1983) 16, 548-558)). The surface-exposed positions can be determined based on the information on the three dimensional structure of antibodies using software suitable for protein modeling. Software which is suitably used for this purpose includes the SYBYL biopolymer module software (Tripos Associates). When the algorithm requires the input size parameter from the user, the "size" of probe for use in computation is generally or preferably set at about 1.4 angstrom or less in radius. Furthermore, a method for determining surface-exposed region and area using personal computer software is described by Pacios (Comput. Chem. (1994) 18 (4), 377-386; and J. Mol. Model. (1995) 1, 46-53).

In still another non-limiting embodiment, a naive library constructed from antibody genes derived from lymphocytes of healthy persons and consisting of naive sequences, which are unbiased repertoire of antibody sequences, can also be particularly suitably used as a randomized variable region library (Gejima et al. (Human Antibodies (2002) 11, 121-129); and Cardoso et al. (Scand. J. Immunol. (2000) 51, 337-344)).

Fc region

An Fc region contains the amino acid sequence derived from the heavy chain constant region of an antibody. An Fc region is a portion of the heavy chain constant region of an antibody, starting from the N terminal end of the hinge region, which corresponds to the papain cleavage site at an amino acid around position 216 according to the EU numbering system, and contains the hinge, CH2, and CH3 domains. While the Fc region may be obtained from human IgG1, it is not limited to a particular subclass of IgG. As described later, a favorable example of the Fc region is an Fc region that has an FcRn-binding activity in an acidic pH range. Furthermore, a favorable example of the Fc region is an Fc region that has an Fcγ receptor-binding activity as described later. A non-limiting embodiment of such an Fc region is, for example, the Fc region of human IgG1 (SEQ ID NO: 9), IgG2 (SEQ ID NO: 10), IgG3 (SEQ ID NO: 11), or IgG4 (SEQ ID NO: 12). A number of allotype sequences of human IgG1, human IgG2, human IgG3, and human IgG4 constant regions due to gene polymorphisms are described in "Sequences of proteins of immunological interest", NIH Publication No. 91-3242. Any of such sequences may be used in the present disclosure. In particular, for the human IgG1 sequence, the amino acid sequence at positions 356 to 358 as indicated by EU numbering may be DEL or EEM. Furthermore, an Fc region does not have to be derived from the above-described human IgG constant region as long as it has a domain that binds to FcyR and/or FcRn.

FcRn

Unlike Fcγ receptor belonging to the immunoglobulin superfamily, human FcRn is structurally similar to polypeptides of major histocompatibility complex (MHC) class I, exhibiting 22% to 29% sequence identity to class I MHC molecules (Ghetie el al., Immunol. Today (1997) 18 (12): 592-598). FcRn is expressed as a heterodimer consisting of soluble β or light chain (β2 microglobulin) complexed with transmembrane α or heavy chain. Like MHC, FcRn α chain comprises three extracellular domains (α1, α2, and α3) and its short cytoplasmic domain anchors the protein onto the cell surface. α1 and α2 domains interact with the FcRn-binding domain of the antibody Fc region (Raghavan et al., Immunity (1994) 1: 303-315).

FcRn is expressed in maternal placenta and york sac of mammals, and is involved in mother-to-fetus IgG transfer. In addition, in neonatal small intestine of rodents, where FcRn is expressed, FcRn is involved in transfer of maternal IgG across brush border epithelium from ingested colostrum or milk. FcRn is expressed in a variety of other tissues and endothelial cell systems of various species. FcRn is also expressed in adult human endothelia, muscular blood vessels, and hepatic sinusoidal capillaries. FcRn is believed to play a role in maintaining the plasma IgG concentration by mediating recycling of IgG to serum upon binding to IgG. Typically, binding of FcRn to IgG molecules is strictly pH dependent. The optimal binding is observed in an acidic pH range below 7.0.

Human FcRn whose precursor is a polypeptide having the signal sequence of SEQ ID NO: 13 (the polypeptide with the signal sequence is shown in SEQ ID NO: 14) forms a complex with human β2-microglobulin *in vivo.* Soluble human FcRn complexed with β2-microglobulin is produced by using conventional recombinant expression techniques. Fc regions as disclosed hereincan be assessed for their binding activity to such a soluble human FcRn complexed with β2-microglobulin. Unless otherwise specified, human FcRn refers to a form capable of binding to an Fc region as disclosed herein. Examples include a complex between human FcRn and human β2-microglobulin.

Binding activity of the Fc region to FcRn, in particular, human FcRn

The binding activity of an Fc region to FcRn, human FcRn in particular, can be measured by methods known to those skilled in the art, as described in the section "Binding Activity" above. Those skilled in the art can appropriately determine the conditions other than pH. The antigen-binding activity and human FcRn-binding activity of an antigen-binding molecule can be assessed based on the dissociation constant (KD), apparent dissociation constant (KD), dissociation rate (kd), apparent dissociation rate (kd), and such. These can be measured by methods known to those skilled in the art. For example, Biacore (GE healthcare), Scatchard plot, or flow cytometer may be used.

When the human FcRn-binding activity of an Fc region is measured, conditions other than the pH are not particularly limited, and can be appropriately selected by those skilled in the art. Measurements can be carried out, for example, at 37°C using MES buffer, as described in International Publication No. WO 2009/125825. Alternatively, the human FcRn-binding activity of an Fc region as disclosed herein can be measured by methods known to those skilled in the art, and may be measured by using, for example, Biacore (GE Healthcare) or such. The binding activity of an Fc region to human FcRn can be assessed by pouring, as an analyte, human FcRn, an Fc region, or an antigen-binding molecule as disclosed herein containing the Fc region into a chip immobilized with an Fc region, an antigen-binding molecule containing the Fc region, or human FcRn.

A neutral pH range as the condition where the Fc region contained in an antigen-binding molecule as disclosed herein has the FcRn-binding activity means pH 6.7 to pH 10.0 in general. Preferably, the neutral pH range is a range indicated with arbitrary pH values between pH 7.0 and pH 8.0, and is preferably selected from pH 7.0, 7.1, 7.2, 7.3, 7.4, 7.5, 7.6, 7.7, 7.8, 7.9, and 8.0, and is particularly preferably pH 7.4 that is close to the pH of plasma (blood) *in vivo.* When the binding affinity between the human FcRn-binding domain and human FcRn at pH 7.4 is too low to assess, pH 7.0 may be used instead of pH 7.4. Herein, an acidic pH range as the condition where the Fc region contained in an antigen-binding molecule as disclosed herein has the FcRn-binding activity means pH 4.0 to pH 6.5 in general. Preferably, the acidic pH range means pH 5.5 to pH 6.5, particularly preferably pH 5.8 to pH 6.0 which is close to the pH in the early endosome *in vivo.* Regarding the temperature used as the measurement condition, the binding affinity between the human FcRn-binding domain and human FcRn may be assessed at any temperature between 10°C and 50°C. Preferably, the binding affinity between the human FcRn-binding domain and human FcRn can be determined at 15°C to 40°C. More preferably, the binding affinity between the human FcRn-binding domain and human FcRn can be determined in the same manner at an arbitrary temperature between 20°C and 35°C, such as any one temperature of 20, 21, 22, 23, 24, 25, 26, 27, 28, 29, 30, 31, 32, 33, 34, and 35°C. In one embodiment, the temperature includes, but is not limited to, for example, 25°C.

According to the Journal of Immunology (2009) 182, 7663-7671, the human FcRn-binding activity of native human IgG1 in an acidic pH range (pH 6.0) is 1.7 μM (KD), and the activity is almost undetectable in a neutral pH range. Thus, in a preferred embodiment, antigen-binding molecules comprising an Fc region of which human FcRn-binding activity in an acidic pH range is 20 μM (KD) or stronger may be screened. In a more preferred embodiment, the antigen-binding molecules comprising an Fc region of which human FcRn-binding activity in an acidic pH range is 2.0 μM (KD) or stronger may be screened. In a still more preferred embodiment, the antigen-binding molecules comprising an Fc region of which human FcRn-binding activity in an acidic pH range is 0.5 μM (KD) or stronger may be screened. The above-mentioned KD values are determined by the method described in the Journal of Immunology (2009) 182: 7663-7671 (by immobilizing the antigen-binding molecule onto a chip and loading human FcRn as an analyte).

Preferred Fc regions have an FcRn-binding activity under an acidic pH range condition. When an Fc region originally has an FcRn-binding activity under an acidic pH range condition, the domain can be used as it is. When the domain has a weak or no FcRn-binding activity under an acidic pH range condition, an Fc region having a desired FcRn-binding activity can be obtained by altering amino acids of an antigen-binding molecule. Fc regions having a desired or enhanced FcRn-binding activity under an acidic pH range condition can also be suitably obtained by altering the amino acids of an Fc region. Amino acid alterations of an Fc region that result in such a desired binding activity can be found by comparing the FcRn-binding activity under an acidic pH range condition before and after amino acid alteration. Those skilled in the art can appropriately alter the amino acids using known techniques similar to the aforementioned techniques used to alter the Fcγ-receptor-binding activity.

Fc regions comprised in the antigen-binding molecules as disclosed herein, which have an FcRn-binding activity under an acidic pH range condition, can be obtained by any method. Specifically, FcRn-binding domains having an FcRn-binding activity or an enhanced FcRn-binding activity under an acidic pH range condition can be obtained by altering the amino acids of an IgG-type human immunoglobulin used as a starting Fc region. Preferred Fc regions of an IgG-type immunoglobulin for alteration include, for example, those of human IgGs (IgG1, IgG2, IgG3, and IgG4, and variants thereof). As long as the Fc region has an FcRn-binding activity under an acidic pH range condition or can increase the human FcRn-binding activity under an acidic pH range condition, amino acids at any position may be altered into other amino acids. When the antigen-binding molecule contains the Fc region of human IgG1 as the Fc region, it is preferable that the resulting Fc region contains an alteration that results in the effect of enhancing FcRn binding under an acidic pH range condition as compared to the binding activity of the starting human IgG1 Fc region. Amino acids that allow such alteration include, for example, at least one or more amino acids selected from the group of positions 252, 254, 256, 309, 311, 315, 433, and 434 according to EU numbering, and their combination amino acids of at least one or more amino acids selected from the group of positions 253, 310, 435, and 426 as described in WO 1997/034631. Favorable examples include at least one or more amino acids selected from the group of positions 238, 252, 253, 254, 255, 256, 265, 272, 286, 288, 303, 305, 307, 309, 311, 312, 317, 340, 356, 360, 362, 376, 378, 380, 382, 386, 388, 400, 413, 415, 424, 433, 434, 435, 436, 439, and 447 as indicated by EU numbering as described in WO 2000/042072. Similarly, favorable examples of amino acids that allow such alteration include, at least one or more amino acids selected from the group of positions 251, 252, 254, 255, 256, 308, 309, 311, 312, 385, 386, 387, 389, 428, 433, 434, and 436 according to EU numbering as described in WO 2002/060919. Furthermore, amino acids that allow such alteration include, for example, amino acids of positions 250, 314, and 428 according to EU numbering as described in WO2004/092219. In addition, favorable examples of amino acids that allow such alteration include at least one or more amino acids selected from the group of positions 238, 244, 245, 249, 252, 256, 257, 258, 260, 262, 270, 272, 279, 283, 285, 286, 288, 293, 307, 311, 312, 316, 317, 318, 332, 339, 341, 343, 375, 376, 377, 378, 380, 382, 423, 427, 430, 431, 434, 436, 438, 440, and 442 as described in WO 2006/020114. Furthermore, favorable examples of amino acids that allow such alteration include amino acids of positions 251, 252, 307, 308, 378, 428, 430, 434, and/or 436 according to EU numbering as described in WO 2010/045193. Alteration of these amino acids enhances FcRn binding of the Fc region of an IgG-type immunoglobulin under an acidic pH range condition.

When the Fc region of human IgG1 is comprised as the Fc region, a non-limiting embodiment of the alteration that results in the effect of enhancing FcRn binding under an acidic pH range condition as compared to the binding activity of the starting Fc region of human IgG1 includes at least one or more amino acid alterations selected from the group consisting of:

Arg or Leu for the amino acid at position 251;
Phe, Ser, Thr, or Tyr for the amino acid at position 252;
Ser or Thr for the amino acid at position 254;
Arg, Gly, Ile, or Leu for the amino acid at position 255;
Ala, Arg, Asn, Asp, Gln, Glu, or Thr for the amino acid at position 256;
Ile or Thr for the amino acid at position 308;
Pro for the amino acid at position 309;
Glu, Leu, or Ser for the amino acid at position 311;
Ala or Asp for the amino acid at position 312;
Ala or Leu for the amino acid at position 314;
Ala, Arg, Asp, Gly, His, Lys, Ser, or Thr for the amino acid at position 385;
Arg, Asp, Ile, Lys, Met, Pro, Ser, or Thr for the amino acid at position 386;
Ala, Arg, His, Pro, Ser, or Thr for the amino acid at position 387;
Asn, Pro, or Ser for the amino acid at position 389;
Leu, Met, Phe, Ser, or Thr for the amino acid at position 428;
Arg, Gln, His, Ile, Lys, Pro, or Ser for the amino acid at position 433;
His, Phe, or Tyr for the amino acid at position 434; and
Arg, Asn, His, Lys, Met, or Thr for the amino acid at position 436, as indicated by EU numbering. Meanwhile, the number of amino acids to be altered is not particularly limited; and amino acid may be altered at only one site or amino acids may be altered at two or more sites.

When the Fc region of human IgG1 is comprised as the Fc region, a non-limiting embodiment of the alteration that results in the effect of enhancing FcRn binding in an acidic pH range condition as compared to the binding activity of the starting Fc region of human IgG1 may be alterations including Ile for the amino acid at position 308, Pro for the amino acid at position 309, and/or Glu for the amino acid at position 311 according to EU numbering. Another non-limiting embodiment of this alteration may include Thr for the amino acid at position 308, Pro for the amino acid at position 309, Leu for the amino acid at position 311, Ala for the amino acid at position 312, and/or Ala for the amino acid at position 314. Furthermore, another non-limiting embodiment of this alteration may include Ile or Thr for the amino acid at position 308, Pro for the amino acid at position 309, Glu, Leu, or Ser for the amino acid at position 311, Ala for the amino acid at position 312, and/or Ala or Leu for the amino acid at position 314. Another non-limiting embodiment of this alteration may include Thr for the amino acid at position 308, Pro for the amino acid at position 309, Ser for the amino acid at position 311, Asp for the amino acid at position 312, and/or Leu for the amino acid at position 314.

When the Fc region of human IgG1 is comprised as the Fc region, a non-limiting embodiment of the alteration that results in the effect of enhancing FcRn binding under an acidic pH range condition as compared to the binding activity of the starting Fc region of human IgG1 may be alterations including Leu for the amino acid at position 251, Tyr for the amino acid at position 252, Ser or Thr for the amino acid at position 254, Arg for the amino acid at position 255, and/or Glu for the amino acid at position 256 according to EU numbering.

When the Fc region of human IgG1 is comprised as the Fc region, a non-limiting embodiment of the alteration that results in the effect of enhancing FcRn binding under an acidic pH range condition as compared to the binding activity of the starting Fc region of human IgG1 may be at least one or more alterations selected from the group of alterations including Leu, Met, Phe, Ser, or Thr for the amino acid at position 428, Arg, Gln, His, Ile, Lys, Pro, or Ser for the amino acid at position 433, His, Phe, or Tyr for the amino acid at position 434, and/or Arg, Asn, His, Lys, Met, or Thr for the amino acid at position 436 according to EU numbering. Another non-limiting embodiment of this alteration may include His or Met for the amino acid at position 428, and/or His or Met for the amino acid at position 434.

When the Fc region of human IgG1 is comprised as the Fc region, a non-limiting embodiment of the alteration that results in the effect of enhancing FcRn binding under an acidic pH range condition as compared to the binding activity of the starting Fc region of human IgG1 may be alterations including Arg for the amino acid at position 385, Thr for the amino acid at position 386, Arg for the amino acid at position 387, and/or Pro for the amino acid at position 389 according to EU numbering. Another non-limiting embodiment of this alteration may include Asp for the amino acid at position 385, Pro for the amino acid at position 386, and/or Ser for the amino acid at position 389.

Furthermore, when the Fc region of human IgG1 is comprised as the Fc region, a non-limiting embodiment of the alteration that results in the effect of enhancing FcRn binding under an acidic pH range condition as compared to the binding activity of the starting Fc region of human IgG1 include at least one or more amino acid alterations selected from the group consisting of:

Gln or Glu for the amino acid at position 250; and
Leu or Phe for the amino acid at position 428 according to EU numbering. The number of amino acids to be altered is not particularly limited; and amino acid may be altered at only one site or amino acids may be altered at two sites.

When the Fc region of human IgG1 is comprised as the Fc region, a non-limiting embodiment of the alteration that results in the effect of enhancing FcRn binding under an acidic pH range condition as compared to the binding activity of the starting Fc region of human IgG1 may be alterations including Gln for the amino acid at position 250, and/or Leu or Phe for the amino acid at position 428 according to EU numbering. Another non-limiting embodiment of this alteration may include Glu for the amino acid at position 250, and/or Leu or Phe for the amino acid at position 428.

When the Fc region of human IgG1 is comprised as the Fc region, a non-limiting embodiment of the alteration that results in the effect of enhancing FcRn binding under an acidic pH range condition as compared to the binding activity of the starting Fc region of human IgG1 include at least two or more amino acid alterations selected from the group consisting of:

Asp or Glu for the amino acid at position 251;
Tyr for the amino acid at position 252;
Gln for the amino acid at position 307;
Pro for the amino acid at position 308;
Val for the amino acid at position 378;
Ala for the amino acid at position 380;
Leu for the amino acid at position 428;
Ala or Lys for the amino acid at position 430;
Ala, His, Ser, or Tyr for the amino acid at position 434; and
Ile for the amino acid at position 436, as indicated by EU numbering. The number of amino acids to be altered is not particularly limited; and amino acid may be altered at only two sites or amino acids may be altered at three or more sites.

When the Fc region of human IgG1 is comprised as the Fc region, a non-limiting embodiment of the alteration that results in the effect of enhancing FcRn binding under an acidic pH range condition as compared to the binding activity of the starting Fc region of human IgG1 may be alterations including Gln for the amino acid at position 307, and Ala or Ser for the amino acid at position 434 according to EU numbering. Another non-limiting embodiment of this alteration may include Pro for the amino acid at position 308, and Ala for the amino acid at position 434. Furthermore, another non-limiting embodiment of this alteration may include Tyr for the amino acid at position 252, and Ala for the amino acid at position 434. A different non-limiting embodiment of this alteration may include Val for the amino acid at position 378, and Ala for the amino acid at position 434. Another different non-limiting embodiment of this alteration may include Leu for the amino acid at position 428, and Ala for the amino acid at position 434. Another different non-limiting embodiment of this alteration may include Ala for the amino acid at position 434, and Ile for the amino acid at position 436. Furthermore, another non-limiting embodiment of this alteration may include Pro for the amino acid at position 308, and Tyr for the amino acid at position 434. In addition, another non-limiting embodiment of this alteration may include Gln for the amino acid at position 307, and Ile for the amino acid at position 436.

When the Fc region of human IgG1 is comprised as the Fc region, a non-limiting embodiment of the alteration that results in the effect of enhancing FcRn binding under an acidic pH range condition as compared to the binding activity of the starting Fc region of human IgG1 may be alterations including any one of Gln for the amino acid at position 307, Ala for the amino acid at position 380, and Ser for the amino acid at position 434 according to EU numbering. Another non-limiting embodiment of this alteration may include Gln for the amino acid at position 307, Ala for the amino acid at position 380, and Ala for the amino acid at position 434. Furthermore, another non-limiting embodiment of this alteration may include Tyr for the amino acid at position 252, Pro for the amino acid at position 308, and Tyr for the amino acid at position 434. A different non-limiting embodiment of this alteration may include Asp for the amino acid at position 251, Gln for the amino acid at position 307, and His for the amino acid at position 434.

When the Fc region of human IgG1 is comprised as the Fc region, a non-limiting embodiment of the alteration that results in the effect of enhancing FcRn binding under an acidic pH range condition as compared to the binding activity of the starting Fc region of human IgG1 include alteration of at least two or more amino acids selected from the group consisting of:

Leu for the amino acid at position 238;
Leu for the amino acid at position 244;
Arg for the amino acid at position 245;
Pro for the amino acid at position 249;
Tyr for the amino acid at position 252;
Pro for the amino acid at position 256;
Ala, Ile, Met, Asn, Ser, or Val for the amino acid at position 257;
Asp for the amino acid at position 258;
Ser for the amino acid at position 260;
Leu for the amino acid at position 262;
Lys for the amino acid at position 270;
Leu or Arg for the amino acid at position 272;
Ala, Asp, Gly, His, Met, Asn, Gln, Arg, Ser, Thr, Trp, or Tyr for the amino acid at position 279;
Ala, Asp, Phe, Gly, His, Ile, Lys, Leu, Asn, Pro, Gln, Arg, Ser, Thr, Trp, or Tyr for the amino acid at position 283;
Asn for the amino acid at position 285;
Phe for the amino acid at position 286;
Asn or Pro for the amino acid at position 288;
Val for the amino acid at position 293;
Ala, Glu, or Met for the amino acid at position 307;
Ala, Ile, Lys, Leu, Met, Val, or Trp for the amino acid at position 311;
Pro for the amino acid at position 312;
Lys for the amino acid at position 316;
Pro for the amino acid at position 317;
Asn or Thr for the amino acid at position 318;
Phe, His, Lys, Leu, Met, Arg, Ser, or Trp for the amino acid at position 332;
Asn, Thr, or Trp for the amino acid at position 339;
Pro for the amino acid at position 341;
Glu, His, Lys, Gln, Arg, Thr, or Tyr for the amino acid at position 343;
Arg for the amino acid at position 375;
Gly, Ile, Met, Pro, Thr, or Val for the amino acid at position 376;
Lys for the amino acid at position 377;
Asp or Asn for the amino acid at position 378;
Asn, Ser, or Thr for the amino acid at position 380;
Phe, His, Ile, Lys, Leu, Met, Asn, Gln, Arg, Ser, Thr, Val, Trp, or Tyr for the amino acid at position 382;
Asn for the amino acid at position 423;
Asn for the amino acid at position 427;

Ala, Phe, Gly, His, Ile, Lys, Leu, Met, Asn, Gln, Arg, Ser, Thr, Val, or Tyr for the amino acid at position 430;

His or Asn for the amino acid at position 431;

Phe, Gly, His, Trp, or Tyr for the amino acid at position 434;

Ile, Leu, or Thr for the amino acid at position 436;

Lys, Leu, Thr, or Trp for the amino acid at position 438;

Lys for the amino acid at position 440; and

Lys for the amino acid at position 442 according to EU numbering. The number of amino acids to be altered is not particularly limited and amino acid at only two sites may be altered and amino acids at three or more sites may be altered.

When the Fc region of human IgG1 is comprised as the Fc region, a non-limiting embodiment of the alteration that results in the effect of enhancing FcRn binding under an acidic pH range condition as compared to the binding activity of the starting Fc region of human IgG1 may be alterations including Ile for the amino acid at position 257, and Ile for the amino acid at position 311 according to EU numbering. Another non-limiting embodiment of this alteration may include Ile for the amino acid at position 257, and His for the amino acid at position 434. Another non-limiting embodiment of this alteration may include Val for the amino acid at position 376, and His for the amino acid at position 434.

Furthermore, in another non-limiting embodiment, one may screen for antigen-binding molecules comprising an Fc region with the characteristic of having a human FcRn-binding activity in the neutral pH range instead of the above-described characteristic of having a human FcRn-binding activity in the acidic pH range. In a preferred embodiment, one may screen for antigen-binding molecules comprising an Fc region whose human FcRn-binding activity in the neutral pH range is 40 $\mu$M (KD) or stronger. In a more preferred embodiment, one may screen for antigen-binding molecules comprising an Fc region whose human FcRn-binding activity in the neutral pH range is 15 $\mu$M (KD) or stronger.

Furthermore, in another non-limiting embodiment, one may screen for antigen-binding molecules comprising an Fc region with the characteristic of having a human FcRn-binding activity in the neutral pH range in addition to the above-described characteristic of having a human FcRn-binding activity in the acidic pH range. In a preferred embodiment, one may screen for antigen-binding molecules comprising an Fc region whose human FcRn-binding activity in the neutral pH range is 40 $\mu$M (KD) or stronger. In a more preferred embodiment, one may screen for antigen-binding molecules comprising an Fc region whose human FcRn-binding activity in the neutral pH range is 15 $\mu$M (KD) or stronger.

Preferred Fc regions have a human FcRn-binding activity in the acidic pH range and/or neutral pH range. When an Fc region originally has a human FcRn-binding activity in the acidic pH range and/or neutral pH range, it can be used as it is. When an Fc region has a weak or no human FcRn-binding activity in the acidic pH range and/or neutral pH range, antigen-binding molecules comprising an Fc region having a desired human FcRn-binding activity can be obtained by altering amino acids of the Fc region comprised in the antigen-binding molecules. Fc regions having a desired human FcRn-binding activity in the acidic pH range and/or neutral pH range can also be suitably obtained by altering amino acids of a human Fc region. Alternatively, antigen-binding molecules comprising an Fc region having a desired human FcRn-binding activity can be obtained by altering amino acids of an Fc region that originally has a human FcRn-binding activity in the acidic pH range and/or neutral pH range. Amino acid alterations of a human Fc region that result in such a desired binding activity can be found by comparing the human FcRn-binding activity in the acidic pH range and/or neutral pH range before and after amino acid alteration. Those skilled in the art can appropriately alter amino acids using known methods.

As used herein, "alteration of amino acids" or "amino acid alteration" of an Fc region includes alteration into an amino acid sequence which is different from that of the starting Fc region. The starting Fc region may be any Fc region, as long as a variant modified from the starting Fc region can bind to human FcRn in an acidic pH range (*i.e.,* the starting Fc region does not necessarily need to have an activity to bind to human FcRn in a neutral pH range). Examples of starting Fc regions preferably include Fc regions of IgG antibodies, *i.e.,* native Fc regions. Furthermore, an altered Fc region modified from a starting Fc region which has been already modified can also be used preferably as an altered Fc region of as disclosed herein. The "starting Fc region" can refer to the polypeptide itself, a composition comprising the starting Fc region, or an amino acid sequence encoding the starting Fc region. Starting Fc regions can comprise a known IgG antibody Fc region produced *via* recombination described briefly in section "Antibody". The origin of starting Fc regions is not limited, and they may be obtained from human or any nonhuman organisms. Such organisms preferably include mice, rats, guinea pigs, hamsters, gerbils, cats, rabbits, dogs, goats, sheep, bovines, horses, camels and organisms selected from nonhuman primates. In another embodiment, starting Fc regions can also be obtained from cynomolgus monkeys, marmosets, rhesus monkeys, chimpanzees, or humans. Starting Fc regions can be obtained preferably from human IgG1; however, they are not limited to any particular IgG subclass. This means that an Fc region represented by human IgG1 (SEQ ID NO: 9), IgG2 (SEQ ID NO: 10), IgG3 (SEQ ID NO: 11), or IgG4 (SEQ ID NO: 12) can be used appropriately as a starting Fc region, and herein also means that an Fc region of an arbitrary IgG class or subclass derived from any organisms described above can be preferably used as a starting Fc region. Examples of naturally-occurring IgG variants or altered forms are described in published documents (Curr. Opin. Biotechnol. (2009) 20(6): 685-91; Curr. Opin. Immunol. (2008) 20(4), 460-470; Protein Eng. Des. Sel. (2010) 23(4): 195-202; International Publication Nos. WO 2009/086320, WO 2008/092117, WO 2007/041635, and WO 2006/105338); however, they are not limited to the examples.

Examples of alterations include those with one or more mutations, for example, mutations by substitution of different amino acid residues for amino acids of starting Fc regions, by insertion of one or more amino acid residues into starting Fc regions, or by deletion of one or more amino acids from starting Fc region. Preferably, the amino acid sequences of altered Fc regions comprise at least a part of the amino acid sequence of a non-native Fc region. Such variants necessarily have sequence identity or similarity less than 100% to their starting Fc region. In a preferred embodiment, the variants have amino acid sequence identity or similarity about 75% to less than 100%, more preferably about 80% to less than 100%, even more preferably about 85% to less than 100%, still more preferably about 90% to less than 100%, and yet more preferably about 95% to less than 100% to the amino acid sequence of their starting Fc region. In a non-limiting embodiment, at least one amino acid is different between a modified Fc region as disclosed herein and its starting Fc region. Amino acid difference between a modified Fc region as disclosed herein and its starting Fc region can also be preferably specified based on amino acid differences at above-described particular amino acid positions according to EU numbering system.

Known methods such as site-directed mutagenesis (Kunkel et al. (Proc. Natl. Acad. Sci. USA (1985) 82, 488-492)) and Overlap extension PCR can be appropriately employed to alter the amino acids of Fc regions. Furthermore, various known methods can also be used as an amino acid alteration method for substituting amino acids by those other than natural amino acids (Annu. Rev. Biophys. Biomol. Struct. (2006) 35, 225-249; Proc. Natl. Acad. Sci. U.S.A. (2003) 100 (11), 6353-6357). For example, a cell-free translation system (Clover Direct (Protein Express)) containing tRNAs in which amber suppressor tRNA, which is complementary to UAG codon (amber codon) that is a stop codon, is linked with an unnatural amino acid may be suitably used.

Fc regions comprised in the antigen-binding molecules as disclosed herein that have a human FcRn-binding activity in the acidic pH range can be obtained by any method. Specifically, one can screen for antigen-binding molecules comprising an Fc region of which human FcRn-binding activity in the acidic pH range is 20 $\mu$M (KD) or stronger; in a more favorable embodiment, an Fc region of which human FcRn-binding activity in the acidic pH range is 2.0 $\mu$M (KD) or stronger; and in an even more favorable embodiment, an Fc region of which human FcRn-binding activity in the acidic pH range is 0.5 $\mu$M (KD) or stronger as a result of altering amino acids of an IgG-type human immunoglobulin used as a starting Fc region. Preferred Fc regions of IgG-type immunoglobulins for modification include, for example, those of human IgGs such as IgG1, IgG2, IgG3, and IgG4 shown in SEQ ID NOs: 9, 10, 11, and 12, respectively, and variants thereof.

When an antigen-binding molecule comprises the Fc region of human IgG1 as the Fc region, suitable examples of amino acids that may be altered to achieve the above-mentioned desired effects on FcRn binding under an acidic pH range condition by altering amino acids of an IgG-type human immunoglobulin as a starting Fc region, include at least one or more amino acids selected from the group of positions 238, 252, 253, 254, 255, 256, 265, 272, 286, 288, 303, 305, 307, 309, 311, 312, 317, 340, 356, 360, 362, 376, 378, 380, 382, 386, 388, 400, 413, 415, 424, 433, 434, 435, 436, 439, and 447 according to EU numbering as described in WO 2000/042072. Similarly, favorable examples of amino acids that allow such alteration include at least one or more amino acids selected from the group of positions 251, 252, 254, 255, 256, 308, 309, 311, 312, 385, 386, 387, 389, 428, 433, 434, and 436 according to EU numbering as described in WO 2002/060919. Furthermore, amino acids that allow such alteration include, for example, amino acids of positions 250, 314, and 428 according to EU numbering as described in WO2004/092219. Furthermore, favorable examples of amino acids that allow such alteration include at least one or more amino acids selected from the group of positions 251, 252, 307, 308, 378, 428, 430, 434, and 436 according to EU numbering as described in WO 2010/045193. Alteration of these amino acids enhances FcRn binding of the Fc region of an IgG-type immunoglobulin under an acidic pH range condition.

Fc regions having human FcRn-binding activity in the neutral pH range can also be obtained by altering amino acids of human immunoglobulin of IgG type used as the starting Fc region. The Fc regions of IgG type immunoglobulins adequate for modification include, for example, those of human IgGs such as IgG1, IgG2, IgG3, and IgG4 respectively represented by SEQ ID NOs: 9, 10, 11, and 12, and modified forms thereof. Amino acids of any positions may be altered into other amino acids, as long as the Fc regions have the human FcRn-binding activity in the neutral pH range or can increase the human FcRn-binding activity in the neutral range. When the antigen-binding molecule contains the Fc region of human IgG1 as the human Fc region, it is preferable that the resulting Fc region contains a modification that results in the effect of enhancing the human FcRn binding in the neutral pH range as compared to the binding activity of the starting Fc region of human IgG1. Amino acids that allow such alteration include, for example, one or more amino acids selected from the group of positions 221 to 225, 227, 228, 230, 232, 233 to 241, 243 to 252, 254 to 260, 262 to 272, 274, 276, 278 to 289, 291 to 312, 315 to 320, 324, 325, 327 to 339, 341, 343, 345, 360, 362, 370, 375 to 378, 380, 382, 385 to 387, 389, 396, 414, 416, 423, 424, 426 to 438, 440, and 442 according to EU numbering. Alteration of these amino acids enhances the human FcRn binding of the Fc region of IgG-type immunoglobulin in the neutral pH range.

From those described above, alterations that enhance the human FcRn binding in the neutral pH range are appropriately selected for use in the present disclosure. Particularly preferred amino acids of the modified Fc regions include, for example, amino acids at positions 237, 248, 250, 252, 254, 255, 256, 257, 258, 265, 286, 289, 297, 298, 303, 305, 307, 308, 309, 311, 312, 314, 315, 317, 332, 334, 360, 376, 380, 382, 384, 385, 386, 387, 389, 424, 428, 433, 434, and 436 according to the EU numbering system. The human FcRn-binding activity in the neutral pH range of the Fc region contained in an antigen-binding molecule can be increased by substituting at least one amino acid selected from the above amino acids into a different amino acid.

Particularly preferred alterations include, for example, at least one or more amino acids selected from the group of:

Met for the amino acid at position 237;
Ile for the amino acid at position 248;
Ala, Phe, Ile, Met, Gln, Ser, Val, Trp, or Tyr for the amino acid at position 250;
Phe, Trp, or Tyr for the amino acid at position 252;
Thr for the amino acid at position 254;
Glu for the amino acid at position 255;
Asp, Asn, Glu, or Gln for the amino acid at position 256;
Ala, Gly, Ile, Leu, Met, Asn, Ser, Thr, or Val for the amino acid at position 257;
His for the amino acid at position 258:

Ala for the amino acid at position 265;
Ala or Glu for the amino acid at position 286;
His for the amino acid at position 289;
Ala for the amino acid at position 297;
Ala for the amino acid at position 303;
Ala for the amino acid at position 305;
Ala, Asp, Phe, Gly, His, Ile, Lys, Leu, Met, Asn, Pro, Gln, Arg, Ser, Val, Trp, or Tyr for the amino acid at position 307;
Ala, Phe, Ile, Leu, Met, Pro, Gln, or Thr for the amino acid at position 308;
Ala, Asp, Glu, Pro, or Arg for the amino acid at position 309;
Ala, His, or Ile for the amino acid at position 311;
Ala or His for the amino acid at position 312;
Lys or Arg for the amino acid at position 314;
Ala, Asp, or His for the amino acid at position 315;
Ala for the amino acid at position 317;
Val for the amino acid at position 332;
Leu for the amino acid at position 334;
His for the amino acid at position 360;
Ala for the amino acid at position 376;
Ala for the amino acid at position 380;
Ala for the amino acid at position 382;
Ala for the amino acid at position 384;
Asp or His for the amino acid at position 385;
Pro for the amino acid at position 386;
Glu for the amino acid at position 387;
Ala or Ser for the amino acid at position 389;
Ala for the amino acid at position 424;
Ala, Asp, Phe, Gly, His, Ile, Lys, Leu, Asn, Pro, Gln, Ser, Thr, Val, Trp, or Tyr for the amino acid at position 428;
Lys for the amino acid at position 433;
Ala, Phe, His, Ser, Trp, or Tyr for the amino acid at position 434; and
His, Ile, Leu, Phe, Thr, or Val for the amino acid at position 436 of the Fc region according to EU numbering. Meanwhile, the number of amino acids to be altered is not particularly limited and amino acid at only one site may be altered and amino acids at two or more sites may be altered. Combinations of these amino acid alterations include, for example, the amino acid alterations shown in Tables 5-1 to 5-32.

[Table 5-1]

| Variant | KD (M) | Site of amino acid alteration |
|---|---|---|
| F1 | 8.10E-07 | N434W |
| F2 | 3.20E-06 | M252YIS254TJ,T256E |
| F3 | 2.50E-06 | N434Y |
| F4 | 5.80E-06 | N434S |
| F5 | 6.80E-55 | N434A |
| F7 | 5.60E-06 | M252Y |
| F8 | 4.20E-06 | M252W |
| F9 | 1.40E-07 | M253Y/S:S254T/T256E/N434Y |
| F10 | 6.90E-08 | M252Y/S254T/T256E/N434W |
| F11 | 3.0E-07 | M252Y/N434Y |
| F12 | 1.70E-07 | M252Y /N434W |
| F13 | 3.20E-07 | M252W/N434Y |
| F14 | 1.80E-07 | M252W/K434W |
| F19 | 4.60E-07 | P257L/N434Y |
| F20 | 4.60E-07 | V308F/N434Y |
| P21 | 3.00E-08 | M252Y/Y308P/N434Y |
| F22 | 2.00E-06 | M428L/N4434S |
| F25 | 9.20E-09 | M252Y/S254T/T256E/V30SP/N434W |
| F26 | 1.00E-06 | I332V |
| F27 | 7.40E-06 | G237M |
| F29 | 1.40E-06 | I332V/N434Y |
| F31 | 2.80E-06 | G237M/V308F |
| F32 | 8.00E-07 | S254T/M434W |
| F33 | 2.30E-06 | S254T/N434Y |
| F34 | 2.80E-07 | T256E/N434W |
| F35 | 8.40E-07 | T256E/M434Y |
| F36 | 3.60E-07 | S254T/T256E/N434W |
| F37 | 1.10E-06 | S254T/T356E/N434Y |
| F38 | 1.00E-07 | M252Y/S254T/N434W |
| P39 | 3.00E-07 | M252Y/S254T/N434Y |
| F40 | 8.20E-08 | M252Y/T256E/N434W |
| F41 | 1.50E-07 | M252Y/T256E/N434Y |

Table 5-2 is a continuation table of Table 5-1.

[Table 5-2]

| F42 | 1.00E-06 | M252Y/S254T/T256E/N434A |
|---|---|---|
| F43 | 1.70E-06 | M252Y/N434A |
| F44 | 1.10E-06 | M252W/N434A |

(continued)

| F47 | 2.40E-07 | M252Y/T256Q/N434W |
|---|---|---|
| F48 | 3.20E-07 | M252Y/T256Q/N434Y |
| F49 | 5.10E-07 | M252F/T256D/N434W |
| F50 | 1.20E-06 | M252F/T256D/N434Y |
| F51 | 8.10E-06 | N434F/Y436H |
| F52 | 3.10E-06 | H433K/N434F/Y436H |
| F53 | 1.00E-06 | I332V/N434W |
| F54 | 8.40E-08 | V308P/N434W |
| F56 | 9.40E-07 | I332V/M428L/N434Y |
| F57 | 1.10E-05 | G385D/Q386P/N389S |
| F58 | 7.70E-07 | G385D/Q386P/N389S/N434W |
| F59 | 2.40E-06 | G385D/Q386P/N389S/N434Y |
| F60 | 1.10E-05 | G385H |
| F61 | 9.70E-07 | G385H/N434W |
| F62 | 1.90E-06 | G385H/N434Y |
| F63 | 2.50E-06 | N434F |
| F64 | 5.30E-06 | N434H |
| F65 | 2.90E-07 | M252Y/S254T/T256E/N434F |
| F66 | 4.30E-07 | M252Y/S254T/T256E/N434H |
| F67 | 6.30E-07 | M252Y/N434F |
| F68 | 9.30E-07 | M252Y/N434H |
| F69 | 5.10E-07 | M428L/N434W |
| F70 | 1.50E-06 | M428L/N434Y |
| F71 | 8.30E-08 | M252Y/S254T/T256E/M428L/N434W |
| F72 | 2.00E-07 | M252Y/S254T/T256E/M428L/N434Y |
| F73 | 1.70E-07 | M252Y/M428L/N434W |
| F74 | 4.60E-07 | M252Y/M428L/N434Y |
| F75 | 1.40E-06 | M252Y/M428L/N434A |
| F76 | 1.00E-06 | M252Y/S254T/T256E/M428L/N434A |
| F77 | 9.90E-07 | T256E/M428L/N434Y |
| F78 | 7.80E-07 | S254T/M428L/N434W |

Table 5-3 is a continuation table of Table 5-2.

[Table 5-3]

| F79 | 5.90E-06 | S254T/T256E/N434A |
|---|---|---|
| F80 | 2.70E-06 | M252Y/T256Q/N434A |
| F81 | 1.60E-06 | M252Y/T256E/N434A |
| F82 | 1.10E-06 | T256Q/N434W |
| F83 | 2.60E-06 | T256Q/N434Y |

(continued)

| F84 | 2.80E-07 | M252W/T256Q/N434W |
|---|---|---|
| F85 | 5.50E-07 | M252W/T256Q/N434Y |
| F86 | 1.50E-06 | S254T/T256Q/N434W |
| F87 | 4.30E-06 | S254T/T256Q/N434Y |
| F88 | 1.90E-07 | M252Y/S254T/T256Q/N434W |
| F89 | 3.60E-07 | M252Y/S254T/T256Q/N434Y |
| F90 | 1.90E-08 | M252Y/T256E/V308P/N434W |
| F91 | 4.80E-08 | M252Y/V308P/M428L/N434Y |
| F92 | 1.10E-08 | M252Y/S254T/T256E/V308P/M428L/N434W |
| F93 | 7.40E-07 | M252W/M428L/N434W |
| F94 | 3.70E-07 | P257L/M428L/N434Y |
| F95 | 2.60E-07 | M252Y/S254T/T256E/M428L/K434F |
| F99 | 6.20E-07 | M252Y/T256E/N434H |
| F101 | 1.10E-07 | M252W /T256Q/P257L/N434Y |
| F103 | 4.40E-08 | P238A/M252Y/V308P/N434Y |
| F104 | 3.70E-08 | M252Y/D265A/V308P/N434Y |
| F105 | 7.50E-08 | M252Y/T307A/V308P/N434Y |
| F106 | 3.70E-08 | M252Y/V303A/V308P/N434Y |
| F107 | 3.40E-08 | M252Y/V308P/D376A/N434Y |
| F108 | 4.10E-08 | M252Y/V305A/V308P/N434Y |
| F109 | 3.20E-08 | M252Y/V308P/Q311A/N434Y |
| F111 | 3.20E-08 | M252Y/V308P/K317A/N434Y |
| F112 | 6.40E-08 | M252Y/V308P/E380A/N434Y |
| F113 | 3.20E-08 | M252Y/V308P/E382A/N434Y |
| F114 | 3.80E-08 | M252Y/V308P/S424A/N434Y |
| F115 | 6.60E-06 | T307A/N434A |
| F116 | 8.70E-06 | E380A/N434A |
| F118 | 1.40E-05 | M428L |
| F119 | 5.40E-06 | T250Q/M428L |

Table 5-4 is a continuation table of Table 5-3.

[Table 5-4]

| F120 | 6.30E-08 | P257L/V308P/M428L/N434Y |
|---|---|---|
| F121 | 1.50E-08 | M252Y/T256E/V308P/M428L/N434W |
| F122 | 1.20E-07 | M252Y/T256E/M428L/N434W |
| F123 | 3.00E-08 | M252Y/T256E/V308P/N434Y |
| F124 | 2.90E-07 | M252Y/T256E/M428L/N434Y |
| F125 | 2.40E-08 | M252Y/S254T/T256E/V308P/M428L/N434Y |
| F128 | 1.70E-07 | P257L/M428L/N434W |

(continued)

| F129 | 2.20E-07 | P257A/M428L/N434Y |
|------|----------|-------------------|
| F131 | 3.00E-06 | P257G/M428L/N434Y |
| F132 | 2.10E-07 | P257I/M428L/N434Y |
| F133 | 4.10E-07 | P257M/M428L/N434Y |
| F134 | 2.70E-07 | P257N/M428L/N434Y |
| F135 | 7.50E-07 | P257S/M428L/N434Y |
| F136 | 3.80E-07 | P257T/M428L/N434Y |
| F137 | 4.60E-07 | P257V/M428L/N434Y |
| F139 | 1.50E-08 | M252W/V308P/N434W |
| F140 | 3.60E-08 | S239K/M252Y/V308P/N434Y |
| F141 | 3.50E-08 | M252Y/S298G/V308P/N434Y |
| F142 | 3.70E-08 | M252Y/D270F/V308P/N434Y |
| F143 | 2.00E-07 | M252Y/V308A/N434Y |
| F145 | 5.30E-08 | M252Y/V308F/N434Y |
| F147 | 2.40E-07 | M252Y/V308I/N434Y |
| F149 | 1.90E-07 | M252Y/V308L/N434Y |
| F150 | 2.00E-07 | M252Y/V308M/N434Y |
| F152 | 2.70E-07 | M252Y/V308Q/N434Y |
| F154 | 1.80E-07 | M252Y/V308T/N434Y |
| F157 | 1.50E-07 | P257A/V308P/M428L/N434Y |
| F158 | 5.90E-08 | P257T/V308P/M428L/N434Y |
| F159 | 4.40E-08 | P257V/V308P/M428L/N434Y |
| F160 | 8.50E-07 | M252W/M428I/N434Y |
| F162 | 1.60E-07 | M252W/M428Y/N434Y |
| F163 | 4.20E-07 | M252W/M428F/N434Y |
| F164 | 3.70E-07 | P238A/M252W/N434Y |
| F165 | 2.90E-07 | M252W/D265A/N434Y |

Table 5-5 is a continuation table of Table 5-4.

[Table 5-5]

| F166 | 1.50E-07 | M252W/T307Q/N434Y |
|------|----------|-------------------|
| F167 | 2.90E-07 | M252W /V303A/N434Y |
| F168 | 3.20E-07 | M252W/D376A/N434Y |
| F169 | 2.90E-07 | M252W/V305A/N434Y |
| F170 | 1.70E-07 | M252W /Q311A/N434Y |
| F171 | 1.90E-07 | M252W /D312A/N434Y |
| F172 | 2.20E-07 | M252W /K317A/N434Y |
| F173 | 7.70E-07 | M252W/E380A/N434Y |
| F174 | 3.40E-07 | M252W/E382A/N434Y |

(continued)

| F175 | 2.70E-07 | M252W/S424A/N434Y |
|---|---|---|
| F176 | 2.90E-07 | S239K/M252W/N434Y |
| F177 | 2.80E-07 | M252W /S298G/N434Y |
| F178 | 2.70E-07 | M252W/D270F/N434Y |
| F179 | 3.0E-07 | M252W/N325G/N434Y |
| F182 | 6.60E-08 | P257A/M428L/N434W |
| F183 | 2.20E-07 | P257T/M428L/N434W |
| F184 | 2.70E-07 | P257V/M428L/N434W |
| F185 | 2.60E-07 | M252W/I332V/N434Y |
| F188 | 3.00E-06 | P2571/Q311I |
| F189 | 1.90E-07 | M252Y/T307A/N434Y |
| F190 | 1.10E-07 | M252Y/T307Q/N434Y |
| F191 | 1.60E-07 | P257L/T307A/M428L/N434Y |
| F192 | 1.10E-07 | P257A/T307A/M428L/N434Y |
| F193 | 8.50E-08 | P257T/T307A/M428L/N434Y |
| F194 | 1.20E-07 | P257V/T307A/M428L/N434Y |
| F195 | 5.60E-08 | P257L/T307Q/M428L/N434Y |
| F196 | 3.50E-08 | P257A/T307Q/M428L/N434Y |
| F197 | 3.30E-08 | P257T/T307Q/M428L/N434Y |
| F198 | 4.80E-08 | P257V/T307Q/M428L/N434Y |
| F201 | 2.10E-07 | M252Y/T307D/N434Y |
| F203 | 2.40E-07 | M252Y/T307F/N434Y |
| F204 | 2.10E-07 | M252Y/T307G/N434Y |
| F205 | 2.00E-07 | M252Y/T307H/N434Y |
| F206 | 2.30E-07 | M252Y/T307I/N434Y |

Table 5-6 is a continuation table of Table 5-5.

[Table 5-6]

| F207 | 9.40E-07 | M252Y/T307K/N434Y |
|---|---|---|
| F208 | 3.90E-07 | M252Y/T307L/N434Y |
| F209 | 1.30E-07 | M252Y/T307M/N434Y |
| F210 | 2.90E-07 | M252Y/T307N/N434Y |
| F211 | 2.40E-07 | M252Y/T307P/N434Y |
| F212 | 6.80E-07 | M252Y/T307R/N434Y |
| F213 | 2.30E-07 | M252Y/T307S/N434Y |
| F214 | 1.70E-07 | M252Y/T307V/N434Y |
| F215 | 9.60E-08 | M252Y/T307W/N434Y |
| F216 | 2.30E-07 | M252Y/T307Y/N434Y |
| F217 | 2.30E-07 | M252Y/K334L/N434Y |

(continued)

| F218 | 2.60E-07 | M252Y/G385H/N434Y |
|------|----------|-------------------|
| F219 | 2.50E-07 | M252Y/T289H/N434Y |
| F220 | 2.50E-07 | M252Y/Q311H/N434Y |
| F221 | 3.10E-07 | M252Y/D312H/N434Y |
| F222 | 3.40E-07 | M252Y/N315H/N434Y |
| F223 | 2.70E-07 | M252Y/K360H/N434Y |
| F225 | 1.50E-06 | M252Y/L314R/N434Y |
| F226 | 5.40E-07 | M252Y/L314K/N434Y |
| F227 | 1.20E-07 | M252Y/N286E/N434Y |
| F228 | 2.30E-07 | M252Y/L309E/N434Y |
| F229 | 5.10E-07 | M252Y/R255E/N434Y |
| F230 | 2.50E-07 | M252Y/P387E/N434Y |
| F236 | 8.90E-07 | K248I/M428L/N434Y |
| F237 | 2.30E-07 | M252Y/M428A/N434Y |
| F238 | 7.40E-07 | M252Y/M428D/N434Y |
| F240 | 7.20E-07 | M252Y/M428F/N434Y |
| F241 | 1.50E-06 | M252Y/M428G/N434Y |
| F242 | 8.50E-07 | M252Y/M428H/N434Y |
| F243 | 1.80E-07 | M252Y/M428I/N434Y |
| F244 | 1.30E-06 | M252Y/M428K/N434Y |
| F245 | 4.70E-07 | M252Y/M428N/N434Y |
| F246 | 1.10E-06 | M252Y/M428P/N434Y |
| F247 | 4.40E-07 | M252Y/M428Q/N434Y |

Table 5-7 is a continuation table of Table 5-6.

[Table 5-7]

| F249 | 6.40E-07 | M252Y/M428S/N434Y |
|------|----------|-------------------|
| F250 | 2.90E-07 | M252Y/M428T/N434Y |
| F251 | 1.90E-07 | M252Y/M428V/N434Y |
| F252 | 1.00E-06 | M252Y/M428W/N434Y |
| F253 | 7.10E-07 | M252Y/M428Y/N434Y |
| F254 | 7.50E-08 | M252W/T307Q/M428Y/N434Y |
| F255 | 1.10E-07 | M252W/Q311A/M428Y/N434Y |
| F256 | 5.40E-08 | M252W/T307Q/Q311A/M428Y/N434Y |
| F257 | 5.00E-07 | M252Y/T307A/M428Y/ N434Y |
| F258 | 3.20E-07 | M252Y/T307Q/M428Y/N434Y |
| F259 | 2.80E-07 | M252Y/D270F/N434Y |
| F260 | 1.30E-07 | M252Y/T307A/Q311A/N434Y |
| F261 | 8.40E-08 | M252Y/T307Q/Q311A/N434Y |

(continued)

| F262 | 1.90E-07 | M252Y/T307A/Q311II/N434Y |
|---|---|---|
| F263 | 1.10E-07 | M252Y/T307Q/Q311H/N434Y |
| F264 | 2.80E-07 | M252Y/E382A/N434Y |
| F265 | 6.80E-07 | M252Y/E382A/M428Y/N434Y |
| F266 | 4.70E-07 | M252Y/T307A/E382A/M428Y/N434Y |
| F267 | 3.20E-07 | M252Y/T307Q/E382A/M428Y/N434Y |
| F268 | 6.30E-07 | P238A/M252Y/M428F/N434Y |
| F269 | 5.20E-07 | M252Y/V305A/M428F/N434Y |
| F270 | 6.60E-07 | M252Y/N325G/M428F/N434Y |
| F271 | 6.90E-07 | M252Y/D376A/M428F/N434Y |
| F272 | 6.80E-07 | M252Y/E380A/M428F/N434Y |
| F273 | 6.50E-07 | M252Y/E382A/M428F/N434Y |
| F274 | 7.60E-07 | M252Y/E380A/E382A/M428F/N434Y |
| F275 | 4.20E-08 | S239K/M252Y/V308P/E382A/N434Y |
| F276 | 4.10E-08 | M252Y/D270F/V308P/E382A/N434Y |
| F277 | 1.30E-07 | S239K/M252Y/V308P/M428Y/N434Y |
| F278 | 3.00E-08 | M252Y/T307Q/V308P/E382A/N434Y |
| F279 | 6.10E-08 | M252Y/V308P/Q311H/E382A/N434Y |
| F280 | 4.10E-08 | S239K/M252Y/D270F/V308P/N434Y |
| F281 | 9.20E-08 | M252Y/V308P/E382A/M428F/N434Y |
| F282 | 2.90E-08 | M252Y/V308P/E382A/M428L/N434Y |

Table 5-8 is a continuation table of Table 5-7.

[Table 5-8]

| F283 | 1.00E-07 | M252Y/V308P/E382A/M428Y/N434Y |
|---|---|---|
| F284 | 1.00E-07 | M252Y/V308P/M428Y/N434Y |
| F285 | 9.90E-08 | M252Y/V308P/M428F/N434Y |
| F286 | 1.20E-07 | S239K/M252Y/V308P/E382A/M428Y/N434Y |
| F287 | 1.00E-07 | M252Y/V308P/E380A/E382A/M428F/N434Y |
| F288 | 1.90E-07 | M252Y/T256E/E382A/N434Y |
| F289 | 4.80E-07 | M252Y/T256E/M428Y/N434Y |
| F290 | 4.60E-07 | M252Y/T256E/E382A/M428Y/N434Y |
| F292 | 2.30E-08 | S239K/V1252Y/V308Y/E382A/M4281/N434Y |
| F293 | 5.30E-08 | M252Y/V308P/E380A/E382A/M428I/N434Y |
| F294 | 1.10E-07 | S239K/M252Y/V308P/M428F/N434Y |
| F295 | 6.80E-07 | S239K/M252Y/E380A/E382A/M428F/N434Y |
| F296 | 4.90E-07 | M252Y/Q311A/M428Y/N434Y |
| F297 | 5.10E-07 | M252Y/D312A/M428Y/N434Y |
| F298 | 4.80E-07 | M252Y/Q311A/D312A/M428Y/N434Y |

(continued)

| F299 | 9.40E-08 | S239K/M252Y/V308P/Q31 1 A/M428Y/N434Y |
|---|---|---|
| F300 | 8.30E-08 | S239K/M252Y/V308P/D312A/M428Y/N434Y |
| F301 | 7.20E-08 | S239K/M252Y/V308P/Q311A/D312A/M428Y/N434Y |
| F302 | 1.90E-07 | M252Y/T256E/T307P/N434Y |
| F303 | 6.70E-07 | M252Y/T307P/M428Y/N434Y |
| F304 | 1.60E-08 | M252W/V308P/M428Y/N434Y |
| F305 | 2.70E-08 | M252Y/T256E/V308P/E382A/N434Y |
| F306 | 3.60E-08 | M252W/V308P/E382A/N434Y |
| F307 | 3.60E-08 | S239K/M252W/V308P/E382A/N434Y |
| F308 | 1.90E-08 | S239K/M252W/V308P/E382A/M428Y/N434Y |
| F310 | 9.40E-08 | S239K/M252W/V308P/E382A/M4281/N434Y |
| F311 | 2.80E-08 | S239K/M252W/V308P/M428F/N434Y |
| F312 | 4.50E-07 | S239K/M252W/E380A/E382A/M428F/N434Y |
| F313 | 6.50E-07 | S239K/M252Y/T307P/M428Y/N434Y |
| F314 | 3.20E-07 | M252Y/T256E/Q311A/D312A/M428Y/N434Y |
| F315 | 6.80E-07 | S239K/M252Y/M428Y/N434Y |
| F316 | 7.00E-07 | S239K/M252Y/D270F/M428Y/N434Y |
| F317 | 1.10E-07 | S239K/M252Y/D270F/V308P/M428Y/N434Y |
| F318 | 1.80E-08 | S239K/M252Y/V308P/M428I/N434Y |

Table 5-9 is a continuation table of Table 5-8.

[Table 5-9]

| F320 | 2.00E-08 | S239K/M252Y/V308P/N325G/E382A/M4281/N434Y |
|---|---|---|
| F321 | 3.20E-08 | S239K/M252Y/D270F/V308P/N325G/N434Y |
| F322 | 9.20E-08 | S239K/M252Y/D270F/T307P/V308P/N434Y |
| F323 | 2.70E-08 | S239K/M252Y/T256E/D270F/V308P/N434Y |
| F324 | 2.80E-08 | S239K/M252Y/D270F/T307Q/V308P/N434Y |
| F325 | 2.10E-08 | S239K/M252Y/D270F/T307Q/V308P/Q311A/N434Y |
| F326 | 7.50E-08 | S239K/M252Y/D270F/T307Q/Q311A/N434Y |
| F327 | 6.50E-08 | S239K/M252Y/T256E/D270F/T307Q/Q311A/N434Y |
| F328 | 1.90E-08 | S239K/M252Y/D270F/V308P/M4281/N434Y |
| F329 | 1.20E-08 | S239K/M252Y/D270F/N286E/V308P/N434Y |
| F330 | 3.60E-08 | S239K/M252Y/D270F/V308P/L309E/N434Y |
| F331 | 3.00E-08 | S239K/M252Y/D270F/V308P/P387E/N434Y |
| F333 | 7.40E-08 | S239K/M252Y/D270F/T307Q/L309E/Q311A/N434Y |
| F334 | 1.90E-08 | S239K/M252Y/D270F/V308P/N325G/M428I/N434Y |
| F335 | 1.50E-08 | S239K/M252Y/T256E/D270F/V308P/M428I/N434Y |
| F336 | 1.40E-08 | S239K/M252Y/D270F/T307Q/V308P/ Q311A/M428I/N434Y |
| F337 | 5.60E-08 | S239K/M252Y/D270F/T307Q/Q311A/M428I/N434Y |

(continued)

| | | |
|---|---|---|
| F338 | 7.70E-09 | S239K/M252Y/D270F/N286E/V308P/M428I/N434Y |
| F339 | 1.90E-08 | S239K/M252Y/D270F/V308P/L309E/M428I/N434Y |
| F343 | 3.20E-08 | S239K/M252Y/D270F/V308P/M428L/N434Y |
| F344 | 3.00E-08 | S239K/M252Y/V308P/M428L/N434Y |
| F349 | 1.50E-07 | S239K/M252Y/V308P/L309P/M428L/N434Y |
| F350 | 1.70E-07 | S239K/M252Y/V308P/L309R/M428L/N434Y |
| F352 | 6.00E-07 | S239K/M252Y/L309P/M428L/N434Y |
| F353 | 1.10E-06 | S239K/M252Y/L309R/M428L/N434Y |
| F354 | 2.80E-08 | S239K/M252Y/T307Q/V308P/M428L/N434Y |
| F356 | 3.40E-08 | S239K/M252Y/D270F/V308P/L309E/P387E/K434Y |
| F357 | 1.60E-08 | S239K/M252Y/T256E/D270F/V308P/N325G/M428I/N434Y |
| F358 | 1.00E-07 | S239K/M252Y/T307Q/N434Y |
| F359 | 4.20E-07 | P257V/T307Q/M428I/N434Y |
| F360 | 1.30E-06 | P257V/T307Q/M428V/N434Y |
| F362 | 5.40E-08 | P257V/T3U7Q/M325G/M428L/N434Y |
| F363 | 4.10E-08 | P257V/T307Q/Q311A/M428L/N434Y |
| F364 | 3.50E-08 | P257V/T307Q/Q311A/N325G/M428L/N434Y |

Table 5-10 is a continuation table of Table 5-9.

[Table 5-10]

| | | |
|---|---|---|
| F365 | 5.10E-08 | P257V/V305A/T307Q/M428L/N434Y |
| F367 | 1.50E-08 | S239K/M252Y/E258H/D270F/T307Q/V308P/Q311A/N434Y |
| F368 | 2.00E-08 | S239K/M252Y/D270F/V308P/N325G/E382A/M428I/N434Y |
| F369 | 7.50E-08 | M252Y/P257V/T307Q/M428I/N434Y |
| F372 | 1.30E-08 | S239K/M252W/V308P/M428Y/N434Y |
| F373 | 1.10E-08 | S239K/M252W/V308P/Q311A/M428Y/N434Y |
| F374 | 1.20E-08 | S239K/M252W/T256E/V308P/M428Y/N434Y |
| F375 | 5.50E-09 | S239K/M252W/N286E/V308P/M428Y/N434Y |
| F376 | 9.60E-09 | S289K/M252Y/T256E/D270F/N286E/V308P/N444Y |
| F377 | 1.30E-07 | S239K/M252W/T307P/M428Y/N434Y |
| F379 | 9.00E-09 | S239K/M252W/T256E/V308P/Q311A/M428Y/N434Y |
| F380 | 5.60E-09 | S239K/M252W/T256E/N286E/V308P/M428Y/N434Y |
| F381 | 1.10E-07 | P257V/T307A/Q311A/M428L/N434Y |
| F382 | 8.70E-08 | P257V/V305A/T307A/M428L/N434Y |
| F386 | 3.20E-08 | M252Y/V308P/L309E/N434Y |
| F387 | 1.50E-07 | M252Y/V308P/L309D/N434Y |
| F388 | 7.00E-08 | M252Y/V308P/L309A/N434Y |
| F389 | 1.70E-08 | M252W/V308P/L309E/M428Y/N434Y |
| F390 | 6.80E-08 | M252W/V308P/L309D/M428Y/N434Y |

(continued)

| F391 | 3.60E-08 | M252W/V308P/L309A/M428Y/N434Y |
|------|----------|-------------------------------|
| F392 | 6.90E-09 | S239K/M252Y/N286E/V308P/M428I/N434Y |
| F393 | 1.20E-08 | S239K/M252Y/N286E/V308P/N434Y |
| F394 | 5.30E-08 | S239K/M252Y/T307Q/Q311A/M428I/N434Y |
| F395 | 2.40E-08 | S239K/M252Y/T256E/V308P/N434Y |
| F396 | 2.00E-08 | S239K/M252Y/D270F/N286E/T307Q/Q311A/M428I/N434Y |
| F397 | 4.50E-08 | S239K/M252Y/D270F/T307Q/Q311A/P387E/M428I/N434Y |
| F398 | 4.40E-09 | S239K/M252Y/D270F/N286E/T307Q/V308P/Q311A/M428I/N434Y |
| F399 | 6.50E-09 | S239K/M252Y/D270F/N286E/T307Q/V308P/M428I/N434Y |
| F400 | 6.10E-09 | S239K/M252Y/D270F/N286E/V308P/Q311A/M428I/N434Y |
| F401 | 6.90E-09 | S239K/M252Y/D270F/N286E/V308P/P387E/M428I/N434Y |
| F402 | 2.30E-08 | P257V/T307Q/M428L/N434W |
| F403 | 5.10E-08 | P257V/T307A/M428L/N434W |
| F404 | 9.40E-08 | P257A/T307Q/L309P/M428L/N434Y |
| F405 | 1.70E-07 | P257V/T307Q/L309P/M428L/N434Y |

Table 5-11 is a continuation table of Table 5-10.

[Table 5-11]

| F406 | 1.50E-07 | P257A/T307Q/L309R/M428L/N434Y |
|------|----------|-------------------------------|
| F407 | 1.60E-07 | P257V/T307Q/L309R/M428L/N434Y |
| F408 | 2.50E-07 | P257V/N286E/M428L/N434Y |
| F409 | 2.00E-07 | P257V/P387E/M428L/N434Y |
| F410 | 2.20E-07 | P257V/T307H/M428L/N434Y |
| F411 | 1.30E-07 | P257V/T307N/M428L/N434Y |
| F412 | 8.80E-08 | P257V/T307G/M428L/N434Y |
| F413 | 1.20E-07 | P257V/T307P/M428L/N434Y |
| F414 | 1.10E-07 | P257V/T307S/M428L/N434Y |
| F415 | 5.60E-08 | P257V/N286E/T307A/M428L/N434Y |
| F416 | 9.40E-08 | P257V/T307A/P387E/M428L/N434Y |
| F418 | 6.20E-07 | S239K/M252Y/T307P/N325G/M428Y/N434Y |
| F419 | 1.60E-07 | M252Y/T307A/Q311H/K360H/N434Y |
| F420 | 1.50E-07 | M252Y/T307A/Q311H/P387E/N434Y |
| F421 | 1.30E-07 | M252Y/T307A/Q311H/M428A/N434Y |
| F422 | 1.80E-07 | M252Y/T307A/Q311H/E382A/N434Y |
| F423 | 8.40E-08 | M252Y/T307W/Q311H/N434Y |
| F424 | 9.40E-08 | S239K/P257A/V308P/M428L/N434Y |
| F425 | 8.00E-08 | P257A/V308P/L309E/M428L/N434Y |
| F426 | 8.40E-08 | P257V/T307Q/N434Y |
| F427 | 1.10E-07 | M252Y/P257V/T307Q/M428V/N434Y |

(continued)

| F428 | 8.00E-08 | M252Y/P257V/T307Q/M428L/N434Y |
|---|---|---|
| F429 | 3.70E-08 | M252Y/P257V/T307Q/N434Y |
| F430 | 8.10E-08 | M252Y/P257V/T307Q/M428Y/N434Y |
| F431 | 6.50E-08 | M252Y/P257V/T307Q/M428F/N434Y |
| F432 | 9.20E-07 | P257V/T307Q/Q311A/N325G/M428V/N434Y |
| F433 | 6.00E-08 | P257V/T307Q/Q311A/N325G/N434Y |
| F434 | 2.00E-08 | P257V/T307Q/Q311A/N325G/M428Y/N434Y |
| F435 | 2.50E-08 | P257V/T307Q/Q311A/N325G/M428F/N434Y |
| F436 | 2.50E-07 | P257A/T307Q/M428V/N434Y |
| F437 | 5.70E-08 | P257A/T307Q/N434Y |
| F438 | 3.60E-08 | P257A/T307Q/M428Y/N434Y |
| F439 | 4.00E-08 | P257A/T307Q/M428F/N434Y |
| F440 | 1.50E-08 | P257V/N286E/T307Q/Q311A/N325G/M428L/N434Y |

Table 5-12 is a continuation table of Table 5-11.

[Table 5-12]

| F441 | 1.80E-07 | P257A/Q311A/M428L/N434Y |
|---|---|---|
| F442 | 2.00E-07 | P257A/Q311H/M428L/N434Y |
| F443 | 5.50E-08 | P257A/T307Q/Q311A/M428L/N434Y |
| F444 | 1.40E-07 | P257A/T307A/Q311A/M428L/N434Y |
| F445 | 6.20E-08 | P257A/T307Q/Q311H/M428L/N434Y |
| F446 | 1.10E-07 | P257A/T307A/Q311H/M428L/N434Y |
| F447 | 1.40E-08 | P257A/N286E/T307Q/M428L/N434Y |
| F448 | 5.30E-08 | P257A/N286E/T307A/M428L/N434Y |
| F449 | 5.70E-07 | S239K/M252Y/D270F/T307P/N325G/M428Y/N434Y |
| F450 | 5.20E-07 | S239K/M252Y/T307P/L309E/N325G/M428Y/N434Y |
| F451 | 1.00E-07 | P257S/T307A/M428L/N434Y |
| F452 | 1.40E-07 | P257M/T307A/M428L/N434Y |
| F453 | 7.80E-08 | P257N/T307A/M428L/N434Y |
| F454 | 9.60E-08 | P257I/T307A/M428L/N434Y |
| F455 | 2.70E-08 | P257V/T307Q/M428Y/N434Y |
| F456 | 3.40E-08 | P257V/T307Q/M428F/N434Y |
| F457 | 4.00E-08 | S239K/P257V/V308P/M428L/N434Y |
| F458 | 1.50E-08 | P257V/T307Q/V308P/N325G/M428L/N434Y |
| F459 | 1.30E-08 | P257V/T307Q/V308P/Q311A/N325G/M428L/N434Y |
| F460 | 4.70E-08 | P257V/T307A/V308P/N325G/M428L/N434Y |
| F462 | 8.50E-08 | P257A/V308P/N325G/M428L/N434Y |
| F463 | 1.30E-07 | P257A/T307A/V308P/M428L/N434Y |
| F464 | 5.50E-08 | P257A/T307Q/V308P/M428L/N434Y |

(continued)

| F465 | 2.10E-08 | P257V/N286E/T307Q/N325G/M428L/N434Y |
| F466 | 3.50E-07 | T256E/P257V/N434Y |
| F467 | 5.70E-07 | T256E/P257T/N434Y |
| F468 | 5.70E-08 | S239K/P257T/V308P/M428L/N434Y |
| F4R9 | S.60E-08 | P257T/V308P/N325G/M428L/N434Y |
| F470 | 5.40E-08 | T256E/P257T/V308P/N325G/M428L/N434Y |
| F471 | 6.60E-08 | P257T/V308P/K325G/E382A/M428L/N434Y |
| F472 | 5.40E-08 | P257T/V308P/K325G/P387E/M428L/N434Y |
| F473 | 4.50E-07 | P257T/V308P/L309P/N325G/M428L/N434Y |
| F474 | 3.50E-07 | P257T/V308P/L309R/N325G/M428L/N434Y |
| F475 | 4.30E-08 | T256E/P257V/T307Q/M428L/N434Y |

Table 5-13 is a continuation table of Table 5-12.

[Table 5-13]

| F476 | 5.50E-08 | P257V/T307Q/E382A/M428L/N434Y |
| F477 | 4.30E-08 | P257V/T307Q/P387E/M428L/N434Y |
| F480 | 3.90E-08 | P257L/V308P/N434Y |
| F481 | 5.60E-08 | P257T/T307Q/N434Y |
| F482 | 7.00E-08 | P257V/T307Q/N325G/N434Y |
| F483 | 5.70E-08 | P257V/T307Q/Q311A/N434Y |
| F484 | 6.20E-08 | P257V/V305A/T307Q/N434Y |
| F485 | 9.70E-08 | P257V/N286E/T307A/N434Y |
| F486 | 3.40E-07 | P257V/T307Q/L309R/Q311H/M428L/N434Y |
| F488 | 3.50E-08 | P257V/V308P/N325G/M428L/N434Y |
| F490 | 7.50E-08 | S239K/P257V/V308P/Q311H/M428L/N434Y |
| F492 | 9.80E-08 | P257V/V305A/T307A/N325G/M428L/N434Y |
| F493 | 4.90E-07 | S239K/D270F/T307P/N325G/M428Y/N434Y |
| F497 | 3.10E-06 | P257T/T307A/M428V/N434Y |
| F498 | 1.30R-06 | P257A/M428V/N434Y |
| F499 | 5.20E-07 | P257A/T307A/M428V/N434Y |
| F500 | 4.30E-08 | P257S/T307Q/M428L/N434Y |
| F506 | 1.90E-07 | P257V/N297A/T307Q/M428L/N434Y |
| F507 | 5.10E-08 | P257V/N286A/T307Q/M428L/N434Y |
| F508 | 1.10E-07 | P257V/T307Q/N315A/M428L/N434Y |
| F509 | 5.80E-08 | P257V/T307Q/N384A/M428L/N434Y |
| F510 | 5.30E-08 | P257V/T307Q/N389A/M428L/N434Y |
| F511 | 4.20E-07 | P257V/N434Y |
| F512 | 5.80E-07 | P257T/N434Y |
| F517 | 3.10E-07 | P257V/N286E/N434Y |

(continued)

| | | |
|---|---|---|
| F518 | 4.20E-07 | P257T/N286E/N434Y |
| F519 | 2.60E-08 | P257V/N286E/T307Q/N434Y |
| F521 | 1.10E-08 | P257V/N286E/T307Q/M428Y/N434Y |
| F523 | 2.60E-08 | P257V/V305A/T307Q/M428Y/N434Y |
| F526 | 1.90E-08 | P257T/T307Q/M428Y/N434Y |
| F527 | 9.40E-09 | P257V/T307Q/V308P/N325G/M428Y/N434Y |
| F529 | 2.50E-08 | P257T/T307Q/M428F/N434Y |
| F533 | 1.20E-08 | P257A/N286E/T307Q/M428F/N434Y |
| F534 | 1.20E-08 | P257A/N286E/T307Q/M428Y/N434Y |

Table 5-14 is a continuation table of Table 5-13.

[Table 5-14]

| | | |
|---|---|---|
| F535 | 3.90E-08 | T250A/P257V/T307Q/M428L/N434Y |
| F538 | 9.90E-08 | T250F/P257V/T307Q/M428L/N434Y |
| F541 | 6.00E-08 | T250I/P257V/T307Q/M428L/N434Y |
| F544 | 3.10E-08 | T250M/P257V/T307Q/M428L/N434Y |
| F549 | 5.40E-08 | T250S/P257V/T307Q/M428L/N434Y |
| F550 | 5.90E-08 | T250V/P257V/T307Q/M428L/N434Y |
| F551 | 1.20E-07 | T250W/P257V/T307Q/M428L/N434Y |
| F552 | 1.10E-07 | T250Y/P257V/T307Q/M428L/N434Y |
| F553 | 1.70E-07 | M252Y/Q311A/N434Y |
| F554 | 2.80E-08 | S239K/M252Y/S254T/V308P/N434Y |
| F556 | 1.50E-06 | M252Y/T307Q/Q311A |
| F559 | 8.00E-08 | M252Y/S254T/N286E/N434Y |
| F560 | 2.80E-08 | M252Y/S254T/V308P/N434Y |
| F561 | 1.40E-07 | M252Y/S254T/T307A/N434Y |
| F562 | 8.30E-08 | M252Y/S254T/T307Q/N434Y |
| F563 | 1.30E-07 | M252Y/S254T/Q311A/N434Y |
| F564 | 1.90E-07 | M252Y/S254T/Q311H/N434Y |
| F565 | 9.20E-08 | M252Y/S254T/T307A/Q311A/N434Y |
| F566 | 6.10E-08 | M252Y/S254T/T307Q/Q311A/N434Y |
| F567 | 2.20E-07 | M252Y/S254T/M428I/N434Y |
| F568 | 1.10E-07 | M252Y/T256E/T307A/Q311H/N434Y |
| F569 | 2.00E-07 | M252Y/T256Q/T307A/Q311H/N434Y |
| F570 | 1.30E-07 | M252Y/S254T/T307A/Q311H/N434Y |
| F571 | 8.10E-08 | M252Y/N286E/T307A/Q311H/N434Y |
| F572 | 1.00E-07 | M252Y/T307A/Q311H/M428I/N434Y |
| F576 | 1.60E-06 | M252Y/T256E/T307Q/Q311H |
| F577 | 1.30E-06 | M252Y/N286E/T307A/Q311A |

(continued)

| F578 | 5.70E-07 | M252Y/N286E/T307Q/Q311A |
|------|----------|--------------------------|
| F580 | 8.60E-07 | M252Y/N286E/T307Q/Q311H |
| F581 | 7.20E-08 | M252Y/T256E/N286E/N434Y |
| F582 | 7.50E-07 | S239K/M252Y/V308P |
| F583 | 7.80E-07 | S239K/M252Y/V308P/E382A |
| F584 | 6.30E-07 | S239K/M252Y/T256E/V308P |
| F585 | 2.90E-07 | S239K/M252Y/N286E/V308P |

Table 5-15 is a continuation table of Table 5-14.

[Table 5-15]

| F586 | 1.40E-07 | S239K/M252Y/N2B6E/V308P/M4281 |
|------|----------|-------------------------------|
| F587 | 1.90E-07 | M252Y/N286E/M428L/N434Y |
| F592 | 2.00E-07 | M252Y/S254T/E382A/N434Y |
| F593 | 3.10E-08 | S239K/M252Y/S254T/V308P/M428I/N434Y |
| F594 | 1.60E-08 | S239K/M252Y/T256E/V308P/M428I/N434Y |
| F595 | 1.80E-07 | S239K/M252Y/M428I/N434Y |
| F596 | 4.00E-07 | M252Y/D312A/E382A/M428Y/N434Y |
| F597 | 2.20E-07 | M252Y/E382A/P387E/N434Y |
| F598 | 1.40E-07 | M252Y/D312A/P387E/N434Y |
| F599 | 5.20E-07 | M252Y/P387E/M428Y/N434Y |
| F600 | 2.80E-07 | M252Y/T256Q/E382A/N434Y |
| F601 | 9.60E-09 | M252Y/N286E/V308P/N434Y |
| F608 |          | G236A/S239D/I332E |
| F611 | 2.80E-07 | M252Y/V305T/T307P/V308I/L309A/N434Y |
| F612 | 3.60E-07 | M252Y/T307P/V308I/L309A/N434Y |
| F613 |          | S239D/A330L/I332E |
| F616 |          | S239D/K326D/L328Y |
| F617 | 7.40E-07 | S239K/N434W |
| F618 | 6.40E-07 | S239K/V308F/N434Y |
| F619 | 3.10E-07 | S239K/M252Y/N434Y |
| F620 | 2.10E-07 | S239K/M252Y/S254T/N434Y |
| F621 | 1.50E-07 | S239K/M252Y/T307A/Q311H/N434Y |
| F622 | 3.50E-07 | S239K/M252Y/T256Q/N434Y |
| F623 | 1.80E-07 | S239K/M252W/N434W |
| F624 | 1.40E-08 | S239K/P257A/N286E/T307Q/M428L/N434Y |
| F625 | 7.60E-08 | S239K/P257A/T307Q/M428L/N434Y |
| F626 | 1.30E-06 | V308P |
| F629 | 3.90E-08 | M252Y/V279L/V308P/N434Y |
| F630 | 3.70E-08 | S239K/M252Y/V279L/V308P/N434Y |

(continued)

| F633 | 2.40E-08 | M252Y/V282D/V308P/N434Y |
|------|----------|-------------------------|
| F634 | 3.20E-08 | S239K/M252Y/V282D/V308P/N434Y |
| F635 | 4.50E-08 | M252Y/V284K/V308P/N434Y |
| F636 | 4.80E-08 | S239K/M252Y/V284K/V308P/N434Y |
| F637 | 1.50E-07 | M252Y/K288S/V308P/N434Y |

Table 5-16 is a continuation table of Table 5-15.

[Table 5-16]

| F638 | 1.40E-07 | S239K/M252Y/K288S/V308P/N434Y |
|------|----------|-------------------------------|
| F639 | 2.70E-08 | M252Y/V308P/G385R/N434Y |
| F640 | 3.60E-08 | S239K/M252Y/V308P/G385R/N434Y |
| F641 | 3.00E-08 | M252Y/V308P/Q386K/N434Y |
| F642 | 3.00E-08 | S239K/M252Y/V308P/Q386K/N434Y |
| F643 | 3.20E-08 | L235G/G236R/S239K/M252Y/V308P/N434Y |
| F644 | 3.00E-08 | G236R/S239K/M252Y/V308P/N434Y |
| F645 | 3.30E-08 | S239K/M252Y/V308P/L328R/N434Y |
| F646 | 3.80E-08 | S239K/M252Y/N297A/V308P/N434Y |
| F647 | 2.90E-08 | P238D/M252Y/V308P/N434Y |
| F648 |          | P238D |
| F649 | 1.20E-07 | S239K/M252Y/N286E/N434Y |
| F650 | 1.70E-07 | S239K/M252Y/T256E/N434Y |
| F651 | 1.80E-07 | S239K/M252Y/Q311A/N434Y |
| F652 | 2.40E-07 | P238D/M252Y/N434Y |
| F654 | 3.20E-08 | L235K/S239K/M252Y/V308P/N434Y |
| F655 | 3.40E-08 | L235R/S239K/M252Y/V308P/N434Y |
| F656 | 3.30E-08 | G237K/S239K/M252Y/V308P/N434Y |
| F657 | 3.20E-08 | G237R/S239K/M252Y/V308P/N434Y |
| F658 | 3.20E-08 | P238K/S239K/M252Y/V308P/N434Y |
| F659 | 3.00E-08 | P238R/S239K/M252Y/V308P/N434Y |
| F660 | 3.10E-08 | S239K/M252Y/V308P/P329K/N434Y |
| F661 | 3.40E-08 | S239K/M252Y/V308P/P329R/N434Y |
| F663 | 6.40E-09 | S239K/M252Y/N286E/T307Q/V308P/Q311A/N434Y |
| F664 | 3.90E-08 | M252Y/N286A/V308P/N434Y |
| F665 | 2.00E-08 | M252Y/N286D/V308P/N434Y |
| F666 | 2.10E-08 | M252Y/N286F/V308P/N434Y |
| F667 | 3.00E-08 | M252Y/N286G/V308P/N434Y |
| F668 | 4.00E-08 | M252Y/N286H/V308P/N434Y |
| F669 | 3.50E-08 | M252Y/N286I/V308P/N434Y |
| F670 | 2.10E-07 | M252Y/N286K/V308P/N434Y |

(continued)

| F671 | 2.20E-08 | M252Y/N286L/V308P/N434Y |
|------|----------|-------------------------|
| F672 | 2.40E-08 | M252Y/N286M/V308P/N434Y |
| F673 | 2.30E-08 | M252Y/N286P/V308P/N434Y |

Table 5-17 is a continuation table of Table 5-16.

[Table 5-17]

| F674 | 3.20E-08 | M252Y/N286Q/V308P/N434Y |
|------|----------|-------------------------|
| F675 | 5.10E-08 | M252Y/N286R/V308P/N434Y |
| F676 | 3.20E-08 | M252Y/N286S/V308P/N434Y |
| F677 | 4.70E-08 | M252Y/N286T/V308P/N434Y |
| F678 | 3.30E-08 | M252Y/N286V/V308P/N434Y |
| F679 | 1.70E-08 | M252Y/N286W/V308P/N434Y |
| F680 | 1.50E-08 | M252Y/N286Y/V308P/N434Y |
| F681 | 4.90E-08 | M252Y/K288A/V308P/N434Y |
| F682 | 8.20E-08 | M252Y/K288D/V308P/N434Y |
| F683 | 5.00E-08 | M252Y/K288E/V308P/N434Y |
| F684 | 5.10E-08 | M252Y/K288F/V308P/N434Y |
| F685 | 5.30E-08 | M252Y/K288G/V308P/N434Y |
| F686 | 4.60E-08 | M252Y/K288H/V308P/N434Y |
| F687 | 4.90E-08 | M252Y/K288I/V308P/N434Y |
| F688 | 2.80E-08 | M252Y/K288L/V308P/N434Y |
| F689 | 4.10E-08 | M252Y/K288M/V308P/N434Y |
| F690 | 1.00E-07 | M252Y/K288N/V308P/N434Y |
| F691 | 3.20E-07 | M252Y/K288P/V308P/N434Y |
| F692 | 3.90E-08 | M252Y/K288Q/V308P/N434Y |
| F693 | 3.60E-08 | M252Y/K288R/V308P/N434Y |
| F694 | 4.70E-08 | M252Y/K288V/V308P/N434Y |
| F695 | 4.00E-08 | M252Y/K288W/V308P/N434Y |
| F696 | 4.40E-08 | M252Y/K288Y/V308P/N434Y |
| F697 | 3.10E-08 | S239K/M252Y/V308P/N325G/N434Y |
| F698 | 2.20E-08 | M252Y/N286E/T307Q/Q311A/N434Y |
| F699 | 2.30E-08 | S239K/M252Y/N286E/T307Q/Q311A/N434Y |
| F700 | 5.20E-08 | M252Y/V308P/L328E/N434Y |
| F705 | 7.10E-09 | M252Y/N286E/V308P/M428I/N434Y |
| F706 | 1.80E-08 | M252Y/N286E/T307Q/Q311A/M428I/N434Y |
| F707 | 5.90E-09 | M252Y/N286E/T307Q/V308P/Q311A/N434Y |
| F708 | 4.10E-09 | M252Y/N286E/T307Q/V308P/Q311A/M428I/N434Y |
| F709 | 2.00E-08 | S239K/M252Y/N286E/T307Q/Q311A/M428I/N434Y |
| F710 | 1.50E-08 | P238D/M252Y/N286E/T307Q/Q311A/M428I/N434Y |

(continued)

| F711 | 6.50E-08 | S239K/M252Y/T307Q/Q311A/N434Y |
|------|----------|-------------------------------|

Table 5-18 is a continuation table of Table 5-17.

[Table 5-18]

| F712 | 6.00E-08 | P238D/M252Y/T307Q/Q311A/N434Y |
|------|----------|-------------------------------|
| F713 | 2.00E-08 | P238D/M252Y/N286E/T307Q/Q311A/N434Y |
| F714 | 2.30E-07 | P238D/M252Y/N325S/N434Y |
| F715 | 2.30E-07 | P238D/M252Y/N325M/N434Y |
| F716 | 2.70E-07 | P238D/M252Y/N325L/N434Y |
| F717 | 2.60E-07 | P238D/M252Y/N325I/N434Y |
| F718 | 2.80E-07 | P238D/M252Y/Q295M/N434Y |
| F719 | 7.40E-08 | P238D/M252Y/N325G/N434Y |
| F720 | 2.40E-08 | M252Y/T307Q/V308P/Q311A/N434Y |
| F721 | 1.50E-08 | M252Y/T307Q/V308P/Q311A/M428I/N434Y |
| F722 | 2.70E-07 | P238D/M252Y/A327G/N434Y |
| F723 | 2.80E-07 | P238D/M252Y/L328D/N434Y |
| F724 | 2.50E-07 | P238D/M252Y/L328E/N434Y |
| F725 | 4.20E-08 | L235K/G237R/S239K/M252Y/V308P/N434Y |
| F726 | 3.70E-08 | L235K/P238K/S239K/M252Y/V308P/N434Y |
| F729 | 9.20E-07 | T307A/Q311A/N434Y |
| F730 | 6.00E-07 | T307Q/Q311A/N434Y |
| F731 | 8.50E-07 | T307A/Q311H/N434Y |
| F732 | 6.80E-07 | T307Q/Q311H/N434Y |
| F733 | 3.20E-07 | M252Y/L328E/N434Y |
| F734 | 3.10E-07 | G236D/M252Y/L328E/N434Y |
| F736 | 3.10E-07 | M252Y/S267M/L328E/N434Y |
| F737 | 3.10E-07 | M252Y/S267L/L328E/N434Y |
| F738 | 3.50E-07 | P238D/M252Y/T307P/N434Y |
| F739 | 2.20E-07 | M252Y/T307P/Q311A/N434Y |
| F740 | 2.90E-07 | M252Y/T307P/Q311H/N434Y |
| F741 | 3.10E-07 | P238D/T250A/M252Y/N434Y |
| F744 | 9.90E-07 | P238D/T250F/M252Y/N434Y |
| F745 | 6.60E-07 | P238D/T250G/M252Y/N434Y |
| F746 | 6.00E-07 | P238D/T250H/M252Y/N434Y |
| F747 | 2.80E-07 | P238D/T250I/M252Y/N434Y |
| F749 | 5.10E-07 | P238D/T250L/M252Y/N434Y |
| F750 | 3.00E-07 | P238D/T250M/M252Y/N434Y |
| F751 | 5.30E-07 | P238D/T250N/M252Y/N434Y |

Table 5-19 is a continuation table of Table 5-18.

[Table 5-19]

| | | |
|---|---|---|
| F753 | 1.80E-07 | P238D/T250Q/M252Y/N434Y |
| F755 | 3.50E-07 | P238D/T250S/M252Y/N434Y |
| F756 | 3.70E-07 | P238D/T250V/M252Y/N434Y |
| F757 | 1.20E-06 | P238D/T250W/M252Y/N434Y |
| F758 | 1.40E-06 | P238D/T250Y/M252Y/N434Y |
| F759 | | L235K/S239K |
| F760 | | L235R/S239K |
| F761 | 1.10E-06 | P238D/N434Y |
| F762 | 3.60E-08 | L235K/S239K/M252Y/N286E/T307Q/Q311A/N434Y |
| F763 | 3.50E-08 | L235R/S239K/M252Y/N286E/T307Q/Q311A/N434Y |
| F764 | 6.30E-07 | P238D/T307Q/Q311A/N434Y |
| F765 | 8.50E-08 | P238D/M252Y/T307Q/L309E/Q311A/N434Y |
| F766 | 6.00E-07 | T307A/L309E/Q311A/N434Y |
| F767 | 4.30E-07 | T307Q/L309E/Q311A/N434Y |
| F768 | 6.40E-07 | T307A/L309E/Q311H/N434Y |
| F769 | 4.60E-07 | T307Q/L309E/Q311H/N434Y |
| F770 | 3.00E-07 | M252Y/T256A/N434Y |
| F771 | 4.00E-07 | M252Y/E272A/N434Y |
| F772 | 3.80R-07 | M252Y/K274A/N434Y |
| F773 | 3.90E-07 | M252Y/V282A/N434Y |
| F774 | 4.00E-07 | M252Y/N286A/N434Y |
| F775 | 6.20E-07 | M252Y/K338A/N434Y |
| F776 | 3.90E-07 | M252Y/K340A/N434Y |
| F777 | 3.90E-07 | M252Y/E345A/N434Y |
| F779 | 3.90E-07 | M252Y/N361A/N434Y |
| F780 | 3.90E-07 | M252Y/Q362A/N434Y |
| F781 | 3.70E-07 | M252Y/S375A/N434Y |
| F782 | 3.50E-07 | M252Y/Y391A/N434Y |
| F783 | 4.00E-07 | M252Y/D413A/N434Y |
| F784 | 5.00E-07 | M252Y/L309A/N434Y |
| F785 | 7.40E-07 | M252Y/L309H/N434Y |
| F786 | 2.80E-08 | M252Y/S254T/N286E/T307Q/Q311A/N434Y |
| F787 | 8.80E-08 | M252Y/S254T/T307Q/L309E/Q311A/N434Y |
| F788 | 4.10E-07 | M252Y/N315A/N434Y |

Table 5-20 is a continuation table of Table 5-19.

[Table 5-20]

| | | |
|---|---|---|
| F789 | 1.50E-07 | M252Y/N315D/N434Y |

(continued)

| F790 | 2.70E-07 | M252Y/N315E/N434Y |
|------|----------|-------------------|
| F791 | 4.40E-07 | M252Y/N315F/N434Y |
| F792 | 4.40E-07 | M252Y/N315G/N434Y |
| F793 | 3.30E-07 | M252Y/N315I/N434Y |
| F794 | 4.10E-07 | M252Y/N315K/N434Y |
| F795 | 3.10E-07 | M252Y/N315L/N434Y |
| F796 | 3.40E-07 | M252Y/N315M/N434Y |
| F798 | 3.50E-07 | M252Y/N315Q/N434Y |
| F799 | 4.10E-07 | M252Y/N315R/N434Y |
| F800 | 3.80E-07 | M252Y/N315S/N434Y |
| F801 | 4.40E-07 | M252Y/N315T/N434Y |
| F802 | 3.30E-07 | M252Y/N315V/N434Y |
| F803 | 3.60E-07 | M252Y/N315W/N434Y |
| F804 | 4.00E-07 | M252Y/N315Y/N434Y |
| F805 | 3.00E-07 | M252Y/N325A/N434Y |
| F806 | 3.10E-07 | M252Y/N384A/N434Y |
| F807 | 3.20E-07 | M252Y/N389A/N434Y |
| F808 | 3.20E-07 | M252Y/N389A/N390A/N434Y |
| F809 | 2.20E-07 | M252Y/S254T/T256S/N434Y |
| F810 | 2.20E-07 | M252Y/A378V/N434Y |
| F811 | 4.90E-07 | M252Y/E380S/N434Y |
| F812 | 2.70E-07 | M252Y/E382V/N434Y |
| F813 | 2.80E-07 | M252Y/S424E/N434Y |
| F814 | 1.20E-07 | M252Y/N434Y/Y436I |
| F815 | 5.50E-07 | M252Y/N434Y/T437R |
| F816 | 3.60E-07 | P238D/T250V/M252Y/T307P/N434Y |
| F817 | 9.80E-08 | P238D/T250V/M252Y/T307Q/Q311A/N434Y |
| F819 | 1.40E-07 | P238D/M252Y/N286E/N434Y |
| F820 | 3.40E-07 | L235K/S239K/M252Y/N434Y |
| F821 | 3.10E-07 | L235R/S239K/M252Y/N434Y |
| F822 | 1.10E-06 | P238D/T250Y/M252Y/W313Y/N434Y |
| F823 | 1.10E-06 | P238D/T250Y/M252Y/W313F/N434Y |
| F828 | 2.50E-06 | P238D/T250V/M252Y/I253V/N434Y |

Table 5-21 is a continuation table of Table 5-20.

[Table 5-21]

| F831 | 1.60E-06 | P238D/T250V/M252Y/R255A/N434Y |
|------|----------|-------------------------------|
| F832 | 2.60E-06 | P238D/T250V/M252Y/R255D/N434Y |
| F833 | 8.00E-07 | P238D/T250V/M252Y/R255E/N434Y |

(continued)

| F834 | 8.10E-07 | P238D/T250V/M252Y/R255F/N434Y |
|---|---|---|
| F836 | 5.00E-07 | P238D/T250V/M252Y/R255H/N434Y |
| F837 | 5.60E-07 | P238D/T250V/M252Y/R255I/N434Y |
| F838 | 4.30E-07 | P238D/T250V/M252Y/R255K/N434Y |
| F839 | 3.40E-07 | P238D/T250V/M252Y/R255L/N434Y |
| F840 | 4.20E-07 | P238D/T250V/M252Y/R255M/N434Y |
| F841 | 1.10E-06 | P238D/T250V/M252Y/R255N/N434Y |
| F843 | 6.60E-07 | P238D/T250V/M252Y/R255Q/N434Y |
| F844 | 1.30E-06 | P238D/T250V/M252Y/R255S/N434Y |
| F847 | 3.40E-07 | P238D/T250V/M252Y/R255W/N434Y |
| F848 | 8.30E-07 | P238D/T250V/M252Y/R255Y/N434Y |
| F849 | 3.30E-07 | M252Y/D280A/N434Y |
| F850 | 2.90E-07 | M252Y/D280E/N434Y |
| F852 | 3.30E-07 | M252Y/D280G/N434Y |
| F853 | 3.20E-07 | M252Y/D280H/N434Y |
| F855 | 3.20E-07 | M252Y/D280K/N434Y |
| F858 | 3.20E-07 | M252Y/D280N/N434Y |
| F860 | 3.30E-07 | M252Y/D280Q/N434Y |
| F861 | 3.20E-07 | M252Y/D280R/N434Y |
| F862 | 3.00E-07 | M252Y/D280S/N434Y |
| F863 | 2.70E-07 | M252Y/D280T/N434Y |
| F867 | 2.80E-07 | M252Y/N384A/N389A/N434Y |
| F868 | 2.00E-08 | G236A/S239D/M252Y/N286E/T307Q/Q311A/N434Y |
| F869 | | G236A/S239D |
| F870 | 7.30E-08 | L235K/S239K/M252Y/T307Q/Q311A/N434Y |
| F871 | 7.10E-08 | L235R/S239K/M252Y/T307Q/Q311A/N434Y |
| F872 | 1.30E-07 | L235K/S239K/M252Y/N286E/N434Y |
| F873 | 1.20E-07 | L235R/S239K/M252Y/N286E/N434Y |
| F875 | 4.80E-07 | M252Y/N434Y/Y436A |
| F877 | 8.30E-07 | M252Y/N434Y/Y436E |
| F878 | 1.90E-07 | M252Y/N434Y/Y436F |

Table 5-22 is a continuation table of Table 5-21.

[Table 5-22]

| F879 | 9.20E-07 | M252Y/N434Y/Y436G |
|---|---|---|
| F880 | 3.90E-07 | M252Y/N434Y/Y436H |
| F881 | 3.10E-07 | M252Y/N434Y/Y436K |
| F882 | 1.30E-07 | M252Y/N434Y/Y436L |
| F883 | 2.10E-07 | M252Y/N434Y/Y436M |

(continued)

| F884 | 4.00E-07 | M252Y/N434Y/Y436N |
|------|----------|-------------------|
| F888 | 4.80E-07 | M252Y/N434Y/Y436S |
| F889 | 2.20E-07 | M252Y/N434Y/Y436T |
| F890 | 1.10E-07 | M252Y/N434Y/Y436V |
| F891 | 1.70E-07 | M252Y/N434Y/Y436W |
| F892 | 7.10E-08 | M252Y/S254T/N434Y/Y436I |
| F893 | 9.80E-08 | L235K/S239K/M252Y/N434Y/Y436I |
| F894 | 9.20E-08 | L235R/S239K/M252Y/N434Y/Y436I |
| F895 | 2.10E-08 | L235K/S239K/M252Y/N286E/T307Q/Q311A/N315E/N434Y |
| F896 | 2.00E-08 | L235R/S239K/M252Y/N286E/T307Q/Q311A/N315E/N434Y |
| F897 | 9.70E-08 | M252Y/N315D/N384A/N389A/N434Y |
| F898 | 1.70E-07 | M252Y/N315E/N384A/N389A/N434Y |
| F899 | 1.10E-07 | M252Y/N315D/G316A/N434Y |
| F900 | 1.70E-07 | M252Y/N315D/G316D/N434Y |
| F901 | 1.30E-07 | M252Y/N315D/G316E/N434Y |
| F902 | 2.20E-07 | M252Y/N315D/G316F/N434Y |
| F903 | 2.30E-07 | M252Y/N315D/G316H/N434Y |
| F904 | 1.00E-07 | M252Y/N315D/G316I/N434Y |
| F905 | 1.30E-07 | M252Y/N315D/G316K/N434Y |
| F906 | 1.50E-07 | M252Y/N315D/G316L/N434Y |
| F907 | 1.30E-07 | M252Y/N315D/G316M/N434Y |
| F908 | 1.50E-07 | M252Y/N315D/G316N/N434Y |
| F909 | 1.30E-07 | M252Y/N315D/G316P/N434Y |
| F910 | 1.40E-07 | M252Y/N315D/G316Q/N434Y |
| F911 | 1.30E-07 | M252Y/N315D/G316R/N434Y |
| F912 | 1.20E-07 | M252Y/N315D/G316S/N434Y |
| F913 | 1.10E-07 | M252Y/N315D/G316T/N434Y |
| F914 | 1.50E-07 | M252Y/N315D/G316V/N434Y |
| F915 | 2.30E-07 | M252Y/N315D/G316W/N434Y |

Table 5-23 is a continuation table of Table 5-22.

[Table 5-23]

| F917 | 2.50E-07 | M252Y/N286S/N434Y |
|------|----------|-------------------|
| F918 | 2.80E-07 | M252Y/D280E/N384A/N389A/N434Y |
| F919 | 3.30E-07 | M252Y/D280G/N384A/N389A/N434Y |
| F920 | 2.50E-07 | M252Y/N286S/N384A/N389A/N434Y |
| F921 | 1.20E-07 | M252Y/N286E/N384A/N389A/N434Y |
| F922 | 5.90E-08 | L235K/S239K/M252Y/N286E/N434Y/Y436I |
| F923 | 6.00E-08 | L235R/S239K/M252Y/N286E/N434Y/Y436I |

(continued)

| | | |
|---|---|---|
| F924 | 3.40E-08 | L235K/S239K/M252Y/T307Q/Q311A/N434Y/Y436I |
| F925 | 3.20E-08 | L235R/S239K/M252Y/T307Q/Q311A/N434Y/Y436I |
| F926 | 1.10E-07 | L235K/S239K/M252Y/S254T/N434Y/Y436I |
| F927 | 1.00E-07 | L235R/S239K/M252Y/S254T/N434Y/Y436I |
| F928 | 2.90E-08 | M252Y/T307Q/Q311A/N434Y/Y436I |
| F929 | 2.90E-08 | M252Y/S254T/T307Q/Q311A/N434Y/Y436I |
| F930 | 1.40E-07 | P238D/T250V/M252Y/N286E/N434Y |
| F931 | 1.20E-07 | P238D/T250V/M252Y/N434Y/Y436I |
| F932 | 3.20E-07 | T250V/M252Y/N434Y |
| F933 | 3.00E-07 | L234R/P238D/T250V/M252Y/N434Y |
| F934 | 3.10E-07 | G236K/P238D/T250V/M252Y/N434Y |
| F935 | 3.20E-07 | G237K/P238D/T250V/M252Y/N434Y |
| F936 | 3.20E-07 | G237R/P238D/T250V/M252Y/N434Y |
| F937 | 3.10E-07 | P238D/S239K/T250V/M252Y/N434Y |
| F938 | 1.60E-07 | L235K/S239K/M252Y/N434Y/Y436V |
| F939 | 1.50E-07 | L235R/S239K/M252Y/N434Y/Y436V |
| F940 | 1.50E-07 | P238D/T250V/M252Y/N434Y/Y436V |
| F941 | 1.20E-08 | M252Y/N286E/T307Q/Q311A/N434Y/Y436V |
| F942 | 4.20E-08 | L235K/S239K/M252Y/T307Q/Q311A/N434Y/Y436V |
| F943 | 4.00E-08 | L235R/S239K/M252Y/T307Q/Q311A/N434Y/Y436V |
| F944 | 1.70E-07 | T250V/M252Y/N434Y/Y436V |
| F945 | 1.70E-08 | T250V/M252Y/V308P/N434Y/Y436V |
| F946 | 4.30E-08 | T250V/M252Y/T307Q/Q311A/N434Y/Y436V |
| F947 | 1. 10E-08 | T250V/M252Y/T307Q/V308P/Q311A/N434Y/Y436V |
| F954 | 5.30E-07 | M252Y/N434Y/H435K/Y436V |
| F957 | 7.70E-07 | M252Y/N434Y/H435N/Y436V |
| F960 | 8.00E-07 | M252Y/N434Y/H435R/Y436V |

Table 5-24 is a continuation table of Table 5-23.

[Table 5-24]

| | | |
|---|---|---|
| F966 | 3.10E-07 | M252Y/S254A/N434Y |
| F970 | 2.50E-06 | M252Y/S254G/N434Y |
| F971 | 2.60E-06 | M252Y/S254H/N434Y |
| F972 | 2.60E-07 | M252Y/S254I/N434Y |
| F978 | 1.30E-06 | M252Y/S254Q/N434Y |
| F980 | 1.80E-07 | M252Y/S254V/N434Y |
| F987 | 4.00E-08 | P238D/T250V/M252Y/T307Q/Q311A/N434Y/Y436V |
| F988 | 6.90E-08 | P238D/T250V/M252Y/N286E/N434Y/Y436V |
| F989 | 1.40E-08 | L235R/S239K/M252Y/V308P/N434Y/Y436V |

(continued)

| F990 | 9.40E-09 | L235R/S239K/M252Y/T307Q/V308P/Q311A/N434Y/Y436V |
|---|---|---|
| F991 | 1.30E-08 | L235R/S239K/M252Y/N286E/T307Q/Q311A/N434Y/Y436V |
| F992 | 5.10E-08 | L235R/S239K/M252Y/T307Q/Q311A/M428I/N434Y/Y436V |
| F993 | 3.80E-08 | M252Y/T307Q/Q311A/N434Y/Y436V |
| F994 | 2.80E-07 | M252Y/N325G/N434Y |
| F995 | 2.90E-07 | L235R/P238D/S239K/M252Y/N434Y |
| F996 | 1.30E-07 | L235R/P238D/S239K/M252Y/N434Y/Y436V |
| F997 | 3.80E-07 | K248I/T250V/M252Y/N434Y/Y436V |
| F998 | 8.50E-07 | K248Y/T250V/M252Y/N434Y/Y436V |
| F999 | 2.10E-07 | T250V/M252Y/E258H/N434Y/Y436V |
| F1005 | | N325G |
| F1008 | 1.70E-07 | L235R/S239K/T250V/M252Y/N434Y/Y436V |
| F1009 | 1.20E-08 | L235R/S239K/T250V/M252Y/T307Q/V308P/Q311A/N434Y/Y436V |
| F1010 | 1.90E-07 | L235R/S239K/M252Y/T307A/Q311H/N434Y |
| F1011 | 4.50E-08 | T250V/M252Y/V308P/N434Y |
| F1012 | 4.70E-08 | L235R/S239K/T250V/M252Y/V308P/N434Y |
| F1013 | 3.00E-08 | T250V/M252Y/T307Q/V308P/Q311A/N434Y |
| F1014 | 3.20E-08 | L235R/S239K/T250V/M252Y/T307Q/V308P/Q311A/N434Y |
| F1015 | 2.20E-08 | L235R/S239K/M252Y/T307Q/V308P/Q311A/N434Y |
| F1016 | 3.80E-09 | T250V/M252Y/N286E/T307Q/V308P/Q311A/N434Y/Y436V |
| F1017 | 4.20E-09 | L235R/S239K/T250V/M252Y/N286E/T307Q/V308P/Q311A/N434Y/Y436 V |
| F1018 | 3.20E-09 | L235R/S239K/M252Y/N286E/T307Q/V308P/Q311A/N434Y/Y436V |
| F1019 | 3.40E-07 | P238D/T250V/M252Y/N325G/N434Y |
| F1020 | 8.50E-08 | P238D/T250V/M252Y/T307Q/Q311A/N325G/N434Y |

Table 5-25 is a continuation table of Table 5-24.

[Table 5-25]

| F1021 | 3.30E-07 | P238D/T250V/M252Y/N325A/N434Y |
|---|---|---|
| F1022 | | K326D/L328Y |
| F1023 | 4.40E-08 | S239D/T250V/M252Y/T307Q/Q311A/N434Y/Y436V |
| F1024 | 4.00E-08 | T250V/M252Y/T307Q/Q311A/K326D/L328Y/N434Y/Y436V |
| F1025 | 3.60E-08 | S239D/T250V/M252Y/T307Q/Q311A/K326D/L328Y/N434Y/Y436V |
| F1026 | 8.40E-08 | M252Y/T307A/Q311H/N434Y/Y436V |
| F1027 | 8.60E-08 | L235R/S239K/M252Y/T307A/Q311H/N434Y/Y436V |
| F1028 | 4.60E-08 | G236A/S239D/T250V/M252Y/T307Q/Q311A/N434Y/Y436V |
| F1029 | 5.10E-08 | T250V/M252Y/T307Q/Q311A/I332E/N434Y/Y436V |
| F1030 | | I332E |
| F1031 | 5.30E-08 | G236A/S239D/T250V/M252Y/T307Q/Q311A/I332E/N434Y/Y436V |
| F1032 | 4.30E-08 | P238D/T250V/M252Y/T307Q/Q311A/N325G/N434Y/Y436V |

(continued)

| F1033 | 1.00E-06 | P238D/N434W |
|---|---|---|
| F1034 | 1.50E-08 | L235K/S239K/M252Y/V308P/N434Y/Y436V |
| F1035 | 1.00E-08 | L235K/S239K/M252Y/T307Q/V308P/Q311A/N434Y/Y436V |
| F1036 | 1.40E-08 | L235K/S239K/M252Y/N286E/T307Q/Q311A/N434Y/Y436V |
| F1037 | 6.10E-08 | L235K/S239K/M252Y/T307Q/Q311A/M428I/N434Y/Y436V |
| F1038 | 2.80E-07 | L235K/P238D/S239K/M252Y/N434Y |
| F1039 | 1.30E-07 | L235K/P238D/S239K/M252Y/N434Y/Y436V |
| F1040 | 2.00E-07 | L235K/S239K/T250V/M252Y/N434Y/Y436V |
| F1041 | 1.40E-08 | L235K/S239K/T250V/M252Y/T307Q/V308P/Q311A/N434Y/Y436V |
| F1042 | 2.00E-07 | L235K/S239K/M252Y/T307A/Q311H/N434Y |
| F1043 | 5.20E-08 | L235K/S239K/T250V/M252Y/V308P/N434Y |
| F1044 | 3.50E-08 | L235K/S239K/T250V/M252Y/T307Q/V308P/Q311A/N434Y |
| F1045 | 2.50E-08 | L235K/S239K/M252Y/T307Q/V308P/Q311A/N434Y |
| F1046 | 4.50E-09 | L235K/S239K/T250V/M252Y/N286E/T307Q/V308P/Q311A/N434Y/Y436 V |
| F1047 | 3.40E-09 | L235K/S239K/M252Y/N286E/T307Q/V308P/Q311A/N434Y/Y436V |
| F1048 | 9.90E-08 | L235K/S239K/M252Y/T307A/Q311H/N434Y/Y436V |
| F1050 | 3.50E-09 | T250V/M252Y/N286E/T307Q/V308P/Q311A/M428I/N434Y/Y436V |
| F1051 | 3.90E-09 | L235R/S239K/T250V/M252Y/N286E/T307Q/V308P/Q311A/M428I/N434 Y/Y436V |
| F1052 | 3.20E-09 | L235R/S239K/M252Y/N286E/T307Q/V308P/Q311A/M428I/N434Y/Y436 V |

Table 5-26 is a continuation table of Table 5-25.

[Table 5-26]

| F1053 | 4.23E-08 | L235R/S239K/T250V/M252Y/T307Q/Q311A/N434Y/Y436V |
|---|---|---|
| F1058 | 1.31E-07 | M252Y/Q386E/N434Y/Y436V |
| F1059 | 1.39E-07 | M252Y/Q386R/N434Y/Y436V |
| F1060 | 1.43E-07 | M252Y/Q386S/N434Y/Y436V |
| F1061 | 1.19E-07 | M252Y/P387E/N434Y/Y436V |
| F1062 | 1.2E-07 | M252Y/P387R/N434Y/Y436V |
| F1063 | 1.43E-07 | M252Y/P387S/N434Y/Y436V |
| F1064 | 1.32E-07 | M252Y/V422E/N434Y/Y436V |
| F1065 | 1.38E-07 | M252Y/V422R/N434Y/Y436V |
| F1066 | 1.45E-07 | M252Y/V422S/N434Y/Y436V |
| F1067 | 1.26E-07 | M252Y/S424E/N434Y/Y436V |
| F1068 | 1.69E-07 | M252Y/S424R/N434Y/Y436V |
| F1069 | 1.39E-07 | M252Y/N434Y/Y436V/Q438E |
| F1070 | 1.73E-07 | M252Y/N434Y/Y436V/Q438R |
| F1071 | 1.24E-07 | M252Y/N434Y/Y436V/Q438S |
| F1072 | 1.35E-07 | M252Y/N434Y/Y436V/S440E |
| F1073 | 1.34E-07 | M252Y/N434Y/Y436V/S440R |

(continued)

| F1074 | 1.32E-07 | S239D/M252Y/N434Y/Y436V |
|---|---|---|
| F1075 | 1.4E07 | M252Y/K326D/L328Y/N434Y/Y436V |
| F1076 | 1.27E-07 | S239D/M252Y/K326D/L328Y/N434Y/Y436V |
| F1077 | 2.03E-06 | K248N/M252Y/N434Y |
| F1078 | 4.7E-07 | M252Y/E380N/E382S/N434Y |
| F1079 | 3.44E-07 | M252Y/E382N/N384S/N434Y |
| F1080 | 3.19E-07 | M252Y/S424N/N434Y |
| F1081 | 6.2E-07 | M252Y/N434Y/Y436N/Q438T |
| F1082 | 2.76E-07 | M252Y/N434Y/Q438N |
| F1083 | 3.45E-07 | M252Y/N434Y/S440N |
| F1094 | 2.6E-07 | M252Y/N434Y/S442N |
| F1095 | 2.86E-07 | M252Y/S383N/G385S/N434Y |
| F1096 | 2.72E-07 | M252Y/Q386T/N434Y |
| F1097 | 2.82E-07 | M252Y/G385N/P387S/N434Y |
| F1098 | 2.58E-07 | S239D/M252Y/N434Y |
| F1099 | 2.57E-07 | M252Y/K326D/L328Y/N434Y |
| F1100 | 2.41E-07 | S239D/M252Y/K326D/L328Y/N434Y |
| F1101 | 6.59E-08 | S239D/M252Y/T307Q/Q311A/N434Y |
| F1102 | 6.46E-08 | M252Y/T307Q/Q311A/K326D/L328Y/N434Y |
| F1103 | 6.11E-08 | S239D/M252Y/T307Q/Q311A/K326D/L328Y/N434Y |
| F1104 | 1.77E-07 | M252Y/V422E/S424R/N434Y/Y436V |
| F1105 | 1.54E-07 | M252Y/V422S/S424R/N434Y/Y436V |
| F1106 | 1.42E-07 | M252Y/N434Y/Y436V/Q438R/S440E |
| F1107 | 1.23E-07 | M252Y/V422D/N434Y/Y436V |

Table 5-27 is a continuation table of Table 5-26.

[Table 5-27]

| F1108 | 1.26E-07 | M252Y/V422K/N434Y/Y436V |
|---|---|---|
| F1109 | 1.27E-07 | M252Y/V422T/N434Y/Y436V |
| F1110 | 1.33E-07 | M252Y/V422Q/N434Y/Y436V |
| F1111 | 1.65E-07 | M252Y/S424K/N434Y/Y436V |
| F1112 | 1.23E-07 | M2 52Y/N434Y/Y436V/ Q438K |
| F1113 | 1.18E-07 | M252Y/N434Y/Y436V/S440D |
| F1114 | 1.31E-07 | M252Y/N434Y/Y436V/S440Q |
| F1115 | 1.35E-07 | M252Y/S424N/N434Y/Y436V |
| F1116 | 7.44E-08 | M252Y/T307Q/Q311A/S424N/N434Y |
| P1117 | 4.87E-08 | T250V/M252Y/T307Q/Q311A/S424N/N434Y/Y436V |
| F1118 | 1.32E-08 | T250V/M252Y/T307Q/V308P/Q311A/S424N/N434Y/Y436V |
| F1119 | 1.03E-08 | T250V/M252Y/T307Q/V308P/Q311A/V422E/N434Y/Y436V |

(continued)

| F1120 | 1.04E-08 | T250V/M252Y/T307Q/V308P/Q311A/S424R/N434Y/Y436V |
|---|---|---|
| F1121 | 1.04E-08 | T250V/M252Y/T307Q/V308P/Q311A/V422E/S424R/N434Y/Y436V |
| F1122 | 1.37E-08 | T250V/M252Y/T307Q/V308P/Q311A/N434Y/Y436V/Q438R |
| F1123 | 9.55E-09 | T250V/M252Y/T307Q/V308P/Q311A/N434Y/Y436V/S440E |
| F1124 | 1.22E-08 | T250V/M252Y/T307Q/V308P/Q311A/N434Y/Y436V/Q438R/S440E |
| F1125 | 5.18E-08 | M252Y/T307Q/N434Y/Y436V |
| F1126 | 8.95E-08 | M252Y/T307A/N434Y/Y436V |
| F1127 | 7.94E-08 | M252Y/Q311A/N434Y/Y436V |
| F1128 | 1.17E-07 | M252Y/Q311H/N434Y/Y436V |
| F1129 | 4.48E-08 | M252Y/T307Q/Q311H/N434Y/Y436V |
| F1130 | 5.54E-08 | M252Y/T307A/Q311A/N434Y/Y436V |
| F1131 | 1.29E-07 | L235R/S239K/M252Y/V422E/N434Y/Y436V |
| F1132 | 1.4E-07 | L235R/S239K/M252Y/V422S/N434Y/Y436V |
| F1133 | 1.58E-07 | L235R/S239K/M252Y/S424R/N434Y/Y436V |
| F1134 | 1.66E-07 | L235R/S239K/M252Y/N434Y/Y436V/Q438R |
| F1135 | 1.26E-07 | L235R/S239K/M252Y/N434Y/Y436V/S440E |
| F1136 | 1.63E-07 | L235R/S239K/M252Y/V422E/S424R/N434Y/Y436V |
| F1137 | 1.58E-07 | L235R/S239K/M252Y/V422S/S424R/N434Y/Y436V |
| F1138 | 1.65E-07 | L235R/S239K/M252Y/N434Y/Y436V/Q438R/S440E |
| F1139 | 1.52E-07 | L235R/S239K/M252Y/S424N/N434Y/Y436V |
| F1140 | 1.62E-07 | M252Y/V422E/S424R/N434Y/Y436V/Q438R/S440E |
| F1141 | 1.77E-07 | M252Y/V422S/S424R/N434Y/Y436V/Q438R/S440E |
| F1142 | 1.87E-07 | L235R/S239K/M252Y/V422E/S424R/N434Y/Y436V/Q438R/S440E |
| F1143 | 1.98E-07 | L235R/S239K/M252Y/V422S/S424R/N434Y/Y436V/Q438R/S440E |
| F1144 | 1.44E-08 | L235R/S239K/T250V/M252Y/T307Q/V308P/Q311A/N434Y/Y436V/Q438R/S440E |
| F1145 | 5.23E-08 | T250V/M252Y/T307Q/Q311A/N434Y/Y436V/Q438R/S440E |
| F1146 | 6.24E-08 | L235R/S239K/T250V/M252Y/T307Q/Q311A/N434Y/Y436V/Q438R/S440E |
| F1147 | 7.19E-08 | M252Y/T307Q/Q311A/N434Y/Q438R/S440E |

Table 5-28 is a continuation table of Table 5-27.

[Table 5-28]

| F1148 | 7.63E-08 | L235R/S239K/M252Y/T307Q/Q311A/N434Y/Q438R/S440E |
|---|---|---|
| F1151 | 2.51E-07 | L235R/S239K/M252Y/S424N/N434Y |
| F1152 | 7.38E-08 | L235R/S239K/M252Y/T307Q/Q311A/S424N/N434Y |
| F1153 | 4.85E-08 | L235R/S239K/T250V/M252Y/T307Q/Q311A/S424N/N434Y/Y436V |
| F1154 | 1.34E-08 | L235R/S239K/T250V/M252Y/T307Q/V308P/Q311A/S424N/N434Y/Y436V |
| F1157 | 2.09E-07 | M252Y/N434Y/Q438R/S440E |
| F1158 | 2.44E-07 | L235R/S239K/M252Y/N434Y/Q438R/S440E |
| F1159 | 4.79E-07 | S424N/N434W |

(continued)

| F1160 | 2.88E-07 | V308F/S424N/N434Y |
|---|---|---|
| F1161 | 1.07E-06 | I332V/S424N/N434Y |
| F1162 | 3.43E-07 | P238D/T250Y/M252Y/N434Y/Y436V |
| F1163 | 1.54E-07 | P238D/T250Y/M252Y/T307Q/Q311A/N434Y |
| F1164 | 6.96E-08 | P238D/T250Y/M252Y/T307Q/Q311A/N434Y/Y436V |
| F1165 | 1.63E-08 | P238D/T250Y/M252Y/T307Q/V308P/Q311A/N434Y/Y436V |
| F1174 | 4.9E-07 | P2571/N434H |
| F1176 | 1.98E-06 | V308F |
| F1178 | 8.72E-07 | V2591/V308F/M428L |
| F1183 | 1.28E-06 | E380A/M428L/N434S |
| F1184 | 1E-06 | T307A/M428L/N434S |
| F1185 | 9.17E-07 | T307A/E380A/M428L/N434S |
| F1188 | 1.72E-06 | T307A/E380A/N434H |
| F1189 | 1.57E-07 | M252Y/H433D/N434Y/Y436V/Q438R/S440E |
| F1190 | 2.4E-07 | M252Y/H433E/N434Y/Y436V/Q438R/S440E |
| F1191 | 2.11E-07 | M252Y/N434Y/Y436V/T437A/Q438R/S440E |
| F1192 | 1.27E-07 | M252Y/N434Y/Y436V/T437G/Q438R/S440E |
| F1194 | 1.55E-07 | M252Y/N434Y/Y436V/Q438R/K439D/S440E |
| F1195 | 1.76E-07 | M252Y/N434Y/Y436V/Q438R/S440E/L441A |
| F1196 | 1.51E-07 | M252Y/N434Y/Y436V/Q438R/S440E/L441E |
| F1197 | 9.46E-08 | M252Y/S254T/N434Y/Y436V/Q438R/S440E |
| F1198 | 7.83E-08 | M252Y/T256E/N434Y/Y436V/Q438R/S440E |
| F1199 | 6.25E-08 | M252Y/S254T/T256E/N434Y/Y436V/Q438R/S440E |
| F1200 | 1.26E-07 | T250V/M252Y/S254T/N434Y/Y436V/Q438R/S440E |
| F1201 | 1.07E-07 | T250V/M252Y/T256E/N434Y/Y436V/Q438R/S440E |
| F1202 | 8.81E-08 | T250V/M252Y/S254T/T256E/N434Y/Y436V/Q438R/S440E |
| F1203 | 1.52E-07 | M252Y/T256Q/N434Y/Y436V/Q438R/S440E |
| F1204 | 1.18E-07 | M252Y/S254T/T256Q/N434Y/Y436V/Q438R/S440E |
| F1205 | 1.98E-07 | T250V/M252Y/T256Q/N434Y/Y436V/Q438R/S440E |
| F1206 | 1.69E-07 | T250V/M252Y/S254T/T256Q/N434Y/Y436V/Q438R/S440E |
| F1207 | 1.11E-06 | I332E/M428L/N434S |
| F1208 | 5.71E-07 | L251A/M252Y/N434Y/Y436V |
| F1211 | 1.23E-06 | L251H/M252Y/N434Y/Y436V |

Table 5-29 is a continuation table of Table 5-28.

[Table 5-29]

| F1213 | 6.33E-07 | L251N/M252Y/N434Y/Y436V |
|---|---|---|
| F1216 | 1.16E-06 | L251S/M252Y/N434Y/Y436V |
| F1217 | 1.14E-06 | L251T/M252Y/N434Y/Y436V |

(continued)

| F1218 | 2.51E-07 | L251V/M252Y/N434Y/Y436V |
|---|---|---|
| F1229 | 2.81E-06 | M252Y/I253V/N434Y/Y436V |
| F1230 | 1.12E-07 | M252Y/N434Y/Y436V/Q438R/S440D |
| F1231 | 9.73E-08 | M252Y/N434Y/Y436V/Q438K/S440E |
| F1232 | 9.79E-08 | M252Y/N434Y/Y436V/Q438K/S440D |
| F1243 | 1.25E-07 | L235R/S239K/M252Y/S254T/N434Y/Y436V/Q438R/S440E |
| F1244 | 1.02E-07 | L235R/S239K/M252Y/T256E/N434Y/Y436V/Q438R/S440E |
| F1245 | 8.2E-08 | L235R/S239K/M252Y/S254T/T256E/N434Y/Y436V/Q438R/S440E |
| F1246 | 1.73E-07 | L235R/S239K/T250V/M252Y/S254T/N434Y/Y436V/Q438R/S440E |
| F1247 | 1.45E-07 | L235R/S239K/T250V/M252Y/T256E/N434Y/Y436V/Q438R/S440E |
| F1248 | 1.2E-07 | L235R/S239K/T250V/M252Y/S254T/T256E/N434Y/Y436V/Q438R/S440E |
| F1249 | 2.06E-07 | L235R/S239K/M252Y/T256Q/N434Y/Y436V/Q438R/S440E |
| F1250 | 1.66E-07 | L235R/S239K/M252Y/S254T/T256Q/N434Y/Y436V/Q438R/S440E |
| F1251 | 2.77E-07 | L235R/S239K/T250V/M252Y/T256Q/N434Y/Y436V/Q438R/S440E |
| F1252 | 2.33E-07 | L235R/S239K/T250V/M252Y/S254T/T256Q/N434Y/Y436V/Q438R/S440E |
| F1253 | 1.12E-07 | L235R/S239K/M252Y/T307A/N434Y/Y436V/Q438R/S440E |
| F1254 | 6.42E-08 | L235R/S239K/M252Y/T307Q/N434Y/Y436V/Q438R/S440E |
| F1255 | 1.11E-07 | L235R/S239K/M252Y/Q311A/N434Y/Y436V/Q438R/S440E |
| F1256 | 1.56E-07 | L235R/S239K/M252Y/Q311H/N434Y/Y436V/Q438R/S440E |
| F1257 | 7.81E-08 | L235R/S239K/M252Y/T307A/Q311A/N434Y/Y436V/Q438R/S440E |
| F1258 | 1.05E-07 | L235R/S239K/M252Y/T307A/Q311H/N434Y/Y436V/Q438R/S440E |
| F1259 | 4.46E-08 | L235R/S239K/M252Y/T307Q/Q311A/N434Y/Y436V/Q438R/S440E |
| F1260 | 6.53E-08 | L235R/S239K/M252Y/T307Q/Q311H/N434Y/Y436V/Q438R/S440E |
| F1261 | 1.35E-07 | L235R/S239K/M252Y/N434Y/Y436V/Q438R/S440D |
| F1262 | 1.26E-07 | L235R/S239K/M252Y/N434Y/Y436V/Q438K/S440E |
| F1263 | 1.24E-07 | L235R/S239K/M252Y/N434Y/Y436V/Q438K/S440D |
| F1264 | 1.27E-07 | L235R/S239K/M252Y/T256A/N434Y/Y436V/Q438R/S440E |
| F1265 | 1.57E-07 | L235R/S239K/M252Y/T256G/N434Y/Y436V/Q438R/S440E |
| F1266 | 9.99E-08 | L235R/S239K/M252Y/T256N/N434Y/Y436V/Q438R/S440E |
| F1267 | 1.5E-07 | L235R/S239K/M252Y/S254A/N434Y/Y436V/Q438R/S440E |
| F1268 | 2E-07 | L235R/S239K/M252Y/H433D/N434Y/Y436V/Q438R/S440E |
| F1269 | 1.69E-07 | L235R/S239K/M252Y/H433D/N434Y/Y436V/Q438K/S440D |
| F1270 | 1.18E-07 | L235R/S239K/M252Y/S254A/N434Y/Y436V/Q438K/S440D |
| F1271 | 2.05E-07 | L235R/S239K/M252Y/S254A/H433D/N434Y/Y436V/Q438R/S440E |
| F1272 | 1.71E-07 | L235R/S239K/M252Y/S254A/H433D/N434Y/Y436V/Q438K/S440D |
| F1273 | 1.53E-07 | L235R/S239K/M252Y/T256Q/N434Y/Y436V/Q438K/S440D |
| F1274 | 2.48E-07 | L235R/S239K/M252Y/T256Q/H433D/N434Y/Y436V/Q438R/S440E |
| F1275 | 2.09E-07 | L235R/S239K/M252Y/T256Q/H433D/N434Y/Y436V/Q438K/S440D |

Table 5-30 is a continuation table of Table 5-29.

[Table 5-30]

| F1276 | 1.02E-07 | L235R/S239K/M252Y/T256A/N434Y/Y436V/Q438K/S440D |
|-------|----------|-------------------------------------------------|
| F1277 | 1.69E-07 | L235R/S239K/M252Y/T256A/H433D/N434Y/Y436V/Q438R/S440E |
| F1278 | 1.4E-07 | L235R/S239K/M252Y/T256A/H433D/N434Y/Y436V/Q438K/S440D |
| F1279 | 1.23E-07 | L235R/S239K/M252Y/T256G/N434Y/Y436V/Q438K/S440D |
| F1280 | 2.09E-07 | L235R/S239K/M252Y/T256G/H433D/N434Y/Y436V/Q438R/S440E |
| F1281 | 1.74E-07 | L235R/S239K/M252Y/T256G/H433D/N434Y/Y436V/Q438K/S440D |
| F1282 | 7.69E-08 | L235R/S239K/M252Y/T256N/N434Y/Y436V/Q438K/S440D |
| F1283 | 1.34E-07 | L235R/S239K/M252Y/T256N/H433D/N434Y/Y436V/Q438R/S440E |
| F1284 | 1.12E-07 | L235R/S239K/M252Y/T256N/H433D/N434Y/Y436V/Q438K/S440D |
| F1285 | 9.36E-08 | L235R/S239K/M252Y/S254T/N434Y/Y436V/Q438K/S440D |
| F1286 | 1.57E-07 | L235R/S239K/M252Y/S254T/H433D/N434Y/Y436V/Q438R/S440E |
| F1287 | 1.5E-07 | L235R/S239K/M252Y/S254T/H433D/N434Y/Y436V/Q438K/S440D |
| F1288 | 7.95E-08 | L235R/S239K/M252Y/T256E/N434Y/Y436V/Q438K/S440D |
| F1289 | 1.33E-07 | L235R/S239K/M252Y/T256E/H433D/N434Y/Y436V/Q438R/S440E |
| F1290 | 1.11E-07 | L235R/S239K/M252Y/T256E/H433D/N434Y/Y436V/Q438K/S440D |
| F1291 | 1.51E-07 | L235R/S239K/M252Y/H433D/N434Y/Y436V |
| F1292 | 4.24E-07 | L235R/S239K/H433D/N434W/Y436V/Q438R/S440E |
| F1293 | 1.61E-07 | L235R/S239K/M252Y/T256E/N434Y/Q438R/S440E |
| F1294 | 2E-07 | L235R/S239K/M252Y/T256E/N434Y/Y436T/Q438R/S440E |
| F1295 | 9.84E-08 | L235R/S239K/M252Y/T256E/N434Y/Y436F/Q438R/S440E |
| F1296 | 2.27E-07 | L235R/S239K/M252Y/T256E/H433D/N434Y/Q438R/S440E |
| F1297 | 2.5E-07 | L235R/S239K/M252Y/T256E/H433D/N434Y/Y436T/Q438R/S440E |
| F1298 | 1.47E-07 | L235R/S239K/M252Y/T256E/H433D/N434Y/Y436F/Q438R/S440E |
| F1299 | 1.5E-07 | L235R/S239K/M252Y/T256E/N434Y/Q438K/S440D |
| F1300 | 1.63E-07 | L235R/S239K/M252Y/T256E/N434Y/Y436T/Q438K/S440D |
| F1301 | 8.3E-08 | L235R/S239K/M252Y/T256E/N434Y/Y436F/Q438K/S440D |
| F1302 | 2.15E-07 | L235R/S239K/M252Y/T256E/H433D/N434Y/Q438K/S440D |
| F1303 | 2.1E-07 | L235R/S239K/M252Y/T256E/H433D/N434Y/Y436T/Q438K/S440D |
| F1304 | 1.24E-07 | L235R/S239K/M252Y/T256E/H433D/N434Y/Y436F/Q438K/S440D |
| F1305 | 2.05E-07 | L235R/S239K/M252Y/H433D/N434Y/Y436V/Q438R/S440D |
| F1306 | 1.92E-07 | L235R/S239K/M252Y/H433D/N434Y/Y436V/Q438K/S440E |
| F1307 | 1.44E-07 | L235R/S239K/M252Y/V422A/S424A/N434Y/Y436V |
| F1308 | 2.06E-07 | L235R/S239K/M252Y/V422L/S424L/N434Y/Y436V |
| F1309 | 1.26E-07 | L235R/S239K/M252Y/N434Y/Y436V/Q438A/S440A |
| F1310 | 2.28E-07 | L235R/S239K/M252Y/N434Y/Y436V/Q438L/S440L |
| F1311 | 1.69E-07 | L235R/S239K/M252Y/V422A/S424A/H433D/N434Y/Y436V |
| F1312 | 1.79E-07 | L235R/S239K/M252Y/V422L/S424L/H433D/N434Y/Y436V |
| F1313 | 1.77E-07 | L235R/S239K/M252Y/H433D/N434Y/Y436V/Q438A/S440A |

(continued)

| F1314 | 2.27E-07 | L235R/S239K/M252Y/H433D/N434Y/Y436V/Q438L/S440L |
|---|---|---|
| F1315 | 1.52E-07 | G237K/S239K/M252Y/N434Y/Y436V |
| F1316 | 1.49E-07 | G237R/S239K/M252Y/N434Y/Y436V |

Table 5-31 is a continuation table of Table 5-30.

[Table 5-31]

| F1317 | 1.38E-07 | S239K/M252Y/P329K/N434Y/Y436V |
|---|---|---|
| F1318 | 1.43E-07 | S239K/M252Y/P329R/N434Y/Y436V |
| F1319 | 2.67E-07 | M252Y/L328Y/N434Y |
| F1320 | 1.22E-07 | L235R/S239K/M252Y/S254T/N434Y/Y436V/Q438R/S440D |
| F1321 | 1.03E-07 | L235R/S239K/M252Y/S254T/N434Y/Y436V/Q438K/S440E |
| F1322 | 1.6E-07 | L235R/S239K/M252Y/S254T/H433D/N434Y/Y436V/Q438R/S440D |
| F1323 | 1.49E-07 | L235R/S239K/M252Y/S254T/H433D/N434Y/Y436V/Q438K/S440E |
| F1324 | 1.32E-07 | L234A/L235A/M252Y/N434Y/Y436V |
| F1325 | 2.13E-07 | L234A/L235A/M252Y/N297A/N434Y/Y436V |
| F1326 | 1.09E-08 | L234A/L235A/T250V/M252Y/T307Q/V308P/Q311A/N434Y/Y436V |
| F1327 | 1.41E-08 | L234A/L235A/T250V/M252Y/N297A/T307Q/V308P/Q311A/N434Y/Y436V |
| F1328 | 1.52E-07 | L235R/G236R/S239K/M252Y/N434Y/Y436V/Q438R/S440E |
| F1329 | 1.29E-07 | L235R/G236R/S239K/M252Y/S254T/N434Y/Y436V/Q438R/S440E |
| F1330 | 1.03E-07 | L235R/G236R/S239K/M252Y/T256E/N434Y/Y436V/Q438R/S440E |
| F1331 | 7.75E-08 | L235R/G236R/S239K/M252Y/S254T/T256E/N434Y/Y436V/Q438R/S440E |
| F1333 | 1.23E-07 | L235R/G236R/S239K/M252Y/N434Y/Y436V |
| F1334 | 1.04E-07 | L235R/G236R/S239K/M252Y/N434Y/Y436V/Q438K/S440D |
| F1335 | 8.78E-08 | L235R/G236R/S239K/M252Y/S254T/N434Y/Y436V/Q438K/S440D |
| F1336 | 7.18E-08 | L235R/G236R/S239K/M252Y/T256E/N434Y/Y436V/Q438K/S440D |
| F1337 | 7.41E-08 | L235R/S239K/M252Y/T256E/N434Y/Y436V/Q438K/S440E |
| F1338 | 1.04E-07 | L235R/S239K/M252Y/T256E/H433D/N434Y/Y436V/Q438K/S440E |
| F1339 | 2.51E-07 | L235R/S239K/M252Y/S254T/T256E/H433D/N434Y/Y436T/Q438K/S440E |
| F1340 | 5.58E-08 | L235R/S239K/M252Y/S254T/T256E/N434Y/Y436V/Q438K/S440E |
| F1341 | 3.22E-07 | L235R/S239K/M252Y/S254T/N434Y/Y436T/Q438K/S440E |
| F1342 | 2.51E-07 | L235R/S239K/M252Y/T256E/N434Y/Y436T/Q438K/S440E |
| F1343 | 2.01E-07 | L235R/S239K/M252Y/S254T/T256E/N434Y/Y436T/Q438K/S440E |
| F1344 | 3.96E-07 | L235R/S239K/M252Y/N434Y/Y436T/Q438K/S440E |
| F1345 | 1.05E-07 | L235R/G236R/S239K/M252Y/N434Y/Y436V/Q438K/S440E |
| F1346 | 8.59E-08 | L235R/G236R/S239K/M252Y/S254T/N434Y/Y436V/Q438K/S440E |
| F1347 | 7.14E-08 | L235R/G236R/S239K/M252Y/T256E/N434Y/Y436V/Q438K/S440E |
| F1348 | 5.52E-08 | L235R/G236R/S239K/M252Y/S254T/T256E/N434Y/Y436V/Q438K/S440E |
| F1349 | 3.36E-07 | L235R/S239K/M252Y/N434Y/Y436T/Q438R/S440E |
| F1350 | 1.18E-07 | L235R/S239K/M252Y/N434Y/Y436F/Q438K/S440E |

(continued)

| F1351 | 1.62E-07 | L235R/S239K/M252Y/N434Y/Y436F/Q438R/S440E |
|---|---|---|
| F1352 | 3.93E-07 | L235R/S239K/M252Y/H433D/N434Y/Y436T/Q438K/S440E |
| F1353 | 4.33E-07 | L235R/S239K/M252Y/H433D/N434Y/Y436T/Q438R/S440E |
| F1354 | 2.29E-07 | L235R/S239K/M252Y/H433D/N434Y/Y436F/Q438K/S440E |
| F1355 | 2.47E-07 | L235R/S239K/M252Y/H433D/N434Y/Y436F/Q438R/S440E |
| F1356 | 1.58E-07 | G236R/M252Y/L328R/N434Y/Y436V |
| F1357 | 2.81E-07 | L235R/S239K/M252Y/S254T/N434Y/Y436T/Q438R/S440E |
| F1358 | 9.07E-08 | L235R/S239K/M252Y/S254T/N434Y/Y436F/Q438K/S440E |

Table 5-32 is a continuation table of Table 5-31.

[Table 5-32]

| F1359 | 1.28E-07 | L235R/S239K/M252Y/S254T/N434Y/Y436F/Q438R/S440E |
|---|---|---|
| F1360 | 3.12E-07 | L235R/S239K/M252Y/S254T/H433D/N434Y/Y436T/Q438K/S440E |
| F1361 | 3.52E-07 | L235R/S239K/M252Y/S254T/H433D/N434Y/Y436T/Q438R/S440E |
| F1362 | 1.41E-07 | L235R/S239K/M252Y/S254T/H433D/N434Y/Y436F/Q438K/S440E |
| F1363 | 1.9E-07 | L235R/S239K/M252Y/S254T/H433D/N434Y/Y436F/Q438R/S440E |
| F1364 | 7.49E-08 | L235R/S239K/M252Y/T256E/N434Y/Y436F/Q438K/S440E |
| F1365 | 3.14E-07 | L235R/S239K/M252Y/T256E/H433D/N434Y/Y436T/Q438K/S440E |
| F1366 | 1.17E-07 | L235R/S239K/M252Y/T256E/H433D/N434Y/Y436F/Q438K/S440E |
| F1367 | 1.79E-07 | L235R/S239K/M252Y/S254T/T256E/N434Y/Y436T/Q438R/S440E |
| F1368 | 5.49E-08 | L235R/S239K/M252Y/S254T/T256E/N434Y/Y436F/Q438K/S440E |
| F1369 | 7.6E-08 | L235R/S239K/M252Y/S254T/T256E/N434Y/Y436F/Q438R/S440E |
| F1370 | 9.14E-08 | L235R/S239K/M252Y/S254T/T256E/H433D/N434Y/Y436V/Q438K/S440E |
| F1371 | 1.09E-07 | L235R/S239K/M252Y/S254T/T256E/H433D/N434Y/Y436V/Q438R/S440E |
| F1372 | 2.28E-07 | L235R/S239K/M252Y/S254T/T256E/H433D/N434Y/Y436T/Q438R/S440E |
| F1373 | 8.67E-08 | L235R/S239K/M252Y/S254T/T256E/H433D/N434Y/Y436F/Q438K/S440E |
| F1374 | 1.2E-07 | L235R/S239K/M252Y/S254T/T256E/H433D/N434Y/Y436F/Q438R/S440E |
| F1375 | 1.03E-07 | L235R/S239K/M252Y/S254T/N434Y/Y436V |
| F1376 | 9.09E-08 | L235R/S239K/M252Y/S254T/T256E/N434Y/Y436V |
| F1377 | 8.27E-08 | L235R/S239K/M252Y/T256E/N434Y/Y436V |
| F1378 | 3.61E-07 | L235R/S239K/M252Y/N434Y/Y436T |
| F1379 | 2.85E-07 | L235R/S239K/M252Y/N434Y/Y436F |

Fcγ receptor

Fcγ receptor (also described as FcγR) refers to a receptor capable of binding to the Fc region of monoclonal IgG1, IgG2, IgG3, or IgG4 antibodies, and includes all members belonging to the family of proteins substantially encoded by an Fcγ receptor gene. In human, the family includes FcγRI (CD64) including isoforms FcγRIa, FcγRIb and FcγRIc; FcγRII (CD32) including isoforms FcγRIIa (including allotypes H131 and R131, *i.e.,* FcγRIIa(H) and FcγRIIa(R)), FcγRIIb (including FcγRIIb-1 and FcγRIIb-2), and FcγRIIc; and FcγRIII (CD16) including isoforms FcγRIIIa (including allotypes V158 and F158, *i.e.,* FcγRIIIa(V) and FcγRIIIa(F)) and FcγRIIIb (including allotypes FcγRIIIb-NA1 and FcγRIIIb-NA2); as well as all unidentified human FcγRs, FcγR isoforms, and allotypes thereof. However, Fcγ receptor is not limited to these examples. Without being limited thereto, FcγR includes those derived from humans, mice, rats, rabbits, and monkeys. FcγR may be derived from any organisms. Mouse FcγR includes, without being limited to, FcγRI (CD64), FcγRII (CD32), FcγRIII (CD16), and FcγRIII-2 (FcγRIV, CD16-2), as well as all unidentified mouse FcγRs, FcγR isoforms, and allotypes thereof. Such preferred Fcγ receptors include, for example, human FcγRI (CD64), FcγRIIa (CD32), FcγRIIb (CD32), FcγRIIIa (CD16), and/or FcγRIIIb (CD16). The polynucleotide sequence and amino acid sequence of FcγRI are shown in SEQ ID NOs: 15 (NM_000566.3) and 16 (NP_000557.1), respectively; the polynucleotide sequence and amino acid sequence of FcγRIIa (allotype H131) are shown in SEQ ID NOs: 17 (BC020823.1) and 18 (AAH20823.1) (allotype R131 is a sequence in which amino acid at position 166 of SEQ ID NO: 18 is substituted with Arg), respectively; the polynucleotide sequence and amino acid sequence of FcγRIIb are shown in SEQ ID NOs: 19 (BC146678.1) and 20 (AAI46679.1), respectively; the polynucleotide sequence and amino acid sequence of FcγRIIIa are shown in SEQ ID NOs: 21 (BC033678.1) and 22 (AAH33678.1), respectively; and the polynucleotide sequence and amino acid sequence of FcγRIIIb are shown in SEQ ID NOs: 23 (BC128562.1) and 24 (AAI28563.1), respectively (RefSeq accession number is shown in each parentheses). For example, as described as FcγRIIIaV when allotype V158 is used, unless otherwise specified, allotype F158 is used; however, the allotype of isoform FcγRIIIa described herein should not be interpreted as being particularly limited.

In FcγRI (CD64) including FcγRIa, FcγRIb, and FcγRIc, and FcγRIII (CD16) including isoforms FcγRIIIa (including allotypes V158 and F158) and FcγRIIIb (including allotypes FcγRIIIb-NA1 and FcγRIIIb-NA2), α chain that binds to the Fc portion of IgG is associated with common γ chain having ITAM responsible for transduction of intracellular activation signal. Meanwhile, the cytoplasmic domain of FcγRII (CD32) including isoforms FcγRIIa (including allotypes H131 and R131) and FcγRIIc contains ITAM. These receptors are expressed on many immune cells such as macrophages, mast cells, and antigen-presenting cells. The activation signal transduced upon binding of these receptors to the Fc portion of IgG results in enhancement of the phagocytic activity of macrophages, inflammatory cytokine production, mast cell degranulation, and the enhanced function of antigen-presenting cells. Fcγ receptors having the ability to transduce the activation signal as described above are also referred to as activating Fcγ receptors.

Meanwhile, the cytoplasmic domain of FcγRIIb (including FcγRIIb-1 and FcγRIIb-2) contains ITIM responsible for transduction of inhibitory signals. The crosslinking between FcγRIIb and B cell receptor (BCR) on B cells suppresses the activation signal from BCR, which results in suppression of antibody production *via* BCR. The crosslinking of FcγRIII and FcγRIIb on macrophages suppresses the phagocytic activity and inflammatory cytokine production. Fcγ receptors having the ability to transduce the inhibitory signal as described above are also referred to as inhibitory Fcγ receptor.

FcγR-binding activity of Fc region

As mentioned above, Fc regions having an Fcγ receptor-binding activity are examples of Fc regions comprised in the antigen-binding molecules as disclosed herein. A non-limiting embodiment of such an Fc region includes the Fc region of human IgG1 (SEQ ID NO: 9), IgG2 (SEQ ID NO: 10), IgG3 (SEQ ID NO: 11), or IgG4 (SEQ ID NO: 12). Whether an Fcγ receptor has binding activity to the Fc region of a monoclonal IgG1, IgG2, IgG3, or IgG4 antibody can be assessed by ALPHA screen (Amplified Luminescent Proximity Homogeneous Assay), surface plasmon resonance (SPR)-based BIACORE method, and others (Proc. Natl. Acad. Sci. USA (2006) 103(11), 4005-4010), in addition to the above-described FACS and ELISA formats.

ALPHA screen is performed by the ALPHA technology based on the principle described below using two types of beads: donor and acceptor beads. A luminescent signal is detected only when molecules linked to the donor beads interact biologically with molecules linked to the acceptor beads and when the two beads are located in close proximity. Excited by laser beam, the photosensitizer in a donor bead converts oxygen around the bead into excited singlet oxygen. When the singlet oxygen diffuses around the donor beads and reaches the acceptor beads located in close proximity, a chemiluminescent reaction within the acceptor beads is induced. This reaction ultimately results in light emission. If molecules linked to the donor beads do not interact with molecules linked to the acceptor beads, the singlet oxygen produced by donor beads do not reach the acceptor beads and chemiluminescent reaction does not occur.

For example, a biotin-labeled antigen-binding molecule comprising Fc region is immobilized to the donor beads and glutathione S-transferase (GST)-tagged Fcγ receptor is immobilized to the acceptor beads. In the absence of an antigen-binding molecule comprising a competitive Fc region variant, Fcγ receptor interacts with a polypeptide complex comprising a wild-type Fc region, inducing a signal of 520 to 620 nm as a result. The antigen-binding molecule having a non-tagged Fc region variant competes with the antigen-binding molecule comprising a native Fc region for the interaction with Fcγ receptor. The relative binding affinity can be determined by quantifying the reduction of fluorescence as a result of competition. Methods for biotinylating the antigen-binding molecules such as antibodies using Sulfo-NHS-biotin or the like are known. Appropriate methods for adding the GST tag to an Fcγ receptor include methods that involve fusing polypeptides encoding Fcγ and GST in-frame, expressing the fused gene using cells introduced with a vector to which the gene is operably linked, and then purifying using a glutathione column. The induced signal can be preferably analyzed, for example, by fitting to a one-site competition model based on nonlinear regression analysis using software such as GRAPHPAD PRISM (GraphPad; San Diego).

One of the substances for observing their interaction is immobilized as a ligand onto the gold thin layer of a sensor chip. When light is shed on the rear surface of the sensor chip so that total reflection occurs at the interface between the gold thin layer and glass, the intensity of reflected light is partially reduced at a certain site (SPR signal). The other substance for observing their interaction is injected as an analyte onto the surface of the sensor chip. The mass of immobilized ligand molecule increases when the analyte binds to the ligand. This alters the refraction index of solvent on the surface of the sensor chip. The change in refraction index causes a positional shift of SPR signal (conversely, the dissociation shifts the signal back to the original position). In the Biacore system, the amount of shift described above (*i.e.,* the change of mass on the sensor chip surface) is plotted on the vertical axis, and thus the change of mass over time is shown as measured data (sensorgram). Kinetic parameters (association rate constant (ka) and dissociation rate constant (kd)) are determined from the curve of sensorgram, and affinity (KD) is determined from the ratio between these two constants. Inhibition assay is preferably used in the BIACORE methods. Examples of such inhibition assay are described in Proc. Natl. Acad. Sci. USA (2006) 103(11), 4005-4010.

In addition to the Fc region of human IgG1 (SEQ ID NO: 9), IgG2 (SEQ ID NO: 10), IgG3 (SEQ ID NO: 11), or IgG4 (SEQ ID NO: 12), an Fc region with modified FcγR binding, which has a higher Fcγ receptor-binding activity than an Fc region of a native human IgG may be appropriately used as an Fc region as disclosed herein. Herein, "Fc region of a native human IgG" refers to an Fc region in which the sugar chain bonded to position 297 (EU numbering) of the Fc region of human IgG1, IgG2, IgG3, or IgG4 shown in SEQ ID NO: 9, 10, 11, or 12 is a fucose-containing sugar chain. Such Fc regions with modified FcγR binding may be produced by altering amino acids of the Fc region of a native human IgG. Whether the FcγR-binding activity of an Fc region with modified FcγR binding is higher than that of an Fc region of a native human IgG can be determined appropriately using methods described in the abovementioned section "Binding Activity".

As used herein, "alteration of amino acids" or "amino acid alteration" of an Fc region includes alteration into an amino acid sequence which is different from that of the starting Fc region. The starting Fc region may be any Fc region, as long as a variant modified from the starting Fc region can bind to human Fcγ receptor in a neutral pH range. Furthermore, an Fc region modified from a starting Fc region which had been already modified can also be used preferably as an Fc region as disclosed herein. The "starting Fc region" can refer to the polypeptide itself, a composition comprising the starting Fc region, or an amino acid sequence encoding the starting Fc region. Starting Fc regions can comprise known Fc regions produced *via* recombination described briefly in the section "Antibody". The origin of starting Fc regions is not limited, and they may be obtained from human or any nonhuman organisms. Such organisms preferably include mice, rats, guinea pigs, hamsters, gerbils, cats, rabbits, dogs, goats, sheep, bovines, horses, camels and organisms selected from nonhuman primates. In another embodiment, starting Fc regions can also be obtained from cynomolgus monkeys, marmosets, rhesus monkeys, chimpanzees, or humans. Starting Fc regions can be obtained preferably from human IgG1; however, they are not limited to any particular IgG class. This means that an Fc region of human IgG1, IgG2, IgG3, or IgG4 can be used appropriately as a starting Fc region, and herein also means that an Fc region of an arbitrary IgG class or subclass derived from any organisms described above can be preferably used as a starting Fc region. Examples of naturally-occurring IgG variants or modified forms are described in published documents (Curr. Opin. Biotechnol. (2009) 20 (6): 685-91; Curr. Opin. Immunol. (2008) 20 (4), 460-470; Protein Eng. Des. Sel. (2010) 23 (4): 195-202; International Publication Nos. WO 2009/086320, WO 2008/092117, WO 2007/041635, and WO 2006/105338); however, they are not limited to the examples.

Examples of alterations include those with one or more mutations, for example, mutations by substitution of different amino acid residues for amino acids of starting Fc regions, by insertion of one or more amino acid residues into starting Fc regions, or by deletion of one or more amino acids from starting Fc region. Preferably, the amino acid sequences of altered Fc regions comprise at least a part of the amino acid sequence of a non-native Fc region. Such variants necessarily have sequence identity or similarity less than 100% to their starting Fc region. In a preferred embodiment, the variants have amino acid sequence identity or similarity about 75% to less than 100%, more preferably about 80% to less than 100%, even more preferably about 85% to less than 100%, still more preferably about 90% to less than 100%, and yet more preferably about 95% to less than 100% to the amino acid sequence of their starting Fc region. In a non-limiting embodiment, at least one amino acid is different between an FcγR-binding modified Fc region as disclosed herein and its starting Fc region. Amino acid difference between an FcγR-binding modified Fc region as disclosed herein and its starting Fc region can also be preferably specified based on the specific amino acid differences at the above-described specific amino acid positions according to EU numbering system.

Known methods such as site-directed mutagenesis (Kunkel et al. (Proc. Natl. Acad. Sci. USA (1985) 82, 488-492)) and Overlap extension PCR can be appropriately employed to alter the amino acids of Fc regions. Furthermore, various known methods can also be used as an amino acid alteration method for substituting amino acids by those other than natural amino acids (Annu. Rev. Biophys. Biomol. Struct. (2006) 35, 225-249; Proc. Natl. Acad. Sci. U.S.A. (2003) 100 (11), 6353-6357). For example, a cell-free translation system (Clover Direct (Protein Express)) containing tRNAs in which amber suppressor tRNA, which is complementary to UAG codon (amber codon) that is a stop codon, is linked with an unnatural amino acid may be suitably used.

Included in the antigen-binding molecules disclosed herein, an Fc region with modified FcγR binding, which has a higher Fcγ receptor-binding activity than that of an Fc region of a native human IgG, (an FcγR binding-modified Fc region) may be obtained by any method. Specifically, the Fc region with modified FcγR binding may be obtained by altering amino acids of an IgG-type human immunoglobulin used as a starting Fc region. Preferred Fc regions of the IgG-type immunoglobulins for alteration include, for example, those of human IgGs shown in SEQ ID NO: 9, 10, 11, or 12 (IgG1, IgG2, IgG3, or IgG4, respectively, and variants thereof).

Amino acids of any positions may be altered to other amino acids, as long as the binding activity toward the Fcγ receptor is higher than that of the Fc region of a native human IgG. When the antigen-binding molecule contains a human IgG1 Fc region as the human Fc region, it preferably contains an alteration that yields the effect of a higher Fcγ receptor-binding activity than that of the Fc region of a native human IgG, in which the sugar chain bound at position 297 (EU numbering) is a fucose-containing sugar chain. Such amino acid alterations have been reported, for example, in international publications such as WO2007/024249, WO2007/021841, WO2006/031370, WO2000/042072, WO2004/029207, WO2004/099249, WO2006/105338, WO2007/041635, WO2008/092117, WO2005/070963, WO2006/020114, WO2006/116260, and WO2006/023403.

Examples of such amino acids that may be altered include at least one or more amino acids selected from the group consisting of positions 221, 222, 223, 224, 225, 227, 228, 230, 231, 232, 233, 234, 235, 236, 237, 238, 239, 240, 241, 243, 244, 245, 246, 247, 249, 250, 251, 254, 255, 256, 258, 260, 262, 263, 264, 265, 266, 267, 268, 269, 270, 271, 272, 273, 274, 275, 276, 278, 279, 280, 281, 282, 283, 284, 285, 286, 288, 290, 291, 292, 293, 294, 295, 296, 297, 298, 299, 300, 301, 302, 303, 304, 305, 311, 313, 315, 317, 318, 320, 322, 323, 324, 325, 326, 327, 328, 329, 330, 331, 332, 333, 334, 335, 336, 337, 339, 376, 377, 378, 379, 380, 382, 385, 392, 396, 421, 427, 428, 429, 434, 436, and 440 (EU numbering). An Fc region (Fc region with modified FcγR binding) having a higher Fcγ receptor-binding activity than that of an Fc region of a native human IgG can be obtained by altering these amino acids.

Examples of particularly preferable alterations for use in the present invention include at least one or more amino acid alterations selected from the group consisting of:

Lys or Tyr for the amino acid at position 221;
Phe, Trp, Glu, or Tyr for the amino acid at position 222;
Phe, Trp, Glu, or Lys for the amino acid at position 223;
Phe, Trp, Glu, or Tyr for the amino acid at position 224;
Glu, Lys, or Trp for the amino acid at position 225;
Glu, Gly, Lys, or Tyr for the amino acid at position 227;
Glu, Gly, Lys, or Tyr for the amino acid at position 228;
Ala, Glu, Gly, or Tyr for the amino acid at position 230;
Glu, Gly, Lys, Pro, or Tyr for the amino acid at position 231;
Glu, Gly, Lys, or Tyr for the amino acid at position 232;
Ala, Asp, Phe, Gly, His, Ile, Lys, Leu, Met, Asn, Gln, Arg, Ser, Thr, Val, Trp, or Tyr for the amino acid at position 233;
Ala, Asp, Glu, Phe, Gly, His, Ile, Lys, Met, Asn, Pro, Gln, Arg, Ser, Thr, Val, Trp, or Tyr for the amino acid at position 234;
Ala, Asp, Glu, Phe, Gly, His, Ile, Lys, Met, Asn, Pro, Gln, Arg, Ser, Thr, Val, Trp, or Tyr for the amino acid at position 235;
Ala, Asp, Glu, Phe, His, Ile, Lys, Leu, Met, Asn, Pro, Gln, Arg, Ser, Thr, Val, Trp, or Tyr for the amino acid at position 236;
Asp, Glu, Phe, His, Ile, Lys, Leu, Met, Asn, Pro, Gln, Arg, Ser, Thr, Val, Trp, or Tyr for the amino acid at position 237;
Asp, Glu, Phe, Gly, His, Ile, Lys, Leu, Met, Asn, Gln, Arg, Ser, Thr, Val, Trp, or Tyr for the amino acid at position 238;
Asp, Glu, Phe, Gly, His, Ile, Lys, Leu, Met, Asn, Pro, Gln, Arg, Thr, Val, Trp, or Tyr for the amino acid at position 239;
Ala, Ile, Met, or Thr for the amino acid at position 240;
Asp, Glu, Leu, Arg, Trp, or Tyr for the amino acid at position 241;
Leu, Glu, Leu, Gln, Arg, Trp, or Tyr for the amino acid at position 243;
His for the amino acid at position 244;
Ala for the amino acid at position 245;
Asp, Glu, His, or Tyr for the amino acid at position 246;
Ala, Phe, Gly, His, Ile, Leu, Met, Thr, Val, or Tyr for the amino acid at position 247;
Glu, His, Gln, or Tyr for the amino acid at position 249;
Glu or Gln for the amino acid at position 250;
Phe for the amino acid at position 251;
Phe, Met, or Tyr for the amino acid at position 254;
Glu, Leu, or Tyr for the amino acid at position 255;
Ala, Met, or Pro for the amino acid at position 256;
Asp, Glu, His, Ser, or Tyr for the amino acid at position 258;

Asp, Glu, His, or Tyr for the amino acid at position 260;

Ala, Glu, Phe, Ile, or Thr for the amino acid at position 262;

Ala, Ile, Met, or Thr for the amino acid at position 263;

Asp, Glu, Phe, Gly, His, Ile, Lys, Leu, Met, Asn, Pro, Gln, Arg, Ser, Thr, Trp, or Tyr for the amino acid at position 264;

Ala, Leu, Phe, Gly, His, Ile, Lys, Leu, Met, Asn, Pro, Gln, Arg, Ser, Thr, Val, Trp, or Tyr for the amino acid at position 265;

Ala, Ile, Met, or Thr for the amino acid at position 266;

Asp, Glu, Phe, His, Ile, Lys, Leu, Met, Asn, Pro, Gln, Arg, Thr, Val, Trp, or Tyr for the amino acid at position 267;

Asp, Glu, Phe, Gly, Ile, Lys, Leu, Met, Pro, Gln, Arg, Thr, Val, or Trp for the amino acid at position 268;

Phe, Gly, His, Ile, Lys, Leu, Met, Asn, Pro, Arg, Ser, Thr, Val, Trp, or Tyr for the amino acid at position 269;

Glu, Phe, Gly, His, Ile, Leu, Met, Pro, Gln, Arg, Ser, Thr, Trp, or Tyr for the amino acid at position 270;

Ala, Asp, Glu, Phe, Gly, His, Ile, Lys, Leu, Met, Asn, Gln, Arg, Ser, Thr, Val, Trp, or Tyr for the amino acid at position 271;

Asp, Phe, Gly, His, Ile, Lys, Leu, Met, Pro, Arg, Ser, Thr, Val, Trp, or Tyr for the amino acid at position 272;

Phe or Ile for the amino acid at position 273;

Asp, Glu, Phe, Gly, His, Ile, Leu, Met, Asn, Pro, Arg, Ser, Thr, Val, Trp, or Tyr for the amino acid at position 274;

Leu or Trp for the amino acid at position 275;

Asp, Glu, Phe, Gly, His, Ile, Leu, Met, Pro, Arg, Ser, Thr, Val, Trp, or Tyr for the amino acid at position 276;

Asp, Glu, Gly, His, Ile, Lys, Leu, Met, Asn, Pro, Gln, Arg, Ser, Thr, Val, or Trp for the amino acid at position 278;

Ala for the amino acid at position 279;

Ala, Gly, His, Lys, Leu, Pro, Gln, Trp, or Tyr for the amino acid at position 280;

Asp, Lys, Pro, or Tyr for the amino acid at position 281;

Glu, Gly, Lys, Pro, or Tyr for the amino acid at position 282;

Ala, Gly, His, Ile, Lys, Leu, Met, Pro, Arg, or Tyr for the amino acid at position 283;

Asp, Glu, Leu, Asn, Thr, or Tyr for the amino acid at position 284;

Asp, Glu, Lys, Gln, Trp, or Tyr for the amino acid at position 285;

Glu, Gly, Pro, or Tyr for the amino acid at position 286;

Asn, Asp, Glu, or Tyr for the amino acid at position 288;

Asp, Gly, His, Leu, Asn, Ser, Thr, Trp, or Tyr for the amino acid at position 290;

Asp, Glu, Gly, His, Ile, Gln, or Thr for the amino acid at position 291;

Ala, Asp, Glu, Pro, Thr, or Tyr for the amino acid at position 292;

Phe, Gly, His, Ile, Leu, Met, Asn, Pro, Arg, Ser, Thr, Val, Trp, or Tyr for the amino acid at position 293;

Phe, Gly, His, Ile, Lys, Leu, Met, Asn, Pro, Arg, Ser, Thr, Val, Trp, or Tyr for the amino acid at position 294;

Asp, Glu, Phe, Gly, His, Ile, Lys, Met, Asn, Pro, Arg, Ser, Thr, Val, Trp, or Tyr for the amino acid at position 295;

Ala, Asp, Glu, Gly, His, Ile, Lys, Leu, Met, Asn, Gln, Arg, Ser, Thr, or Val for the amino acid at position 296;

Asp, Glu, Phe, Gly, His, Ile, Lys, Leu, Met, Pro, Gln, Arg, Ser, Thr, Val, Trp, or Tyr for the amino acid at position 297;

Ala, Asp, Glu, Phe, His, Ile, Lys, Met, Asn, Gln, Arg, Thr, Val, Trp, or Tyr for the amino acid at position 298;

Ala, Asp, Glu, Phe, Gly, His, Ile, Lys, Leu, Met, Asn, Pro, Gln, Arg, Ser, Val, Trp, or Tyr for the amino acid at position 299;

Ala, Asp, Glu, Gly, His, Ile, Lys, Leu, Met, Asn, Pro, Gln, Arg, Ser, Thr, Val, or Trp for the amino acid at position 300;

Asp, Glu, His, or Tyr for the amino acid at position 301;

Ile for the amino acid at position 302;

Asp, Gly, or Tyr for the amino acid at position 303;

Asp, His, Leu, Asn, or Thr for the amino acid at position 304;

Glu, Ile, Thr, or Tyr for the amino acid at position 305;

Ala, Asp, Asn, Thr, Val, or Tyr for the amino acid at position 311;

Phe for the amino acid at position 313;

Leu for the amino acid at position 315;

Glu or Gln for the amino acid at position 317;

His, Leu, Asn, Pro, Gln, Arg, Thr, Val, or Tyr for the amino acid at position 318;

Asp, Phe, Gly, His, Ile, Leu, Asn, Pro, Ser, Thr, Val, Trp, or Tyr for the amino acid at position 320;

Ala, Asp, Phe, Gly, His, Ile, Pro, Ser, Thr, Val, Trp, or Tyr for the amino acid at position 322;

Ile for the amino acid at position 323;

Asp, Phe, Gly, His, Ile, Leu, Met, Pro, Arg, Thr, Val, Trp, or Tyr for the amino acid at position 324;

Ala, Asp, Glu, Phe, Gly, His, Ile, Lys, Leu, Met, Pro, Gln, Arg, Ser, Thr, Val, Trp, or Tyr for the amino acid at position 325;

Ala, Asp, Glu, Gly, Ile, Leu, Met, Asn, Pro, Gln, Ser, Thr, Val, Trp, or Tyr for the amino acid at position 326;

Ala, Asp, Glu, Phe, Gly, His, Ile, Lys, Leu, Met, Asn, Pro, Arg, Thr, Val, Trp, or Tyr for the amino acid at position 327;

Ala, Asp, Glu, Phe, Gly, His, Ile, Lys, Met, Asn, Pro, Gln, Arg, Ser, Thr, Val, Trp, or Tyr for the amino acid at position 328;

Asp, Glu, Phe, Gly, His, Ile, Lys, Leu, Met, Asn, Gln, Arg, Ser, Thr, Val, Trp, or Tyr for the amino acid at position 329;

Cys, Glu, Phe, Gly, His, Ile, Lys, Leu, Met, Asn, Pro, Arg, Ser, Thr, Val, Trp, or Tyr for the amino acid at position 330;

Asp, Phe, His, Ile, Leu, Met, Gln, Arg, Thr, Val, Trp, or Tyr for the amino acid at position 331;

Ala, Asp, Glu, Phe, Gly, His, Lys, Leu, Met, Asn, Pro, Gln, Arg, Ser, Thr, Val, Trp, or Tyr for the amino acid at position 332;

Ala, Asp, Glu, Phe, Gly, His, Ile, Leu, Met, Pro, Ser, Thr, Val, or Tyr for the amino acid at position 333;

Ala, Glu, Phe, Ile, Leu, Pro, or Thr for the amino acid at position 334;

Asp, Phe, Gly, His, Ile, Leu, Met, Asn, Pro, Arg, Ser, Val, Trp, or Tyr for the amino acid at position 335;

Glu, Lys, or Tyr for the amino acid at position 336;

Glu, His, or Asn for the amino acid at position 337;

Asp, Phe, Gly, Ile, Lys, Met, Asn, Gln, Arg, Ser, or Thr for the amino acid at position 339;

Ala or Val for the amino acid at position 376;

Gly or Lys for the amino acid at position 377;

Asp for the amino acid at position 378;

Asn for the amino acid at position 379;

Ala, Asn, or Ser for the amino acid at position 380;

Ala or Ile for the amino acid at position 382;

Glu for the amino acid at position 385;

Thr for the amino acid at position 392;

Leu for the amino acid at position 396;

Lys for the amino acid at position 421;

Asn for the amino acid at position 427;

Phe or Leu for the amino acid at position 428;

Met for the amino acid at position 429;

Trp for the amino acid at position 434;

Ile for the amino acid at position 436; and

Gly, His, Ile, Leu, or Tyr for the amino acid at position 440;

as indicated by EU numbering. The number of amino acids to be altered is not particularly limited; and amino acid may be altered at only one site or amino acids may be altered at two or more sites. Examples of combinations for amino acid alterations at two or more sites include those described in Table 6 (Tables 6-1 to 6-3).

[Table 6-1]

| Combination of amino acids | Combination of amino acids |
| --- | --- |
| K370E/P396L/D270E | S239Q/I332Q |
| Q419H/P396L/D270E | S267D/I332E |
| V240A/P396L/D270E | S267E/I332E |
| R255L/P396L/D270E | S267L/A327S |
| R255L/P396L/D270E | S267Q/A327S |
| R255L/P396L/D270E/R292G | S298A/I332E |
| R255L/P396L/D270E | S304T/I332E |
| R255L/P396L/D270E/Y300L | S324G/I332D |
| F343L/D270E/K392N/P396L | S324G/I332E |
| F243L/R255L/D270E/P396L | S324I/I332D |
| F243L/R292P/Y300L/V305I/P396L | S324I/I332E |
| F243L/R292P/Y300L/P396L | T260H/I332E |
| F243L/R292P/Y300L | T335D/I332E |
| F243L/R292P/P396L | V240I/V266I |
| F243L/R292P/V305I | V264I/I332E |
| F243L/R292P | D265F/N297E/I332E |
| S298A/E333A/K334A | D26SY/N297D/I332E |

(continued)

| Combination of amino acids | Combination of amino acids |
|---|---|
| E380A/T307A | F243L/V262I/V264W |
| K326M/E333S | N297D/A330Y/I332E |
| K326A/ES33A | N297D/T299E/I332E |
| S317A/K353A | N297D/T299F/I332E |
| A327D/I332E | N297D/T299H/1332E |
| A33OL/1332E | N297D/T299I/I332E |
| A330Y/I332E | N297D/T299L/I332E |
| E258H/I332E | N297D/T299V/I332E |
| E272H/I332E | P230A/E233D/I332E |
| E272I/N276D | P244H/P245A/P247V |
| E272R/I332E | S239D/A330L/I332E |
| E283H/I332E | S239D/A330Y/I332E |
| E293R/I332E | S239D/H268E/A330Y |
| F241L/V262I | S239D/I332E/A327A |
| F241W/F243W | S239D/I332E/A330I |

Table 6-2 is a continuation of Table 6-1.

[Table 6-2]

| F243L/V264I | S239D/N297D/I332E |
|---|---|
| H268D/A330Y | S239D/S298A/I332E |
| H268E/A330Y<br>K246H/I332E | S239D/V264I/I332E<br>S239E/N297D/I332E |
| L234D/I332E | S239E/V264I/I332E |
| L234E/I332E | S239N/A330L/I332E |
| L234G/I332E | S239N/A330Y/I332E |
| L234I/I332E | S239N/S298A/I332E |
| L234I/L235D | S239Q/V264I/I332E |
| L234Y/I332E | V264E/N297D/I332E |
| L235D/I332E | V264I/A330L/I332E |
| L235E/I332E | V264I/A330Y/I332E |
| L235I/I332E | V264I/S298A/I332E |
| L235S/I332E | Y296D/N297D/I332E |
| L328A/I332D | Y296E/N297D/I332E |
| L328D/I332D | Y296H/N297D/I332E |
| L328D/I332E | Y296N/N297D/I332E |
| L328E/I332D | Y296Q/N297D/I332E |
| L328E/I332E | Y296T/N297D/I332E |
| L328F/I332D | D265Y /N297D/T299L/I332E |

(continued)

| | |
|---|---|
| L328F/I332E | F241E/F243Q/V262T/V264E |
| L328H/I332E | F241E/F243R/V262E/V264R |
| L328I/I332D | F241E/F243Y/V262T/V264R |
| L328I/I332E | F241L/F243L/V262I/V264I |
| L328M/I332D | F241R/F243Q/V262T/V264R |
| L328M/I332E | F241S/F243H/V262T/V264T |
| L328N/I332D | F241W/F243W/V262A/V264A |
| L328N/I332E | F241Y/F243Y/V262T/V264T |
| L328Q/I332D | I332E/A330Y/H268E/A327A |
| L328Q/I332E | N297D/I332E/S239D/A330L |
| L328T/I332D | N297D/S298A/A330Y/I332E |
| L328T/I332E | S239D/A330Y/I332E/K326E |
| L328V/I332D | S239D/A330Y/I332E/K326T |
| L328V/I332E | S239D/A330Y/I332E/L234I |
| L328Y/I332D | S239D/A330Y/I332E/L235D |

Table 6-3 is a continuation of Table 6-2.

[Table 6-3]

| | |
|---|---|
| L328Y/I332E | S239D/A330Y/I332E/V240I |
| N297D/I332E | S239D/A330Y/I332E/V264T |
| N297E/I332E | S239D/A330Y/I332E/V266I |
| N297S/I332E | S239D/D265F/N297D/I332E |
| P227G/I332E | S239D/D265H/N297D/I332E |
| P230A/E233D | S239D/D265I/N297D/I332E |
| Q295E/I332E | S239D/D265L/N297D/I332E |
| R255Y/I332E | S239D/D265T/N297D/I332E |
| S239D/I332D | S239D/D265V/N297D/I332E |
| S239D/I332E | S239D/D265Y/N297D/I332E |
| S239D/I332N | S239D/I332E/A330Y/A327A |
| S239D/I332Q | S239D/I332E/H268E/A327A |
| S239E/D265G | S239D/I332E/H268E/A330Y |
| S239E/D265N | S239D/N297D/I332E/A330Y |
| S239E/D265Q | S239D/N297D/I332E/K326E |
| S239E/I332D | S239D/N297D/I332E/L235D |
| S239E/I332E | S239D/V264I/A330L/I332E |
| S239E/I332N | S239D/V264I/S298A/I332E |
| S239E/I332Q | S239E/V264I/A330Y/I332E |
| S239N/I332D | F241E/F243Q/V262T/V264E/I332E |
| S239N/I332E | F241E/F243R/V262E/V264R/I332E |

(continued)

| S239N/I332N | F241E/F243Y/V262T/V264R/I332E |
|---|---|
| S239N/I332Q | F241R/F243Q/V262T/V264R/I332E |
| S239Q/I332D | S239D/I332E/H268E/A330Y/A327A |
| S239Q/I332E | S239E/V264I/S298A/A330Y/I332E |
| S239Q/I332N | F241Y/F243Y/V262T/V264T/N297D/I332E |
| S267E/L328F | G236D/S267E |
| S239D/S267E | |

For the pH conditions to measure the binding activity of the Fc region contained in the antigen-binding molecule as disclosed herein and the Fcγ receptor, conditions in an acidic pH range or in a neutral pH range may be suitably used. The neutral pH range, as a condition to measure the binding activity of the Fc region and the Fcγ receptor contained in the antigen-binding molecule as disclosed herein, generally indicates pH 6.7 to pH 10.0. Preferably, it is a range indicated with arbitrary pH values between pH 7.0 and pH8.0; and preferably, it is selected from pH 7.0, pH 7.1, pH 7.2, pH 7.3, pH 7.4, pH 7.5, pH 7.6, pH 7.7, pH 7.8, pH 7.9, and pH 8.0; and particularly preferably, it is pH 7.4, which is close to the pH of plasma (blood) *in vivo*. Herein, the acidic pH range, as a condition for having a binding activity of the Fc region and the Fcγ receptor contained in the antigen-binding molecule as disclosed herein, generally indicates pH 4.0 to pH 6.5. Preferably, it indicates pH 5.5 to pH 6.5, and particularly preferably, it indicates pH 5.8 to pH 6.0, which is close to the pH in the early endosome *in vivo*. With regard to the temperature used as a measurement condition, the binding affinity between an Fc region and an Fcγ receptor can be evaluated at any temperature between 10°C and 50°C. Preferably, a temperature between 15°C and 40°C is used to determine the binding affinity between an Fc region and an Fcγ receptor. More preferably, any temperature between 20°C and 35°C, such as any single temperature from 20°C, 21°C, 22°C, 23°C, 24°C, 25°C, 26°C, 27°C, 28°C, 29°C, 30°C, 31°C, 32°C, 33°C, 34°C, and 35°C, can be similarly used to determine the binding affinity between an Fc region and an Fcγ receptor. A temperature of 25°C is a non-limiting example in an embodiment of the present disclosure.

Herein, "Fc region with modified FcγR binding has a higher Fcγ receptor-binding activity than the native Fc region" means that the human Fcγ receptor-binding activity of the Fc region with modified FcγR binding toward any of the human Fcγ receptors of FcγRI, FcγRIIa, FcγRIIb, FcγRIIIa, and/or FcγRIIIb is higher than the binding activity of the native Fc region toward these human Fcγ receptors. For example, it means that based on an above-described analytical method, in comparison to the binding activity of an antigen-binding molecule containing a native human IgG Fc region as a control, the binding activity of the antigen-binding molecule comprising an Fc region with modified FcγR binding is 105% or more, preferably 110% or more, 115% or more, 120% or more, 125% or more, particularly preferably 130% or more, 135% or more, 140% or more, 145% or more, 150% or more, 155% or more, 160% or more, 165% or more, 170% or more, 175% or more, 180% or more, 185% or more, 190% or more, 195% or more, 2-fold or more, 2.5-fold or more, 3-fold or more, 3.5-fold or more, 4-fold or more, 4.5-fold or more, 5-fold or more, 7.5-fold or more, 10-fold or more, 20-fold or more, 30-fold or more, 40-fold or more, 50-fold or more, 60-fold or more, 70-fold or more, 80-fold or more, 90-fold or more, or 100-fold or more. The starting Fc region may be used as a native Fc region, and native Fc regions of antibodies of the same subclass may also be used.

An Fc region of a native human IgG in which the sugar chain bonded to the amino acid at position 297 (EU numbering) is a fucose-containing sugar chain, is suitably used as a native Fc region of human IgG to be used as a control. Whether or not the sugar chain bonded to the amino acid at position 297 (EU numbering) is a fucose-containing sugar chain can be determined using the technique described in Non-patent Document 6. For example, it is possible to determine whether or not the sugar chain bonded to the native human IgG Fc region is a fucose-containing sugar chain by a method such as the one below. Sugar chain is dissociated from a native human IgG to be tested, by reacting the test native human IgG with *N*-Glycosidase F (Roche diagnostics) (Weitzhandler et al. (J. Pharma. Sciences (1994) 83, 12, 1670-1675)). Next, a dried concentrate of a reaction solution from which protein has been removed by reaction with ethanol (Schenk et al. (J. Clin. Investigation (2001) 108 (11) 1687-1695)) is fluorescently labeled with 2-aminopyridine (Bigge et al. (Anal. Biochem. (1995) 230 (2) 229-238)). Reagents are removed by solid extraction using a cellulose cartridge, and the fluorescently labeled 2-AB-modified sugar chain is analyzed by normal-phase chromatography. It is possible to determine whether or not the sugar chain bonded to the native Fc region of a human IgG is a fucose-containing sugar chain by observing the detected chromatogram peaks.

As an antigen-binding molecule containing an Fc region of a native antibody of the same subclass, which is to be used as a control, an antigen-binding molecule having an Fc region of a monoclonal IgG antibody may be suitably used. The structures of the Fc regions are described in SEQ ID NO: 9 (A is added to the N terminus of RefSeq Accession No. AAC82527.1), SEQ ID NO: 10 (A is added to the N terminus of RefSeq Accession No. AAB59393.1), SEQ ID NO: 11 (RefSeq Accession No. CAA27268.1), and SEQ ID NO: 12 (A is added to the N terminus of RefSeq Accession No. AAB59394.1). Further, when an antigen-binding molecule containing an Fc region of a particular antibody isotype is used as the test substance, the effect of the antigen-binding molecule containing the test Fc region on Fcγ receptor-binding activity is tested by using as a control an antigen-binding molecule having an Fc region of a monoclonal IgG antibody of that particular isotype. In this way, antigen-binding molecules containing an Fc region of which Fcγ receptor-binding activity is demonstrated to be high are suitably selected.

Fc regions having a selective binding activity to an Fcγ receptor

Examples of Fc regions suitable for use in the present disclosure include Fc regions having a higher binding activity to a particular Fcγ receptor than to other Fcγ receptors (Fc regions having a selective binding activity to an Fcγ receptor). When an antibody is used as the antigen-binding molecule, a single antibody molecule can only bind to a single Fcγ receptor molecule. Therefore, a single antigen-binding molecule cannot bind to other activating FcγRs in an inhibitory Fcγ receptor-bound state, and cannot bind to other activating Fcγ receptors or inhibitory Fcγ receptors in an activating Fcγ receptor-bound state.

As described above, suitable examples of activating Fcγ receptors include FcγRI (CD64) which includes FcγRIa, FcγRIb, and FcγRIc; FcγRIII (CD16) which includes isoforms FcγRIIIa (including allotypes V158 and F158) and FcγRIIIb (including allotypes FcγRIIIb-NA1 and FcγRIIIb-NA2); and FcγRIIa (including allotypes H131 and R131). Meanwhile, suitable examples of inhibitory Fcγ receptors include FcγRIIb (including FcγRIIb-1 and FcγRIIb-2).

Herein, an example of a case where the binding activity toward a certain Fcγ receptor is higher than the binding activity toward another Fcγ receptor is the case where the binding activity toward an inhibitory Fcγ receptor is higher than the binding activity toward an activating Fcγ receptor. In this case, the binding activity of the Fc region toward FcγRIIb is said to be higher than the binding activity toward any of the human Fcγ receptors of FcγRIa, FcγRIIa, FcγRIIIa, and/or FcγRIIIb. For example, this means that, based on an above-described analytical method, the binding activity of an antigen-binding molecule containing the Fc region toward FcγRIIb is 105% or more, preferably 110% or more, 120% or more, 130% or more, 140% or more, particularly preferably 150% or more, 160% or more, 170% or more, 180% or more, 190% or more, 200% or more, 250% or more, 300% or more, 350% or more, 400% or more, 450% or more, 500% or more, 750% or more, 10-fold or more, 20-fold or more, 30-fold or more, 40-fold or more, 50-fold, 60-fold, 70-fold, 80-fold, 90-fold, or 100-fold or more as compared with the binding activity toward any of the human Fcγ receptors of FcγRIa, FcγRIIa, FcγRIIIa, and/or FcγRIIIb.

In a non-limiting embodiment, a preferred example of an Fc region having a higher inhibitory Fcγ receptor-binding activity than an activating Fcγ receptor-binding activity (having a selective binding activity toward an inhibitory Fcγ receptor) is an Fc region in which the amino acid at position 238 or 328 as indicated by EU numbering in the aforementioned Fc region has been altered to an amino acid different from that of the native Fc region.

In a non-limiting embodiment, a suitable example of an Fc region that has a higher binding activity toward an inhibitory Fcγ receptor than toward an activating Fcγ receptor (*i.e.,* having a selective binding activity toward an inhibitory Fcγ receptor) is an Fc region with one or more of the following alterations in the amino acids (indicated by EU numbering) of the aforementioned Fc region: the amino acid at position 238 is altered to Asp and the amino acid at position 328 is altered to Glu. The Fc regions and alterations described in US2009/0136485 may be selected appropriately as the Fc region having a selective binding activity to an inhibitory Fcγ receptor.

In a non-limiting embodiment, a suitable example is an Fc region in which one or more of the amino acids indicated by EU numbering at positions 238 and 328 according to EU numbering are respectively altered to Asp or Glu in the aforementioned Fc region.

Furthermore, in a non-limiting embodiment, suitable examples of the Fc regions are those with substitution of Asp for Pro at position 238 (EU numbering), and one or more alterations selected from among Trp for the amino acid at position 237, Phe for the amino acid at position 237, Val for the amino acid at position 267, Gln for the amino acid at position 267, Asn for the amino acid at position 268, Gly for the amino acid at position 271, Leu for the amino acid at position 326, Gln for the amino acid at position 326, Glu for the amino acid at position 326, Met for the amino acid at position 326, Asp for the amino acid at position 239, Ala for the amino acid at position 267, Trp for the amino acid at position 234, Tyr for the amino acid at position 234, Ala for the amino acid at position 237, Asp for the amino acid at position 237, Glu for the amino acid at position 237, Leu for the amino acid at position 237, Met for the amino acid at position 237, Tyr for the amino acid at position 237, Lys for the amino acid at position 330, Arg for the amino acid at position 330, Asp for the amino acid at position 233, Asp for the amino acid at position 268, Glu for the amino acid at position 268, Asp for the amino acid at position 326, Ser for the amino acid at position 326, Thr for the amino acid at position 326, Ile for the amino acid at position 323, Leu for the amino acid at position 323, Met for the amino acid at position 323, Asp for the amino acid at position 296, Ala for the amino acid at position 326, Asn for the amino acid at position 326, and Met for the amino acid at position 330, according to EU numbering.

Antigen-binding molecule

As used herein, an "antigen-binding molecule" is used in the broadest sense to refer to a molecule containing an antigen-binding domain and an Fc region. Specifically, the antigen-binding molecules include various types of molecules as long as they exhibit the antigen-binding activity. Molecules in which an antigen-binding domain is linked to an Fc region include, for example, antibodies. Antibodies may include single monoclonal antibodies (including agonistic antibodies and antagonistic antibodies), human antibodies, humanized antibodies, chimeric antibodies, and such. Alternatively, when used as antibody fragments, they preferably include antigen-binding domains and antigen-binding fragments (for example, Fab, F(ab')2, scFv, and Fv). Scaffold molecules where three dimensional structures, such as already-known stable α/β barrel protein structure, are used as a scaffold (base) and only some portions of the structures are made into libraries to construct antigen-binding domains are also included in antigen-binding molecules as disclosed herein.

An antigen-binding molecule as disclosed herein may contain at least some portions of an Fc region that mediates the binding to FcRn and Fcγ receptor. In a non-limiting embodiment, the antigen-binding molecule includes, for example, antibodies and Fc fusion proteins. A fusion protein refers to a chimeric polypeptide comprising a polypeptide having a first amino acid sequence that is linked to a polypeptide having a second amino acid sequence that would not naturally link in nature. For example, a fusion protein may comprise the amino acid sequence encoding at least a portion of an Fc region (for example, a portion of an Fc region responsible for the binding to FcRn or a portion of an Fc region responsible for the binding to Fcγ receptor) and a non-immunoglobulin polypeptide containing, for example, the amino acid sequence encoding the ligand-binding domain of a receptor or a receptor-binding domain of a ligand. The amino acid sequences may be present in separate proteins that are transported together to a fusion protein, or generally may be present in a single protein; however, they are included in a new rearrangement in a fusion polypeptide. Fusion proteins can be produced, for example, by chemical synthesis, or by genetic recombination techniques to express a polynucleotide encoding peptide regions in a desired arrangement.

Each of the domains of the antigen-binding domain, Fc region, and such can be linked together *via* linkers or directly *via* polypeptide binding. The linkers comprise arbitrary peptide linkers that can be introduced by genetic engineering, synthetic linkers, and linkers disclosed in, for example, Protein Engineering (1996) 9(3), 299-305. However, peptide linkers are preferred. The length of the peptide linkers is not particularly limited, and can be suitably selected by those skilled in the art according to the purpose. The length is preferably five amino acids or more (without particular limitation, the upper limit is generally 30 amino acids or less, preferably 20 amino acids or less), and particularly preferably 15 amino acids.

For example, such peptide linkers preferably include:

Ser
Gly·Ser
Gly·Gly·Ser
Ser·Gly·Gly
Gly·Gly·Gly·Ser (SEQ ID NO: 25)
Ser·Gly·Gly·Gly (SEQ ID NO: 26)
Gly·Gly·Gly·Gly·Ser (SEQ ID NO: 27)
Ser·Gly·Gly·Gly·Gly (SEQ ID NO: 28)
Gly·Gly·Gly·Gly·Gly·Ser (SEQ ID NO: 29)
Ser·Gly·Gly·Gly·Gly·Gly (SEQ ID NO: 30)
Gly·Gly·Gly·Gly·Gly·Gly·Ser (SEQ ID NO: 31)
Ser·Gly·Gly·Gly·Gly·Gly·Gly (SEQ ID NO: 32)
(Gly·Gly·Gly·Gly·Ser (SEQ ID NO: 27))n
(Ser·Gly·Gly·Gly·Gly (SEQ ID NO: 28))n
[where n is an integer of 1 or larger]. The length or sequences of peptide linkers can be selected accordingly by those skilled in the art depending on the purpose.

Synthetic linkers (chemical crosslinking agents) is routinely used to crosslink peptides, and for example:

N-hydroxy succinimide (NHS),
disuccinimidyl suberate (DSS),
bis(sulfosuccinimidyl) suberate (BS3),
dithiobis(succinimidyl propionate) (DSP),
dithiobis(sulfosuccinimidyl propionate) (DTSSP),
ethylene glycol bis(succinimidyl succinate) (EGS),

ethylene glycol bis(sulfosuccinimidyl succinate) (sulfo-EGS),
disuccinimidyl tartrate (DST), disulfosuccinimidyl tartrate (sulfo-DST),
bis[2-(succinimidoxycarbonyloxy)ethyl] sulfone (BSOCOES),
and bis[2-(sulfosuccinimidoxycarbonyloxy)ethyl] sulfone (sulfo-BSOCOES). These crosslinking agents are commercially available.

When multiple linkers for linking the respective domains are used, they may all be of the same type, or may be of different types.

In addition to the linkers exemplified above, linkers with peptide tags such as His tag, HA tag, myc tag, and FLAG tag may also be suitably used. Furthermore, hydrogen bonding, disulfide bonding, covalent bonding, ionic interaction, and properties of binding with each other as a result of combination thereof may be suitably used. For example, the affinity between CH1 and CL of antibody may be used, and Fc regions originating from the above-described bispecific antibodies may also be used for hetero Fc region association. Moreover, disulfide bonds formed between domains may also be suitably used.

In order to link the respective domains *via* peptide linkage, polynucleotides encoding the domains are linked in frame. Known methods for linking polynucleotides in frame include techniques such as ligation of restriction fragments, fusion PCR, and overlapping PCR. Such methods can be appropriately used alone or in combination to produce the antigen-binding molecules as disclosed herein. Herein, the terms "linked" and "fused", or "linkage" and "fusion" are used interchangeably. These terms mean that two or more elements or components such as polypeptides are linked together to form a single structure by any means including the above-described chemical linking means and recombination techniques. When two or more elements or components are polypeptides, fusing in frame means linking two or more units of reading frames to form a longer continuous reading frame while maintaining the correct reading frames of the polypeptides. When two molecules of Fab are used as the antigen-binding domain, an antibody which is an antigen-binding molecule as disclosed herein in which the antigen-binding domain is linked in frame to an Fc region by a peptide bond and not *via* a linker can be used as a suitable antigen-binding molecule of the present application.

Antigen-binding molecule which eliminates aggregated antigens in preference to unaggregated antigens from plasma

By selecting an antigen-binding molecule with higher binding activity to an aggregated antigen than to an unaggregated antigen as the antigen-binding molecule of the present disclosure, its elimination effect can further be increased. Such antigen-binding molecules can be obtained from among antigen-binding molecules produced according to the later-described method of producing antigen-binding molecules by selecting antigen-binding molecules with larger difference in their binding activities for aggregated antigens and unaggregated antigens.

The elimination effect can also be enhanced by selecting antigen-binding molecules that bind to epitopes which are specific to aggregated antigens. Antigen-binding molecules that bind to such epitopes can be obtained using known methods. For example, they can be obtained by obtaining molecules that bind to an aggregated antigen, and then comparing binding of those antigen-binding molecules to the aggregated antigen and unaggregated antigen, and selecting antigen-binding molecules that have higher binding to the aggregated antigen (WO 2006/016644; EMBO J. (1994) 13, 1166-75; WO 2009/008529; and such).

Furthermore, the elimination effect can be enhanced by selecting an antigen-binding molecule that has a higher binding activity to an Fcγ receptor or to the FcRn of a complex formed between an aggregated antigen and an antigen-binding molecule than its binding activity to an Fcγ receptor or to the FcRn of a complex formed between an unaggregated antigen and the antigen-binding molecule. Such an antigen-binding molecule can be obtained, for example, by introducing into an antigen-binding molecule obtained according to the later-described method for producing antigen-binding molecules, alterations that yield the aforementioned effect of enhancing binding to an Fcγ receptor or an FcRn, allowing formation of complexes between the altered antigen-binding molecule and an aggregated antigen or between the altered antigen-binding molecule and an unaggregated antigen, and then selecting an antigen-binding molecule that shows large difference in its binding activity to the Fcγ receptor or FcRn of the complexes.

Whether an aggregated antigen is eliminated in preference to an unaggregated antigen can be confirmed by comparing plasma clearance of the aggregated antigen and plasma clearance of the unaggregated antigen. Specifically, if the ratio of clearance of an aggregated antigen in the presence of a test antigen-binding molecule to clearance of an aggregated antigen in the absence of the antigen-binding molecule (clearance of an aggregated antigen in the presence of the antigen-binding molecule / clearance of an aggregated antigen in the absence of the antigen-binding molecule) is higher than the clearance ratio for the unaggregated antigen (clearance of unaggregated antigen in the presence of the antigen-binding molecule / clearance of unaggregated antigen in the absence of the antigen-binding molecule), the antigen-binding molecule can be determined to be eliminating the aggregated antigen in preference to the unaggregated antigen. In the present invention, the clearance ratio of the aggregated antigen is preferably at least 1.5 times the clearance ratio of the unaggregated antigen (clearance ratio for aggregated antigen / clearance ratio for unaggregated antigen).

As long as use of the antigen-binding molecule can eliminate the aggregated antigen from plasma, the embodiment of its use is not particularly limited. Examples of a non-limiting embodiment of such use are pharmaceutical compositions containing an antigen-binding molecule as disclosed herein and methods comprising administering to a subject such an antigen-binding molecule. An example of another non-limiting embodiment is use of the antigen-binding molecule in an *ex vivo* method for eliminating aggregated antigens from plasma, which includes contacting an immune complex formed through contact of an antigen-binding molecule as disclosed herein with plasma isolated from a subject, and containing the antigen-binding molecules and aggregated antigens with FcRn- or Fcγ receptor-expressing cells.

Improvement of preference

As used herein, "eliminate the aggregated antigen from plasma" refers to the ability of eliminating from plasma antigens present in the plasma when an antigen-binding molecule is administered *in vivo* or when the antigen-binding molecule is secreted *in vivo*. Therefore, in the present invention, one can say that "plasma clearance of aggregated antigen by the antigen-binding molecule takes place preferentially" when plasma clearance of aggregated antigen is promoted as compared to that of unaggregated antigen when the antigen-binding molecule is administered. Whether plasma clearance of aggregated antigen by the antigen-binding molecule is taking place preferentially can be determined, for example, by measuring the above-mentioned clearance ratio. The above-mentioned clearance ratio is measured for an antigen-binding molecule that can bind to an aggregated antigen and which shows change in antigen-binding activity according to ion concentration, such as decreased antigen-binding activity in an acidic pH range compared to antigen-binding activity in a neutral pH range (or decreased antigen-binding activity in a low calcium ion concentration compared to antigen-binding activity in a high calcium ion concentration); and when the difference relative to the clearance ratio for unaggregated antigen (clearance ratio (aggregated antigen) / clearance ratio (unaggregated antigen)) becomes large, one can determine that preference has been improved.

Furthermore, if the antigen-binding molecule is administered to or secreted in an organism having biological fluids in which aggregated and unaggregated antigens coexist, and this results in a larger decrease in plasma concentration of the aggregated antigen than the decrease in plasma concentration of the unaggregated antigen, in comparison to before administration of the antigen-binding molecule, one can determine that the preference has been improved.

Improvement of pharmacokinetics

As used herein, "enhancement of pharmacokinetics", "improvement of pharmacokinetics", and "superior pharmacokinetics" can be restated as "enhancement of plasma (blood) retention", "improvement of plasma (blood) retention", "superior plasma (blood) retention", and "prolonged plasma (blood) retention". These terms are synonymous.

As used herein, "improvement of pharmacokinetics" means not only prolongation of the period until elimination from the plasma (for example, until the antigen-binding molecule is degraded intracellularly or the like and cannot return to the plasma) after administration of the antigen-binding molecule to humans, or non-human animals such as mice, rats, monkeys, rabbits, and dogs, but also prolongation of the plasma retention of the antigen-binding molecule in a form that allows antigen binding (for example, in an antigen-free form of the antigen-binding molecule) during the period of administration to elimination due to degradation. Human IgG having wild-type Fc region can bind to FcRn from non-human animals. For example, mouse can be preferably used to be administered in order to confirm the property of the antigen-binding molecule as disclosed herein since human IgG having wild-type Fc region can bind to mouse FcRn stronger than to human FcRn (Int Immunol. (2001) 13(12): 1551-1559). As another example, mouse in which its native FcRn genes are disrupted and a transgene for human FcRn gene is harbored to be expressed (Methods Mol Biol. 2010; 602: 93-104) can also be preferably used to be administered in order to confirm the property of the antigen-binding molecule as disclosed herein. Specifically, "improvement of pharmacokinetics" also includes prolongation of the period until elimination due to degradation of the antigen-binding molecule not bound to antigens (the antigen-free form of antigen-binding molecule). The antigen-binding molecule in plasma cannot bind to a new antigen if the antigen-binding molecule has already bound to an antigen. Thus, the longer the period that the antigen-binding molecule is not bound to an antigen, the longer the period that it can bind to a new antigen (the higher the chance of binding to another antigen). This enables reduction of the time period that an antigen is free of the antigen-binding molecule *in vivo* and prolongation of the period that an antigen is bound to the antigen-binding molecule. The plasma concentration of the antigen-free form of antigen-binding molecule can be increased and the period that the antigen is bound to the antigen-binding molecule can be prolonged by accelerating the antigen elimination from the plasma by administration of the antigen-binding molecule. Specifically, herein "improvement of the pharmacokinetics of antigen-binding molecule" includes the improvement of a pharmacokinetic parameter of the antigen-free form of the antigen-binding molecule (any of prolongation of the half-life in plasma, prolongation of mean retention time in plasma, and impairment of plasma clearance), prolongation of the period that the antigen is bound to the antigen-binding molecule after administration of the antigen-binding molecule, and acceleration of antigen-binding molecule-mediated antigen elimination from the plasma. The improvement of pharmacokinetics of antigen-binding molecule can be assessed by determining any one of the parameters, half-life in plasma, mean plasma retention time, and plasma clearance for the antigen-binding molecule or the antigen-free form thereof ("Pharmacokinetics: Enshu-niyoru Rikai (Understanding through practice)" Nanzando). For example, the plasma concentration of the antigen-binding molecule or antigen-free form thereof is determined after administration of the antigen-binding molecule to mice, rats, monkeys, rabbits, dogs, or humans. Then, each parameter is determined. When the plasma half-life or mean plasma retention time is prolonged, the pharmacokinetics of the antigen-binding molecule can be judged to be improved. The parameters can be determined by methods known to those skilled in the art. The parameters can be appropriately assessed, for example, by noncompartmental analysis using the pharmacokinetics analysis software WinNonlin (Pharsight) according to the appended instruction manual. The plasma concentration of antigen-free antigen-binding molecule can be determined by methods known to those skilled in the art, for example, using the assay method described in Clin Pharmacol. 2008 Apr; 48(4): 406-417.

As used herein, "improvement of pharmacokinetics" also includes prolongation of the period that an antigen is bound to an antigen-binding molecule after administration of the antigen-binding molecule. Whether the period that an antigen is bound to the antigen-binding molecule after administration of the antigen-binding molecule is prolonged can be assessed by determining the plasma concentration of free antigen. The prolongation can be judged based on the determined plasma concentration of free antigen or the time period required for an increase in the ratio of free antigen concentration to the total antigen concentration.

The plasma concentration of free antigen not bound to the antigen-binding molecule or the ratio of free antigen concentration to the total antigen concentration can be determined by methods known to those skilled in the art, for example, by the method used in Pharm Res. (2006) Jan; 23(1): 95-103. Alternatively, when an antigen exhibits a particular function *in vivo,* whether the antigen is bound to an antigen-binding molecule that neutralizes the antigen function (antagonistic molecule) can be assessed by testing whether the antigen function is neutralized. Whether the antigen function is neutralized can be assessed by assaying an *in vivo* marker that reflects the antigen function. Whether the antigen is bound to an antigen-binding molecule that activates the antigen function (agonistic molecule) can be assessed by assaying an *in vivo* marker that reflects the antigen function.

Determination of the plasma concentration of free antigen and ratio of the amount of free antigen in plasma to the amount of total antigen in plasma, *in vivo* marker assay, and such measurements are not particularly limited; however, the assays are preferably carried out after a certain period of time has passed after administration of the antigen-binding molecule. The period after administration of the antigen-binding molecule is not particularly limited; those skilled in the art can determine the appropriate period depending on the properties and the like of the administered antigen-binding molecule. Such periods include, for example, one day after administration of the antigen-binding molecule, three days after administration of the antigen-binding molecule, seven days after administration of the antigen-binding molecule, 14 days after administration of the antigen-binding molecule, and 28 days after administration of the antigen-binding molecule. As used herein, the concept "plasma antigen concentration" comprises both "total antigen concentration in plasma" which is the sum of antigen-binding molecule bound antigen and non-bound antigen concentration or "free antigen concentration in plasma" which is antigen-binding molecule non-bound antigen concentration.

The total antigen concentration in the plasma can be lowered by administration, as antigen-binding molecule, of the antigen-binding molecule as disclosed herein by 2-fold, 5-fold, 10-fold, 20-fold, 50-fold, 100-fold, 200-fold, 500-fold, 1,000-fold, or even higher as compared to administration of an antigen-binding molecule containing an antigen-binding domain whose antigen-binding activity is ion concentration-independent or an antigen-binding molecule containing an Fc region with an impaired FcγR-binding activity, or compared to when the antigen-binding domain molecule as disclosed herein is not administered.

Molar antigen/antigen-binding molecule ratio can be calculated as shown below:

value A: Molar antigen concentration at each time point
value B: Molar antigen-binding molecule concentration at each time point
value C: Molar antigen concentration per molar antigen-binding molecule concentration (molar antigen/antigen-binding molecule ratio) at each time point

$$C = A/B.$$

Smaller value C indicates higher efficiency of antigen elimination per antigen-binding molecule whereas higher value C indicates lower efficiency of antigen elimination per antigen-binding molecule.

Administering an antigen-binding molecule as disclosed herein can lower the molar antigen/antigen-binding molecule ratio by 2-fold, 5-fold, 10-fold, 20-fold, 50-fold, 100-fold, 200-fold, 500-fold, 1000-fold or more as compared to administration of antigen-binding molecules that cannot form the immune complexes disclosed as disclosed herein, antigen-binding molecules containing antigen-binding domains of which antigen-binding activity is independent of ion concentrations, or antigen-binding molecules containing Fc regions with compromised binding activity toward FcγR or FcRn.

As reference for comparison with the antigen-binding molecules as disclosed herein, antigen-binding molecules that cannot form the immune complexes disclosed herein, antigen-binding molecules containing antigen-binding domains of which antigen-binding activity is independent of ion concentrations, or antigen-binding molecules containing Fc regions with compromised binding activity toward FcγR or FcRn.

When an FcRn-mediated pathway is used in the incorporation of antigen-binding molecules as disclosed herein from the plasma into cells, reduction in the total antigen concentration in plasma or the molar antigen/antibody ratio can also be assessed using human FcRn transgenic mouse line 32 or line 276 (Jackson Laboratories, Methods Mol Biol. (2010) 602: 93-104) by either antigen-antibody co-injection model or steady-state antigen infusion model when the antigen-binding molecule do not cross-react to the mouse counterpart antigen. When the antigen-binding molecule cross-react with the mouse counterpart, they can also be assessed by simply injecting the antigen-binding molecule to human FcRn transgenic mouse line 32 or line 276 (Jackson Laboratories). In the co-injection model, mixture of the antigen-binding molecule and antigen is administered to mice. In the steady-state antigen infusion model, infusion pump filled with an antigen solution is embedded into mice to achieve a constant plasma antigen concentration, and then the antigen-binding molecule is injected to the mice. Test antigen-binding molecules are administered at the same dosage. The total antigen concentration in plasma, free antigen concentration in plasma, and antigen-binding molecule concentration in plasma are measured at appropriate time points using method known to those skilled in the art.

When an FcγR-mediated pathway is used in the incorporation of antigen-binding molecules as disclosed herein from the plasma into cells, reduction in the total antigen concentration in plasma or the molar antigen/antibody ratio can be assessed by either the antigen-antibody co-injection model or the steady-state antigen infusion model using the conventionally used C57BL/6J mice (Charles River Japan) when the antigen-binding molecule does not cross-react with the mouse counterpart antigen. If the antigen-binding molecule cross-reacts with the mouse counterpart, assessment can be carried out simply by injecting the antigen-binding molecule into the conventionally used C57BL/6J mice (Charles River Japan).

In the co-injection model, a mixture of the antigen-binding molecule and antigen is administered to mice. In the steady-state antigen infusion model, an infusion pump filled with an antigen solution is embedded into mice to achieve a constant plasma antigen concentration, and then the antigen-binding molecule is injected into the mice. Test antigen-binding molecules are administered at the same dose. The total antigen concentration in plasma, free antigen concentration in plasma, and antigen-binding molecule concentration in plasma are measured at appropriate time points using methods known to those skilled in the art.

Total or free antigen concentration in plasma and molar antigen/antigen-binding molecule ratio can be measured at 2, 4, 7, 14, 28, 56, or 84 days after administration to evaluate the long-term effect. In other words, a long term plasma antigen concentration is determined by measuring total or free antigen concentration in plasma and molar antigen/antigen-binding molecule ratio at 2, 4, 7, 14, 28, 56, or 84 days after administration of an antigen-binding molecule in order to evaluate the property of the antigen-binding molecule as disclosed herein. Whether the reduction of plasma antigen concentration or molar antigen/antigen-binding molecule ratio is achieved by antigen-binding molecule as disclosed herein can be determined by the evaluation of the reduction at any one or more of the time points described above.

Total or free antigen concentration in plasma and molar antigen/antigen-binding molecule ratio can be measured at 15 min, 1, 2, 4, 8, 12, or 24 hours after administration to evaluate the short-term effect. In other words, a short term plasma antigen concentration is determined by measuring total or free antigen concentration in plasma and molar antigen/antigen-binding molecule ratio at 15 min, 1, 2, 4, 8, 12, or 24 hours after administration of an antigen-binding molecule in order to evaluate the property of the antigen-binding molecule as disclosed herein.

The route of administration of an antigen-binding molecule as disclosed herein can be selected from intradermal, intravenous, intravitreal, subcutaneous, intraperitoneal, parenteral and intramuscular injection.

It is preferable that the pharmacokinetics of the antigen-binding molecule in human is improved. When the plasma retention in human is difficult to determine, it may be predicted based on the plasma retention in mice (for example, normal mice, human antigen-expressing transgenic mice, human FcRn-expressing transgenic mice) or monkeys (for example, cynomolgus monkeys).

As used herein, "the improvement of the pharmacokinetics and prolonged plasma retention of an antigen-binding molecule" means improvement of any pharmacokinetic parameter (any of prolongation of the half-life in plasma, prolongation of mean retention time in plasma, reduction of plasma clearance, and bioavailability) after *in vivo* administration of the antigen-binding molecule, or an increase in the concentration of the antigen-binding molecule in the plasma in an appropriate time after administration. It may be determined by measuring any parameter such as half-life in plasma, mean retention time in plasma, plasma clearance, and bioavailability of the antigen-binding molecule (Pharmacokinetics: Enshu-niyoru Rikai (Understanding through practice), (Nanzando)). For example, when an antigen-binding molecule is administered to mice (normal mice and human FcRn transgenic mice), rats, monkeys, rabbits, dogs, humans, and so on, and the concentration of the antigen-binding molecule in the plasma is determined and each of the parameters is calculated, the pharmacokinetics of the antigen-binding molecule can be judged to be improved when the plasma half-life or mean retention time in the plasma is prolonged. These parameters can be determined by methods known to those skilled in the art. For example, the parameters can be appropriately assessed by non-compartmental analysis using pharmacokinetics analysis software WinNonlin (Pharsight) according to the attached instruction manual.

While it is not bound to a particular theory, a mechanism such as that described in the Discussion of the later-described Examples is presented as an example of a mechanism that may take place when an antigen-binding molecule as disclosed herein, which binds to an aggregated antigen and comprises an Fc region and an antigen-binding domain whose antigen-binding activity varies depending on ion concentration, eliminates the aggregated antigen from plasma.

If an antibody that contains a native IgG1-type constant region against an aggregated antigen and shows pH- or Ca-dependent binding can form a large immune complex and bind to FcgR, FcRn, complement receptors, and such with avidity, it is thought that aggregated antigen elimination can be preferentially and greatly accelerated. It is thought that when GA2-IgG1 which binds to aggregated human IgA is administered, such large immune complexes are formed. It is thought that GA2-IgG1 was able to greatly accelerate elimination of aggregated human IgA because the immune complex comprising GA2-IgG1 and human IgA, which is an aggregated antigen bound to an Fc receptor such as FcRn or FcγR with avidity, and was quickly incorporated into Fc receptor-expressing cells. The IgA that dissociates from the immune complex in the endosomes of cells that have taken up the immune complex is degraded in the lysosomes. At the same time, the IgA-dissociated antibody, which was bound to FcRn in the endosomes, is subsequently recycled to the plasma and can bind again to IgA in the plasma. Elimination of human IgA in the plasma is thought to be greatly accelerated in this manner. A method using an amino-acid-variant of the Fc region which binds to FcRn in the pH neutral range is described in WO2011/122011 as a method for accelerating elimination of antigens from the plasma. The present disclosure is useful as a method for accelerating the elimination from the plasma of aggregated antigens without using the above-mentioned variants, and can further accelerate the elimination of the aggregated antigens from the plasma through combination with the above-mentioned variants. Moreover, aggregated antigens may be eliminated, other than from the plasma, from the interstitial fluid, synovial fluid, peritoneal fluid, pleural fluid, and pericardial fluid, as long as the cells contacting interstitial fluid, synovial fluid, peritoneal fluid, pleural fluid, or pericardial fluid express FcγR or FcRn. A non-limiting embodiment of such cells includes immune cells and such present in the interstitial fluid, synovial fluid, peritoneal fluid, pleural fluid, and pericardial fluid.

*Ex vivo* method for eliminating aggregated antigens from the plasma

An example of a non-limiting embodiment of the use of an antigen-binding molecule in the method provided by the present disclosure for eliminating aggregated antigens from the plasma includes use of the antigen-binding molecule in a so-called *ex vivo* method for eliminating aggregated antigens from the plasma, which includes forming an immune complex containing the antigen-binding molecules and aggregated antigens by allowing the antigen-binding molecules as disclosed herein to contact plasma isolated from a subject, and allowing the immune complex to contact cells expressing FcRn and/or Fcγ receptors.

Furthermore, an example of a non-limiting embodiment of the use of an antigen-binding molecule in the method provided herein for eliminating aggregated antigens from plasma includes use of the antigen-binding molecule in a so-called *ex vivo* method for eliminating aggregated antigens from the plasma, which includes contacting an immune complex containing the antigen-binding molecules and aggregated antigens present in the plasma isolated from a subject to whom the antigen-binding molecules as disclosed herein are administered with cells expressing FcRn and/or Fcγ receptors.

Whether an aggregated antigen is eliminated in preference to an unaggregated antigen from plasma can be confirmed, for example, by comparing and evaluating the aforementioned plasma clearance ratio for aggregated antigen (clearance of aggregated antigen in the presence of the antigen-binding molecule / clearance of aggregated antigen in the absence of the antigen-binding molecule) and the clearance ratio for unaggregated antigen (clearance of unaggregated antigen in the presence of the antigen-binding molecule / clearance of unaggregated antigen in the absence of the antigen-binding molecule).

Method of producing antigen-binding molecules containing an Fc region and an antigen-binding domain whose antigen-binding activity is ion concentration-dependent

In a non-limiting embodiment, after isolating a polynucleotide encoding an antigen-binding domain whose binding activity changes depending on the condition selected as described above, the polynucleotide is inserted into an appropriate expression vector. For example, when the antigen-binding domain is an antibody variable region, once a cDNA encoding the variable region is obtained, the cDNA is digested with restriction enzymes that recognize the restriction sites inserted at the two ends of the cDNA. Preferably, the restriction enzymes recognize and digest a nucleotide sequence that appears at a low frequency in the nucleotide sequence composing the gene of the antigen-binding molecule. Furthermore, restriction enzymes that provide cohesive ends are preferably inserted to insert a single copy of a digested fragment into the vector in the correct orientation. The cDNA encoding a variable region of an antigen-binding molecule digested as described above is inserted into an appropriate expression vector to obtain an expression vector for the antigen-binding molecule as disclosed herein. At this time, a gene encoding an antibody constant region (C region) may be fused in frame with the gene encoding the variable region.

To produce an antigen-binding molecule of interest, a polynucleotide encoding the antigen-binding molecule is inserted in a manner operably linked to a regulatory sequence into an expression vector. Regulatory sequences include, for example, enhancers and promoters. Furthermore, an appropriate signal sequence may be linked to the amino terminus so that the expressed antigen-binding molecule is secreted to the outside of the cells. As signal sequence, for example, a peptide having the amino acid sequence MGWSCIILFLVATATGVHS (SEQ ID NO: 1) is used; however, it is also possible to link other appropriate signal sequences. The expressed polypeptide is cleaved at the carboxyl terminus of the above-described sequence, and the cleaved polypeptide is secreted as a mature polypeptide to the outside of cells. Then, appropriate host cells are transformed with this expression vector so that recombinant cells expressing the polynucleotide encoding the antigen-binding molecule of interest can be obtained. The antigen-binding molecules as disclosed hereincan be produced from the recombinant cells by following the methods described above in the section "Antibody".

For a nucleic acid, "operably linked" means that the nucleic acid has a functional relationship with another nucleic acid sequence. For example, a DNA encoding a presequence or a secretory leader is operably linked to a DNA encoding a certain polypeptide if it is to be expressed as a precursor protein involved in the secretion of the polypeptide. A promoter or enhancer is operably linked to a coding sequence if it affects the transcription of the coding sequence. A ribosome binding site is operably linked to a coding sequence if it is in a position that facilitates translation. Generally, "operably linked" means that the linked DNA sequences are contiguous, and in the case of a secretory leader, it means that the linked DNA sequences are contiguous and in a reading frame. However, enhancers do not have to be contiguous. Linking is accomplished by ligation at suitable restriction sites. If such sites do not exist, synthetic oligonucleotide adaptors or linkers are used in accordance with conventional practice. Furthermore, linked nucleic acids may be produced by the above-mentioned overlap extension PCR technique.

In a non-limiting embodiment, after isolating a polynucleotide encoding the above-described antigen-binding molecule whose binding activity varies depending on a selected condition, a variant of the polynucleotide is inserted into an appropriate expression vector. Such variants preferably include those prepared *via* humanization based on the polynucleotide sequence encoding an antigen-binding molecule as disclosed herein obtained by screening as a randomized variable region library a synthetic library or an immune library constructed originating from nonhuman animals. The same methods as described above for producing above-described humanized antibodies can be used as a method for producing humanized antigen-binding molecule variants.

In another embodiment, such variants preferably include those obtained by introducing an alteration that increases the antigen affinity (affinity maturation) of an antigen-binding molecule as disclosed herein into an isolated polynucleotide sequence for the molecule obtained by screening using a synthetic library or a naive library as a randomized variable region library. Such variants can be obtained by various known procedures for affinity maturation, including CDR mutagenesis (Yang et al. (J. Mol. Biol. (1995) 254, 392-403)), chain shuffling (Marks et al. (Bio/Technology (1992) 10, 779-783)), use of *E. coli* mutant strains (Low et al. (J. Mol. Biol. (1996) 250, 359-368)), DNA shuffling (Patten et al. (Curr. Opin. Biotechnol. (1997) 8, 724-733)), phage display (Thompson et al. (J. Mol. Biol. (1996) 256, 77-88)), and sexual PCR (Clameri et al. (Nature (1998) 391, 288-291)).

As described above, antigen-binding molecules that are produced by the production methods disclosed herein include antigen-binding molecules having an Fc region. Various variants can be used as Fc regions. In an embodiment, variants of the present disclosure preferably include polynucleotides encoding antigen-binding molecules having a heavy chain in which a polynucleotide encoding an Fc region variant as described above is linked in frame to a polynucleotide encoding the above-described antigen-binding molecule whose binding activity varies depending on a selected condition.

In a non-limiting embodiment of the present invention, Fc regions preferably include, for example, Fc constant regions of antibodies such as IgG1 of SEQ ID NO: 9 (Ala is added to the N terminus of AAC82527.1), IgG2 of SEQ ID NO: 10 (Ala is added to the N terminus of AAB59393.1), IgG3 of SEQ ID NO: 11 (CAA27268.1), and IgG4 of SEQ ID NO: 12 (Ala is added to the N terminus ofAAB59394.1). A number of allotype sequences of human IgG1, human IgG2, human IgG3, and human IgG4 constant regions due to gene polymorphisms are described in "Sequences of proteins of immunological interest", NIH Publication No. 91-3242. Any of such sequences may be used in the present invention. In particular, for the human IgG1 sequence, the amino acid sequence at positions 356 to 358 as indicated by EU numbering may be DEL or EEM. The plasma retention of IgG molecules is relatively long (the elimination from plasma is slow) since FcRn, particularly human FcRn, functions as a salvage receptor for IgG molecules. IgG molecules incorporated into endosomes by pinocytosis bind under the endosomal acidic condition to FcRn, particularly human FcRn, expressed in endosomes. IgG molecules that cannot bind to FcRn, particularly human FcRn, are transferred to lysosomes, and degraded there. Meanwhile, IgG molecules bound to FcRn, particularly human FcRn, are transferred to cell surface, and then return to plasma as a result of dissociation from FcRn, particularly human FcRn, under the neutral condition in plasma.

Since antibodies comprising a typical Fc region do not have a binding activity to FcRn, particularly to human FcRn, under the plasma neutral pH range condition, typical antibodies and antibody-antigen complexes are incorporated into cells by non-specific endocytosis and transferred to cell surface by binding to FcRn, particularly human FcRn, in the endosomal acidic pH range condition. FcRn, particularly human FcRn, transports antibodies from the endosome to the cell surface. Thus, some of FcRn, particularly human FcRn, is thought to be also present on the cell surface. However, antibodies are recycled to plasma, since they dissociated from FcRn, particularly human FcRn, in the neutral pH range condition on cell surface.

Fc regions having human FcRn-binding activity in the neutral pH range, which can be included in the antigen-binding molecules as disclosed herein, can be obtained by any method. Specifically, Fc regions having human FcRn-binding activity in the neutral pH range can be obtained by altering amino acids of human IgG-type immunoglobulin as a starting Fc region. Preferred Fc regions of human IgG-type immunoglobulin for alteration include, for example, those of human IgGs (IgG1, IgG2, IgG3, and IgG4, and variants thereof). Amino acids at any positions may be altered to other amino acids as long as the resulting regions have the human FcRn-binding activity in the neutral pH range or increased human FcRn-binding activity in the neutral range. When an antigen-binding molecule comprises the Fc region of human IgG1 as human Fc region, it is preferable that the resulting region comprises an alteration that results in the effect to enhance the human FcRn binding in the neutral pH range as compared to the binding activity of the starting Fc region of human IgG1. Amino acids that allow such alterations include, for example, amino acids at positions 221 to 225, 227, 228, 230, 232, 233 to 241, 243 to 252, 254 to 260, 262 to 272, 274, 276, 278 to 289, 291 to 312, 315 to 320, 324, 325, 327 to 339, 341, 343, 345, 360, 362, 370, 375 to 378, 380, 382, 385 to 387, 389, 396, 414, 416, 423, 424, 426 to 438, 440, and 442 (indicated by EU numbering). More specifically, such amino acid alterations include those listed in Table 5. Alteration of these amino acids enhances the human FcRn binding of the Fc region of IgG-type immunoglobulin in the neutral pH range.

Among those described above, appropriate alterations that enhance the human FcRn binding in the neutral pH range are selected for use in the present invention. Particularly preferred amino acids for such Fc region variants include, for example, amino acids at positions 237, 248, 250, 252, 254, 255, 256, 257, 258, 265, 286, 289, 297, 298, 303, 305, 307, 308, 309, 311, 312, 314, 315, 317, 332, 334, 360, 376, 380, 382, 384, 385, 386, 387, 389, 424, 428, 433, 434, and 436 (indicated by EU numbering). The human FcRn-binding activity of the Fc region included in an antigen-binding molecule can be increased in the neutral pH range by substituting at least one amino acid with a different amino acid.

Particularly preferred alterations in the Fc region include, for example, at least one or more amino acid alterations selected from the group of:

Met for the amino acid at position 237;
Ile for the amino acid at position 248;
Ala, Phe, Ile, Met, Gln, Ser, Val, Trp, or Tyr for the amino acid at position 250;
Phe, Trp, or Tyr for the amino acid at position 252;
Thr for the amino acid at position 254;
Glu for the amino acid at position 255;
Asp, Asn, Glu, or Gln for the amino acid at position 256;
Ala, Gly, Ile, Leu, Met, Asn, Ser, Thr, or Val for the amino acid at position 257;
His for the amino acid at position 258;
Ala for the amino acid at position 265;
Ala or Glu for the amino acid at position 286;
His for the amino acid at position 289;
Ala for the amino acid at position 297;
Ala for the amino acid at position 303;
Ala for the amino acid at position 305;
Ala, Asp, Phe, Gly, His, Ile, Lys, Leu, Met, Asn, Pro, Gln, Arg, Ser, Val, Trp, or Tyr for the amino acid at position 307;
Ala, Phe, Ile, Leu, Met, Pro, Gln, or Thr for the amino acid at position 308;
Ala, Asp, Glu, Pro, or Arg for the amino acid at position 309;
Ala, His, or Ile for the amino acid at position 311;
Ala or His for the amino acid at position 312;
Lys or Arg for the amino acid at position 314;
Ala, Asp, or His for the amino acid at position 315;
Ala for the amino acid at position 317;

Val for the amino acid at position 332;

Leu for the amino acid at position 334;

His for the amino acid at position 360;

Ala for the amino acid at position 376;

Ala for the amino acid at position 380;

Ala for the amino acid at position 382;

Ala for the amino acid at position 384;

Asp or His for the amino acid at position 385;

Pro for the amino acid at position 386;

Glu for the amino acid at position 387;

Ala or Ser for the amino acid at position 389;

Ala for the amino acid at position 424;

Ala, Asp, Phe, Gly, His, Ile, Lys, Leu, Asn, Pro, Gln, Ser, Thr, Val, Trp, or Tyr for the amino acid at position 428;

Lys for the amino acid at position 433;

Ala, Phe, His, Ser, Trp, or Tyr for the amino acid at position 434; and

His, Ile, Leu, Phe, Thr, or Val for the amino acid at position 436 in the EU numbering system. Meanwhile, the number of altered amino acids is not particularly limited; such amino acid alterations include single amino acid alteration and alteration of amino acids at two or more sites. Combinations of amino acid alterations at two or more sites include, for example, those described in Tables 5-1 to 5-32.

In addition to the Fc region of human IgG1(SEQ ID NO: 9), IgG2 (SEQ ID NO: 10), IgG3 (SEQ ID NO: 11), or IgG4 (SEQ ID NO: 12), as Fc regions included in the present invention, Fc regions with modified FcγR binding, which have a higher Fcγ receptor-binding activity than the Fc region of a native human IgG in which the sugar chain bound at position 297 (EU numbering) is a fucose-containing sugar chain, may be suitably used. Such Fc regions with modified FcγR binding may be produced by altering amino acids in the Fc region of a native human IgG. Whether the FcγR-binding activity of an Fc region is higher than that of the Fc region of a native human IgG, in which the sugar chain bound at position 297 (EU numbering) is a fucose-containing sugar chain, can be appropriately determined using methods such as those described above.

As used herein, "alteration of amino acids" or "amino acid alteration" of an Fc region includes alteration into an amino acid sequence which is different from that of the starting Fc region. The starting Fc region may be any Fc region, as long as a variant altered from the starting Fc region can bind to human Fcγ receptor in a neutral pH range. Furthermore, an Fc region altered from a starting Fc region which had been already altered can also be used preferably as an Fc region as disclosed herein. The "starting Fc region" can refer to the polypeptide itself, a composition comprising the starting Fc region, or an amino acid sequence encoding the starting Fc region. Starting Fc regions can comprise a known Fc region produced *via* recombination described briefly in the section "Antibody". The origin of starting Fc regions is not limited, and they may be obtained from human or any nonhuman organisms. Such organisms preferably include mice, rats, guinea pigs, hamsters, gerbils, cats, rabbits, dogs, goats, sheep, bovines, horses, camels and organisms selected from nonhuman primates. In another embodiment, starting Fc regions can also be obtained from cynomolgus monkeys, marmosets, rhesus monkeys, chimpanzees, or humans. Starting Fc regions can be obtained preferably from human IgG1; however, they are not limited to any particular IgG class. This means that an Fc region of human IgG1, IgG2, IgG3, or IgG4 can be used appropriately as a starting Fc region, and herein also means that an Fc region of an arbitrary IgG class or subclass derived from any organisms described above can be preferably used as a starting Fc region. Examples of naturally-occurring IgG variants or modified forms are described in published documents (Curr. Opin. Biotechnol. (2009) 20(6): 685-91; Curr. Opin. Immunol. (2008) 20(4): 460-470; Protein Eng. Des. Sel. (2010) 23(4): 195-202; International Publication Nos. WO 2009/086320, WO 2008/092117, WO 2007/041635, and WO 2006/105338); however, they are not limited to the examples.

Examples of alterations include those with one or more mutations, for example, mutations by substitution of different amino acid residues for amino acids of starting Fc regions, by insertion of one or more amino acid residues into starting Fc regions, or by deletion of one or more amino acids from starting Fc region. Preferably, the amino acid sequences of altered Fc regions comprise at least a part of the amino acid sequence of a non-native Fc region. Such variants necessarily have sequence identity or similarity less than 100% to their starting Fc region. In a preferred embodiment, the variants have amino acid sequence identity or similarity about 75% to less than 100%, more preferably about 80% to less than 100%, even more preferably about 85% to less than 100%, still more preferably about 90% to less than 100%, and yet more preferably about 95% to less than 100% to the amino acid sequence of their starting Fc region. In a non-limiting embodiment, at least one amino acid is different between an FcγR-binding modified Fc region as disclosed herein and its starting Fc region. The amino acid differences between an FcγR-binding modified Fc region as disclosed herein and its starting Fc region can also be suitably specified based on the amino acid differences at the above-described particular amino acid positions specified by the EU numbering system.

**EP 2 857 419 B1**

The Fc region with modified FcγR binding, which has a higher Fcγ receptor-binding activity than that of the Fc region of a native human IgG in which the sugar chain bound at position 297 (EU numbering) is a fucose-containing sugar chain, contained in the antigen-binding molecules as disclosed herein may be obtained by any method. Specifically, the Fc region with modified FcγR binding can be obtained by altering amino acids in a human IgG-type immunoglobulin, and is used as the starting Fc region. Preferred Fc regions of IgG-type immunoglobulins for alteration include, for example, Fc regions of human IgGs (IgG1, IgG2, IgG3, IgG4, and variants thereof).

Amino acids of any positions may be altered to other amino acids, as long as the binding activity toward the Fcγ receptor is higher than that of the Fc region of a native human IgG, in which the sugar chain bound at position 297 (EU numbering) is a fucose-containing sugar chain. When the antigen-binding molecule contains a human IgG1 Fc region as the human Fc region, it preferably contains an alteration that yields the effect of a higher Fcγ receptor-binding activity than that of the Fc region of a native human IgG, in which the sugar chain bound at position 297 (EU numbering) is a fucose-containing sugar chain. Such amino acid alterations have been reported, for example, in international publications such as WO 2007/024249, WO 2007/021841, WO 2006/031370, WO 2000/042072, WO 2004/029207, WO 2004/099249, WO 2006/105338, WO 2007/041635, WO 2008/092117, WO 2005/070963, WO 2006/020114, WO 2006/116260, and WO 2006/023403.

Examples of such amino acids that can be altered include at least one or more amino acids selected from the group of positions 221, 222, 223, 224, 225, 227, 228, 230, 231, 232, 233, 234, 235, 236, 237, 238, 239, 240, 241, 243, 244, 245, 246, 247, 249, 250, 251, 254, 255, 256, 258, 260, 262, 263, 264, 265, 266, 267, 268, 269, 270, 271, 272, 273, 274, 275, 276, 278, 279, 280, 281, 282, 283, 284, 285, 286, 288, 290, 291, 292, 293, 294, 295, 296, 297, 298, 299, 300, 301, 302, 303, 304, 305, 311, 313, 315, 317, 318, 320, 322, 323, 324, 325, 326, 327, 328, 329, 330, 331, 332, 333, 334, 335, 336, 337, 339, 376, 377, 378, 379, 380, 382, 385, 392, 396, 421, 427, 428, 429, 434, 436, and 440 (EU numbering). Alteration of these amino acids can yield Fc regions (Fc regions with modified FcγR binding) having a higher Fcγ receptor-binding activity than the Fcγ receptor-binding activity of an Fc region of a native human IgG, in which the sugar chain bound at position 297 (EU numbering) is a fucose-containing sugar chain.

Examples of particularly preferred alterations for use in the present invention include at least one or more amino acid alterations in the Fc region selected from the group of:

Lys or Tyr for the amino acid at position 221;
Phe, Trp, Glu, or Tyr for the amino acid at position 222;
Phe, Trp, Glu, or Lys for the amino acid at position 223;
Phe, Trp, Glu, or Tyr for the amino acid at position 224;
Glu, Lys, or Trp for the amino acid at position 225;
Glu, Gly, Lys, or Tyr for the amino acid at position 227;
Glu, Gly, Lys, or Tyr for the amino acid at position 228;
Ala, Glu, Gly, or Tyr for the amino acid at position 230;
Glu, Gly, Lys, Pro, or Tyr for the amino acid at position 231;
Glu, Gly, Lys, or Tyr for the amino acid at position 232;
Ala, Asp, Phe, Gly, His, Ile, Lys, Leu, Met, Asn, Gln, Arg, Ser, Thr, Val, Trp, or Tyr for the amino acid at position 233;
Ala, Asp, Glu, Phe, Gly, His, Ile, Lys, Met, Asn, Pro, Gln, Arg, Ser, Thr, Val, Trp, or Tyr for the amino acid at position 234;
Ala, Asp, Glu, Phe, Gly, His, He, Lys, Met, Asn, Pro, Gln, Arg, Ser, Thr, Val, Trp, or Tyr for the amino acid at position 235;
Ala, Asp, Glu, Phe, His, Ile, Lys, Leu, Met, Asn, Pro, Gln, Arg, Ser, Thr, Val, Trp, or Tyr for the amino acid at position 236;
Asp, Glu, Phe, His, He, Lys, Leu, Met, Asn, Pro, Gln, Arg, Ser, Thr, Val, Trp, or Tyr for the amino acid at position 237;
Asp, Glu, Phe, Gly, His, He, Lys, Leu, Met, Asn, Gln, Arg, Ser, Thr, Val, Trp, or Tyr for the amino acid at position 238;
Asp, Glu, Phe, Gly, His, He, Lys, Leu, Met, Asn, Pro, Gln, Arg, Thr, Val, Trp, or Tyr for the amino acid at position 239;
Ala, He, Met, or Thr for the amino acid at position 240;
Asp, Glu, Leu, Arg, Trp, or Tyr for the amino acid at position 241;
Leu, Glu, Leu, Gln, Arg, Trp, or Tyr for the amino acid at position 243;
His for the amino acid at position 244;
Ala for the amino acid at position 245;
Asp, Glu, His, or Tyr for the amino acid at position 246;
Ala, Phe, Gly, His, Ile, Leu, Met, Thr, Val, or Tyr for the amino acid at position 247;
Glu, His, Gln, or Tyr for the amino acid at position 249;
Glu or Gln for the amino acid at position 250;
Phe for the amino acid at position 251;
Phe, Met, or Tyr for the amino acid at position 254;
Glu, Leu, or Tyr for the amino acid at position 255;

Ala, Met, or Pro for the amino acid at position 256;

Asp, Glu, His, Ser, or Tyr for the amino acid at position 258;

Asp, Glu, His, or Tyr for the amino acid at position 260;

Ala, Glu, Phe, Ile, or Thr for the amino acid at position 262;

Ala, He, Met, or Thr for the amino acid at position 263;

Asp, Glu, Phe, Gly, His, He, Lys, Leu, Met, Asn, Pro, Gln, Arg, Ser, Thr, Trp, or Tyr for the amino acid at position 264;

Ala, Leu, Phe, Gly, His, He, Lys, Leu, Met, Asn, Pro, Gln, Arg, Ser, Thr, Val, Trp, or Tyr for the amino acid at position 265;

Ala, He, Met, or Thr for the amino acid at position 266;

Asp, Glu, Phe, His, Ile, Lys, Leu, Met, Asn, Pro, Gln, Arg, Thr, Val, Trp, or Tyr for the amino acid at position 267;

Asp, Glu, Phe, Gly, He, Lys, Leu, Met, Pro, Gln, Arg, Thr, Val, or Trp for the amino acid at position 268;

Phe, Gly, His, He, Lys, Leu, Met, Asn, Pro, Arg, Ser, Thr, Val, Trp, or Tyr for the amino acid at position 269;

Glu, Phe, Gly, His, He, Leu, Met, Pro, Gln, Arg, Ser, Thr, Trp, or Tyr for the amino acid at position 270;

Ala, Asp, Glu, Phe, Gly, His, He, Lys, Leu, Met, Asn, Gln, Arg, Ser, Thr, Val, Trp, or Tyr for the amino acid at position 271;

Asp, Phe, Gly, His, He, Lys, Leu, Met, Pro, Arg, Ser, Thr, Val, Trp, or Tyr for the amino acid at position 272;

Phe or Ile for the amino acid at position 273;

Asp, Glu, Phe, Gly, His, Ile, Leu, Met, Asn, Pro, Arg, Ser, Thr, Val, Trp, or Tyr for the amino acid at position 274;

Leu or Trp for the amino acid at position 275;

Asp, Glu, Phe, Gly, His, Ile, Leu, Met, Pro, Arg, Ser, Thr, Val, Trp, or Tyr for the amino acid at position 276;

Asp, Glu, Gly, His, Ile, Lys, Leu, Met, Asn, Pro, Gln, Arg, Ser, Thr, Val, or Trp for the amino acid at position 278;

Ala for the amino acid at position 279;

Ala, Gly, His, Lys, Leu, Pro, Gln, Trp, or Tyr for the amino acid at position 280;

Asp, Lys, Pro, or Tyr for the amino acid at position 281;

Glu, Gly, Lys, Pro, or Tyr for the amino acid at position 282;

Ala, Gly, His, He, Lys, Leu, Met, Pro, Arg, or Tyr for the amino acid at position 283;

Asp, Glu, Leu, Asn, Thr, or Tyr for the amino acid at position 284;

Asp, Glu, Lys, Gln, Trp, or Tyr for the amino acid at position 285;

Glu, Gly, Pro, or Tyr for the amino acid at position 286;

Asn, Asp, Glu, or Tyr for the amino acid at position 288;

Asp, Gly, His, Leu, Asn, Ser, Thr, Trp, or Tyr for the amino acid at position 290;

Asp, Glu, Gly, His, He, Gln, or Thr for the amino acid at position 291;

Ala, Asp, Glu, Pro, Thr, or Tyr for the amino acid at position 292;

Phe, Gly, His, He, Leu, Met, Asn, Pro, Arg, Ser, Thr, Val, Trp, or Tyr for the amino acid at position 293;

Phe, Gly, His, He, Lys, Leu, Met, Asn, Pro, Arg, Ser, Thr, Val, Trp, or Tyr for the amino acid at position 294;

Asp, Glu, Phe, Gly, His, He, Lys, Met, Asn, Pro, Arg, Ser, Thr, Val, Trp, or Tyr for the amino acid at position 295;

Ala, Asp, Glu, Gly, His, He, Lys, Leu, Met, Asn, Gln, Arg, Ser, Thr, or Val for the amino acid at position 296;

Asp, Glu, Phe, Gly, His, He, Lys, Leu, Met, Pro, Gln, Arg, Ser, Thr, Val, Trp, or Tyr for the amino acid at position 297;

Ala, Asp, Glu, Phe, His, Ile, Lys, Met, Asn, Gln, Arg, Thr, Val, Trp, or Tyr for the amino acid at position 298;

Ala, Asp, Glu, Phe, Gly, His, Ile, Lys, Leu, Met, Asn, Pro, Gln, Arg, Ser, Val, Trp, or Tyr for the amino acid at position 299;

Ala, Asp, Glu, Gly, His, He, Lys, Leu, Met, Asn, Pro, Gln, Arg, Ser, Thr, Val, or Trp for the amino acid at position 300;

Asp, Glu, His, or Tyr for the amino acid at position 301;

Ile for the amino acid at position 302;

Asp, Gly, or Tyr for the amino acid at position 303;

Asp, His, Leu, Asn, or Thr for the amino acid at position 304;

Glu, Ile, Thr, or Tyr for the amino acid at position 305;

Ala, Asp, Asn, Thr, Val, or Tyr for the amino acid at position 311;

Phe for the amino acid at position 313;

Leu for the amino acid at position 315;

Glu or Gln for the amino acid at position 317;

His, Leu, Asn, Pro, Gln, Arg, Thr, Val, or Tyr for the amino acid at position 318;

Asp, Phe, Gly, His, Ile, Leu, Asn, Pro, Ser, Thr, Val, Trp, or Tyr for the amino acid at position 320;

Ala, Asp, Phe, Gly, His, He, Pro, Ser, Thr, Val, Trp, or Tyr for the amino acid at position 322;

Ile for the amino acid at position 323;

Asp, Phe, Gly, His, He, Leu, Met, Pro, Arg, Thr, Val, Trp, or Tyr for the amino acid at position 324;

Ala, Asp, Glu, Phe, Gly, His, He, Lys, Leu, Met, Pro, Gln, Arg, Ser, Thr, Val, Trp, or Tyr for the amino acid at position 325;

Ala, Asp, Glu, Gly, He, Leu, Met, Asn, Pro, Gln, Ser, Thr, Val, Trp, or Tyr for the amino acid at position 326;

Ala, Asp, Glu, Phe, Gly, His, He, Lys, Leu, Met, Asn, Pro, Arg, Thr, Val, Trp, or Tyr for the amino acid at position 327;

Ala, Asp, Glu, Phe, Gly, His, He, Lys, Met, Asn, Pro, Gln, Arg, Ser, Thr, Val, Trp, or Tyr for the amino acid at position 328;

Asp, Glu, Phe, Gly, His, He, Lys, Leu, Met, Asn, Gln, Arg, Ser, Thr, Val, Trp, or Tyr for the amino acid at position 329;

Cys, Glu, Phe, Gly, His, He, Lys, Leu, Met, Asn, Pro, Arg, Ser, Thr, Val, Trp, or Tyr for the amino acid at position 330;

Asp, Phe, His, He, Leu, Met, Gln, Arg, Thr, Val, Trp, or Tyr for the amino acid at position 331; Ala, Asp, Glu, Phe, Gly, His, Lys, Leu, Met, Asn, Pro, Gln, Arg, Ser, Thr, Val, Trp, or Tyr for the amino acid at position 332;

Ala, Asp, Glu, Phe, Gly, His, He, Leu, Met, Pro, Ser, Thr, Val, or Tyr for the amino acid at position 333;

Ala, Glu, Phe, He, Leu, Pro, or Thr for the amino acid at position 334;

Asp, Phe, Gly, His, He, Leu, Met, Asn, Pro, Arg, Ser, Val, Trp, or Tyr for the amino acid at position 335;

Glu, Lys, or Tyr for the amino acid at position 336;

Glu, His, or Asn for the amino acid at position 337;

Asp, Phe, Gly, Ile, Lys, Met, Asn, Gln, Arg, Ser, or Thr for the amino acid at position 339;

Ala or Val for the amino acid at position 376;

Gly or Lys for the amino acid at position 377;

Asp for the amino acid at position 378;

Asn for the amino acid at position 379;

Ala, Asn, or Ser for the amino acid at position 380;

Ala or Ile for the amino acid at position 382;

Glu for the amino acid at position 385;

Thr for the amino acid at position 392;

Leu for the amino acid at position 396;

Lys for the amino acid at position 421;

Asn for the amino acid at position 427;

Phe or Leu for the amino acid at position 428;

Met for the amino acid at position 429;

Trp for the amino acid at position 434;

Ile for the amino acid at position 436; and

Gly, His, He, Leu, or Tyr for the amino acid at position 440;

as indicated by EU numbering. Meanwhile, the number of amino acids to be altered is not particularly limited, and an amino acid at only one site may be altered or amino acids at two or more sites may be altered. Examples of combinations for the amino acid alterations at two or more sites include those described in Table 6 (Tables 6-1 to 6-3).

Among the Fc regions suitable for use in the present disclosure, a suitable example of an Fc region that has a higher binding activity toward an inhibitory Fcγ receptor than toward an activating Fcγ receptor (*i.e.,* having a selective binding activity toward an inhibitory Fcγ receptor), which is used as a non-limiting embodiment of an Fc region with the property of having a higher binding activity toward a specific Fcγ receptor than toward other Fcγ receptors (*i.e.,* an Fc region having a selective Fcγ receptor-binding activity), is an Fc region with one or more of the following alterations in the amino acids (indicated by EU numbering) of the aforementioned Fc region: the amino acid at position 238 is altered to Asp and the amino acid at position 328 is modified to Glu. The Fc regions and alterations described in US2009/0136485 may be selected appropriately as the Fc region having a selective binding activity to an inhibitory Fcγ receptor.

In a non-limiting embodiment, a suitable example is an Fc region in which one or more of the amino acids indicated by EU numbering at positions 238 and 328 according to EU numbering are respectively altered to Asp or Glu in the aforementioned Fc region.

Furthermore, in a non-limiting embodiment, suitable examples of the Fc regions are those with substitution of Asp for Pro at position 238 (EU numbering), and one or more alterations selected from among Trp for the amino acid at position 237, Phe for the amino acid at position 237, Val for the amino acid at position 267, Gln for the amino acid at position 267, Asn for the amino acid at position 268, Gly for the amino acid at position 271, Leu for the amino acid at position 326, Gln for the amino acid at position 326, Glu for the amino acid at position 326, Met for the amino acid at position 326, Asp for the amino acid at position 239, Ala for the amino acid at position 267, Trp for the amino acid at position 234, Tyr for the amino acid at position 234, Ala for the amino acid at position 237, Asp for the amino acid at position 237, Glu for the amino acid at position 237, Leu for the amino acid at position 237, Met for the amino acid at position 237, Tyr for the amino acid at position 237, Lys for the amino acid at position 330, Arg for the amino acid at position 330, Asp for the amino acid at position 233, Asp for the amino acid at position 268, Glu for the amino acid at position 268, Asp for the amino acid at position 326, Ser for the amino acid at position 326, Thr for the amino acid at position 326, Ile for the amino acid at position 323, Leu for the amino acid at position 323, Met for the amino acid at position 323, Asp for the amino acid at position 296, Ala for the amino acid at position 326, Asn for the amino acid at position 326, and Met for the amino acid at position 330, according to EU numbering.

Known methods such as site-directed mutagenesis (Kunkel et al. (Proc. Natl. Acad. Sci. USA (1985) 82, 488-492)) and Overlap extension PCR can be appropriately employed to alter the amino acids of Fc regions. Furthermore, various known methods can also be used as an amino acid alteration method for substituting amino acids by those other than natural amino acids (Annu. Rev. Biophys. Biomol. Struct. (2006) 35: 225-249; Proc. Natl. Acad. Sci. U.S.A. (2003) 100(11): 6353-6357). For example, a cell-free translation system (Clover Direct (Protein Express)) containing tRNAs in which amber suppressor tRNA, which is complementary to UAG codon (amber codon) that is a stop codon, is linked with an unnatural amino acid may be suitably used.

In an embodiment of variants of the present disclosure, polynucleotides encoding antigen-binding molecules which have a heavy chain where a polynucleotide encoding an Fc region modified to have an amino acid mutation as described above is linked in frame to a polynucleotide encoding the above-described antigen-binding molecule whose binding activity varies depending on a selected condition.

The present disclosure provides methods for producing antigen-binding molecules, comprising collecting the antigen-binding molecules from culture media of cells introduced with vectors in which a polynucleotide encoding an Fc region is operably linked in frame to a polynucleotide encoding an antigen-binding domain whose binding activity varies depending on ion concentration condition. Furthermore, the present disclosure also provides methods for producing antigen-binding molecules, comprising collecting the antigen-binding molecules from culture media of cells introduced with vectors constructed by operably linking a polynucleotide encoding an antigen-binding domain whose binding activity varies depending on ion concentration condition to a polynucleotide encoding an Fc region which is in advance operably linked to a vector.

Pharmaceutical composition

Also disclosed herein are pharmaceutical compositions comprising antigen-binding molecules of the present disclosure, antigen-binding molecules produced by alteration methods of the present disclosure, or antigen-binding molecules produced by production methods disclosed herein. Antigen-binding molecules as disclosed herein or antigen-binding molecules produced by production methods as disclosed herein are useful as pharmaceutical compositions since they, when administered, have the strong effect to reduce the plasma antigen concentration as compared to typical antigen-binding molecules, and exhibit the improved *in vivo* immune response, pharmacokinetics, and others in animals administered with the molecules. The pharmaceutical compositions as disclosed herein may comprise pharmaceutically acceptable carriers.

As used herein, pharmaceutical compositions generally refer to agents for treating or preventing, or testing and diagnosing diseases.

The pharmaceutical compositions as disclosed herein can be formulated by methods known to those skilled in the art. For example, they can be used parenterally, in the form of injections of sterile solutions or suspensions including water or other pharmaceutically acceptable liquid. For example, such compositions can be formulated by mixing in the form of unit dose required in the generally approved medicine manufacturing practice, by appropriately combining with pharmacologically acceptable carriers or media, specifically with sterile water, physiological saline, vegetable oil, emulsifier, suspension, surfactant, stabilizer, flavoring agent, excipient, vehicle, preservative, binder, or such. In such formulations, the amount of active ingredient is adjusted to obtain an appropriate amount in a pre-determined range.

Sterile compositions for injection can be formulated using vehicles such as distilled water for injection, according to standard formulation practice. Aqueous solutions for injection include, for example, physiological saline and isotonic solutions containing dextrose or other adjuvants (for example, D-sorbitol, D-mannose, D-mannitol, and sodium chloride). It is also possible to use in combination appropriate solubilizers, for example, alcohols (ethanol and such), polyalcohols (propylene glycol, polyethylene glycol, and such), non-ionic surfactants (polysorbate 80(TM), HCO-50, and such).

Oils include sesame oil and soybean oils. Benzyl benzoate and/or benzyl alcohol can be used in combination as solubilizers. It is also possible to combine buffers (for example, phosphate buffer and sodium acetate buffer), soothing agents (for example, procaine hydrochloride), stabilizers (for example, benzyl alcohol and phenol), and/or antioxidants. Appropriate ampules are filled with the prepared injections.

The pharmaceutical compositions as disclosed herein are preferably administered parenterally. For example, the compositions in the dosage form for injections, transnasal administration, transpulmonary administration, or transdermal administration are administered. For example, they can be administered systemically or locally by intravenous injection, intramuscular injection, intraperitoneal injection, subcutaneous injection, or such.

Administration methods can be appropriately selected in consideration of the patient's age and symptoms. The dose of a pharmaceutical composition containing an antigen-binding molecule can be, for example, from 0.0001 mg to 1000 mg/kg for each administration. Alternatively, the dose can be, for example, from 0.001 to 100000 mg per patient. However, the present disclosure is not limited by the numeric values described above. The doses and administration methods vary depending on the patient's weight, age, symptoms, and such. Those skilled in the art can set appropriate doses and administration methods in consideration of the factors described above.

Furthermore, provided are kits for use in the methods disclosed herein, which comprise at least an antigen-binding molecule as disclosed herein. In addition to the above, pharmaceutically acceptable carriers, media, instruction manuals describing the using method, and such may be packaged into the kits.

Amino acids contained in the amino acid sequences as disclosed herein may be post-translationally modified (for example, the modification of an N-terminal glutamine into a pyroglutamic acid by pyroglutamylation is well-known to those skilled in the art). Naturally, such post-translationally modified amino acids are included in the amino acid sequences as disclosed herein.

Screening method and production method

In one embodiment, an antigen-binding molecule that binds to an aggregated antigen and has a function of eliminating the aggregated antigen from plasma may be obtained by screening antigen-binding molecules, which comprises the following step of:

(a) selecting an antigen-binding molecule whose antigen-binding activity to an aggregated antigen under an intracellular ion concentration condition is lower than the binding activity under an extracellular ion concentration condition.

In the screening method disclosed herein, the above-mentioned ion concentration can be used as the ion concentration. For the intracellular ion concentration condition, for example, in the case of ionized calcium, the above-described low calcium concentration condition is applicable; and in the case of hydrogen ion or pH, the above-described high hydrogen ion concentration or low pH, *i.e.,* an acidic pH range condition, is applicable. On the other hand, for the extracellular ion concentration, for example, the above-described high calcium concentration condition is applicable in the case of ionized calcium, and the above-described low hydrogen ion concentration or high pH, *i.e.,* a neutral pH range condition is applicable in the case of hydrogen ion or pH. Whether the antigen-binding activity of the antigen-binding molecule is lower under an intracellular ion concentration condition than under an extracellular ion concentration condition can be confirmed by taking measurements under each of the ion concentration conditions according to known measurement methods such as those described in the above-described section "Binding Activity".

The screening method includes a method that further comprises the step(s) of:

(b):

(i) selecting an antigen-binding molecule whose binding activity to an aggregated antigen becomes higher than the binding activity to an unaggregated antigen under an extracellular ion concentration condition; and/or

(ii) selecting an antigen-binding molecule whose binding activity to an Fcγ receptor or an FcRn of a complex formed between an aggregated antigen and an antigen-binding molecule becomes higher than the binding activity to an Fcγ receptor or an FcRn of a complex formed between an unaggregated antigen and an antigen-binding molecule under an extracellular ion concentration condition.

Carrying out these step(s) can yield an antigen-binding molecule that has a function of eliminating aggregated antigens in preference to unaggregated antigens.

Whether an antigen-binding molecule will show higher binding activity to an aggregated antigen than to an unaggregated antigen under an extracellular ion concentration condition can be confirmed by measuring its binding activity to the aggregated antigen and to the unaggregated antigen, respectively, under an extracellular ion concentration condition according to the above-described methods for measuring binding activity to an Fcγ receptor or FcRn.

Furthermore, "binding activity of an antigen-binding molecule to an aggregated antigen is higher than the binding activity to an unaggregated antigen under an extracellular ion concentration condition" can be reworded as "dissociation of an antigen-binding molecule from an aggregated antigen becomes slower than dissociation from an unaggregated antigen under an extracellular ion concentration condition". Whether dissociation is becoming slow can be confirmed, for example, through comparison of the dissociation phases for the aggregated antigen and the unaggregated antigen, by using Biacore T200 (GE Healthcare) to obtain the slope of dissociation phase when the condition is changed from an extracellular ion concentration condition to an intracellular ion concentration condition. A specific method is as described in Example 2(2).

In one of the embodiments provided herein, the antigen-binding molecule that binds to an aggregated antigen and has a function of eliminating the aggregated antigen from plasma may be obtained by a method of producing an antigen-binding molecule which comprises in addition to the above-described step (a) or steps (a) and (b), the following steps of:

(c) culturing a host cell comprising a vector carrying a gene encoding the antigen-binding molecule selected in step (a) or steps (a) and (b) mentioned above; and

(d) isolating an antigen-binding molecule from the culture obtained in step (c) mentioned above.

These steps can be carried out using a known antibody production method such as described in the above-mentioned section "Antibody".

All prior art documents cited in the specification are incorporated herein by reference.

[Table 7]

| LIST OF APPLICABLE DISEASES | |
|---|---|
| DISEASE NAME | CAUSAL PROTEIN |
| Huntington('s) disease | huntingtin |
| Spinocerebellar ataxia type 1 | ataxin-1 |
| Spinocerebellar ataxia type 2 | ataxin-2 |
| Spinocerebellar ataxia type 6 | Ca channel $\alpha 1$ A |
| Spinocerebellar ataxia type 7 | ataxin-7 |
| Spinocerebellar ataxia type 17 | TATA binding protein |
| Machado-Joseph disease | MDJ |
| Dentatorubropallidoluysian atrophy | DRPLA |
| Spinal and bulbar muscular atrophy | Androgen receptor |
| $\alpha 1$-antitrypsin deficiency | $\alpha 1$-antitrypsin |
| Emphysema | $\alpha 1$-antichymotrypsin |
| Premature senility | neuroserpin |
| Angioedema | C1 inhibitor |
| Generalized thrombus | Antithrombin III |
| Alzheimer('s) disease | A$\beta$ |
| AL amyloidosis | L-ch |
| Familial polyneuropathy (FAP) | trans thyretin |
| Reactive AA amyloidosis | SAA |
| Dialysis amyloidosis | $\beta$2M |
| AH amyloidosis | H-ch |
| Cerebral amyloid angiopathy | cystatin C |
| Parkinson('s) disease | $\alpha$ synuclein |
| Dementia with Lewy bodies | $\alpha$ synuclein |
| Multiple system degeneration | $\alpha$ synuclein |
| Type 2 diabetes | amylin |
| Sickle-cell anemia | hemoglobin |
| Cataract | crystallin |
| IgA nephropathy | IgA |
| Tauopathy | Tau protein |
| Frontotemporal lobar degeneration (FTLD) | TAR DNA-binding protein 43 kDa (TDP-43) |
| Amyotrophic lateral sclerosis (ALS) | TAR DNA-binding protein 43 kDa (TDP-43), Superoxide dismutase 1 (SOD1), FUS (Fused in Sarcoma gene) |

(continued)

| LIST OF APPLICABLE DISEASES | |
|---|---|
| DISEASE NAME | CAUSAL PROTEIN |
| Creutzfeldt-Jakob disease: CJD | Prion |
| Gerstmann-Sträussler-Scheinker syndrome: GSS | Prion |
| Fatal Familial Insomnia: FFI | Prion |
| Kuru | Prion |
| Charcot-Marie-Tooth disease: CMT1A | Prion |
| Congenital central hypoventilation syndrome | PHOX2B |
| X-linked epilepsia nutans | ARX |
| Oculopharyngeal muscular dystrophy | Poly-adenylate binding protein nuclear 1 (PABPN1) |
| Limb-Girdle (distal/Miyoshi) muscular dystrophy | dysferlin |
| Desmin myopathy | desmin |
| Leukodystrophies (Alexander disease) | GFAP |
| Epidermolysis bullosa Köbner (ichthyosis) | Keratin 5/14 |

Examples

[Example 1] Preparation of antibodies that show calcium-dependent binding to human IgA (1-1) Preparation of MRA-hIgA, GC-hIgA-FLAG, and GC-hIgA-MYC

As human IgA, MRA-hIgA (heavy chain SEQ ID NO: 33; light chain SEQ ID NO: 36), GC-hIgA-FLAG (heavy chain SEQ ID NO: 34; light chain SEQ ID NO: 37), and GC-hIgA-MYC (heavy chain SEQ ID NO: 35; light chain SEQ ID NO: 37) were prepared as follows.

Preparation of MRA-hIgA

MRA-hIgA which is a recombinant of human IgA (hereinafter, MRA-hIgA) was prepared as follows. A gene fragment encoding MRA-hIgA (heavy chain SEQ ID NO: 33; light chain SEQ ID NO: 36) was inserted into an animal cell expression vector. The constructed plasmid vector was transfected into FreeStyle 293 (Invitrogen) using 293Fectin (Invitrogen) along with an EBNA1-expressing gene. Then, the transfected cells were cultured at 37°C under 8% $CO_2$ to secrete the MRA-IgA protein into the culture supernatant. The protein was purified using ion exchange chromatography and gel filtration chromatography according to a method known to those skilled in the art.

Preparation of GC-hIgA-FLAG

GC-hIgA-FLAG, which is a recombinant of human IgA, (hereinafter, GC-hIgA-FLAG) was prepared as follows. A gene fragment encoding GC-hIgA-FLAG (heavy chain SEQ ID NO: 34; light chain SEQ ID NO: 37) was inserted into an animal cell expression vector. The constructed plasmid vector was transfected into FreeStyle 293 (Invitrogen) using 293Fectin (Invitrogen) along with an EBNA1-expressing gene. Then, the transfected cells were cultured at 37°C under 8% $CO_2$ for six days to secrete the GC-hIgA protein into the culture supernatant.
The cell culture containing GC-hIgA-FLAG was filtered through a 0.22-$\mu$m bottle top filter to obtain culture supernatant. Purified GC-hIgA-FLAG was obtained using ion exchange chromatography and gel filtration chromatography according to a method known to those skilled in the art.

Preparation of GC-hIgA-MYC

GC-hIgA-MYC, which is a recombinant of human IgA, (hereinafter, GC-hIgA-MYC) was prepared as follows. A gene fragment encoding GC-hIgA-MYC (heavy chain SEQ ID NO: 35; light chain SEQ ID NO: 37) was inserted into an animal cell expression vector. The constructed plasmid vector was transfected into FreeStyle 293 (Invitrogen) using 293Fectin (Invitrogen) along with an EBNA1-expressing gene. Then, the transfected cells were cultured at 37°C under

8% $CO_2$ for six days to secrete the GC-hIgA protein into the culture supernatant.

The cell culture containing GC-hIgA-MYC was filtered through a 0.22-$\mu$m bottle top filter to obtain culture supernatant. Purified GC-hIgA-MYC was obtained using ion exchange chromatography and gel filtration chromatography according to a method known to those skilled in the art.

(1-2) Antibodies with calcium-dependent binding

H54/L28-IgGI described in International Publication No. WO 2009/125825 is a humanized anti-IL-6 receptor antibody. Fv4-IgG1 is a humanized anti-IL-6 receptor antibody that results from conferring H54/L28-IgG1 with the property of binding to soluble human IL-6 receptor in a pH-dependent manner (*i.e.,* of binding under neutral condition and dissociating under acidic condition). The *in vivo* test described in International Publication No. WO 2009/125825 using mice demonstrated that elimination of soluble human IL-6 receptor is greatly accelerated in a group administered with a mixture of Fv4-IgG1 and soluble human IL-6 receptor as antigen as compared to a group administered with a mixture of H54/L28-IgGI and soluble human IL-6 receptor as antigen.

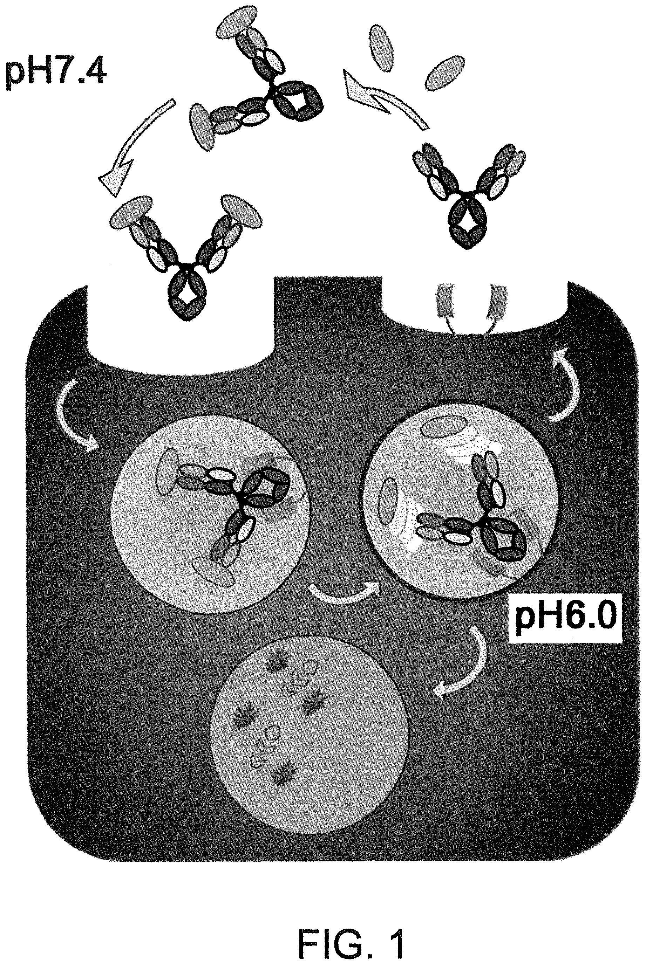

Soluble human IL-6 receptor bound to a general antibody that binds to soluble human IL-6 receptor is recycled to the plasma along with the antibody *via* FcRn. Meanwhile, an antibody that binds to soluble human IL-6 receptor in a pH-dependent manner dissociates under the acidic conditions in the endosome from the soluble human IL-6 receptor that was bound to the antibody. The dissociated soluble human IL-6 receptor is degraded in the lysosome. Thus, this can greatly accelerate the elimination of soluble human IL-6 receptor. Moreover, the antibody that binds to soluble human IL-6 receptor in a pH-dependent manner is recycled to the plasma *via* FcRn, so that the recycled antibody can bind to a soluble human IL-6 receptor again. By repeating this, a single antibody molecule can repeatedly bind to soluble human IL-6 receptors multiple times (Fig. 1).

Meanwhile, as described in International Publication No. WO 2009/125825, H54/L28-IgG1 is a humanized anti-IL-6 receptor antibody and Fv4-IgG1 is a humanized anti-IL-6 receptor antibody that results from conferring H54/L28-IgG1 with the property of binding to soluble human IL-6 receptor in a pH-dependent manner (*i.e.,* binding under neutral condition and dissociating under acidic condition). Fv4-IgG1-v2 is a humanized anti-IL-6 receptor antibody in which FcRn binding is increased over Fv4-IgG1 under neutral conditions. The *in vivo* test described in International Publication No. WO 2011/122011 using mice demonstrated that elimination of soluble human IL-6 receptor is greatly accelerated in a group administered with a mixture of Fv4-IgGl-v2 and soluble human IL-6 receptor as antigen as compared to a group administered with a mixture of Fv4-IgGl and soluble human IL-6 receptor as antigen. Thus, it was reported that, by enhancing the binding toward FcRn under neutral condition (pH 7.4) of an antibody that binds to antigens in a pH-dependent manner, the effect of the antibody to repeatedly bind to antigens and the effect of promoting elimination of antigens from the plasma can be further improved, and antigen elimination can be eliminated from the plasma through administration of the antibody (Fig. 2).

In the mechanism of a pH-dependent binding antibody shown in Figs. 1 and 2, it is important that the antibody strongly binds to an antigen in plasma and dissociates from the antigen in the endosome based on the environmental difference between plasma and endosome, i.e., pH difference (pH 7.4 in plasma; pH 6.0 in endosome). The degree of environmental difference between plasma and endosome is important for differentiating the antigen-binding ability of a pH-dependent binding antibody in plasma and endosome. A pH difference is due to a difference in the hydrogen ion concentration. Specifically, the hydrogen ion concentration in plasma (pH 7.4) is about 40 nM, while the concentration in the endosome (pH 6.0) is about 1000 nM. The factor (hydrogen ion) concentration differs by about 25 times between plasma and endosome.

The present inventors conceived that, in order to achieve the mechanism illustrated in Figs. 1 and 2 easily or to enhance the mechanism, it would be beneficial to use an antibody that depends on a factor that has a greater concentration difference between plasma and endosome than the difference of hydrogen ion concentration between the two. Thus, the inventors searched for a factor whose concentration is considerably different between plasma and endosome. As a result, calcium was identified. The ionized calcium concentration is about 1.1 to 1.3 mM in plasma and about 3 $\mu$M in the endosome. The factor (calcium) concentration differs by about 400 times between the two. Thus, the ratio was found to be greater than the difference in hydrogen ion concentration (25 times). Specifically, antigen dissociation from an antibody which is equivalent to or more efficiently than a pH-dependent binding antibody is expected to be achieved by using an ionized calcium concentration-dependent binding antibody, which binds to an antigen under a high calcium concentration condition (1.1 to 1.3 mM) but dissociates from the antigen under a low calcium concentration condition (3 $\mu$M).

(1-3) Expression and purification of antibodies that bind to human IgA

GA2-IgG1 (heavy chain SEQ ID NO: 38; light chain SEQ ID NO: 39) is a newly obtained antibody that bind to human IgA. DNA sequence encoding GA2-IgG1 (heavy chain SEQ ID NO: 38; light chain SEQ ID NO: 39) were inserted into animal cell expression plasmids by a method known to those skilled in the art. Antibodies were expressed by the following method. Cells of human fetal kidney cell-derived FreeStyle 293-F (Invitrogen) were suspended in the FreeStyle 293 Expression Medium (Invitrogen), and plated at a cell density of $1.33 \times 10^6$ cells/ml (3 ml) into each well of a 6-well plate. The constructed plasmids were transfected into cells by a lipofection method. The cells were cultured for five days in a $CO_2$ incubator (37°C, 8% $CO_2$, 90 rpm). From the prepared culture supernatants, antibodies were purified using the rProtein A Sepharose™ Fast Flow (Amersham Biosciences) by a method known to those skilled in the art. The concentrations of purified antibodies were determined by measuring absorbance at 280 nm using a spectrophotometer. Antibody concentrations were calculated from the determined values using an extinction coefficient calculated by the PACE method (Protein Science (1995) 4: 2411-2423).

(1-4) Assessment of prepared antibodies for calcium-dependent human IgA-binding activity

Using Biacore T200 (GE Healthcare), the obtained antibodies were assessed for their binding activity to human IgA (dissociation constant $K_D$ (M)). The measurement was carried out using as a running buffer 0.05% tween20, 20 mmol/l ACES, 150 mmol/l NaCl (pH 7.4 or pH 5.8) containing 3 $\mu$M or 1.2 mM $CaCl_2$, or 0.05% tween20, 20 mmol/l ACES, 150 mmol/l NaCl (pH 8.0) containing 0.1 $\mu$M or 10 mM $CaCl_2$.

After an adequate amount of recombinant Protein A/G (Thermo Scientific) was immobilized onto the Sensor chip CM5 (GE Healthcare) by an amino coupling method, antibodies were allowed to bind onto the sensor chip. An appropriate concentration of MRA-hIgA (described in (1-1)) was injected as an analyte to interact with antibodies on the sensor chip. Then, the sensor chip was regenerated by injecting 10 mmol/l glycine-HCl, pH 1.5. The measurement was carried out at 37°C. From the assay result, the dissociation constant $K_D$ (M) was calculated based on curve-fitting analysis and equilibrium constant analysis using Biacore T200 Evaluation Software (GE Healthcare). The result is shown in Table 8. It was revealed that GA2-IgG1 bound strongly to human IgA at a $Ca^{2+}$ concentration of 1.2 mM whereas the antibody bound weakly to human IgA at a $Ca^{2+}$ concentration of 3 $\mu$M. Furthermore, under a 1.2 mM $Ca^{2+}$ concentration condition, GA2-IgG1 was shown to bind to human IgA strongly at pH 7.4 but weakly at pH 5.8. More specifically, GA2-IgGI was revealed to bind to human IgA in a pH- and calcium-dependent manner.

[Table 8]

| Antibody Name | Conditions | Fit | ka | kd | KD [M] |
|---|---|---|---|---|---|
| GA2-IgG1 | pH 7.4, 1.2 mM Ca | 1:1 binding model | 4.0E+05 | 1.6E-02 | 3.9E-08 |
| | pH 7.4, 3 $\mu$M Ca | Steady State Affinity | - | - | 6.7E-06 |
| | pH 5.8, 1.2 mM Ca | Steady State Affinity | - | - | 4.0E-06 |
| | pH 5.8, 3 $\mu$M Ca | Steady State Affinity | - | - | 5.0E-06 |

[Example 2]

(2-1) Preparation of aggregated hIgA

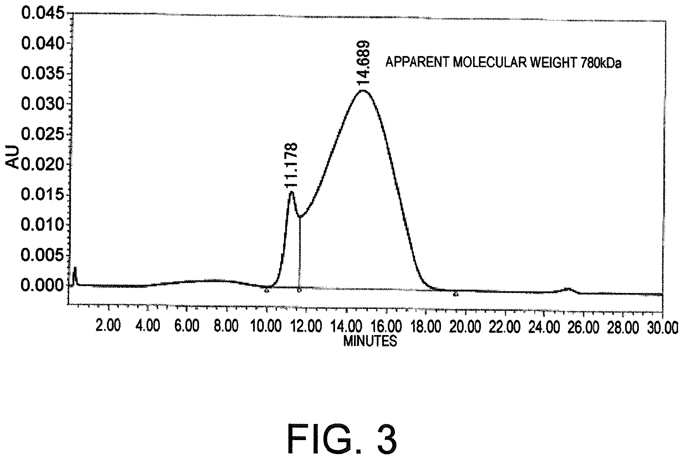

Aggregated hIgA was prepared using the crosslinking agent SPDP (N-Succinimidyl 3-(2-pyridyldithio)propionate, Thermo Scientific). GC-hIgA-MYC prepared in Example 1 was modified using SPDP, and hIgAs were cross-linked with each other by mixing a fraction that has not been treated subsequently with a fraction that has been treated under reducing conditions. After the crosslinking reaction, the macromolecular component was fractioned by gel filtration chromatography to obtain aggregated hIgA. The result of gel filtration chromatographic analysis on aggregated hIgA is shown in Fig. 3. The apparent molecular weight of the aggregated hIgA, calculated from the elution position of the molecular-weight marker, was 780 kDa. Since the elution peak is broad, aggregates of various sizes are conceivably being formed. As a comparison, GC-hIgA-FLAG that has not been treated with SPDP was used as the unaggregated hIgA.

(2-2) Assessment of the obtained antibodies for their pH/Ca-dependent binding ability to aggregated hIgA

The obtained antibodies were assessed for their binding to unaggregated hIgA and aggregated hIgA using Biacore T200 (GE Healthcare). The measurement was carried out using as a running buffer, 0.05% tween20, 20 mmol/l ACES, 150 mmol/L NaCl (pH 7.4 and pH 5.8) containing 3 $\mu$M or 1.2 mM $CaCl_2$.

After an adequate amount of recombinant Protein A/G (Thermo Scientific) was immobilized onto Sensor chip CM5 (GE Healthcare) by an amino coupling method, antibodies were allowed to bind onto the sensor chip. An appropriate concentration of hIgA (described in Example 1) was injected as an analyte to interact with antibodies on the sensor chip.

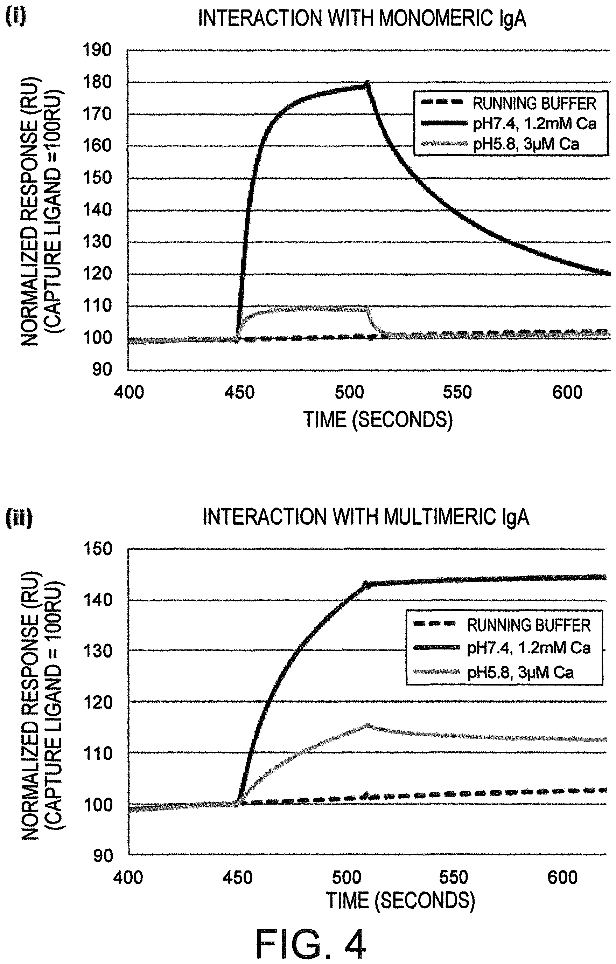

Then, the sensor chip was regenerated by injecting 10 mmol/L Glycine-HCl, pH 1.5. The measurement was carried out at 37°C. The obtained sensorgram is shown in Fig. 4.

Binding of GA2-IgG1 to unaggregated hIgA was clearly shown to be strong at a $Ca^{2+}$ concentration of 1.2 mM but weak at a $Ca^{2+}$ concentration of 3 $\mu$M. Comparison of the results from unaggregated hIgA and aggregated hIgA showed that the slope of the dissociation phase was becoming shallow for aggregated hIgA, and the dissociation of GA2-IgG1 was getting difficult. The reason for this delay of dissociation may be because a single aggregated hIgA molecule contains multiple binding sites and GA2-IgG1 binds to the molecule with avidity.

[Example 3] Assessment of human IgA-binding antibodies for their effect on plasma retention of aggregated hIgA and unaggregated hIgA using normal mice

(3-1) *In vivo* test using normal mice

Normal mice (C57BL/6J mouse; Charles River Japan) were administered with unaggregated hIgA (prepared in Example 1) or aggregated hIgA (prepared in Example 2) alone or administered with an anti-hIgA antibody one day prior to administration of unaggregated hIgA or aggregated hIgA; and then they were assessed for the *in vivo* dynamics of hIgA and the anti-hIgA antibody. An anti-hIgA antibody, an unaggregated hIgA solution (300 $\mu$g/mL) and an aggregated hIgA solution (300 $\mu$g/mL) were administered at 10 mL/kg into the caudal vein. The anti-hIgA antibody used was GA2-IgG1 described above.

The concentration of human IgA was 300 $\mu$g/mL in all of the mixed solutions. Meanwhile, the anti-hIgA antibody concentration was 30 $\mu$g/mL (0.3 mg/kg), 100 $\mu$g/mL (1 mg/kg), or 300 $\mu$g/mL (3 mg/kg). When the anti-hIgA antibody concentration was 30 $\mu$g/mL or 100 $\mu$g/mL, hIgA was present in excess relative to the anti-hIgA antibody, and therefore it was assumed that majority of the anti-hIgA antibody was bound to hIgA. Conversely, more than half of hIgA was not bound with the antibody. Meanwhile, when the anti-hIgA antibody concentration was 300 $\mu$g/mL, the anti-hIgA antibody and hIgA were present in nearly equal amounts; and when the affinity of GA2-IgGI was taken into account, 80% or so of both the anti-hIgA antibody and hIgA were thought to be bound.

Blood was collected 5 minutes, 1 hour, 2 hours, 4 hours, 7 hours, 1 day, 2 days, and 3 days, after administration. Immediately after the collection, the blood was centrifuged at 4°C and 12000 rpm for 15 minutes to isolate plasma. The isolated plasma was stored in a freezer at - 20°C or below before measurements.

(3-2) Determination of plasma concentration of anti-human IgA antibody in normal mice by ELISA

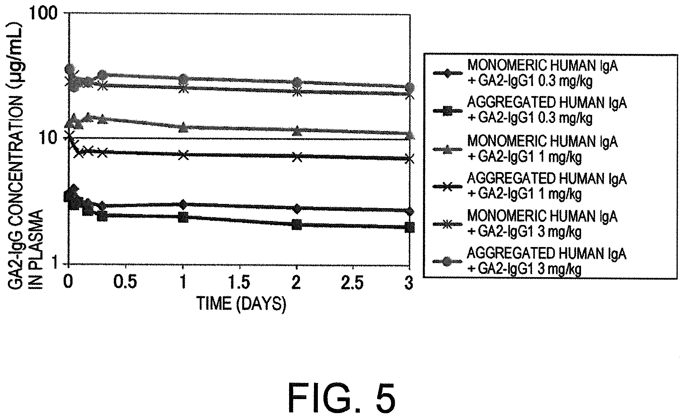

Anti-human IgA antibody concentrations in mouse plasma were determined by ELISA. First, Anti-Human IgG (y-chain specific) F(ab')2 Fragment of Antibody (SIGMA) was aliquoted into Nunc-Immuno Plate, MaxiSorp (Nalge nunc International). The plate was left overnight at 4°C to prepare an anti-human IgG antibody-immobilized plate. Standard samples were prepared at plasma concentrations of 2, 1, 0.5, 0.25, 0.125, 0.0625, and 0.03125 $\mu$g/ml. Mouse plasma assay samples were prepared by diluting 100 times or more. The samples were aliquoted into the Anti-Human IgG antibody-immobilized plate. After one hour of incubation at room temperature, the samples were reacted with the Goat Anti-Human IgG (y chain specific) Biotin (BIOT) Conjugate (Southern Biotechnology Associats Inc.) at room temperature for one hour. Then, the samples were reacted with Streptavidin-PolyHRP80 (Stereospecific Detection Technologies) at room temperature for one hour. Chromogenic reaction was carried out using the TMB One Component HRP Microwell Substrate (BioFX Laboratories) as a substrate. After the reaction was terminated with IN sulfuric acid (Showa Chemical), the absorbance at 450 nm was measured using a microplate reader. Using the analysis software SOFTmax PRO (Molecular Devices), the concentrations in mouse plasma were calculated based on the absorbance from the standard curve. A time course of plasma concentrations of antibody GA2-IgG1 determined by the above-described method after intravenous administration to normal mice is shown in Fig. 5.

(3-3) Determination of plasma human IgA concentration by ELISA

The concentration of unaggregated human IgA (monomeric human IgA) in mouse plasma was determined by ELISA. First, the Mouse anti-FLAG Antibody (SIGMA) was dispensed into the Nunc-Immuno Plates, MaxiSorp (Nalge nunc International), and allowed to stand overnight at 4°C to prepare Anti-FLAG-immobilized plates. Human IgA calibration curve samples of plasma concentrations 20, 10, 5, 2.5, 1.25, 0.625, and 0.3125 $\mu$g/mL, and mouse plasma assay samples diluted 100-fold or more were prepared and aliquoted into the Anti-FLAG-immobilized plates. The plates were incubated overnight at room temperature. 100 $\mu$L of 500 ng/mL GPC3 (R&D Systems) was added and reacted at room temperature for one hour. 100 $\mu$L of 1 $\mu$g/mL Anti-GPC3 Antibody Biotinylated was added and reacted at room temperature for one hour, and then with the Streptavidin-PolyHRP80 (Stereospecific Detection Technologies) at room temperature for one hour. Chromogenic reaction was carried out using the TMB One Component HRP Microwell Substrate (BioFX Laboratories) as a substrate. After the reaction was terminated with IN sulfuric acid (Showa Chemical), the absorbance at 450 nm was measured using a microplate reader. Using analysis software SOFTmax PRO (Molecular Devices), the concentrations in mouse plasma were calculated based on the absorbance from the standard curve.

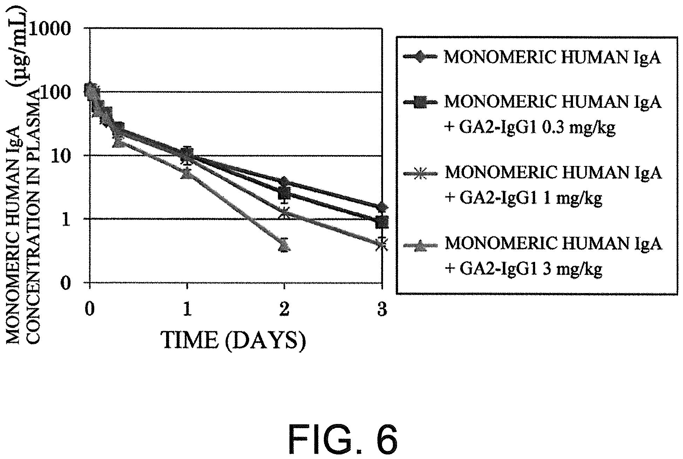

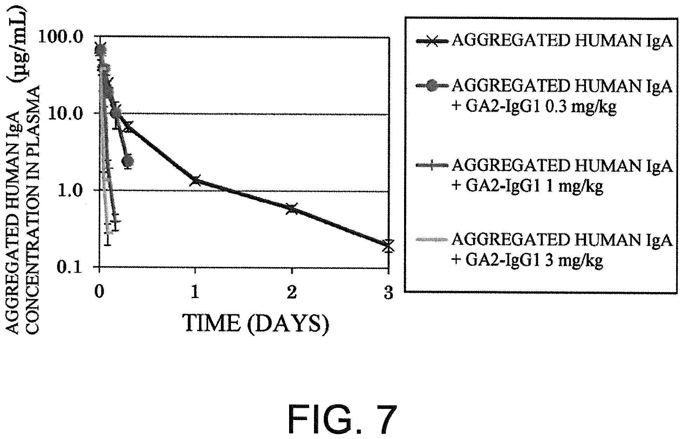

The human IgA-SPDP-Polymer (aggregated human IgA) concentration in mouse plasma was measured by the ECL method. First, a Mouse Anti-c-MYC Antibody (SIGMA) was dispensed into the MULTI-ARRAY-96-well Plate (MSD), and allowed to stand for one hour at room temperature to prepare Anti-MYC-immobilized plates. Human IgA-SPDP-Polymer calibration curve samples with plasma concentrations of 10, 5, 2.5, 1.25, 0.625, 0.3125, and 0.1563 μg/mL, and mouse plasma assay samples diluted 100-fold or more were prepared. In preparation, first, mouse plasma assay samples and 50-fold-diluted plasma containing STD were prepared using a buffer containing 10 mM EDTA, and then this was mixed with an equal amount of 20 μg/mL antibody to be administered (in 10 mM EDTA buffer) and incubated at room temperature for 30 minutes to prepare samples for addition to plates. These samples were aliquoted into the Anti-MYC-immobilized plate and then incubated at room temperature for one hour. Subsequently, 100 μL of 1 μg/mL Rabbit Anti-human-IgA-Antibody Biotinylated (BETHYL) was added and reacted at room temperature for one hour, and this was further reacted with Streptavidin-PolyHRP80 (Stereospecific Detection Technologies) at room temperature for one hour. Next, after addition of Read Buffer, measurements were taken on Sector Imager 2400 (MSD). The concentration in mouse plasma was calculated based on the response in the calibration curve using the analytical software, SOFTmax PRO (Molecular Devices). A time course of human IgA (unaggregated human IgA (monomeric human IgA)) concentration and human IgA-SPDP-Polymer (aggregated human IgA) concentration in normal mouse plasma after intraveous administration, which were measured by this method, are shown in Figures 6 and 7, respectively, and their clearance values and such are shown in Table 9.

[Table 9]

| Human IgA | GA2-IgG1 dose (mg/kg) | Human IgA clearance (mL/day/kg) | Clearance ratio CL (human IgA + GA2-IgG1) / CL (human IgA alone) | Clearance ratio (aggregated human IgA) / clearance rate (monomeric human IgA) |
|---|---|---|---|---|
| Monomeric human IgA | - | 80.6 ± 5.2 | - | - |
|  | 0.3 | 85.5 ± 20.6 | 1.1 | - |
|  | 1.0 | 98.5 ± 5.3 | 1.2 | - |
|  | 3.0 | 128 ± 6 | 1.6 | - |
| Aggregated human IgA | - | 307 ± 19 | - | - |
|  | 0.3 | 557 ± 42 | 1.8 | 1.7 |
|  | 1.0 | 1599 ± 359 | 5.2 | 4 |
|  | 3.0 | 4967 ± 411 | 16.2 | 10.2 |

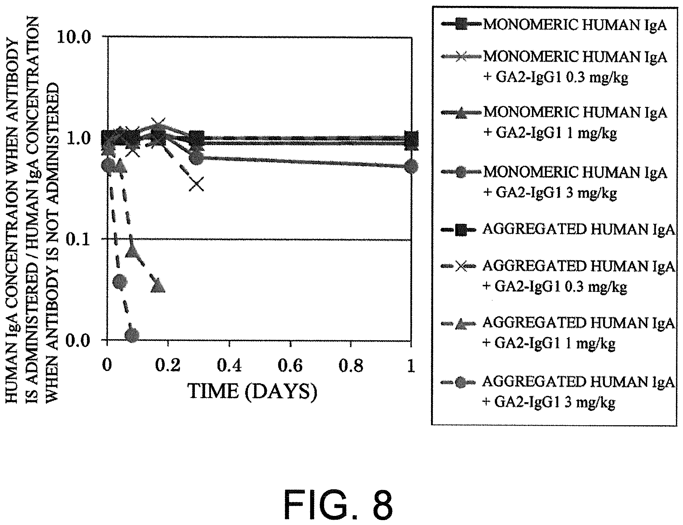

Time course of "concentration of unaggregated human IgA after GA2-IgG1 administration / concentration of unaggregated human IgA when human IgA alone is administered" and "concentration of aggregated human IgA after GA2-IgG1 administration / concentration of aggregated human IgA when human IgA alone is administered" are shown in Fig. 8 as indicators that present the degree at which elimination of both types of human IgA due to administration of GA2-IgG1 was accelerated as compared to administration of monomeric human IgA (unaggregated human IgA) alone or aggregated human IgA alone.

These results show that at any dose, GA2-IgG1 accelerates elimination of aggregated human IgA in preference to unaggregated human IgA. In particular in GA2-IgG1 at 3 mg/kg, clearance of aggregated human IgA could be accelerated preferentially by ten times or more in terms of clearance ratio.

Discussion

As a result, while clearance of unaggregated human IgA was only slightly faster when co-administered with GA2-IgG1 than when administered alone, clearance of aggregated human IgA was greatly accelerated when co-administered with GA2-IgG1 as compared to when administered alone. While the clearance ratio of unaggregated human IgA between single-administration and co-administration with an antibody is 1.1 to 1.6, the clearance ratio of aggregated human IgA between single administration and co-administration with an antibody is 1.8 to 16.2. This shows that the degree of acceleration of clearance is greater for aggregated human IgA than for unaggregated human IgA when co-administered with an antibody.

Regarding the pharmacokinetics of GA2-IgG1, also when it was co-administered with either unaggregated human IgA or aggregated human IgA, elimination was slow one day after administration and onwards.

The following mechanism may be the reason why clearance of aggregated human IgA was greatly accelerated as compared to clearance of unaggregated human IgA.

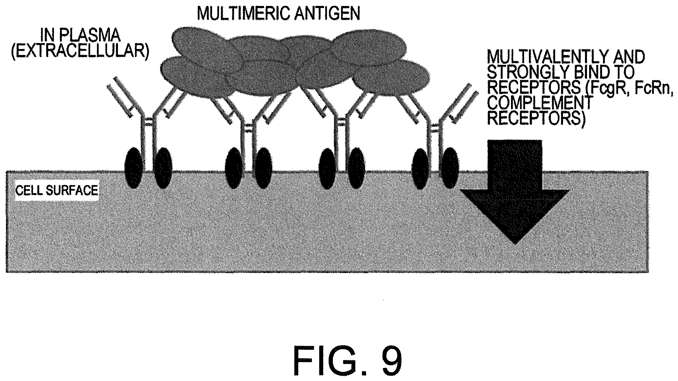

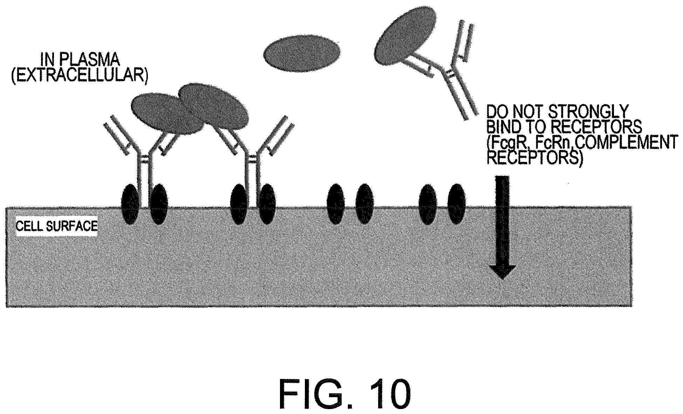

Since an aggregated antigen contains multiple antibody-binding sites in a single molecule, multiple antibody molecules are polyvalently and strongly bound; and it hardly dissociates compared to the unaggregated monomeric antigen, and forms huge immune complexes. As shown in Fig. 9, since huge immune complexes containing multiple antibody molecules can bind polyvalently and strongly to cell surface receptors (FcγR, FcRn, complement receptors, and such) *via* a polyvalent Fc region, they are efficiently taken up by cells expressing these receptors. Then, dissociation of the aggregated antigen from the antibody showing pH- or Ca-dependent binding dissolves the formation of immune complexes in the endosome. Since the aggregated antigen cannot bind to FcRn, it is transferred to a lysosome and then degraded. Meanwhile, since the antibody cannot form an immune complex, it is thought to be recycled into plasma by FcRn. In contrast, since small immune complexes not containing multiple antibodies do not have a sufficient affinity to natural IgG1-type receptors as shown in Fig. 10, their incorporation into cells is low in efficiency or takes place only non-specifically.

As shown in Fig. 4, when GA2-IgG1 is allowed to interact with aggregated human IgA, the dissociation phase becomes shallow; and compared to dissociation from unaggregated human IgA, GA2-IgG1 has the property of not being easily dissociated from aggregated human IgA. More specifically, since multiple GA2-IgG1 molecules bind multivalently to an aggregated human IgA and dissociation is difficult, they may form a larger immune complex than the immune complex formed between unaggregated human IgA and GA2-IgG1. Since huge immune complexes bind strongly *via* multivalent Fc regions to FcγR, FcRn, complement receptors, or such, it is thought that they are quickly taken up by cells expressing these receptors, and then the aggregated human IgA dissociates from GA2-IgG1 in the endosome and is transferred to the lysosome and degraded, and thus the clearance was substantially accelerated compared to when human IgA was administered alone. Furthermore, GA2-IgG1 is considered to have been recycled into plasma by FcRn after dissociation of aggregated human IgA and dissolution of the immune complex.

More specifically, GA2-IgG1 may have accomplished substantial acceleration of clearance of aggregated human IgA by two multivalent-binding effects: forming a huge immune complex by strongly and multivalently binding to aggregated human IgA more than to unaggregated human IgA, and strongly and multivalently binding to various Fc receptors *via* multiple Fc's.

As such, it was shown that GA2-IgG1 has the effect of eliminating aggregated human IgA from plasma in preference to unaggregated human IgA.

Therefore, clearance of aggregated antigens can be preferentially accelerated in the co-presence of unaggregated and aggregated antigens, by conferring an antigen-binding molecule that binds to aggregated antigens and comprises an Fc region with the function of having a pH- and/or Ca-concentration-dependent binding activity. Furthermore, it may be possible to increase preference for aggregated antigens and accelerate clearance of aggregated antigens if an appropriate pH- and Ca-dependent antibody that binds to aggregated antigens more strongly than to unaggregated antigens can be selected.

Antigen-binding molecules that have an effect of eliminating aggregated antigens in preference to unaggregated antigens from plasma in this manner may be applied as therapeutic agents for diseases caused by aggregates such as amyloidosis, polyglutamine disease, serpin disease, IgA nephropathy, and such.

**Claims**

1. A method for producing an antibody which is capable of selectively eliminating disease-causing protein aggregates in preference to protein monomers from plasma, said method comprising the following steps:

(a) selecting an antibody:

(A) wherein the ratio of the KD (dissociation constant) of the antibody to said protein aggregates under an intracellular pH 5.8 and a calcium ion concentration of 3 μM to an extracellular pH 7.4 and a calcium ion concentration of 1.2 mM is 2 or more:

(B) wherein the binding activity to the FcRn receptor of a complex formed between said protein aggregates and the antibody is higher than

the binding activity to the FcRn receptor of a complex formed between the unaggregated protein and the antibody; and

(C) wherein the ratio of plasma clearance of said aggregated protein in the absence of said antibody to plasma clearance of said aggregated protein in the presence of said antibody is 1.5 times or more than the same plasma clearance ratio for said unaggregated protein;

(b) culturing a host cell comprising a vector that carries a gene encoding the antibody selected in step (a); and

(c) isolating the antibody from the culture obtained in step (b).

2. The method of claim 1, wherein the protein is huntingtin, ataxin-1, ataxin-2, Ca channel α1A, ataxin-7, TATA binding protein, MDJ, DRPLA, androgen receptor, α1-antitrypsin, α1-antichymotrypsin, neuroserpin, Cl inhibitor, antithrombin III, Aβ, L-ch, transthyretin, SAA, β2M, H-ch, cystatin C, α synuclein, amylin, hemoglobin, crystalline, IgA, Tau protein, TAR DNA-binding protein 43 kDa (TDP-43), Superoxide dismutase (SOD1), FUS (Fused in Sarcoma gene), Prion, PHOX2B, ARX, poly-adenylate binding protein nuclear 1 (PABPN1), dysferlin, desmin, GFAP, or keratin 5/14.

3. The method of claim 1 or 2, wherein the antibody comprises an Fc region which is an Fc region represented by any one of SEQ ID NO: 9, 10, 11, or 12.

4. The method of claim 1 or 2, wherein under an acidic pH condition, FcRn-binding activity of the Fc region comprised in the antibody is enhanced compared to that of the Fc region represented by any one of SEQ ID NO: 9, 10, 11, or 12.

5. The method of claim 4, wherein the Fc region is an Fc region in which at least one or more amino acids selected from the group consisting of amino acids at positions 238, 244, 245, 249, 250, 251, 252, 253, 254, 255, 256, 257, 258, 260, 262, 265, 270, 272, 279, 283, 285, 286, 288, 293, 303, 305, 307, 308, 309, 311, 312, 314, 316, 317, 318, 332, 339, 340, 341, 343, 356, 360, 362, 375, 376, 377, 378, 380, 382, 385, 386, 387, 388, 389, 400, 413, 415, 423, 424, 427, 428, 430, 431, 433, 434, 435, 436, 438, 439, 440, 442, and 447, according to EU numbering, are substituted in the amino acid sequence of the Fc region represented by any one of SEQ ID NO: 9, 10, 11, or 12.

6. The method of claim 5, wherein the Fc region comprises at least one or more amino acids selected from the group consisting of:

Leu for the amino acid at position 238;
Leu for the amino acid at position 244;
Arg for the amino acid at position 245;
Pro for the amino acid at position 249;
Gln or Glu for the amino acid at position 250, or
Arg, Asp, Glu, or Leu for the amino acid at position 251;
Phe, Ser, Thr, or Tyr for the amino acid at position 252;
Ser or Thr for the amino acid at position 254;
Arg, Gly, Ile, or Leu for the amino acid at position 255;
Ala, Arg, Asn, Asp, Gln, Glu, Pro, or Thr for the amino acid at position 256;
Ala, Ile, Met, Asn, Ser, or Val for the amino acid at position 257;
Asp for the amino acid at position 258;
Ser for the amino acid at position 260;
Leu for the amino acid at position 262;
Lys for the amino acid at position 270;
Leu or Arg for the amino acid at position 272;
Ala, Asp, Gly, His, Met, Asn, Gln, Arg, Ser, Thr, Trp, or Tyr for the amino acid at position 279;
Ala, Asp, Phe, Gly, His, Ile, Lys, Leu, Asn, Pro, Gln, Arg, Ser, Thr, Trp, or Tyr for the amino acid at position 283;
Asn for the amino acid at position 285;

Phe for the amino acid at position 286;
Asn or Pro for the amino acid at position 288;
Val for the amino acid at position 293;
Ala, Glu, Gln, or Met for the amino acid at position 307;
Ala, Glu, Ile, Lys, Leu, Met, Ser, Val, or Trp for the amino acid at position 311;
Pro for the amino acid at position 309;
Ala, Asp, or Pro for the amino acid at position 312;
Ala or Leu for the amino acid at position 314;
Lys for the amino acid at position 316;
Pro for the amino acid at position 317;
Asn or Thr for the amino acid at position 318;
Phe, His, Lys, Leu, Met, Arg, Ser, or Trp for the amino acid at position 332;
Asn, Thr, or Trp for the amino acid at position 339;
Pro for the amino acid at position 341;
Glu, His, Lys, Gln, Arg, Thr, or Tyr for the amino acid at position 343;
Arg for the amino acid at position 375;
Gly, Ile, Met, Pro, Thr, or Val for the amino acid at position 376;
Lys for the amino acid at position 377;
Asp, Asn, or Val for the amino acid at position 378;
Ala, Asn, Ser, or Thr for the amino acid at position 380;
Phe, His, Ile, Lys, Leu, Met, Asn, Gln, Arg, Ser, Thr, Val, Trp, or Tyr for the amino acid at position 382;
Ala, Arg, Asp, Gly, His, Lys, Ser, or Thr for the amino acid at position 385;
Arg, Asp, Ile, Lys, Met, Pro, Ser, or Thr for the amino acid at position 386;
Ala, Arg, His, Pro, Ser, or Thr for the amino acid at position 387;
Asn, Pro, or Ser for the amino acid at position 389;
Asn for the amino acid at position 423;
Asn for the amino acid at position 427;
Leu, Met, Phe, Ser, or Thr for the amino acid at position 428;
Ala, Phe, Gly, His, Ile, Lys, Leu, Met, Asn, Gln, Arg, Ser, Thr, Val, or Tyr for the amino acid at position 430;
His or Asn for the amino acid at position 431;
Arg, Gln, His, Ile, Lys, Pro, or Ser for the amino acid at position 433;
Ala, Gly, His, Phe, Ser, Trp, or Tyr for the amino acid at position 434;
Arg, Asn, His, Ile, Leu, Lys, Met, or Thr for the amino acid at position 436;
Lys, Leu, Thr, or Trp for the amino acid at position 438;
Lys for the amino acid at position 440, or
Lys for the amino acid at position 442; and
Ile, Pro, or Thr for the amino acid at position 308;
as indicated by EU numbering, in the amino acid sequence of the Fc region represented by any one of SEQ ID NO: 9, 10, 11, or 12.

7. The method of claim 1 or 2, wherein under a neutral pH range condition, an FcRn-binding activity of the Fc region comprised in the antibody is enhanced compared to that of the Fc region represented by any one of SEQ ID NO: 9, 10, 11, or 12.

8. The method of claim 7, wherein the Fc region is an Fc region in which at least one or more amino acids selected from the group consisting of amino acids at positions 237, 248, 250, 252, 254, 255, 256, 257, 258, 265, 286, 289, 297, 298, 303, 305, 307, 308, 309, 311, 312, 314, 315, 317, 332, 334, 360, 376, 380, 382, 384, 385, 386, 387, 389, 424, 428, 433, 434, and 436, according to EU numbering, are substituted in the amino acid sequence of the Fc region represented by any one of SEQ ID NO: 9, 10, 11, or 12.

9. The method of claim 8, wherein the Fc region comprises at least one or more amino acids selected from the group of:

Met for the amino acid at position 237;
Ile for the amino acid at position 248;
Ala, Phe, Ile, Met, Gln, Ser, Val, Trp, or Tyr for the amino acid at position 250;
Phe, Trp, or Tyr for the amino acid at position 252;
Thr for the amino acid at position 254;
Glu for the amino acid at position 255;

Asp, Asn, Glu, or Gln for the amino acid at position 256;
Ala, Gly, Ile, Leu, Met, Asn, Ser, Thr, or Val for the amino acid at position 257;
His for the amino acid at position 258;
Ala for the amino acid at position 265;
Ala or Glu for the amino acid at position 286;
His for the amino acid at position 289;
Ala for the amino acid at position 297;
Ala for the amino acid at position 303;
Ala for the amino acid at position 305;
Ala, Asp, Phe, Gly, His, Ile, Lys, Leu, Met, Asn, Pro, Gln, Arg, Ser, Val, Trp, or Tyr for the amino acid at position 307;
Ala, Phe, Ile, Leu, Met, Pro, Gln, or Thr for the amino acid at position 308;
Ala, Asp, Glu, Pro, or Arg for the amino acid at position 309;
Ala, His, or Ile for the amino acid at position 311;
Ala or His for the amino acid at position 312;
Lys or Arg for the amino acid at position 314;
Ala, Asp, or His for the amino acid at position 315;
Ala for the amino acid at position 317;
Val for the amino acid at position 332;
Leu for the amino acid at position 334;
His for the amino acid at position 360;
Ala for the amino acid at position 376;
Ala for the amino acid at position 380;
Ala for the amino acid at position 382;
Ala for the amino acid at position 384;
Asp or His for the amino acid at position 385;
Pro for the amino acid at position 386;
Glu for the amino acid at position 387;
Ala or Ser for the amino acid at position 389;
Ala for the amino acid at position 424;
Ala, Asp, Phe, Gly, His, Ile, Lys, Leu, Asn, Pro, Gln, Ser, Thr, Val, Trp, or Tyr for the amino acid at position 428;
Lys for the amino acid at position 433;
Ala, Phe, His, Ser, Trp, or Tyr for the amino acid at position 434; and
His, Ile, Leu, Phe, Thr, or Val for the amino acid at position 436;
as indicated by EU numbering in the amino acid sequence of the Fc region represented by any one of SEQ ID NOs: 9, 10, 11, and 12.

10. The method of any one of claims 1 to 6, wherein the antibody comprises an Fc region which includes an Fc region that has a higher Fcγ receptor-binding activity than that of the Fc region of a native human IgG.

11. The method of claim 10, wherein the Fc region comprises in its amino acid sequence at least one or more amino acids that are different from amino acids of the native human IgG Fc region selected from the group of positions 221, 222, 223, 224, 225, 227, 228, 230, 231, 232, 233, 234, 235, 236, 237, 238, 239, 240, 241, 243, 244, 245, 246, 247, 249, 250, 251, 254, 255, 256, 258, 260, 262, 263, 264, 265, 266, 267, 268, 269, 270, 271, 272, 273, 274, 275, 276, 278, 279, 280, 281, 282, 283, 284, 285, 286, 288, 290, 291, 292, 293, 294, 295, 296, 297, 298, 299, 300, 301, 302, 303, 304, 305, 311, 313, 315, 317, 318, 320, 322, 323, 324, 325, 326, 327, 328, 329, 330, 331, 332, 333, 334, 335, 336, 337, 339, 376, 377, 378, 379, 380, 382, 385, 392, 396, 421, 427, 428, 429, 434, 436, and 440 (EU numbering).

12. The method of claim 11, wherein the Fc region comprises in its amino acid sequence at least one or more amino acids selected from the group of:

Lys or Tyr for the amino acid at position 221;
Phe, Trp, Glu, or Tyr for the amino acid at position 222;
Phe, Trp, Glu, or Lys for the amino acid at position 223;
Phe, Trp, Glu, or Tyr for the amino acid at position 224;
Glu, Lys, or Trp for the amino acid at position 225;
Glu, Gly, Lys, or Tyr for the amino acid at position 227;
Glu, Gly, Lys, or Tyr for the amino acid at position 228;

Ala, Glu, Gly, or Tyr for the amino acid at position 230;

Glu, Gly, Lys, Pro, or Tyr for the amino acid at position 231;

Glu, Gly, Lys, or Tyr for the amino acid at position 232;

Ala, Asp, Phe, Gly, His, Ile, Lys, Leu, Met, Asn, Gln, Arg, Ser, Thr, Val, Trp, or Tyr for the amino acid at position 233;

Ala, Asp, Glu, Phe, Gly, His, Ile, Lys, Met, Asn, Pro, Gln, Arg, Ser, Thr, Val, Trp, or Tyr for the amino acid at position 234;

Ala, Asp, Glu, Phe, Gly, His, Ile, Lys, Met, Asn, Pro, Gln, Arg, Ser, Thr, Val, Trp, or Tyr for the amino acid at position 235;

Ala, Asp, Glu, Phe, His, Ile, Lys, Leu, Met, Asn, Pro, Gln, Arg, Ser, Thr, Val, Trp, or Tyr for the amino acid at position 236;

Asp, Glu, Phe, His, Ile, Lys, Leu, Met, Asn, Pro, Gln, Arg, Ser, Thr, Val, Trp, or Tyr for the amino acid at position 237;

Asp, Glu, Phe, Gly, His, Ile, Lys, Leu, Met, Asn, Gln, Arg, Ser, Thr, Val, Trp, or Tyr for the amino acid at position 238;

Asp, Glu, Phe, Gly, His, Ile, Lys, Leu, Met, Asn, Pro, Gln, Arg, Thr, Val, Trp, or Tyr for the amino acid at position 239;

Ala, Ile, Met, or Thr for the amino acid at position 240;

Asp, Glu, Leu, Arg, Trp, or Tyr for the amino acid at position 241;

Leu, Glu, Leu, Gln, Arg, Trp, or Tyr for the amino acid at position 243;

His for the amino acid at position 244;

Ala for the amino acid at position 245;

Asp, Glu, His, or Tyr for the amino acid at position 246;

Ala, Phe, Gly, His, Ile, Leu, Met, Thr, Val, or Tyr for the amino acid at position 247;

Glu, His, Gln, or Tyr for the amino acid at position 249;

Glu or Gln for the amino acid at position 250;

Phe for the amino acid at position 251;

Phe, Met, or Tyr for the amino acid at position 254;

Glu, Leu, or Tyr for the amino acid at position 255;

Ala, Met, or Pro for the amino acid at position 256;

Asp, Glu, His, Ser, or Tyr for the amino acid at position 258;

Asp, Glu, His, or Tyr for the amino acid at position 260;

Ala, Glu, Phe, Ile, or Thr for the amino acid at position 262;

Ala, Ile, Met, or Thr for the amino acid at position 263;

Asp, Glu, Phe, Gly, His, Ile, Lys, Leu, Met, Asn, Pro, Gln, Arg, Ser, Thr, Trp, or Tyr for the amino acid at position 264;

Ala, Leu, Phe, Gly, His, Ile, Lys, Leu, Met, Asn, Pro, Gln, Arg, Ser, Thr, Val, Trp, or Tyr for the amino acid at position 265;

Ala, Ile, Met, or Thr for the amino acid at position 266;

Asp, Glu, Phe, His, Ile, Lys, Leu, Met, Asn, Pro, Gln, Arg, Thr, Val, Trp, or Tyr for the amino acid at position 267;

Asp, Glu, Phe, Gly, Ile, Lys, Leu, Met, Pro, Gln, Arg, Thr, Val, or Trp for the amino acid at position 268;

Phe, Gly, His, Ile, Lys, Leu, Met, Asn, Pro, Arg, Ser, Thr, Val, Trp, or Tyr for the amino acid at position 269;

Glu, Phe, Gly, His, Ile, Leu, Met, Pro, Gln, Arg, Ser, Thr, Trp, or Tyr for the amino acid at position 270;

Ala, Asp, Glu, Phe, Gly, His, Ile, Lys, Leu, Met, Asn, Gln, Arg, Ser, Thr, Val, Trp, or Tyr for the amino acid at position 271;

Asp, Phe, Gly, His, Ile, Lys, Leu, Met, Pro, Arg, Ser, Thr, Val, Trp, or Tyr for the amino acid at position 272;

Phe or Ile for the amino acid at position 273;

Asp, Glu, Phe, Gly, His, Ile, Leu, Met, Asn, Pro, Arg, Ser, Thr, Val, Trp, or Tyr for the amino acid at position 274;

Leu or Trp for the amino acid at position 275;

Asp, Glu, Phe, Gly, His, Ile, Leu, Met, Pro, Arg, Ser, Thr, Val, Trp, or Tyr for the amino acid at position 276;

Asp, Glu, Gly, His, Ile, Lys, Leu, Met, Asn, Pro, Gln, Arg, Ser, Thr, Val, or Trp for the amino acid at position 278;

Ala for the amino acid at position 279;

Ala, Gly, His, Lys, Leu, Pro, Gln, Trp, or Tyr for the amino acid at position 280;

Asp, Lys, Pro, or Tyr for the amino acid at position 281;

Glu, Gly, Lys, Pro, or Tyr for the amino acid at position 282;

Ala, Gly, His, Ile, Lys, Leu, Met, Pro, Arg, or Tyr for the amino acid at position 283;

Asp, Glu, Leu, Asn, Thr, or Tyr for the amino acid at position 284;

Asp, Glu, Lys, Gln, Trp, or Tyr for the amino acid at position 285;

Glu, Gly, Pro, or Tyr for the amino acid at position 286;

Asn, Asp, Glu, or Tyr for the amino acid at position 288;

Asp, Gly, His, Leu, Asn, Ser, Thr, Trp, or Tyr for the amino acid at position 290;

Asp, Glu, Gly, His, Ile, Gln, or Thr for the amino acid at position 291;

Ala, Asp, Glu, Pro, Thr, or Tyr for the amino acid at position 292;

Phe, Gly, His, Ile, Leu, Met, Asn, Pro, Arg, Ser, Thr, Val, Trp, or Tyr for the amino acid at position 293;

Phe, Gly, His, Ile, Lys, Leu, Met, Asn, Pro, Arg, Ser, Thr, Val, Trp, or Tyr for the amino acid at position 294;

Asp, Glu, Phe, Gly, His, Ile, Lys, Met, Asn, Pro, Arg, Ser, Thr, Val, Trp, or Tyr for the amino acid at position 295;

Ala, Asp, Glu, Gly, His, Ile, Lys, Leu, Met, Asn, Gln, Arg, Ser, Thr, or Val for the amino acid at position 296;

Asp, Glu, Phe, Gly, His, Ile, Lys, Leu, Met, Pro, Gln, Arg, Ser, Thr, Val, Trp, or Tyr for the amino acid at position 297;

Ala, Asp, Glu, Phe, His, Ile, Lys, Met, Asn, Gln, Arg, Thr, Val, Trp, or Tyr for the amino acid at position 298;

Ala, Asp, Glu, Phe, Gly, His, Ile, Lys, Leu, Met, Asn, Pro, Gln, Arg, Ser, Val, Trp, or Tyr for the amino acid at position 299;

Ala, Asp, Glu, Gly, His, Ile, Lys, Leu, Met, Asn, Pro, Gln, Arg, Ser, Thr, Val, or Trp for the amino acid at position 300;

Asp, Glu, His, or Tyr for the amino acid at position 301;

Ile for the amino acid at position 302;

Asp, Gly, or Tyr for the amino acid at position 303;

Asp, His, Leu, Asn, or Thr for the amino acid at position 304;

Glu, Ile, Thr, or Tyr for the amino acid at position 305;

Ala, Asp, Asn, Thr, Val, or Tyr for the amino acid at position 311;

Phe for the amino acid at position 313;

Leu for the amino acid at position 315;

Glu or Gln for the amino acid at position 317;

His, Leu, Asn, Pro, Gln, Arg, Thr, Val, or Tyr for the amino acid at position 318;

Asp, Phe, Gly, His, Ile, Leu, Asn, Pro, Ser, Thr, Val, Trp, or Tyr for the amino acid at position 320;

Ala, Asp, Phe, Gly, His, Ile, Pro, Ser, Thr, Val, Trp, or Tyr for the amino acid at position 322;

Ile for the amino acid at position 323;

Asp, Phe, Gly, His, Ile, Leu, Met, Pro, Arg, Thr, Val, Trp, or Tyr for the amino acid at position 324;

Ala, Asp, Glu, Phe, Gly, His, Ile, Lys, Leu, Met, Pro, Gln, Arg, Ser, Thr, Val, Trp, or Tyr for the amino acid at position 325;

Ala, Asp, Glu, Gly, Ile, Leu, Met, Asn, Pro, Gln, Ser, Thr, Val, Trp, or Tyr for the amino acid at position 326;

Ala, Asp, Glu, Phe, Gly, His, Ile, Lys, Leu, Met, Asn, Pro, Arg, Thr, Val, Trp, or Tyr for the amino acid at position 327;

Ala, Asp, Glu, Phe, Gly, His, Ile, Lys, Met, Asn, Pro, Gln, Arg, Ser, Thr, Val, Trp, or Tyr for the amino acid at position 328;

Asp, Glu, Phe, Gly, His, Ile, Lys, Leu, Met, Asn, Gln, Arg, Ser, Thr, Val, Trp, or Tyr for the amino acid at position 329;

Cys, Glu, Phe, Gly, His, Ile, Lys, Leu, Met, Asn, Pro, Arg, Ser, Thr, Val, Trp, or Tyr for the amino acid at position 330;

Asp, Phe, His, Ile, Leu, Met, Gln, Arg, Thr, Val, Trp, or Tyr for the amino acid at position 331;

Ala, Asp, Glu, Phe, Gly, His, Lys, Leu, Met, Asn, Pro, Gln, Arg, Ser, Thr, Val, Trp, or Tyr for the amino acid at position 332;

Ala, Asp, Glu, Phe, Gly, His, Ile, Leu, Met, Pro, Ser, Thr, Val, or Tyr for the amino acid at position 333;

Ala, Glu, Phe, Ile, Leu, Pro, or Thr for the amino acid at position 334;

Asp, Phe, Gly, His, Ile, Leu, Met, Asn, Pro, Arg, Ser, Val, Trp, or Tyr for the amino acid at position 335;

Glu, Lys, or Tyr for the amino acid at position 336;

Glu, His, or Asn for the amino acid at position 337;

Asp, Phe, Gly, Ile, Lys, Met, Asn, Gln, Arg, Ser, or Thr for the amino acid at position 339;

Ala or Val for the amino acid at position 376;

Gly or Lys for the amino acid at position 377;

Asp for the amino acid at position 378;

Asn for the amino acid at position 379;

Ala, Asn, or Ser for the amino acid at position 380;

Ala or Ile for the amino acid at position 382;

Glu for the amino acid at position 385;

Thr for the amino acid at position 392;

Leu for the amino acid at position 396;

Lys for the amino acid at position 421;

Asn for the amino acid at position 427;

Phe or Leu for the amino acid at position 428;

Met for the amino acid at position 429;

Trp for the amino acid at position 434;

Ile for the amino acid at position 436; and

Gly, His, Ile, Leu, or Tyr for the amino acid at position 440;

as indicated by EU numbering.

13. The method of any one of claims 1 to 9, wherein the antibody comprises an Fc region which has a higher binding activity toward an inhibitory Fcγ receptor than toward an activating Fcγ receptor.

14. The method of claim 13, wherein the inhibitory Fcγ receptor is human FcγRIIb.

15. The method of claim 13 or 14, wherein the activating Fcγ receptor is human FcγRIa, human FcγRIIa (R), human FcγRIIa (H), human FcγRIIIa (V), or human FcγRIIIa (F).

16. The method of any one of claims 13 to 15, wherein the amino acid at position 238 or 328 (EU numbering) in the Fc region is different from the amino acid in the native human IgG Fc region.

17. The method of claim 16, wherein the amino acid at position 238 of the Fc region is Asp or the amino acid at position 328 of the Fc region is Glu as indicated by EU numbering.

18. The method of claim 16 or 17, wherein the amino acid sequence of the Fc region comprises at least one or more amino acids selected from the group consisting of:

    Asp for the amino acid at position 233;
    Trp or Tyr for the amino acid at position 234;
    Ala, Asp, Glu, Leu, Met, Phe, Trp, or Tyr for the amino acid at position 237;
    Asp for the amino acid at position 239;
    Ala, Gln, or Val for the amino acid at position 267;
    Asn, Asp, or Glu for the amino acid at position 268;
    Gly for the amino acid at position 271;
    Ala, Asn, Asp, Gln, Glu, Leu, Met, Ser, or Thr for the amino acid at position 326;
    Arg, Lys, or Met for the amino acid at position 330;
    Ile, Leu, or Met for the amino acid at position 323; and
    Asp for the amino acid at position 296;
    as indicated by EU numbering.

**Patentansprüche**

1. Verfahren zur Herstellung eines Antikörpers, der in der Lage ist, selektiv krankheitserregende Proteinaggregate anstelle von Proteinmonomeren aus Plasma zu beseitigen; wobei das Verfahren die folgenden Schritte umfasst:

    (a) Auswählen eines Antikörpers:

        (A) wobei das Verhältnis der KD (Dissoziationskonstante) des Antikörpers zu den Proteinaggregaten bei einem intrazellulären pH-Wert von 5,8 und einer Calciumionenkonzentration von 3 μM zu einem extrazellulären pH-Wert von 7,4 und einer Calciumionenkonzentration von 1,2 mM 2 oder höher ist;
        (B) wobei die Bindungsaktivität an den FcRn-Rezeptor eines Komplexes, der zwischen den Proteinaggregaten und dem Antikörper gebildet wird, höher ist als die Bindungsaktivität an den FcRn-Rezeptor eines Komplexes, der zwischen dem nichtaggregierten Protein und dem Antikörper gebildet wird; und
        (C) wobei das Verhältnis der Plasma-Clearance des aggregierten Proteins in Abwesenheit des Antikörpers zu der Plasma-Clearance des aggregierten Proteins in Anwesenheit des Antikörpers 1,5 mal oder höher ist als das gleiche Plasma-Clearance-Verhältnis für das nicht-aggregierte Protein;

    (b) Züchten einer Wirtszelle, die einen Vektor umfasst, der ein Gen trägt, das den in Schritt (a) ausgewählten Antikörper codiert; und
    (c) Isolieren des Antikörpers aus der in Schritt (b) erhaltenen Kultur.

2. Verfahren nach Anspruch 1, wobei das Protein Huntingtin, Ataxin-1, Ataxin-2, Ca-Kanal α1A, Ataxin-7, TATA-Bindungsprotein, MDJ, DRPLA, Androgenrezeptor, α1-Antitrypsin, α1-Antichymotrypsin, Neuroserpin, C1-Inhibitor, Antithrombin III, Aß, L-ch, Transthyretin, SAA, β2M, H-ch, Cystatin C, α-Synuclein, Amylin, Hämoglobin, Crystallin, IgA, Tau-Protein, TAR DNAbindendes Protein 43 kDa (TDP-43), Superoxiddismutase (SOD1), FUS (Fused in Sarcoma gene), Prion, PHOX2B, ARX, nukleäres Polyadenylatbindendes Protein 1 (PABPN1), Dysferlin, Desmin, GFAP oder Keratin 5/14 ist.

3. Verfahren nach Anspruch 1 oder 2, wobei der Antikörper eine Fc-Region umfasst, die eine von einer der durch SEQ ID NOs:9, 10, 11 oder 12 dargestellten Fc-Region ist.

4. Verfahren nach Anspruch 1 oder 2, wobei unter einer sauren-pH-Wert-Bedingung die FcRn-Bindungsaktivität der im Antikörper umfassten Fc-Region im Vergleich zu der von einer der durch SEQ ID NOs:9, 10, 11 oder 12 dargestellten Fc-Region verbessert ist.

5. Verfahren nach Anspruch 4, wobei die Fc-Region eine Fc-Region ist, in der mindestens eine oder mehr Aminosäure(n) ausgewählt aus der Gruppe bestehend aus Aminosäuren an den Positionen 238, 244, 245, 249, 250, 251, 252, 253, 254, 255, 256, 257, 258, 260, 262, 265, 270, 272, 279, 283, 285, 286, 288, 293, 303, 305, 307, 308, 309, 311, 312, 314, 316, 317, 318, 332, 339, 340, 341, 343, 356, 360, 362, 375, 376, 377, 378, 380, 382, 385, 386, 387, 388, 389, 400, 413, 415, 423, 424, 427, 428, 430, 431, 433, 434, 435, 436, 438, 439, 440, 442 und 447 nach EU-Nummerierung in der Aminosäuresequenz der von einer der durch SEQ ID NOs:9, 10, 11 oder 12 dargestellten Fc-Region substituiert ist/sind.

6. Verfahren nach Anspruch 5, wobei die Fc-Region mindestens eine oder mehr Aminosäuren umfasst, die ausgewählt ist/sind aus der Gruppe bestehend aus:

Leu für die Aminosäure an Position 238;
Leu für die Aminosäure an Position 244;
Arg für die Aminosäure an Position 245;
Pro für die Aminosäure an Position 249;
Gln oder Glu für die Aminosäure an Position 250, oder
Arg, Asp, Glu oder Leu für die Aminosäure an Position 251;
Phe, Ser, Thr oder Tyr für die Aminosäure an Position 252;
Ser oder Thr für die Aminosäure an Position 254;
Arg, Gly, Ile oder Leu für die Aminosäure an Position 255;
Ala, Arg, Asn, Asp, Gln, Glu, Pro oder Thr für die Aminosäure an Position 256;
Ala, Ile, Met, Asn, Ser oder Val für die Aminosäure an Position 257;
Asp für die Aminosäure an Position 258;
Ser für die Aminosäure an Position 260,
Leu für die Aminosäure an Position 262;
Lys für die Aminosäure an Position 270;
Leu oder Arg für die Aminosäure an Position 272;
Ala, Asp, Gly, His, Met, Asn, Gln, Arg, Ser, Thr, Trp oder Tyr für die Aminosäure an Position 279;
Ala, Asp, Phe, Gly, His, Ile, Lys, Leu, Asn, Pro, Gln, Arg, Ser, Thr, Trp oder
Tyr für die Aminosäure an Position 283;
Asn für die Aminosäure an Position 285;
Phe für die Aminosäure an Position 286;
Asn oder Pro für die Aminosäure an Position 288;
Val für die Aminosäure an Position 293;
Ala, Glu, Gln oder Met für die Aminosäure an Position 307;
Ala, Glu, Ile, Lys, Leu, Met, Ser, Val oder Trp für die Aminosäure an Position 311;
Pro für die Aminosäure an Position 309;
Ala, Asp oder Pro für die Aminosäure an Position 312;
Ala oder Leu für die Aminosäure an Position 314;
Lys für die Aminosäure an Position 316;
Pro für die Aminosäure an Position 317;
Asn oder Thr für die Aminosäure an Position 318;
Phe, His, Lys, Leu, Met, Arg, Ser oder Trp für die Aminosäure an Position 332;
Asn, Thr oder Trp für die Aminosäure an Position 339;
Pro für die Aminosäure an Position 341;
Glu, His, Lys, Gln, Arg, Thr oder Tyr für die Aminosäure an Position 343;
Arg für die Aminosäure an Position 375;

Gly, Ile, Met, Pro, Thr oder Val für die Aminosäure an Position 376;

Lys für die Aminosäure an Position 377;

Asp, Asn oder Val für die Aminosäure an Position 378;

Ala, Asn, Ser oder Thr für die Aminosäure an Position 380;

Phe, His, Ile, Lys, Leu, Met, Asn, Gln, Arg, Ser, Thr, Val, Trp oder Tyr für die Aminosäure an Position 382;

Ala, Arg, Asp, Gly, His, Lys, Ser oder Thr für die Aminosäure an Position 385;

Arg, Asp, Ile, Lys, Met, Pro, Ser oder Thr für die Aminosäure an Position 386;

Ala, Arg, His, Pro, Ser, oder Thr für die Aminosäure an Position 387;

Asn, Pro oder Ser für die Aminosäure an Position 389;

Asn für die Aminosäure an Position 423;

Asn für die Aminosäure an Position 427;

Leu, Met, Phe, Ser oder Thr für die Aminosäure an Position 428;

Ala, Phe, Gly, His, Ile, Lys, Leu, Met, Asn, Gln, Arg, Ser, Thr, Val oder Tyr für die Aminosäure an Position 430;

His oder Asn für die Aminosäure an Position 431;

Arg, Gln, His, Ile, Lys, Pro oder Ser für die Aminosäure an Position 433;

Ala, Gly, His, Phe, Ser, Trp oder Tyr für die Aminosäure an Position 434;

Arg, Asn, His, Ile, Leu, Lys, Met oder Thr für die Aminosäure an Position 436;

Lys, Leu, Thr oder Trp für die Aminosäure an Position 438;

Lys für die Aminosäure an Position 440; oder

Lys für die Aminosäure an Position 442; und

Ile, Pro oder Thr für die Aminosäure an Position 308;

wie durch EU-Nummerierung angegeben, in der Aminosäuresequenz der von einer der durch SEQ ID NOs:9, 10, 11 oder 12 dargestellten Fc-Region.

7. Verfahren nach Anspruch 1 oder 2, wobei unter einer Bedingung eines neutralen pH-Bereichs eine FcRn-Bindungsaktivität der im Antikörper umfassten Fc-Region im Vergleich zu der von einer der durch SEQ ID NOs:9, 10, 11 oder 12 dargestellten Fc-Region verbessert ist.

8. Verfahren nach Anspruch 7, wobei die Fc-Region eine Fc-Region ist, in der mindestens eine oder mehr Aminosäuren ausgewählt aus der Gruppe bestehend aus Aminosäuren an den Positionen 237, 248, 250, 252, 254, 255, 256, 257, 258, 265, 286, 289, 297, 298, 303, 305, 307, 308, 309, 311, 312, 314, 315, 317, 332, 334, 360, 376, 380, 382, 384, 385, 386, 387, 389, 424, 428, 433, 434 und 436 nach EU-Nummerierung, in der Aminosäuresequenz der von einer der durch SEQ ID NOs:9, 10, 11 oder 12 dargestellten Fc-Region substituiert ist/sind.

9. Verfahren nach Anspruch 8, wobei die Fc-Region mindestens eine oder mehr Aminosäuren umfasst, ausgewählt aus der Gruppe aus:

Met für die Aminosäure an Position 237;

Ile für die Aminosäure an Position 248;

Ala, Phe, Ile, Met, Gln, Ser, Val, Trp oder Tyr für die Aminosäure an Position 250;

Phe, Trp oder Tyr für die Aminosäure an Position 252;

Thr für die Aminosäure an Position 254;

Glu für die Aminosäure an Position 255;

Asp, Asn, Glu oder Gln für die Aminosäure an Position 256;

Ala, Gly, Ile, Leu, Met, Asn, Ser, Thr oder Val für die Aminosäure an Position 257;

His für die Aminosäure an Position 258;

Ala für die Aminosäure an Position 265;

Ala oder Glu für die Aminosäure an Position 286;

His für die Aminosäure an Position 289;

Ala für die Aminosäure an Position 297;

Ala für die Aminosäure an Position 303;

Ala für die Aminosäure an Position 305;

Ala, Asp, Phe, Gly, His, Ile, Lys, Leu, Met, Asn, Pro, Gln, Arg, Ser, Val, Trp oder Tyr für die Aminosäure an Position 307;

Ala, Phe, Ile, Leu, Met, Pro, Gln oder Thr für die Aminosäure an Position 308;

Ala, Asp, Glu, Pro oder Arg für die Aminosäure an Position 309;

Ala, His oder Ile für die Aminosäure an Position 311;

Ala oder His für die Aminosäure an Position 312;

Lys oder Arg für die Aminosäure an Position 314;

Ala, Asp oder His für die Aminosäure an Position 315;

Ala für die Aminosäure an Position 317;

Val für die Aminosäure an Position 332;

Leu für die Aminosäure an Position 334;

His für die Aminosäure an Position 360;

Ala für die Aminosäure an Position 376;

Ala für die Aminosäure an Position 380;

Ala für die Aminosäure an Position 382;

Ala für die Aminosäure an Position 384;

Asp oder His für die Aminosäure an Position 385;

Pro für die Aminosäure an Position 386;

Glu für die Aminosäure an Position 387;

Ala oder Ser für die Aminosäure an Position 389;

Ala für die Aminosäure an Position 424;

Ala, Asp, Phe, Gly, His, Ile, Lys, Leu, Asn, Pro, Gln, Ser, Thr, Val, Trp oder Tyr für die Aminosäure an Position 428;

Lys für die Aminosäure an Position 433;

Ala, Phe, His, Ser, Trp oder Tyr für die Aminosäure an Position 434; und

His, Ile, Leu, Phe, Thr oder Val für die Aminosäure an Position 436;

wie durch EU-Nummerierung angegeben, in der Aminosäuresequenz der von einer der durch SEQ ID NOs:9, 10, 11 und 12 dargestellten Fc-Region.

10. Verfahren nach einem der Ansprüche 1 bis 6, wobei der Antikörper eine Fc-Region umfasst, die eine höhere Fcγ-Rezeptor-Bindungsaktivität aufweist als die Fc-Region eines nativen humanen IgG.

11. Verfahren nach Anspruch 10, wobei die Fc-Region in ihrer Aminosäuresequenz mindestens eine oder mehr Aminosäuren umfasst, die sich von Aminosäuren der Fc-Region von nativem humanen IgG unterscheiden, ausgewählt aus der Gruppe der Positionen 221, 222, 223, 224, 225, 227, 228, 230, 231, 232, 233, 234, 235, 236, 237, 238, 239, 240, 241, 243, 244, 245, 246, 247, 249, 250, 251, 254, 255, 256, 258, 260, 262, 263, 264, 265, 266, 267, 268, 269, 270, 271, 272, 273, 274, 275, 276, 278, 279, 280, 281, 282, 283, 284, 285, 286, 288, 290, 291, 292, 293, 294, 295, 296, 297, 298, 299, 300, 301, 302, 303, 304, 305, 311, 313, 315, 317, 318, 320, 322, 323, 324, 325, 326, 327, 328, 329, 330, 331, 332, 333, 334, 335, 336, 337, 339, 376, 377, 378, 379, 380, 382, 385, 392, 396, 421, 427, 428, 429, 434, 436 und 440 (EU-Nummerierung).

12. Verfahren nach Anspruch 11, wobei die Fc-Region in ihrer Aminosäuresequenz mindestens eine oder mehr Aminosäuren umfasst, ausgewählt aus der Gruppe aus:

Lys oder Tyr für die Aminosäure an Position 221;

Phe, Trp, Glu oder Tyr für die Aminosäure an Position 222;

Phe, Trp, Glu oder Lys für die Aminosäure an Position 223;

Phe, Trp, Glu oder Tyr für die Aminosäure an Position 224;

Glu, Lys oder Trp für die Aminosäure an Position 225;

Glu, Gly, Lys oder Tyr für die Aminosäure an Position 227;

Glu, Gly, Lys oder Tyr für die Aminosäure an Position 228;

Ala, Glu, Gly oder Tyr für die Aminosäure an Position 230;

Glu, Gly, Lys, Pro oder Tyr für die Aminosäure an Position 231;

Glu, Gly, Lys oder Tyr für die Aminosäure an Position 232;

Ala, Asp, Phe, Gly, His, Ile, Lys, Leu, Met, Asn, Gln, Arg, Ser, Thr, Val, Trp oder Tyr für die Aminosäure an Position 233;

Ala, Asp, Glu, Phe, Gly, His, Ile, Lys, Met, Asn, Pro, Gln, Arg, Ser, Thr, Val, Trp oder Tyr für die Aminosäure an Position 234;

Ala, Asp, Glu, Phe, Gly, His, Ile, Lys, Met, Asn, Pro, Gln, Arg, Ser, Thr, Val, Trp oder Tyr für die Aminosäure an Position 235;

Ala, Asp, Glu, Phe, His, Ile, Lys, Leu, Met, Asn, Pro, Gln, Arg, Ser, Thr, Val, Trp oder Tyr für die Aminosäure an Position 236;

Asp, Glu, Phe, His, Ile, Lys, Leu, Met, Asn, Pro, Gln, Arg, Ser, Thr, Val, Trp oder Tyr für die Aminosäure an Position 237;

Asp, Glu, Phe, Gly, His, Ile, Lys, Leu, Met, Asn, Gln, Arg, Ser, Thr, Val, Trp, oder Tyr für die Aminosäure an Position 238;

Asp, Glu, Phe, Gly, His, Ile, Lys, Leu, Met, Asn, Pro, Gln, Arg, Thr, Val, Trp oder Tyr für die Aminosäure an Position 239;

Ala, Ile, Met oder Thr für die Aminosäure an Position 240;

Asp, Glu, Leu, Arg, Trp oder Tyr für die Aminosäure an Position 241;

Leu, Glu, Leu, Gln, Arg, Trp oder Tyr für die Aminosäure an Position 243;

His für die Aminosäure an Position 244;

Ala für die Aminosäure an Position 245;

Asp, Glu, His oder Tyr für die Aminosäure an Position 246;

Ala, Phe, Gly, His, Ile, Leu, Met, Thr, Val oder Tyr für die Aminosäure an Position 247;

Glu, His, Gln oder Tyr für die Aminosäure an Position 249;

Glu oder Gln für die Aminosäure an Position 250;

Phe für die Aminosäure an Position 251;

Phe, Met oder Tyr für die Aminosäure an Position 254;

Glu, Leu oder Tyr für die Aminosäure an Position 255;

Ala, Met oder Pro für die Aminosäure an Position 256;

Asp, Glu, His, Ser oder Tyr für die Aminosäure an Position 258;

Asp, Glu, His oder Tyr für die Aminosäure an Position 260;

Ala, Glu, Phe, Ile oder Thr für die Aminosäure an Position 262;

Ala, Ile, Met oder Thr für die Aminosäure an Position 263;

Asp, Glu, Phe, Gly, His, Ile, Lys, Leu, Met, Asn, Pro, Gln, Arg, Ser, Thr, Trp oder Tyr für die Aminosäure an Position 264;

Ala, Leu, Phe, Gly, His, Ile, Lys, Leu, Met, Asn, Pro, Gln, Arg, Ser, Thr, Val, Trp oder Tyr für die Aminosäure an Position 265;

Ala, Ile, Met oder Thr für die Aminosäure an Position 266;

Asp, Glu, Phe, His, Ile, Lys, Leu, Met, Asn, Pro, Gln, Arg, Thr, Val, Trp oder Tyr für die Aminosäure an Position 267;

Asp, Glu, Phe, Gly, Ile, Lys, Leu, Met, Pro, Gln, Arg, Thr, Val oder Trp für die Aminosäure an Position 268;

Phe, Gly, His, Ile, Lys, Leu, Met, Asn, Pro, Arg, Ser, Thr, Val, Trp oder Tyr für die Aminosäure an Position 269;

Glu, Phe, Gly, His, Ile, Leu, Met, Pro, Gln, Arg, Ser, Thr, Trp oder Tyr für die Aminosäure an Position 270;

Ala, Asp, Glu, Phe, Gly, His, Ile, Lys, Leu, Met, Asn, Gln, Arg, Ser, Thr, Val, Trp oder Tyr für die Aminosäure an Position 271;

Asp, Phe, Gly, His, Ile, Lys, Leu, Met, Pro, Arg, Ser, Thr, Val, Trp oder Tyr für die Aminosäure an Position 272;

Phe oder Ile für die Aminosäure an Position 273;

Asp, Glu, Phe, Gly, His, Ile, Leu, Met, Asn, Pro, Arg, Ser, Thr, Val, Trp oder Tyr für die Aminosäure an Position 274;

Leu oder Trp für die Aminosäure an Position 275;

Asp, Glu, Phe, Gly, His, Ile, Leu, Met, Pro, Arg, Ser, Thr, Val, Trp oder Tyr für die Aminosäure an Position 276;

Asp, Glu, Gly, His, Ile, Lys, Leu, Met, Asn, Pro, Gln, Arg, Ser, Thr, Val oder Trp für die Aminosäure an Position 278;

Ala für die Aminosäure an Position 279;

Ala, Gly, His, Lys, Leu, Pro, Gln, Trp oder Tyr für die Aminosäure an Position 280;

Asp, Lys, Pro oder Tyr für die Aminosäure an Position 281;

Glu, Gly, Lys, Pro oder Tyr für die Aminosäure an Position 282;

Ala, Gly, His, Ile, Lys, Leu, Met, Pro, Arg oder Tyr für die Aminosäure an Position 283;

Asp, Glu, Leu, Asn, Thr oder Tyr für die Aminosäure an Position 284;

Asp, Glu, Lys, Gln, Trp oder Tyr für die Aminosäure an Position 285;

Glu, Gly, Pro oder Tyr für die Aminosäure an Position 286;

Asn, Asp, Glu oder Tyr für die Aminosäure an Position 288;

Asp, Gly, His, Leu, Asn, Ser, Thr, Trp oder Tyr für die Aminosäure an Position 290;

Asp, Glu, Gly, His, Ile, Gln oder Thr für die Aminosäure an Position 291;

Ala, Asp, Glu, Pro, Thr oder Tyr für die Aminosäure an Position 292;

Phe, Gly, His, Ile, Leu, Met, Asn, Pro, Arg, Ser, Thr, Val, Trp oder Tyr für die Aminosäure an Position 293;

Phe, Gly, His, Ile, Lys, Leu, Met, Asn, Pro, Arg, Ser, Thr, Val, Trp oder Tyr für die Aminosäure an Position 294;

Asp, Glu, Phe, Gly, His, Ile, Lys, Met, Asn, Pro, Arg, Ser, Thr, Val, Trp oder Tyr für die Aminosäure an Position 295;

Ala, Asp, Glu, Gly, His, Ile, Lys, Leu, Met, Asn, Gln, Arg, Ser, Thr oder Val für die Aminosäure an Position 296;

Asp, Glu, Phe, Gly, His, Ile, Lys, Leu, Met, Pro, Gln, Arg, Ser, Thr, Val, Trp oder Tyr für die Aminosäure an Position 297;

Ala, Asp, Glu, Phe, His, Ile, Lys, Met, Asn, Gln, Arg, Thr, Val, Trp oder Tyr für die Aminosäure an Position 298;

Ala, Asp, Glu, Phe, Gly, His, Ile, Lys, Leu, Met, Asn, Pro, Gln, Arg, Ser, Val, Trp oder Tyr für die Aminosäure an Position 299;

Ala, Asp, Glu, Gly, His, Ile, Lys, Leu, Met, Asn, Pro, Gln, Arg, Ser, Thr, Val oder Trp für die Aminosäure an Position 300;

Asp, Glu, His oder Tyr für die Aminosäure an Position 301;

Ile für die Aminosäure an Position 302;

Asp, Gly oder Tyr für die Aminosäure an Position 303;

Asp, His, Leu, Asn oder Thr für die Aminosäure an Position 304;

Glu, Ile, Thr oder Tyr für die Aminosäure an Position 305;

Ala, Asp, Asn, Thr, Val oder Tyr für die Aminosäure an Position 311;

Phe für die Aminosäure an Position 313;

Leu für die Aminosäure an Position 315;

Glu oder Gln für die Aminosäure an Position 317;

His, Leu, Asn, Pro, Gln, Arg, Thr, Val oder Tyr für die Aminosäure an Position 318;

Asp, Phe, Gly, His, Ile, Leu, Asn, Pro, Ser, Thr, Val, Trp oder Tyr für die Aminosäure an Position 320;

Ala, Asp, Phe, Gly, His, Ile, Pro, Ser, Thr, Val, Trp oder Tyr für die Aminosäure an Position 322;

Ile für die Aminosäure an Position 323;

Asp, Phe, Gly, His, Ile, Leu, Met, Pro, Arg, Thr, Val, Trp oder Tyr für die Aminosäure an Position 324;

Ala, Asp, Glu, Phe, Gly, His, Ile, Lys, Leu, Met, Pro, Gln, Arg, Ser, Thr, Val, Trp oder Tyr für die Aminosäure an Position 325;

Ala, Asp, Glu, Gly, Ile, Leu, Met, Asn, Pro, Gln, Ser, Thr, Val, Trp oder Tyr für die Aminosäure an Position 326;

Ala, Asp, Glu, Phe, Gly, His, Ile, Lys, Leu, Met, Asn, Pro, Arg, Thr, Val, Trp oder Tyr für die Aminosäure an Position 327;

Ala, Asp, Glu, Phe, Gly, His, Ile, Lys, Met, Asn, Pro, Gln, Arg, Ser, Thr, Val, Trp oder Tyr für die Aminosäure an Position 328;

Asp, Glu, Phe, Gly, His, Ile, Lys, Leu, Met, Asn, Gln, Arg, Ser, Thr, Val, Trp oder Tyr für die Aminosäure an Position 329;

Cys, Glu, Phe, Gly, His, Ile, Lys, Leu, Met, Asn, Pro, Arg, Ser, Thr, Val, Trp oder Tyr für die Aminosäure an Position 330;

Asp, Phe, His, Ile, Leu, Met, Gln, Arg, Thr, Val, Trp oder Tyr für die Aminosäure an Position 331;

Ala, Asp, Glu, Phe, Gly, His, Lys, Leu, Met, Asn, Pro, Gln, Arg, Ser, Thr, Val, Trp oder Tyr für die Aminosäure an Position 332;

Ala, Asp, Glu, Phe, Gly, His, Ile, Leu, Met, Pro, Ser, Thr, Val oder Tyr für die Aminosäure an Position 333;

Ala, Glu, Phe, Ile, Leu, Pro oder Thr für die Aminosäure an Position 334;

Asp, Phe, Gly, His, Ile, Leu, Met, Asn, Pro, Arg, Ser, Val, Trp oder Tyr für die Aminosäure an Position 335;

Glu, Lys oder Tyr für die Aminosäure an Position 336;

Glu, His oder Asn für die Aminosäure an Position 337;

Asp, Phe, Gly, Ile, Lys, Met, Asn, Gln, Arg, Ser oder Thr für die Aminosäure an Position 339;

Ala oder Val für die Aminosäure an Position 376;

Gly oder Lys für die Aminosäure an Position 377;

Asp für die Aminosäure an Position 378:

Asn für die Aminosäure an Position 379;

Ala, Asn oder Ser für die Aminosäure an Position 380;

Ala oder Ile für die Aminosäure an Position 382;

Glu für die Aminosäure an Position 385;

Thr für die Aminosäure an Position 392;

Leu für die Aminosäure an Position 396;

Lys für die Aminosäure an Position 421;

Asn für die Aminosäure an Position 427;

Phe oder Leu für die Aminosäure an Position 428;

Met für die Aminosäure an Position 429;

Trp für die Aminosäure an Position 434;

Ile für die Aminosäure an Position 436; und

Gly, His, Ile, Leu oder Tyr für die Aminosäure an Position 440;

wie durch EU-Nummerierung angegeben.

13. Verfahren nach einem der Ansprüche 1 bis 9, wobei der Antikörper eine Fc-Region umfasst, die eine höhere Bindungsaktivität gegenüber einem inhibitorischen Fcγ-Rezeptor aufweist als gegenüber einem aktivierenden Fcγ-Rezeptor.

**14.** Verfahren nach Anspruch 13, wobei der inhibitorische Fcγ-Rezeptor humaner FcγRIIb ist.

**15.** Verfahren nach Anspruch 13 oder 14, wobei der aktivierende Fcγ-Rezeptor humaner FcγRIa, humaner FcγRIIA (R), humaner FcγRIIa (H), humaner FcγRIIIa (V) oder humaner FcγRIIIa (F) ist.

**16.** Verfahren nach einem der Ansprüche 13 bis 15, wobei sich die Aminosäure an Position 238 oder 328 (EU-Nummerierung) in der Fc-Region von der Aminosäure in der nativen Fc-Region von humanem IgG unterscheidet.

**17.** Verfahren nach Anspruch 16, wobei die Aminosäure an Position 238 der Fc-Region Asp oder die Aminosäure an Position 328 der Fc-Region Glu ist, wie durch EU-Nummerierung angegeben.

**18.** Verfahren nach Anspruch 16 oder 17, wobei die Aminosäuresequenz der Fc-Region mindestens eine oder mehr Aminosäuren umfasst, ausgewählt aus der Gruppe bestehend aus:

> Asp für die Aminosäure an Position 233;
> Trp oder Tyr für die Aminosäure an Position 234;
> Ala, Asp, Glu, Leu, Met, Phe, Trp oder Tyr für die Aminosäure an Position 237;
> Asp für die Aminosäure an Position 239;
> Ala, Gln oder Val für die Aminosäure an Position 267;
> Asn, Asp oder Glu für die Aminosäure an Position 268;
> Gly für die Aminosäure an Position 271;
> Ala, Asn, Asp, Gln, Glu, Leu, Met, Ser oder Thr für die Aminosäure an Position 326;
> Arg, Lys oder Met für die Aminosäure an Position 330;
> Ile, Leu oder Met für die Aminosäure an Position 323; und
> Asp für die Aminosäure an Position 296;
> wie durch EU-Nummmerierung angeben.

## Revendications

**1.** Méthode pour produire un anticorps qui est capable d'éliminer sélectivement des agrégats de protéine provoquant une maladie de préférence à des monomères de protéine dans le plasma, ladite méthode comprenant les étapes suivantes :

> (a) sélection d'un anticorps :
>
> > (A) dans laquelle le rapport de la KD (constante de dissociation) de l'anticorps dirigé contre lesdits agrégats de protéine à un pH intracellulaire de 5,8 et à une concentration d'ions calcium de 3 μM jusqu'à un pH extracellulaire de 7,4 et une concentration d'ions calcium de 1,2 mM, est de 2 ou plus ;
> > (B) dans laquelle l'activité de liaison au récepteur FcRn d'un complexe formé entre lesdits agrégats de protéines et l'anticorps est supérieure à l'activité de liaison au récepteur FcRn d'un complexe formé entre la protéine non agrégée et l'anticorps ; et
> > (C) dans laquelle le rapport de la clairance plasmatique de ladite protéine agrégée en l'absence dudit anticorps sur la clairance plasmatique de ladite protéine agrégée en présence dudit anticorps est de 1,5 fois ou plus que le même rapport de clairance plasmatique pour ladite protéine non agrégée ;
>
> (b) culture d'une cellule hôte comprenant un vecteur qui porte un gène codant l'anticorps sélectionné dans l'étape (a) ; et
> (c) isolation de l'anticorps à partir de la culture obtenue dans l'étape (b).

**2.** Méthode selon la revendication 1, dans laquelle la protéine est la huntingtine, l'ataxine-1, l'ataxine-2, la sous-unité α1A du canal calcique, l'ataxine-7, la protéine de liaison à TATA, MDJ, DRPLA, le récepteur d'androgène, l'antitrypsine α1, l'antichymotrypsine α1, la neuroserpine, l'inhibiteur de C1, l'antithrombine III, Aβ, L-ch, la transthyrétine, SAA, β2M, H-ch, la cystatine C, la synucléine α, l'amyline, l'hémoglobine, la cristalline, l'IgA, la protéine Tau, la protéine se liant à l'ADN de TAR 43 kDa (TDP-43), la superoxyde dismutase (SOD1), FUS (fusionnée dans le gène de sarcome), le prion, PHOX2B, ARX, la protéine nucléaire 1 se liant au polyadénylate (PABPN1), la dysferline, la desmine, GFAP, ou la kératine 5/14.

**3.** Méthode selon la revendication 1 ou 2, dans laquelle l'anticorps comprend une région Fc qui est une région Fc représentée par l'une quelconque des SEQ ID NO : 9, 10, 11 ou 12.

**4.** Méthode selon la revendication 1 ou 2, dans laquelle, dans une condition de pH acide, l'activité de liaison à FcRn de la région Fc comprise dans l'anticorps est amplifiée en comparé à celle de la région Fc représentée par l'une quelconque des SEQ ID NO : 9, 10, 11 ou 12.

**5.** Méthode selon la revendication 4, dans laquelle la région Fc est une région Fc dans laquelle au moins un ou plusieurs acides aminés choisis dans le groupe constitué par les acides aminés aux positions 238, 244, 245, 249, 250, 251, 252, 253, 254, 255, 256, 257, 258, 260, 262, 265, 270, 272, 279, 283, 285, 286, 288, 293, 303, 305, 307, 308, 309, 311, 312, 314, 316, 317, 318, 332, 339, 340, 341, 343, 356, 360, 362, 375, 376, 377, 378, 380, 382, 385, 386, 387, 388, 389, 400, 413, 415, 423, 424, 427, 428, 430, 431, 433, 434, 435, 436, 438, 439, 440, 442, et 447, conformément à la numérotation EU, sont substitués dans la séquence d'acides aminés de la région Fc représentée par l'une quelconque des SEQ ID NO: 9, 10, 11 ou 12.

**6.** Méthode selon la revendication 5, dans laquelle la région Fc comprend au moins un ou plusieurs acides aminés choisis dans le groupe constitué par :

Leu pour l'acide aminé à la position 238 ;
Leu pour l'acide aminé à la position 244 ;
Arg pour l'acide aminé à la position 245 ;
Pro pour l'acide aminé à la position 249 ;
Gln ou Glu pour l'acide aminé à la position 250, ou
Arg, Asp, Glu ou Leu pour l'acide aminé à la position 251 ;
Phe, Ser, Thr ou Tyr pour l'acide aminé à la position 252 ;
Ser ou Thr pour l'acide aminé à la position 254 ;
Arg, Gly, Ile ou Leu pour l'acide aminé à la position 255 ;
Ala, Arg, Asn, Asp, Gln, Glu, Pro ou Thr pour l'acide aminé à la position 256 ;
Ala, Ile, Met, Asn, Ser ou Val pour l'acide aminé à la position 257 ;
Asp pour l'acide aminé à la position 258 ;
Ser pour l'acide aminé à la position 260 ;
Leu pour l'acide aminé à la position 262 ;
Lys pour l'acide aminé à la position 270 ;
Leu ou Arg pour l'acide aminé à la position 272 ;
Ala, Asp, Gly, His, Met, Asn, Gln, Arg, Ser, Thr, Trp ou Tyr pour l'acide aminé à la position 279 ;
Ala, Asp, Phe, Gly, His, Ile, Lys, Leu, Asn, Pro, Gln, Arg, Ser, Thr, Trp ou Tyr pour l'acide aminé à la position 283 ;
Asn pour l'acide aminé à la position 285 ;
Phe pour l'acide aminé à la position 286 ;
Asn ou Pro pour l'acide aminé à la position 288 ;
Val pour l'acide aminé à la position 293 ;
Ala, Glu, Gln ou Met pour l'acide aminé à la position 307 ;
Ala, Glu, Ile, Lys, Leu, Met, Ser, Val ou Trp pour l'acide aminé à la position 311 ;
Pro pour l'acide aminé à la position 309 ;
Ala, Asp ou Pro pour l'acide aminé à la position 312 ;
Ala ou Leu pour l'acide aminé à la position 314 ;
Lys pour l'acide aminé à la position 316 ;
Pro pour l'acide aminé à la position 317 ;
Asn ou Thr pour l'acide aminé à la position 318 ;
Phe, His, Lys, Leu, Met, Arg, Ser ou Trp pour l'acide aminé à la position 332 ;
Asn, Thr ou Trp pour l'acide aminé à la position 339 ;
Pro pour l'acide aminé à la position 341 ;
Glu, His, Lys, Gln, Arg, Thr ou Tyr pour l'acide aminé à la position 343 ;
Arg pour l'acide aminé à la position 375 ;
Gly, Ile, Met, Pro, Thr ou Val pour l'acide aminé à la position 376 ;
Lys pour l'acide aminé à la position 377 ;
Asp, Asn ou Val pour l'acide aminé à la position 378 ;
Ala, Asn, Ser ou Thr pour l'acide aminé à la position 380 ;
Phe, His, Ile, Lys, Leu, Met, Asn, Gln, Arg, Ser, Thr, Val, Trp ou Tyr pour l'acide aminé à la position 382 ;

Ala, Arg, Asp, Gly, His, Lys, Ser ou Thr pour l'acide aminé à la position 385 ;
Arg, Asp, Ile, Lys, Met, Pro, Ser ou Thr pour l'acide aminé à la position 386 ;
Ala, Arg, His, Pro, Ser ou Thr pour l'acide aminé à la position 387 ;
Asn, Pro ou Ser pour l'acide aminé à la position 389 ;
Asn pour l'acide aminé à la position 423 ;
Asn pour l'acide aminé à la position 427 ;
Leu, Met, Phe, Ser ou Thr pour l'acide aminé à la position 428 ;
Ala, Phe, Gly, His, Ile, Lys, Leu, Met, Asn, Gln, Arg, Ser, Thr, Val ou Tyr pour l'acide aminé à la position 430 ;
His ou Asn pour l'acide aminé à la position 431 ;
Arg, Gln, His, Ile, Lys, Pro ou Ser pour l'acide aminé à la position 433 ;
Ala, Gly, His, Phe, Ser, Trp ou Tyr pour l'acide aminé à la position 434 ;
Arg, Asn, His, Ile, Leu, Lys, Met ou Thr pour l'acide aminé à la position 436 ;
Lys, Leu, Thr ou Trp pour l'acide aminé à la position 438 ;
Lys pour l'acide aminé à la position 440, ou
Lys pour l'acide aminé à la position 442 ; et
Ile, Pro ou Thr pour l'acide aminé à la position 308 ;
comme indiqué par la numérotation EU, dans la séquence d'acides aminés de la région Fc représentée par l'une quelconque des SEQ ID NO : 9, 10, 11 ou 12.

7. Méthode selon la revendication 1 ou 2, dans laquelle, dans une condition de plage de pH neutre, une activité de liaison à FcRn de la région Fc comprise dans l'anticorps est amplifiée comparé à celle de la région Fc représentée par l'une quelconque des SEQ ID NO : 9, 10, 11 ou 12.

8. Méthode selon la revendication 7, dans laquelle la région Fc est une région Fc dans laquelle au moins un ou plusieurs acides aminés choisis dans le groupe constitué par les acides aminés aux positions 237, 248, 250, 252, 254, 255, 256, 257, 258, 265, 286, 289, 297, 298, 303, 305, 307, 308, 309, 311, 312, 314, 315, 317, 332, 334, 360, 376, 380, 382, 384, 385, 386, 387, 389, 424, 428, 433, 434, et 436, conformément à la numérotation EU, sont substitués dans la séquence d'acides aminés de la région Fc représentée par l'une quelconque des SEQ ID NO : 9, 10, 11 ou 12.

9. Méthode selon la revendication 8, dans laquelle la région Fc comprend au moins un ou plusieurs acides aminés choisis dans le groupe constitué par :

Met pour l'acide aminé à la position 237 ;
Ile pour l'acide aminé à la position 248 ;
Ala, Phe, Ile, Met, Gln, Ser, Val, Trp ou Tyr pour l'acide aminé à la position 250 ;
Phe, Trp ou Tyr pour l'acide aminé à la position 252 ;
Thr pour l'acide aminé à la position 254 ;
Glu pour l'acide aminé à la position 255 ;
Asp, Asn, Glu ou Gln pour l'acide aminé à la position 256 ;
Ala, Gly, Ile, Leu, Met, Asn, Ser, Thr ou Val pour l'acide aminé à la position 257 ;
His pour l'acide aminé à la position 258 ;
Ala pour l'acide aminé à la position 265 ;
Ala ou Glu pour l'acide aminé à la position 286 ;
His pour l'acide aminé à la position 289 ;
Ala pour l'acide aminé à la position 297 ;
Ala pour l'acide aminé à la position 303 ;
Ala pour l'acide aminé à la position 305 ;
Ala, Asp, Phe, Gly, His, Ile, Lys, Leu, Met, Asn, Pro, Gln, Arg, Ser, Val, Trp ou Tyr pour l'acide aminé à la position 307 ;
Ala, Phe, Ile, Leu, Met, Pro, Gln ou Thr pour l'acide aminé à la position 308 ;
Ala, Asp, Glu, Pro ou Arg pour l'acide aminé à la position 309 ;
Ala, His ou Ile pour l'acide aminé à la position 311 ;
Ala ou His pour l'acide aminé à la position 312 ;
Lys ou Arg pour l'acide aminé à la position 314 ;
Ala, Asp ou His pour l'acide aminé à la position 315 ;
Ala pour l'acide aminé à la position 317 ;
Val pour l'acide aminé à la position 332 ;
Leu pour l'acide aminé à la position 334 ;

His pour l'acide aminé à la position 360 ;
Ala pour l'acide aminé à la position 376 ;
Ala pour l'acide aminé à la position 380 ;
Ala pour l'acide aminé à la position 382 ;
Ala pour l'acide aminé à la position 384 ;
Asp ou His pour l'acide aminé à la position 385 ;
Pro pour l'acide aminé à la position 386 ;
Glu pour l'acide aminé à la position 387 ;
Ala ou Ser pour l'acide aminé à la position 389 ;
Ala pour l'acide aminé à la position 424 ;
Ala, Asp, Phe, Gly, His, Ile, Lys, Leu, Asn, Pro, Gln, Ser, Thr, Val, Trp ou Tyr pour l'acide aminé à la position 428 ;
Lys pour l'acide aminé à la position 433 ;
Ala, Phe, His, Ser, Trp ou Tyr pour l'acide aminé à la position 434 ; et
His, Ile, Leu, Phe, Thr ou Val pour l'acide aminé à la position 436 ;
comme indiqué par la numérotation EU dans la séquence d'acides aminés de la région Fc représentée par l'une quelconque des SEQ ID NO : 9, 10, 11 et 12.

**10.** Méthode selon l'une quelconque des revendications 1 à 6, dans laquelle l'anticorps comprend une région Fc qui inclut une région Fc qui a une activité de liaison au récepteur de Fcγ supérieure à celle de la région Fc d'une IgG humaine native.

**11.** Méthode selon la revendication 10, dans laquelle la région Fc comprend dans sa séquence d'acides aminés au moins un ou plusieurs acides aminés qui sont différents des acides aminés de la région Fc d'IgG humaine native, choisis dans le groupe de positions 221, 222, 223, 224, 225, 227, 228, 230, 231, 232, 233, 234, 235, 236, 237, 238, 239, 240, 241, 243, 244, 245, 246, 247, 249, 250, 251, 254, 255, 256, 258, 260, 262, 263, 264, 265, 266, 267, 268, 269, 270, 271, 272, 273, 274, 275, 276, 278, 279, 280, 281, 282, 283, 284, 285, 286, 288, 290, 291, 292, 293, 294, 295, 296, 297, 298, 299, 300, 301, 302, 303, 304, 305, 311, 313, 315, 317, 318, 320, 322, 323, 324, 325, 326, 327, 328, 329, 330, 331, 332, 333, 334, 335, 336, 337, 339, 376, 377, 378, 379, 380, 382, 385, 392, 396, 421, 427, 428, 429, 434, 436, et 440 (numérotation EU).

**12.** Méthode selon la revendication 11, dans laquelle la région Fc comprend dans sa séquence d'acides aminés au moins un ou plusieurs acides aminés choisis dans le groupe constitué par :

Lys ou Tyr pour l'acide aminé à la position 221 ;
Phe, Trp, Glu ou Tyr pour l'acide aminé à la position 222 ;
Phe, Trp, Glu ou Lys pour l'acide aminé à la position 223 ;
Phe, Trp, Glu ou Tyr pour l'acide aminé à la position 224 ;
Glu, Lys ou Trp pour l'acide aminé à la position 225 ;
Glu, Gly, Lys ou Tyr pour l'acide aminé à la position 227 ;
Glu, Gly, Lys ou Tyr pour l'acide aminé à la position 228 ;
Ala, Glu, Gly ou Tyr pour l'acide aminé à la position 230 ;
Glu, Gly, Lys, Pro ou Tyr pour l'acide aminé à la position 231 ;
Glu, Gly, Lys ou Tyr pour l'acide aminé à la position 232 ;
Ala, Asp, Phe, Gly, His, Ile, Lys, Leu, Met, Asn, Gln, Arg, Ser, Thr, Val, Trp ou Tyr pour l'acide aminé à la position 233 ;
Ala, Asp, Glu, Phe, Gly, His, Ile, Lys, Met, Asn, Pro, Gln, Arg, Ser, Thr, Val, Trp ou Tyr pour l'acide aminé à la position 234 ;
Ala, Asp, Glu, Phe, Gly, His, Ile, Lys, Met, Asn, Pro, Gln, Arg, Ser, Thr, Val, Trp ou Tyr pour l'acide aminé à la position 235 ;
Ala, Asp, Glu, Phe, His, Ile, Lys, Leu, Met, Asn, Pro, Gln, Arg, Ser, Thr, Val, Trp ou Tyr pour l'acide aminé à la position 236 ;
Asp, Glu, Phe, His, Ile, Lys, Leu, Met, Asn, Pro, Gln, Arg, Ser, Thr, Val, Trp ou Tyr pour l'acide aminé à la position 237 ;
Asp, Glu, Phe, Gly, His, Ile, Lys, Leu, Met, Asn, Gln, Arg, Ser, Thr, Val, Trp ou Tyr pour l'acide aminé à la position 238 ;
Asp, Glu, Phe, Gly, His, Ile, Lys, Leu, Met, Asn, Pro, Gln, Arg, Thr, Val, Trp ou Tyr pour l'acide aminé à la position 239 ;
Ala, Ile, Met ou Thr pour l'acide aminé à la position 240 ;

Asp, Glu, Leu, Arg, Trp ou Tyr pour l'acide aminé à la position 241 ;

Leu, Glu, Leu, Gln, Arg, Trp ou Tyr pour l'acide aminé à la position 243 ;

His pour l'acide aminé à la position 244 ;

Ala pour l'acide aminé à la position 245 ;

Asp, Glu, His ou Tyr pour l'acide aminé à la position 246 ;

Ala, Phe, Gly, His, Ile, Leu, Met, Thr, Val ou Tyr pour l'acide aminé à la position 247 ;

Glu, His, Gln ou Tyr pour l'acide aminé à la position 249 ;

Glu ou Gln pour l'acide aminé à la position 250 ;

Phe pour l'acide aminé à la position 251 ;

Phe, Met ou Tyr pour l'acide aminé à la position 254 ;

Glu, Leu ou Tyr pour l'acide aminé à la position 255 ;

Ala, Met ou Pro pour l'acide aminé à la position 256 ;

Asp, Glu, His, Ser ou Tyr pour l'acide aminé à la position 258 ;

Asp, Glu, His ou Tyr pour l'acide aminé à la position 260 ;

Ala, Glu, Phe, Ile ou Thr pour l'acide aminé à la position 262 ;

Ala, Ile, Met ou Thr pour l'acide aminé à la position 263 ;

Asp, Glu, Phe, Gly, His, Ile, Lys, Leu, Met, Asn, Pro, Gln, Arg, Ser, Thr, Trp ou Tyr pour l'acide aminé à la position 264 ;

Ala, Leu, Phe, Gly, His, Ile, Lys, Leu, Met, Asn, Pro, Gln, Arg, Ser, Thr, Val, Trp ou Tyr pour l'acide aminé à la position 265 ;

Ala, Ile, Met ou Thr pour l'acide aminé à la position 266 ;

Asp, Glu, Phe, His, Ile, Lys, Leu, Met, Asn, Pro, Gln, Arg, Thr, Val, Trp ou Tyr pour l'acide aminé à la position 267 ;

Asp, Glu, Phe, Gly, Ile, Lys, Leu, Met, Pro, Gln, Arg, Thr, Val ou Trp pour l'acide aminé à la position 268 ;

Phe, Gly, His, Ile, Lys, Leu, Met, Asn, Pro, Arg, Ser, Thr, Val, Trp ou Tyr pour l'acide aminé à la position 269 ;

Glu, Phe, Gly, His, Ile, Leu, Met, Pro, Gln, Arg, Ser, Thr, Trp ou Tyr pour l'acide aminé à la position 270 ;

Ala, Asp, Glu, Phe, Gly, His, Ile, Lys, Leu, Met, Asn, Gln, Arg, Ser, Thr, Val, Trp ou Tyr pour l'acide aminé à la position 271 ;

Asp, Phe, Gly, His, Ile, Lys, Leu, Met, Pro, Arg, Ser, Thr, Val, Trp, ou Tyr pour l'acide aminé à la position 272 ;

Phe ou Ile pour l'acide aminé à la position 273 ;

Asp, Glu, Phe, Gly, His, Ile, Leu, Met, Asn, Pro, Arg, Ser, Thr, Val, Trp, ou Tyr pour l'acide aminé à la position 274 ;

Leu ou Trp pour l'acide aminé à la position 275 ;

Asp, Glu, Phe, Gly, His, Ile, Leu, Met, Pro, Arg, Ser, Thr, Val, Trp, ou Tyr pour l'acide aminé à la position 276 ;

Asp, Glu, Gly, His, Ile, Lys, Leu, Met, Asn, Pro, Gln, Arg, Ser, Thr, Val, ou Trp pour l'acide aminé à la position 278 ;

Ala pour l'acide aminé à la position 279 ;

Ala, Gly, His, Lys, Leu, Pro, Gln, Trp, ou Tyr pour l'acide aminé à la position 280 ;

Asp, Lys, Pro, ou Tyr pour l'acide aminé à la position 281 ;

Glu, Gly, Lys, Pro, ou Tyr pour l'acide aminé à la position 282 ;

Ala, Gly, His, Ile, Lys, Leu, Met, Pro, Arg, ou Tyr pour l'acide aminé à la position 283 ;

Asp, Glu, Leu, Asn, Thr, ou Tyr pour l'acide aminé à la position 284 ;

Asp, Glu, Lys, Gln, Trp, ou Tyr pour l'acide aminé à la position 285 ;

Glu, Gly, Pro, ou Tyr pour l'acide aminé à la position 286 ;

Asn, Asp, Glu, ou Tyr pour l'acide aminé à la position 288 ;

Asp, Gly, His, Leu, Asn, Ser, Thr, Trp, ou Tyr pour l'acide aminé à la position 290 ;

Asp, Glu, Gly, His, Ile, Gln, ou Thr pour l'acide aminé à la position 291 ;

Ala, Asp, Glu, Pro, Thr, ou Tyr pour l'acide aminé à la position 292 ;

Phe, Gly, His, Ile, Leu, Met, Asn, Pro, Arg, Ser, Thr, Val, Trp, ou Tyr pour l'acide aminé à la position 293 ;

Phe, Gly, His, Ile, Lys, Leu, Met, Asn, Pro, Arg, Ser, Thr, Val, Trp, ou Tyr pour l'acide aminé à la position 294 ;

Asp, Glu, Phe, Gly, His, Ile, Lys, Met, Asn, Pro, Arg, Ser, Thr, Val, Trp, ou Tyr pour l'acide aminé à la position 295 ;

Ala, Asp, Glu, Gly, His, Ile, Lys, Leu, Met, Asn, Gln, Arg, Ser, Thr, ou Val pour l'acide aminé à la position 296 ;

Asp, Glu, Phe, Gly, His, Ile, Lys, Leu, Met, Pro, Gln, Arg, Ser, Thr, Val, Trp, ou Tyr pour l'acide aminé à la position 297 ;

Ala, Asp, Glu, Phe, His, Ile, Lys, Met, Asn, Gln, Arg, Thr, Val, Trp, ou Tyr pour l'acide aminé à la position 298 ;

Ala, Asp, Glu, Phe, Gly, His, Ile, Lys, Leu, Met, Asn, Pro, Gln, Arg, Ser, Val, Trp, ou Tyr pour l'acide aminé à la position 299 ;

Ala, Asp, Glu, Gly, His, Ile, Lys, Leu, Met, Asn, Pro, Gln, Arg, Ser, Thr, Val, ou Trp pour l'acide aminé à la position 300 ;

Asp, Glu, His, ou Tyr pour l'acide aminé à la position 301 ;

Ile pour l'acide aminé à la position 302 ;

Asp, Gly, ou Tyr pour l'acide aminé à la position 303 ;

Asp, His, Leu, Asn, ou Thr pour l'acide aminé à la position 304 ;

Glu, Ile, Thr, ou Tyr pour l'acide aminé à la position 305 ;

Ala, Asp, Asn, Thr, Val, ou Tyr pour l'acide aminé à la position 311 ;

Phe pour l'acide aminé à la position 313 ;

Leu pour l'acide aminé à la position 315 ;

Glu ou Gln pour l'acide aminé à la position 317 ;

His, Leu, Asn, Pro, Gln, Arg, Thr, Val, ou Tyr pour l'acide aminé à la position 318 ;

Asp, Phe, Gly, His, Ile, Leu, Asn, Pro, Ser, Thr, Val, Trp, ou Tyr pour l'acide aminé à la position 320 ;

Ala, Asp, Phe, Gly, His, Ile, Pro, Ser, Thr, Val, Trp, ou Tyr pour l'acide aminé à la position 322 ;

Ile pour l'acide aminé à la position 323 ;

Asp, Phe, Gly, His, Ile, Leu, Met, Pro, Arg, Thr, Val, Trp, ou Tyr pour l'acide aminé à la position 324 ;

Ala, Asp, Glu, Phe, Gly, His, Ile, Lys, Leu, Met, Pro, Gln, Arg, Ser, Thr, Val, Trp, ou Tyr pour l'acide aminé à la position 325 ;

Ala, Asp, Glu, Gly, Ile, Leu, Met, Asn, Pro, Gln, Ser, Thr, Val, Trp, ou Tyr pour l'acide aminé à la position 326 ;

Ala, Asp, Glu, Phe, Gly, His, Ile, Lys, Leu, Met, Asn, Pro, Arg, Thr, Val, Trp, ou Tyr pour l'acide aminé à la position 327 ;

Ala, Asp, Glu, Phe, Gly, His, Ile, Lys, Met, Asn, Pro, Gln, Arg, Ser, Thr, Val, Trp, ou Tyr pour l'acide aminé à la position 328 ;

Asp, Glu, Phe, Gly, His, Ile, Lys, Leu, Met, Asn, Gln, Arg, Ser, Thr, Val, Trp, ou Tyr pour l'acide aminé à la position 329 ;

Cys, Glu, Phe, Gly, His, Ile, Lys, Leu, Met, Asn, Pro, Arg, Ser, Thr, Val, Trp, ou Tyr pour l'acide aminé à la position 330 ;

Asp, Phe, His, Ile, Leu, Met, Gln, Arg, Thr, Val, Trp, ou Tyr pour l'acide aminé à la position 331 ;

Ala, Asp, Glu, Phe, Gly, His, Lys, Leu, Met, Asn, Pro, Gln, Arg, Ser, Thr, Val, Trp, ou Tyr pour l'acide aminé à la position 332 ;

Ala, Asp, Glu, Phe, Gly, His, Ile, Leu, Met, Pro, Ser, Thr, Val, ou Tyr pour l'acide aminé à la position 333 ;

Ala, Glu, Phe, Ile, Leu, Pro, ou Thr pour l'acide aminé à la position 334 ;

Asp, Phe, Gly, His, Ile, Leu, Met, Asn, Pro, Arg, Ser, Val, Trp, ou Tyr pour l'acide aminé à la position 335 ;

Glu, Lys, ou Tyr pour l'acide aminé à la position 336 ;

Glu, His, ou Asn pour l'acide aminé à la position 337 ;

Asp, Phe, Gly, Ile, Lys, Met, Asn, Gln, Arg, Ser, ou Thr pour l'acide aminé à la position 339 ;

Ala ou Val pour l'acide aminé à la position 376 ;

Gly ou Lys pour l'acide aminé à la position 377 ;

Asp pour l'acide aminé à la position 378 ;

Asn pour l'acide aminé à la position 379 ;

Ala, Asn, ou Ser pour l'acide aminé à la position 380 ;

Ala ou Ile pour l'acide aminé à la position 382 ;

Glu pour l'acide aminé à la position 385 ;

Thr pour l'acide aminé à la position 392 ;

Leu pour l'acide aminé à la position 396 ;

Lys pour l'acide aminé à la position 421 ;

Asn pour l'acide aminé à la position 427 ;

Phe ou Leu pour l'acide aminé à la position 428 ;

Met pour l'acide aminé à la position 429 ;

Trp pour l'acide aminé à la position 434 ;

Ile pour l'acide aminé à la position 436 ; et

Gly, His, Ile, Leu, ou Tyr pour l'acide aminé à la position 440 ;

comme indiqué par la numérotation EU.

13. Méthode selon l'une quelconque des revendications 1 à 9, dans laquelle l'anticorps comprend une région Fc qui a une activité de liaison vis-à-vis d'un récepteur de Fcγ inhibiteur supérieure à celle vis-à-vis d'un récepteur de Fcγ activateur.

14. Méthode selon la revendication 13, dans laquelle le récepteur de Fcγ inhibiteur est le FcγRIIb humain.

15. Méthode selon la revendication 13 ou 14, dans laquelle le récepteur de Fcγ d'activation est le FcγRIa humain, le FcγRIIa humain (R), le FcγRIIa humain (H), le FcγRIIIa humain (V), ou le FcγRIIIa humain (F).

**16.** Méthode selon l'une quelconque des revendications 13 à 15, dans laquelle l'acide aminé à la position 238 ou 328 (numérotation EU) dans la région Fc est différent de l'acide aminé dans la région Fc d'IgG humaine native.

**17.** Méthode selon la revendication 16, dans laquelle l'acide aminé à la position 238 de la région Fc est Asp ou l'acide aminé à la position 328 de la région Fc est Glu, comme indiqué par la numérotation EU.

**18.** Méthode selon la revendication 16 ou 17, dans laquelle la séquence d'acides aminés de la région Fc comprend au moins un ou plusieurs acides aminés choisis dans le groupe constitué par :

Asp pour l'acide aminé à la position 233 ;
Trp ou Tyr pour l'acide aminé à la position 234 ;
Ala, Asp, Glu, Leu, Met, Phe, Trp, ou Tyr pour l'acide aminé à la position 237 ;
Asp pour l'acide aminé à la position 239 ;
Ala, Gln, ou Val pour l'acide aminé à la position 267 ;
Asn, Asp, ou Glu pour l'acide aminé à la position 268 ;
Gly pour l'acide aminé à la position 271 ;
Ala, Asn, Asp, Gln, Glu, Leu, Met, Ser, ou Thr pour l'acide aminé à la position 326 ;
Arg, Lys, ou Met pour l'acide aminé à la position 330 ;
Ile, Leu, ou Met pour l'acide aminé à la position 323 ; et
Asp pour l'acide aminé à la position 296 ;
comme indiqué par la numérotation EU.

FIG. 1

FIG. 2

FIG. 3

FIG. 4

FIG. 5

FIG. 6

EP 2 857 419 B1

FIG. 7

183

FIG. 8

FIG. 9

FIG. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2009125825 A
- WO 2011122011 A
- WO 2004044011 A
- WO 2005040229 A
- WO 2002032925 A
- WO 1995001937 A
- WO 2002020565 A
- WO 2003029462 A
- WO 2008016854 A
- WO 1994011523 A
- EP 239400 A
- WO 1996002576 A
- WO 1993012227 A
- WO 1992003918 A
- WO 1994002602 A
- WO 1994025585 A
- WO 1996034096 A
- WO 1996033735 A
- WO 1992001047 A
- WO 1992020791 A
- WO 1993006213 A
- WO 1993011236 A
- WO 1993019172 A
- WO 1995001438 A
- WO 1995015388 A
- WO 2008081008 A

- US 20090035836 A
- WO 1997034631 A
- WO 2000042072 A
- WO 2002060919 A
- WO 2004092219 A
- WO 2006020114 A
- WO 2010045193 A
- WO 2009086320 A
- WO 2008092117 A
- WO 2007041635 A
- WO 2006105338 A
- WO 2007024249 A
- WO 2007021841 A
- WO 2006031370 A
- WO 2004029207 A
- WO 2004099249 A
- WO 2005070963 A
- WO 2006116260 A
- WO 2006023403 A
- US 20090136485 A
- WO 2006016644 A
- WO 2009008529 A
- JP 2012123782 A

### Non-patent literature cited in the description

- **JANICE M REICHERT ; CLARK J ROSENSWEIG ; LAURA B FADEN ; MATTHEW C DEWITZ.** Monoclonal antibody successes in the clinic. *Nature Biotechnology,* 2005, vol. 23, 1073-1078
- **PAVLOU AK ; BELSEY MJ.** The therapeutic antibodies market to 2008. *Eur J Pharm Biopharm.,* April 2005, vol. 59 (3), 389-96
- **RAJPAL A ; BEYAZ N ; HABER L ; CAPPUCCILLI G ; YEE H ; BHATT RR ; TAKEUCHI T ; LERNER RA ; CREA R.** A general method for greatly improving the affinity of antibodies by using combinatorial libraries. *Proc. Natl. Acad. Sci. U. S. A.,* 2005, vol. 102 (24), 8466-8471

- **RATHANASWAMI P ; ROALSTAD S ; ROSKOS L ; SU QJ ; LACKIE S ; BABCOOK J.** Demonstration of an in vivo generated sub-picomolar affinity fully human monoclonal antibody to interleukin-8. *Biochem. Biophys. Res. Commun.,* 2005, vol. 334 (4), 1004-1013
- **IGAWA T et al.** Antibody recycling by engineered pH-dependent antigen binding improves the duration of antigen neutralization. *Nat Biotechnol.,* 2010, vol. 28, 1203-7
- **KUNKEL et al.** *Proc. Natl. Acad. Sci. USA,* 1985, vol. 82, 488-492
- *Annu. Rev. Biophys. Biomol. Struct.,* 2006, vol. 35, 225-249
- *Proc. Natl. Acad. Sci. U.S.A.,* 2003, vol. 100 (11), 6353-6357

- Epitope Mapping Protocols in Methods in Molecular Biology. 1996, vol. 66
- Antibodies: A Laboratory Manual. Cold Spring Harbor Laboratory, 1988, 359-420
- Sequences of proteins of immunological interest. NIH Publication
- *J. Immunol.,* 1979, vol. 123 (4), 1548-1550
- *Current Topics in Microbiology and Immunology,* 1978, vol. 81, 1-7
- *C. Eur. J. Immunol.,* 1976, vol. 6 (7), 511-519
- *Cell,* 1976, vol. 8 (3), 405-415
- *Nature,* 1978, vol. 276 (5685), 269-270
- *J. Immunol. Methods,* 1980, vol. 35 (1-2), 1-21
- *J. Exp. Med.,* 1978, vol. 148 (1), 313-323
- *Nature,* 1979, vol. 277 (5692), 131-133
- **KOHLER ; MILSTEIN et al.** *Methods Enzymol.,* 1981, vol. 73, 3-46
- **VANDAMME et al.** *Eur. J. Biochem.,* 1990, vol. 192 (3), 767-775
- *Biochemistry,* 1979, vol. 18 (24), 5294-5299
- *Anal. Biochem.,* 1987, vol. 162 (1), 156-159
- *Proc. Natl. Acad. Sci. USA,* 1988, vol. 85 (23), 8998-9002
- *Nucleic Acids Res.,* 1989, vol. 17 (8), 2919-2932
- **HELA ; VERO.** *Current Protocols in Protein Science,* May 2001
- **EBERT, K. M. et al.** *Bio/Technology,* 1994, vol. 12 (7), 699-702
- *Cancer Res.,* 1993, vol. 53, 851-856
- **BERNASCONI et al.** *Science,* 2002, vol. 298, 2199-2202
- Sequences of Proteins of Immunological Interest. National Institute of Health, 1987
- **SPRINGER et al.** *Cell,* 2000, vol. 102, 275-277
- **KAWASAKI ; KRETSINGER.** *Protein Prof.,* 1995, vol. 2, 305-490
- **MONCRIEF et al.** *J. Mol. Evol.,* 1990, vol. 30, 522-562
- **CHAUVAUX et al.** *Biochem. J.,* 1990, vol. 265, 261-265
- **BAIROCH ; COX.** *FEBS Lett.,* 1990, vol. 269, 454-456
- **DAVIS.** *New Biol.,* 1990, vol. 2, 410-419
- **SCHAEFER et al.** *Genomics,* 1995, vol. 25, 638-643
- **ECONOMOU et al.** *EMBO J.,* 1990, vol. 9, 349-354
- **WURZBURG et al.** *Structure,* 2006, vol. 14 (6), 1049-1058
- **TOMLINSON et al.** *J. Mol. Biol.,* 1992, vol. 227, 776-798
- **WILLIAMS ; WINTER.** *Eur. J. Immunol.,* 1993, vol. 23, 1456-1461
- **COX et al.** *Nat. Genetics,* 1994, vol. 7, 162-168
- **MATSUDA et al.** *J. Exp. Med.,* 1998, vol. 188, 1973-1975
- **KAWASAKI et al.** *Eur. J. Immunol.,* 2001, vol. 31, 1017-1028
- **SCHABLE ; ZACHAU.** *Biol. Chem. Hoppe Seyler,* 1993, vol. 374, 1001-1022
- **BRENSING-KUPPERS et al.** *Gene,* 1997, vol. 191, 173-181
- **KAWASAKI et al.** *Genome Res.,* 1997, vol. 7, 250-261
- **WU et al.** *J. Exp. Med.,* 1970, vol. 132, 211-250
- **KABAT.** Sequences of Proteins of Immunological Interest. National Institute of Health, 1987
- **LEE ; RICHARDS.** *J. Mol. Biol.,* 1971, vol. 55, 379-400
- **CONNOLLY.** *J. Appl. Cryst.,* 1983, vol. 16, 548-558
- **PACIOS.** *Comput. Chem.,* 1994, vol. 18 (4), 377-386
- *J. Mol. Model.,* 1995, vol. 1, 46-53
- **GEJIMA et al.** *Human Antibodies,* 2002, vol. 11, 121-129
- **CARDOSO et al.** *Scand. J. Immunol.,* 2000, vol. 51, 337-344
- *Bioorg. Med. Chem.,* 2003, vol. 11 (17), 3761-3768
- **GHETIE.** *Immunol. Today,* 1997, vol. 18 (12), 592-598
- **RAGHAVAN et al.** *Immunity,* 1994, vol. 1, 303-315
- *Journal of Immunology,* 2009, vol. 182, 7663-7671
- *Curr. Opin. Biotechnol.,* 2009, vol. 20 (6), 685-91
- *Curr. Opin. Immunol.,* 2008, vol. 20 (4), 460-470
- *Protein Eng. Des. Sel.,* 2010, vol. 23 (4), 195-202
- *Proc. Natl. Acad. Sci. USA,* 2006, vol. 103 (11), 4005-4010
- **WEITZHANDLER et al.** *J. Pharma. Sciences,* 1994, vol. 83 (12), 1670-1675
- **SCHENK et al.** *J. Clin. Investigation,* 2001, vol. 108 (11), 1687-1695
- **BIGGE et al.** *Anal. Biochem.,* 1995, vol. 230 (2), 229-238
- *Protein Engineering,* 1996, vol. 9 (3), 299-305
- *EMBO J.,* 1994, vol. 13, 1166-75
- *Int Immunol.,* 2001, vol. 13 (12), 1551-1559
- *Methods Mol Biol.,* 2010, vol. 602, 93-104
- *Clin Pharmacol.,* April 2008, vol. 48 (4), 406-417
- *Pharm Res.,* 2006, vol. 23 (1), 95-103
- Methods Mol Biol. Jackson Laboratories, 2010, vol. 602, 93-104
- **YANG et al.** *J. Mol. Biol.,* 1995, vol. 254, 392-403

- **MARKS et al.** *Bio/Technology,* 1992, vol. 10, 779-783
- **LOW et al.** *J. Mol. Biol.,* 1996, vol. 250, 359-368
- **PATTEN et al.** *Curr. Opin. Biotechnol.,* 1997, vol. 8, 724-733
- **THOMPSON et al.** *J. Mol. Biol.,* 1996, vol. 256, 77-88
- **CLAMERI et al.** *Nature,* 1998, vol. 391, 288-291
- *Protein Science,* 1995, vol. 4, 2411-2423